(19) Europäisches Patentamt
European Patent Office
Office européen des brevets

(11) EP 3 649 123 B1

# (12) EUROPÄISCHE PATENTSCHRIFT

(45) Veröffentlichungstag und Bekanntmachung des Hinweises auf die Patenterteilung:
**15.03.2023 Patentblatt 2023/11**

(21) Anmeldenummer: **18737229.7**

(22) Anmeldetag: **02.07.2018**

(51) Internationale Patentklassifikation (IPC):
***C07D 405/14*** (2006.01) ***C07D 409/14*** (2006.01)
***C07D 471/04*** (2006.01) ***C07D 471/06*** (2006.01)
***C07D 471/14*** (2006.01) ***C07D 487/04*** (2006.01)
***C07D 491/04*** (2006.01) ***C07D 495/04*** (2006.01)
***C07D 498/06*** (2006.01) ***H01L 51/00*** (2006.01)
***H01L 51/50*** (2006.01)

(52) Gemeinsame Patentklassifikation (CPC):
**C07D 405/14; C07D 409/14; C07D 471/04; C07D 471/06; C07D 471/14; C07D 487/04; C07D 491/04; C07D 495/04; C07D 498/06; H10K 50/11; H10K 85/657; H10K 85/6572; H10K 85/6574;** H10K 50/16; H10K 71/12; (Forts.)

(86) Internationale Anmeldenummer:
**PCT/EP2018/067732**

(87) Internationale Veröffentlichungsnummer:
**WO 2019/007866 (10.01.2019 Gazette 2019/02)**

## (54) ZUSAMMENSETZUNG FÜR ORGANISCHE ELEKTRONISCHE VORRICHTUNGEN

COMPOSITION FOR ORGANIC ELECTRONIC DEVICES

COMPOSITION POUR DISPOSITIFS ÉLECTRONIQUES ORGANIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.07.2017 EP 17179775**
**05.10.2017 EP 17195036**

(43) Veröffentlichungstag der Anmeldung:
**13.05.2020 Patentblatt 2020/20**

(60) Teilanmeldung:
**23150252.7**

(73) Patentinhaber: **Merck Patent GmbH**
**64293 Darmstadt (DE)**

(72) Erfinder:
- **PARHAM, Amir**
  **60486 Frankfurt am Main (DE)**
- **KROEBER, Jonas**
  **60311 Frankfurt am Main (DE)**
- **GROSSMANN, Tobias**
  **64297 Darmstadt (DE)**
- **JATSCH, Anja**
  **60489 Frankfurt am Main (DE)**
- **EICKHOFF, Christian**
  **68259 Mannheim (DE)**
- **EHRENREICH, Christian**
  **64285 Darmstadt (DE)**

(56) Entgegenhaltungen:
**WO-A1-2015/165563** **WO-A1-2015/169412**
**WO-A1-2018/101691** **KR-A- 20170 113 320**
**KR-B1- 101 744 248** **US-A1- 2014 158 992**
**US-A1- 2015 318 487**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
H10K 71/16; H10K 85/654; H10K 2102/103;
Y02P 70/50

## Beschreibung

Die vorliegende Erfindung betrifft eine Zusammensetzung, die einen elektronentransportierenden Host und einen lochtransportierenden Host gemäß Anspruch 1 umfasst, deren Verwendung in elektronischen Vorrichtungen sowie elektronische Vorrichtungen enthaltend diese Zusammensetzung. Der elektronentransportierende Host wird besonders bevorzugt aus der Klasse der Triazin-Dibenzofuran-Carbazol-Systeme oder der Klasse der Triazin-Dibenzothiophen-Carbazol-Systeme ausgewählt. Der lochtransportierende Host wird bevorzugt aus der Klasse der Biscarbazole ausgewählt.

Der Aufbau organischer Elektrolumineszenzvorrichtungen (z.B. OLEDs - organic light emitting diodes oder OLECs - organic light emitting electrochemical cells), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfach gesteigerte Energie- und Leistungseffizienz möglich. Generell gibt es bei OLEDs, insbesondere auch bei OLEDs die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung, und davon insbesondere die Host bzw. Matrixmaterialien. Verbesserungen dieser Materialien können zu deutlichen Verbesserungen elektrolumineszierender Vorrichtungen führen.

Hostmaterialien zur Verwendung in organischen elektronischen Vorrichtungen sind dem Fachmann gut bekannt. Im Stand der Technik wird häufig auch der Begriff Matrixmaterial verwendet, wenn ein Hostmaterial für phosphoreszierende Emitter gemeint ist. Diese Verwendung des Begriffs gilt auch für die vorliegende Erfindung. Mittlerweile wurde eine Vielzahl von Hostmaterialien sowohl für fluoreszierende als auch für phosphoreszierende elektronische Vorrichtungen entwickelt.

Gemäß dem Stand der Technik werden unter anderem Ketone (z. B. gemäß WO 2004/093207 oder WO 2010/006680) oder Phosphinoxide (z. B. gemäß WO 2005/003253) als Matrixmaterialien für phosphoreszierende Emitter verwendet. Weitere Matrixmaterialien gemäß dem Stand der Technik repräsentieren Triazine (bspw. WO 2008/056746, EP 0906947, EP 0908787, EP 0906948) sowie Lactame (bspw. WO 2011/116865 oder WO 2011/137951). Weiterhin werden gemäß dem Stand der Technik unter anderem Carbazolderivate (z. B. gemäß WO 2005/039246, US 2005/0069729 oder WO 2014/015931), Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Aus der WO 2011/057706 sind Carbazolderivate bekannt, welche mit zwei Triphenyltriazingruppen substituiert sind. Aus der WO 2011/046182 sind Carbazol-Arylen-TriazinDerivate bekannt, welche am Triazin mit einer Fluorenylgruppe substituiert sind. WO 2009/069442 offenbart Tricyclen, wie Carbazol, Dibenzofuran oder Dibenzothiophen, die hochgradig mit elektronenarmen Heteroaromaten (z.B. Pyridin, Pyrimidin oder Triazin) substituiert sind, als Hostmaterialien. Aus WO 2011/057706, WO 2015/014434 und WO 2015/169412 sind weitere Hostmaterialien bekannt, die unter anderem Triazin-Dibenzofuran-Carbazolderivate und Triazin-Dibenzothiophen-Carbazolderivate umfassen, wobei das Triazin gegebenenfalls mit einem Linker an das Dibenzofuran oder Dibenzothiophen gebunden ist.

Eine weitere Möglichkeit, die Leistungsdaten elektronischer Vorrichtungen, insbesondere von organischen Elektrolumineszenzvorrichtungen, zu verbessern, besteht darin, Kombinationen aus zwei oder mehr Materialien, insbesondere Host-materialien bzw. Matrixmaterialien, zu verwenden.

US 6,392,250 B1 offenbart die Verwendung einer Mischung bestehend aus einem Elektronentransportmaterial, einem Lochtransportmaterial und einem fluoreszierenden Emitter in der Emissionsschicht einer OLED. Mit Hilfe dieser Mischung konnte die Lebensdauer der OLED gegenüber dem Stand der Technik verbessert werden.

US 6,803,720 B1 offenbart die Verwendung einer Mischung enthaltend einen phosphoreszierenden Emitter sowie ein Loch- und ein Elektronentransportmaterial in der Emissionsschicht einer OLED. Dabei sind sowohl das Loch- und das Elektrontransportmaterial kleine organische Moleküle.

US 9,601,698 offenbart die Verwendung einer Mischung zweier Hostmaterialien und eines phosphoreszierenden Emitters, beispielsweise eine Mischung eines Pyridin-Carbazol-Dibenzothiophen-Derivats mit einem Triarylamino-substituierten Biscarbazol in der emittierenden Schicht einer OLED.

Gemäß WO 2015/156587 können spezielle Carbazol-Derivate in einer Mischung mit Biscarbazolen als Hostmaterialien verwendet werden.

Gemäß WO 2015/169412 können beispielsweise Triazin-Dibenzofuran-Carbazolderivate und Triazin-Dibenzothiophen-Carbazolderivate ebenfalls in einer Mischung verwendet werden. So wird beispielsweise die Herstellung

der OLED mit Kennzeichnung E34 beschrieben, die in der emittierenden Schicht die Hostmaterialien EG1, IC6 und den phosphoreszierenden Emitter TEG1 enthält. Die Strukturen der verwendeten Verbindungen sind im Folgenden aufgeführt:

| | | |
|---|---|---|
| 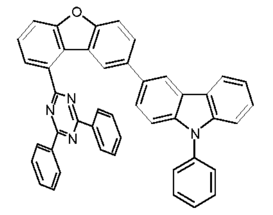 | 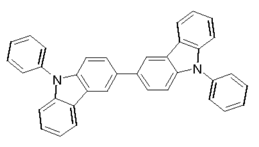 | 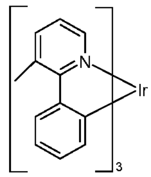 |
| EG1 | IC6 | TEG1. |

In dem Patent KR101744248 B1 wird eine spezielle Abfolge von zwei emittierenden Schichten in einer Vorrichtung beschrieben, wobei jede emittierende Schicht zwei Hostmaterialien enthält. Die erste emittierende Schicht enthält Host 1-1 und Host 1-2. Die zweite emittierende Schicht enthält Host 2-1 und Host 2-2, wobei Host 1-2 und Host 2-1 die gleichen Materialien sind. Anspruch 7 beschreibt spezielle 1-2-Hostmaterialien. Anspruch 10 beschreibt die Verbindung, zuvor abgekürzt als EG1, als 2-2-Hostmaterial.
Gemäß der nachveröffentlichten Patentanmeldung KR20170113320 kann die Verbindung, zuvor abgekürzt mit EG1, im Dokument H-6 genannt, zusammen mit einem di(1 ,3-biphenyl)-substituierten Biscarbazol in einer Mischung zusammen verwendet werden. Das entsprechende Biscarbazol (3-(9'-1,3-Biphenyl-9H-carbazol-3'-yl)-9-(1,3-biphenyl)-9H-carbazol ist im Dokument mit H-2 bezeichnet.
Allerdings besteht bei Verwendung dieser Materialien oder bei der Verwendung von Mischungen der Materialien noch Verbesserungsbedarf, insbesondere in Bezug auf die Lebensdauer der organischen elektronischen Vorrichtung.
Aufgabe der vorliegenden Erfindung ist daher die Bereitstellung von Materialien, welche sich für den Einsatz in einer organischen elektronischen Vorrichtung, insbesondere in einer organischen Elektrolumineszenz-vorrichtung, und insbesondere in einer fluoreszierenden oder phosphoreszierenden OLED eignen und zu guten Device-Eigenschaften, insbesondere im Hinblick auf eine verbesserte Lebensdauer, führen, sowie die Bereitstellung der entsprechenden elektronischen Vorrichtung.
Es wurde nun gefunden, dass Zusammensetzungen, die Verbindungen der Formel (1e) enthalten und einen lochtransportierenden Host der Formel (2a) umfassen, diese Aufgabe lösen und die Nachteile aus dem Stand der Technik beseitigen. Derartige Zusammensetzungen führen zu sehr guten Eigenschaften organischer elektronischer Vorrichtungen, insbesondere organischer Elektrolumineszenzvorrichtungen, insbesondere hinsichtlich der Lebensdauer und insbesondere auch bei Anwesenheit einer lichtemittierenden Komponente in der Emissionsschicht bei Konzentrationen zwischen 2 und 15 Gew.-%.
Ein erster Gegenstand der vorliegenden Erfindung ist daher eine Zusammensetzung umfassend mindestens eine Verbindung der Formel (1e) und mindestens eine Verbindung der Formel (2a)

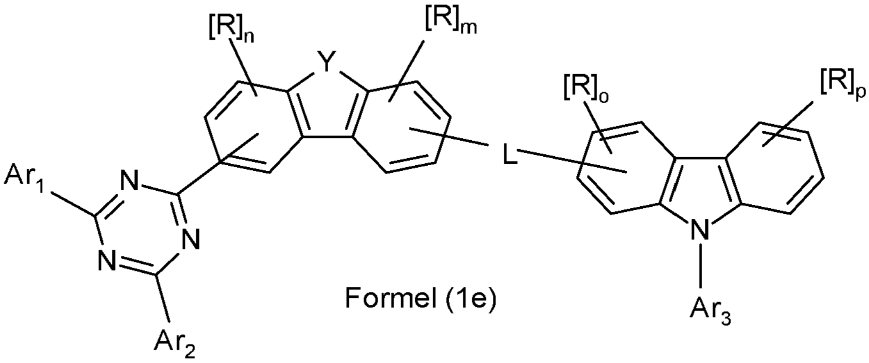

Formel (1e)

,

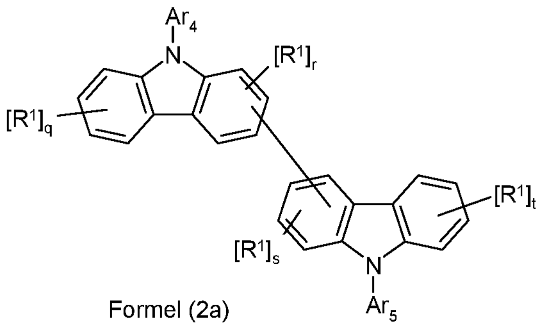

Formel (2a) ,

wobei für die verwendeten Symbole und Indizes gilt:

| | |
|---|---|
| Y | ist ausgewählt aus O oder S; |
| L | ist bei jedem Auftreten gleich oder verschieden ein aromatisches oder heteroaromatisches Ringsystem mit 5 bis 30 aromatischen Ringatomen, das durch einen oder mehrere Reste $R^3$ substituiert sein kann, bevorzugt ein aromatisches oder heteroaromatisches Ringsystem mit 6 bis 18 C-Atomen, das durch einen oder mehrere Reste $R^3$ substituiert sein kann; |
| Ar1, $Ar_2$ | sind jeweils unabhängig voneinander bei jedem Auftreten eine Aryl- oder Heteroarylgruppe mit 5 bis 40 aromatischen Ringatomen, die mit einem oder mehreren Resten $R^3$ substituiert sein können; |
| $Ar_3$ | ist ein aromatisches Ringsystem mit 6 bis 40 aromatischen Ringatomen oder ein heteroaromatisches Ringsystem mit 10 bis 40 aromatischen Ringatomen, das mit einem oder mehreren Resten $R^3$ substituiert sein kann; |
| $Ar_4$ und $Ar_5$ | sind jeweils unabhängig voneinander ein aromatisches Ringsystem mit 6 bis 40 aromatischen Ringatomen oder ein heteroaromatisches Ringsystem mit 10 bis 40 aromatischen Ringatomen, das mit einem oder mehreren Resten $R^3$ substituiert sein kann, in der Gesamtheit nur ein N-Atom enthält oder in der Gesamtheit ein oder mehrere O- und/oder S-Atome enthält, mit der Maßgabe, dass $Ar_4$ und $Ar_5$ nicht gleichzeitig Phenyl sein können; |
| R | ist bei jedem Auftreten gleich oder verschieden ausgewählt aus der Gruppe bestehend aus H, D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C{=}CR^2$, $Si(R^2)_2$, C=O, C=S, $C{=}NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 aromatischen Ringatomen, das jeweils mit einem oder mehreren Resten $R^2$ substituiert sein kann, einer Aryloxy- oder Heteroaryloxygruppe mit 5 bis 40 aromatischen Ringatomen, die mit einem oder mehreren Resten $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^2$ substituiert sein kann; |
| $R^1$ in $[R^1]_q$ | ist bei jedem Auftreten unabhängig Phenyl oder Biphenyl, wenn q größer 0 bedeutet; |
| $R^1$ in $[R^1]$t | ist bei jedem Auftreten unabhängig Phenyl, Biphenyl oder Terphenyl, wenn t größer 0 bedeutet; |
| $R^2$ | ist bei jedem Auftreten gleich oder verschieden ausgewählt aus der Gruppe bestehend aus H, D, F, Cl, Br, I, CN, $NO_2$, $N(Ar)_2$, $NH_2$, $N(R^3)_2$, C(=O)Ar, C(=O)H, $C(=O)R^3$, $P(=O)(Ar)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nicht-benachbarte $CH_2$-Gruppen durch HC=CH, $R^3C{=}CR^3$, C=C, $Si(R^3)_2$, $Ge(R^3)_2$, $Sn(R^3)_2$, C=O, C=S, C=Se, $C{=}NR^3$, $P(=O)(R^3)$, SO, $SO_2$, NH, $NR^3$, O, S, CONH oder $CONR^3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 aromatischen Ringatomen, das jeweils mit einem oder mehreren Resten $R^3$ substituiert sein kann, einer Aryloxy- oder Heteroaryloxygruppe mit 5 bis 60 aromatischen Ringatomen, die mit einem oder mehreren Resten $R^3$ substituiert sein kann, oder einer Kombination dieser Systeme, wobei optional zwei oder mehr benachbarte Substituenten $R^2$ ein monocycli- |

sches oder polycyclisches, aliphatisches, aromatisches oder heteroaromatisches Ringsystem bilden können, das mit einem oder mehreren Resten $R^3$ substituiert sein kann;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden;

Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^3$ substituiert sein kann; dabei können zwei Reste Ar, welche an dasselbe N-Atom, P-Atom oder B-Atom binden, auch durch eine Einfachbindung oder eine Brücke, ausgewählt aus $N(R^3)$, $C(R^3)_2$, O oder S, miteinander verbrückt sein und

n und m bedeuten unabhängig voneinander 0, 1, 2 oder 3;

p und o bedeuten jeweils unabhängig voneinander 0, 1, 2 oder 3;

q und t bedeuten jeweils unabhängig voneinander 0, 1, 2, 3 oder 4;

r ist 0 und

s ist 0.

$R^0$ ist bei jedem Auftreten gleich oder verschieden ausgewählt aus der Gruppe bestehend aus H, D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nicht-benachbarte $CH_2$-Gruppen durch $R^2C{=}CR^2$, $Si(R^2)_2$, C=O, C=S, $C{=}NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 aromatischen Ringatomen, das jeweils mit einem oder mehreren Resten $R^2$ substituiert sein kann, einer Aryloxy- oder Heteroaryloxygruppe mit 5 bis 40 aromatischen Ringatomen, die mit einem oder mehreren Resten $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann; dabei können optional zwei Substituenten $R^0$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^2$ substituiert sein kann.

L ist erfindungsgemäß bevorzugt ein aromatisches oder heteroaromatisches Ringsystem mit 6 bis 18 C-Atomen, das durch einen oder mehrere Reste $R^3$ substituiert sein kann. Das aromatische oder heteroaromatische Ringsystem mit 6 bis 18 C-Atomen ist bevorzugt ein Linker, ausgewählt aus L-1 bis L-40 wie nachfolgend beschrieben, der durch einen oder mehrere Reste $R^3$ substituiert sein kann. L ist erfindungsgemäß besonders bevorzugt ein aromatisches Ringsystem mit 6 bis 18 C-Atomen, das durch einen oder mehrere Reste $R^3$ substituiert sein kann, ganz besonders bevorzugt ausgewählt aus Phenylen, Naphthylen, Biphenylen, Phenanthrenylen oder Triphenylenylen, wobei die Anbindung an die weiteren Substituenten nicht eingeschränkt ist. Das aromatische Ringsystem mit 6 bis 18 C-Atomen ist bevorzugt Phenylen, wobei die Anbindung an die weiteren Substituenten nicht eingeschränkt ist. Phenylen kann hierbei in ortho, meta oder para-Stellung mit der Dibenzofuran/Dibenzothiophen-Einheit und der Carbazol-Einheit verknüpft sein. Bevorzugt wird L als Phenylen in meta-Stellung verknüpft.

Weitere Gegenstände der Erfindung umfassen Formulierungen, die derartige Zusammensetzungen enthalten, gemäß Anspruch 7, die Verwendung dieser Zusammensetzungen in einer organischen elektronischen Vorrichtung gemäß den Ansprüchen 8 und 9, organische elektronische Vorrichtungen, bevorzugt Elektrolumineszenzvorrichtungen, die derartige Zusammensetzungen enthalten, und dabei die Zusammensetzung bevorzugt in einer Schicht enthalten, gemäß den Ansprüchen 10 bis 14, sowie Verfahren zur Herstellung derartiger Vorrichtungen gemäß den Ansprüchen 15 bis 18. Die überraschenden und vorteilhaften Effekte werden durch spezifische Selektion bekannter Materialien erreicht, insbesondere, was die Auswahl elektronenleitenden Materialien der Formel (1e) und der lochtransportierenden Materialien der Formel (2a) betrifft.

Die Schicht, die die Zusammensetzung umfassend mindestens eine Verbindung der Formel (1e) und mindestens eine Verbindung der Formel (2a), wie zuvor beschrieben oder nachfolgend bevorzugt beschrieben, enthält, ist insbesondere eine emittierende Schicht (EML), eine Elektronentransportschicht (ETL), eine Elektroneninjektionsschicht (EIL) und/oder eine Lochblockierschicht (HBL).

Wenn es sich um eine emittierende Schicht handelt, dann ist es bevorzugt eine phosphoreszierende Schicht, die dadurch gekennzeichnet ist, dass sie zusätzlich zu der Zusammensetzung umfassend die Matrixmaterialien der Formel (1e) und Formel (2a), wie zuvor beschrieben, einen phosphoreszierenden Emitter enthält.

Benachbarte Kohlenstoffatome im Sinne der vorliegenden Erfindung sind Kohlenstoffatome, die direkt miteinander verknüpft sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

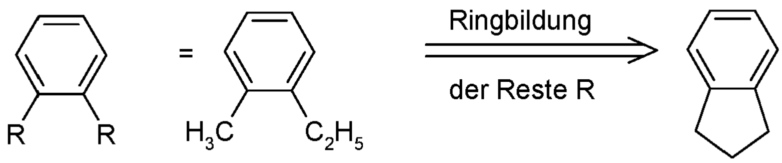


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

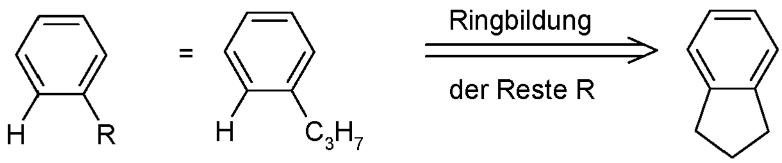


Eine Arylgruppe im Sinne dieser Erfindung enthält 6 bis 40 aromatische Ringatome, bevorzugt C-Atome. Eine Heteroarylgruppe im Sinne dieser Erfindung enthält 5 bis 40 aromatische Ringatome, wobei die Ringatome C-Atome und mindestens ein Heteroatom umfassen, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Phenyl, abgeleitet von Benzol, bzw. ein einfacher heteroaromatischer Cyclus, beispielsweise abgeleitet von Pyridin, Pyrimidin oder Thiophen, oder eine kondensierte Aryl- oder Heteroarylgruppe, beispielsweise abgeleitet von Naphthalin, Anthracen, Phenanthren, Chinolin oder Isochinolin, verstanden. Eine Arylgruppe mit 6 bis 10 C-Atomen ist daher vorzugsweise Phenyl oder Naphthyl, wobei die Anbindung der Arylgruppe als Substituent dabei nicht eingeschränkt ist. Eine Arylengruppe mit 6 bis 10 C-Atomen ist daher vorzugsweise Phenylen oder Naphthylen, wobei die Verknüpfung der Arylengruppe als Linker dabei nicht eingeschränkt ist.

Ein aromatisches Ringsystem im Sinne dieser Erfindung enthält 6 bis 40 C-Atome im Ringsystem und kann durch einen oder mehrere Reste $R^3$ substituiert sein, wobei $R^3$ eine nachfolgend beschriebene Bedeutung hat.

Ein aromatisches Ringsystem enthält auch Arylgruppen, wie zuvor beschrieben.

Ein heteroaromatisches Ringsystem im Sinne dieser Erfindung enthält 5 bis 40 Ringatome und mindestens ein Heteroatom und kann durch einen oder mehrere Reste $R^3$ substituiert sein, wobei $R^3$ eine nachfolgend beschriebene Bedeutung hat. Ein bevorzugtes heteroaromatische Ringsystem hat 10 bis 40 Ringatome und mindestens ein Heteroatom und kann durch einen oder mehrere Reste $R^3$ substituiert sein, wobei $R^3$ eine nachfolgend beschriebene Bedeutung hat. Ein heteroaromatisches Ringsystem enthält auch Heteroarylgruppen, wie zuvor beschrieben. Die Heteroatome im heteroaromatischen Ringsystem sind bevorzugt ausgewählt aus N, O und/oder S.

Unter einem aromatischen oder heteroaromatischen Ringsystem im Sinne dieser Erfindung wird ein System verstanden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische bzw. heteroaromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sind Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls von der Definition des aromatischen bzw. heteroaromatischen Ringsystems umfasst.

Unter einem aromatischen oder heteroaromatischen Ringsystem mit 5 - 40 aromatischen Ringatomen, welches noch jeweils mit den genannten Resten $R^3$ substituiert sein kann und welches über beliebige Positionen am Aromaten bzw. Heteroaromaten verknüpft sein kann, werden beispielsweise Gruppen verstanden, die abgeleitet sind von Benzol,

Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die Abkürzung Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^3$ substituiert sein kann; dabei können zwei Reste Ar, welche an dasselbe N-Atom, P-Atom oder B-Atom binden, auch durch eine Einfachbindung oder eine Brücke, ausgewählt aus $N(R^3)$, $C(R^3)_2$, O oder S, miteinander verbrückt sein. Der Substituent $R^3$ wurde zuvor beschrieben oder wird nachfolgend bevorzugt beschrieben.

Unter einer cyclischen Alkyl-, Alkoxy- oder Thioalkylgruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden.

Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden.

Unter einer Alkinylgruppe werden beispielsweise Ethinyl, Propinyl, Butinyl, Pentinyl, Hexinyl, Heptinyl oder Octinyl verstanden.

Unter einer $C_1$- bis $C_{20}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einer $C_1$- bis $C_{20}$-Thioalkylgruppe werden beispielsweise S-Alkylgruppen verstanden, beispielsweise Thiomethyl, 1-Thioethyl, 1-Thio-i-propyl, 1-Thio-n-propoyl, 1-Thio-i-butyl, 1-Thio-n-butyl oder 1-Thio-t-butyl.

Eine Aryloxy- oder Heteroaryloxygruppe mit 5 bis 40 aromatischen Ringatomen bedeutet O-Aryl oder O-Heteroaryl und bedeutet, dass die Aryl- bzw. Heteroarylgruppe über ein Sauerstoffatom gebunden wird.

Eine Aralkyl- oder Heteroaralkylgruppe mit 5 bis 40 aromatischen Ringatomen bedeutet, dass eine Alkylgruppe, wie zuvor beschrieben, mit einer Arylgruppe bzw. Heteroarylgruppe substituiert ist.

Ein phosphoreszierender Emitter im Sinne der vorliegenden Erfindung ist eine Verbindung, die Lumineszenz aus einem angeregten Zustand mit höherer Spinmultiplizität zeigt, also einem Spinzustand > 1, insbesondere aus einem angeregten Triplettzustand. Im Sinne dieser Anmeldung sollen alle lumineszierenden Komplexe mit Übergangsmetallen oder Lanthaniden als phosphoreszierende Emitter angesehen werden. Eine genauere Definition erfolgt weiter unten.

Wenn die Zusammensetzung umfassend mindestens eine Verbindung der Formel (1e), wie zuvor beschrieben oder nachfolgend bevorzugt beschrieben, und mindestens eine Verbindung der Formel (2a), wie zuvor beschrieben oder nachfolgend beschrieben, als Matrixmaterial für einen phosphoreszierenden Emitter eingesetzt wird, ist es bevorzugt, wenn deren Triplettenergie nicht wesentlich kleiner als die Triplettenergie des phosphoreszierenden Emitters ist. Dabei gilt bevorzugt für das Triplettniveau $T_1$(Emitter) - $T_1$(Matrix) $\leq 0.2$ eV, besonders bevorzugt $\leq 0.15$ eV, ganz besonders bevorzugt $\leq 0.1$ eV. Dabei ist $T_1$(Matrix) das Triplettniveau des Matrixmaterials in der Emissionsschicht, wobei diese Bedingung für jedes der beiden Matrixmaterialien gilt, und $T_1$(Emitter) ist das Triplettniveau des phosphoreszierenden Emitters. Enthält die Emissionsschicht mehr als zwei Matrixmaterialien, so gilt die oben genannte Beziehung

bevorzugt auch für jedes weitere Matrixmaterial.

Elektronentransportierende Hosts der Formel (1e):

In einer Ausführungsform der Erfindung werden Verbindungen der Formel (1e) ausgewählt, in denen Y ausgewählt ist aus O oder S und der Substituent

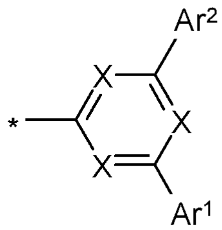

ist in Position 1, 2, 3 oder 4 des Dibenzofurans oder Dibenzothiophens gebunden und bedeutet ein Triazin, wobei Y, L, $Ar_1$, $Ar_2$, $Ar_3$, R, n und m eine zuvor angegebene Bedeutung haben oder eine nachfolgend angegebene Bedeutung haben und * die Verknüpfungsstelle mit dem Dibenzofuran bzw. Dibenzothiophen kennzeichnet.

Verbindungen der Formel (1e), in denen sich der Substituent

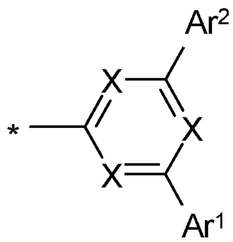

in Position 1 oder 2 des Dibenzofurans oder Dibenzothiophens befindet und ein Triazin bedeutet, werden durch die Formeln (1a) und (1b) dargestellt,

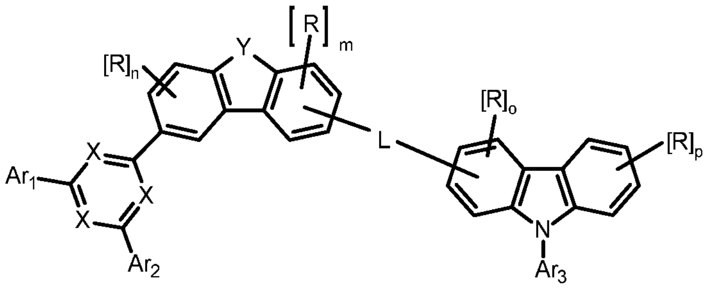

Formel (1a) ,

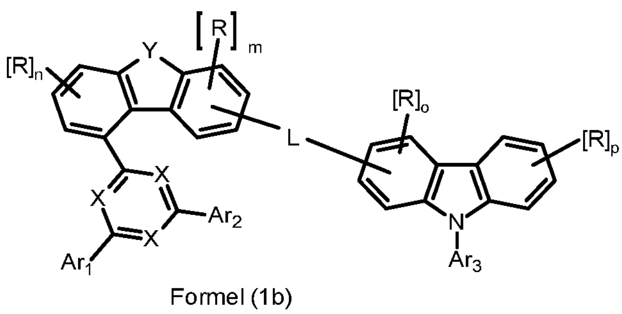

Formel (1b) ,

wobei Y, L, $Ar_1$, $Ar_2$, $Ar_3$, R, n und m eine zuvor angegebene Bedeutung haben oder eine nachfolgend angegebene Bedeutung haben und p und o jeweils unabhängig voneinander 0, 1, 2 oder 3 bedeuten.

Verbindungen der Formel (1e), in denen sich der Substituent

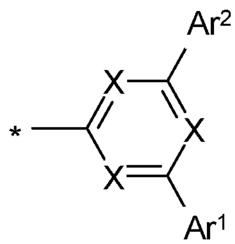

in Position 3 oder 4 des Dibenzofurans oder Dibenzothiophens befindet und ein Triazin bedeutet, werden durch die Formeln (1c) und (1d) dargestellt,

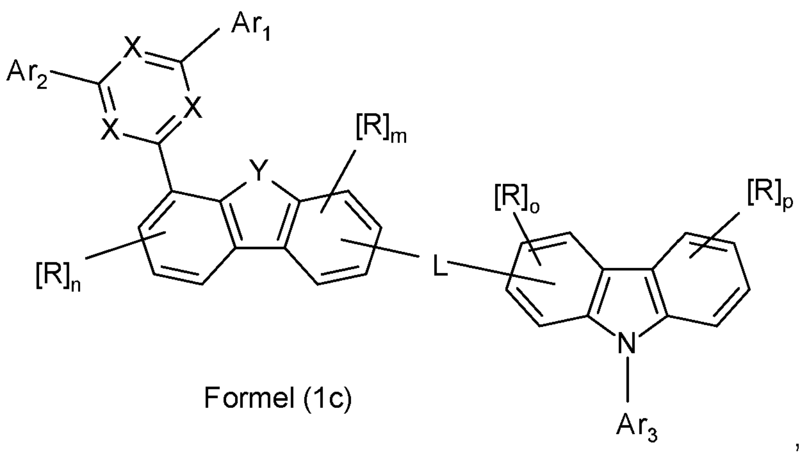

Formel (1c)

,

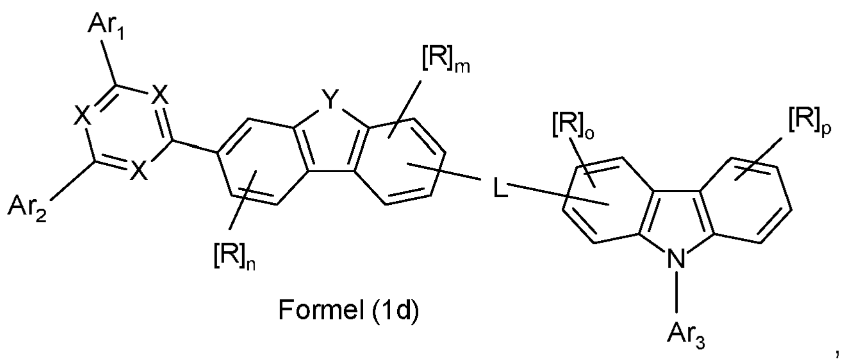

Formel (1d)

,

wobei Y, L, $Ar_1$, $Ar_2$, $Ar_3$, R, n und m eine zuvor angegebene Bedeutung haben oder eine nachfolgend angegebene Bedeutung haben und p und o jeweils unabhängig voneinander 0, 1, 2 oder 3 bedeuten.

Bevorzugt wird mindestens eine Verbindung der Formel (1a) für die Zusammensetzung ausgewählt, mit Substituenten, die zuvor beschrieben oder nachfolgend bevorzugt beschrieben werden.

Bevorzugt wird mindestens eine Verbindung der Formel (1b) für die Zusammensetzung ausgewählt, mit Substituenten, die zuvor beschrieben oder nachfolgend bevorzugt beschrieben werden.

Bevorzugt wird mindestens eine Verbindung der Formel (1c) für die Zusammensetzung ausgewählt, mit Substituenten, die zuvor beschrieben oder nachfolgend bevorzugt beschrieben werden.

Bevorzugt wird mindestens eine Verbindung der Formel (1d) für die Zusammensetzung ausgewählt, mit Substituenten, die zuvor beschrieben oder nachfolgend bevorzugt beschrieben werden.

Ein weiterer Gegenstand der Erfindung ist demzufolge eine Zusammensetzung, wie zuvor beschrieben, wobei die Verbindung der Formel (1e) der Verbindung der Formel (1a), (1b), (1c) oder (1d) entspricht, bevorzugt der Formel (1b) oder (1c) entspricht.

Verbindungen der Formel (1e) werden für die Zusammensetzung ausgewählt,

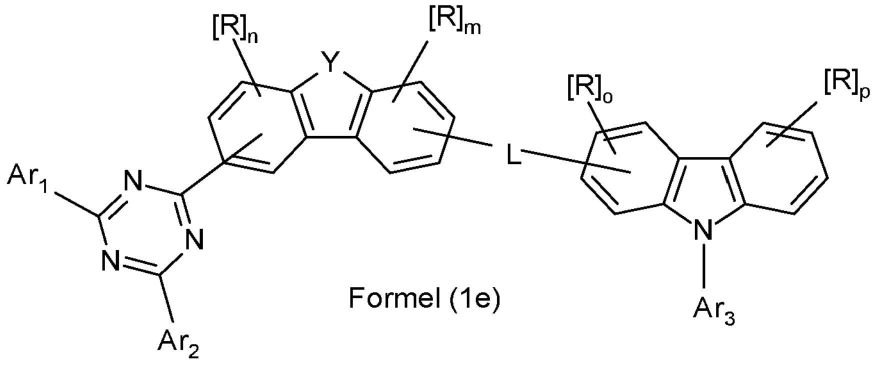

Formel (1e)

,

wobei Y, L, $Ar_1$, $Ar_2$, $Ar_3$, R, n und m eine zuvor angegebene Bedeutung haben oder eine nachfolgend angegebene Bedeutung haben,
der Triazinsubstituent in 1, 2, 3 oder 4-Position angeknüpft ist und p und o jeweils unabhängig voneinander 0, 1, 2 oder 3 bedeuten.

In Verbindungen der Formeln (1a), (1b), (1c), (1d) oder (1e) oder bevorzugt beschriebenen Verbindungen der Formeln (1a), (1b), (1c), (1d) oder (1e) stehen $Ar_1$ und $Ar_2$ jeweils unabhängig voneinander bevorzugt für eine Arylgruppe mit 6 bis 40 C-Atomen, wie zuvor beschrieben oder bevorzugt beschrieben, die mit einem oder mehreren Resten $R^3$ substituiert sein kann. Besonders bevorzugt steht mindestens ein $Ar_1$ oder $Ar_2$ für Phenyl und der andere aromatische Substituent steht für eine Arylgruppe mit 6 bis 40 C-Atomen, die mit einem oder mehreren Resten $R^3$ substituiert sein kann. Besonders bevorzugt steht mindestens ein $Ar_1$ oder $Ar_2$ für Phenyl und der andere aromatische Substituent steht für eine Phenylgruppe, die mit einem oder mehreren Resten $R^3$ substituiert sein kann. Ganz besonders bevorzugt sind beide Gruppen $Ar_1$ und $Ar_2$ gleich. Ganz besonders bevorzugt stehen beide Gruppen $Ar_1$ und $Ar_2$ für Phenyl.

Wenn in Verbindungen der Formeln (1a), (1b), (1c), (1d) oder (1e) oder bevorzugt beschriebenen Verbindungen der Formeln (1a), (1b), (1c), (1d) oder (1e) $Ar_1$ und $Ar_2$, wie zuvor beschrieben oder bevorzugt beschrieben, eine Aryl- oder Heteroarylgruppe bedeutet, die mit einem oder mehreren Resten $R^3$ substituiert sind, so wird der Substituent $R^3$ bei jedem Auftreten gleich oder verschieden bevorzugt ausgewählt aus der Gruppe bestehend aus D, F oder einem aromatischen oder heteroaromatischen Ringsystem mit 5 bis 40 aromatischen Ringatomen. Das heteroaromatische Ringsystem mit 5 bis 40 aromatischen Ringatomen ist für diese/diesen Substituenten $R^3$ bevorzugt abgeleitet von Dibenzofuran oder Dibenzothiophen. Das aromatische Ringsystem mit 6 bis 40 aromatischen Ringatomen ist für diese/diesen Substituenten $R^3$ bevorzugt Phenyl, Biphenyl oder Terphenyl, besonders bevorzugt Phenyl oder [1,1',2',1"]-Terphenyl-5'-yl. Bevorzugt ist die Arylgruppe oder Heteroarylgruppe in $Ar_1$ und $Ar_2$ jeweils unabhängig voneinander ein Mal mit $R^3$ substituiert. Besonders bevorzugt ist die Arylgruppe oder Heteroarylgruppe in $Ar_1$ oder $Ar_2$ ein Mal mit $R^3$ substituiert. Ganz besonders bevorzugt sind die Arylgruppe oder Heteroarylgruppe in $Ar_1$ und $Ar_2$ unsubstituiert.

In Verbindungen der Formeln(1a), (1b), (1c), (1d) oder (1e) oder bevorzugt beschriebenen Verbindungen der Formeln (1a), (1b), (1c), (1d) oder (1e) wird Y ausgewählt aus O oder S. Besonders bevorzugt steht Y für O.

In Verbindungen der Formeln (1a), (1b), (1c), (1d) oder (1e) oder bevorzugt beschriebenen Verbindungen der Formeln (1a), (1b), (1c), (1d) oder (1e) ist n bevorzugt 0 oder 1, wobei R eine zuvor angegebene Bedeutung oder eine nachfolgend angegebene Bedeutung hat. Besonders bevorzugt ist n 0.

In Verbindungen der Formeln (1a), (1b), (1c), (1d) oder (1e) oder in bevorzugt beschriebenen Verbindungen der Formeln (1a), (1b), (1c), (1d) oder (1e) ist m bevorzugt 0 oder 1, wobei R eine zuvor angegebene Bedeutung oder eine nachfolgend angegebene Bedeutung hat. Besonders bevorzugt ist m 0.

In Verbindungen der Formeln (1a), (1b), (1c), (1d) oder (1e) oder in bevorzugt beschriebenen Verbindungen der Formeln(1a), (1b), (1c), (1d) oder (1e) ist die Summe von n und m, abgekürzt (n+m), bevorzugt 0, 1 oder 2, wobei R eine zuvor angegebene Bedeutung oder eine nachfolgend angegebene Bedeutung hat. Besonders bevorzugt ist (n+m) 0 oder 1. Ganz besonders bevorzugt ist (n+m) 0.

Wenn in Verbindungen der Formeln (1a), (1b), (1c), (1d) oder (1e) oder in bevorzugt beschriebenen Verbindungen der Formeln (1a), (1b), (1c), (1d) oder (1e) n und m größer 0 oder n oder m größer 0 sind, so wird der Substituent R bei jedem Auftreten gleich oder verschieden bevorzugt ausgewählt aus der Gruppe bestehend aus D, F, einer Alkylgruppe mit 1 bis 40 C-Atomen oder einem aromatischen oder heteroaromatischen Ringsystem mit 5 bis 40 aromatischen Ringatomen. Das heteroaromatische Ringsystem mit 5 bis 40 aromatischen Ringatomen ist für diesen Substituenten R bevorzugt abgeleitet von Dibenzofuran oder Dibenzothiophen. Das aromatische Ringsystem mit 6 bis 40 aromatischen Ringatomen ist für diesen Substituenten R bevorzugt Phenyl, Biphenyl oder Terphenyl, besonders bevorzugt Phenyl oder [1,1',2',1"]-Terphenyl-5'-yl. Die Alkylgruppe mit 1 bis 40 C-Atomen ist für diesen Substituenten R bevorzugt eine

lineare oder verzweigte Alkylgruppe mit 1 bis 4 C-Atomen, besonders bevorzugt Methyl, Ethyl, n-Propyl oder n-Butyl, ganz besonders bevorzugt Methyl.

In Verbindungen der Formeln (1a), (1b), (1c), (1d) oder (1e) oder bevorzugt beschriebenen Verbindungen der Formeln (1a), (1b), (1c), (1d) oder (1e) ist L bevorzugt bei jedem Auftreten gleich oder verschieden ein aromatisches oder heteroaromatisches Ringsystem mit 6 bis 18 C-Atomen, das durch einen oder mehrere Reste $R^3$ substituiert sein kann.

In Verbindungen der Formeln (1a), (1b), (1c), (1d) oder (1e) oder bevorzugt beschriebenen Verbindungen der Formeln (1a), (1b), (1c), (1d) oder (1e) ist L ganz besonders bevorzugt ein aromatisches Ringsystem mit 6 bis 18 C-Atomen, das durch einen oder mehrere Reste $R^3$ substituiert sein kann. $R^3$ ist dabei bevorzugt aus der Gruppe bestehend aus D oder Phenyl ausgewählt.

Das aromatische oder heteroaromatische Ringsystem mit 6 bis 18 C-Atomen ist bevorzugt ein Linker, ausgewählt aus L-1 bis L-40, die unsubstituiert sind oder durch $R^3$, wie zuvor beschrieben, substituiert sein können:

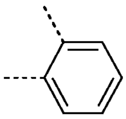
L-1

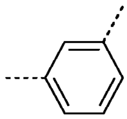
L-2

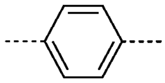
L-3

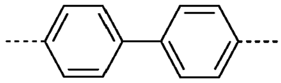
L-4

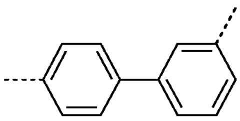
L-5

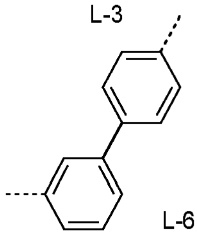
L-6

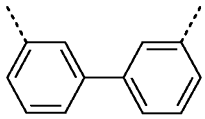
L-7

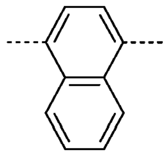
L-8

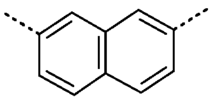
L-9

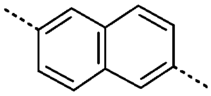
L-10

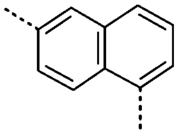
L-11

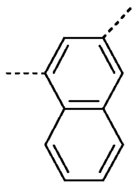
L-12

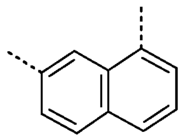
L-13

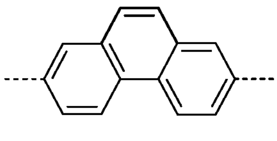
L-14

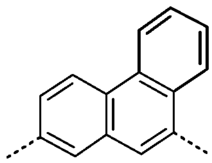
L-15

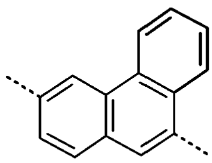
L-16

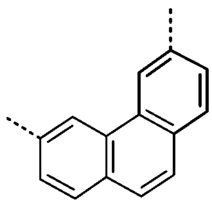
L-17

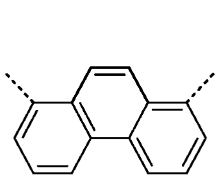
L-18

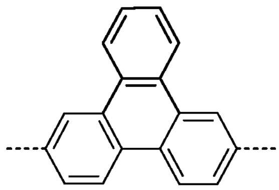
L-19

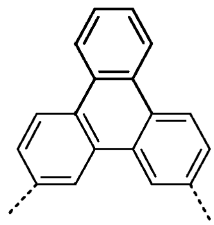
L-20

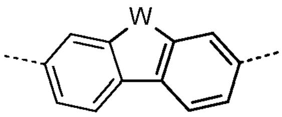

L-21

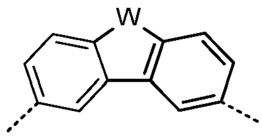

L-22

,

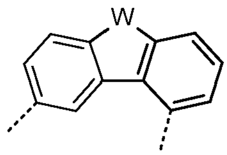

L-23

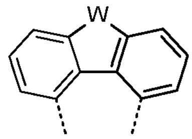

L-24

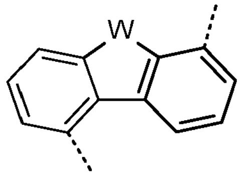

L-25

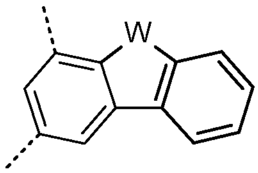

L-26

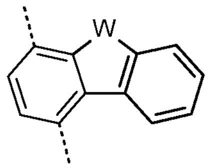

L-27

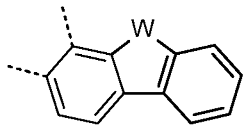

L-28

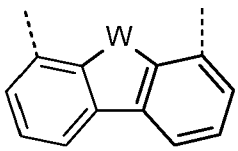

L-29

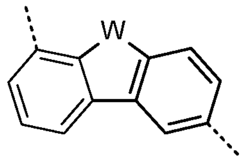

L-30

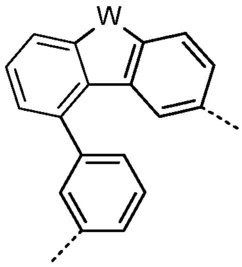

L-31

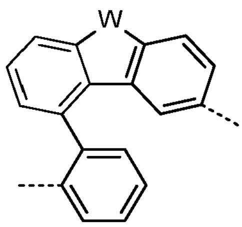

L-32

L-33

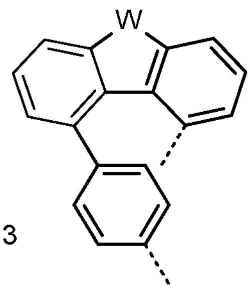

L-34

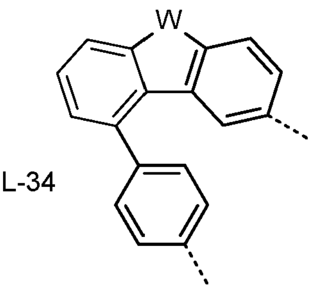

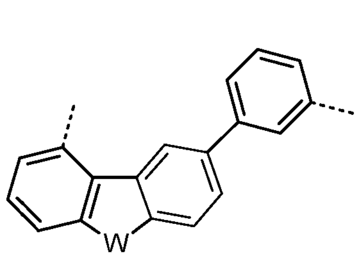

L-35

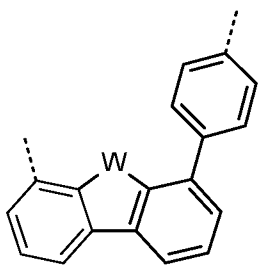

L-36

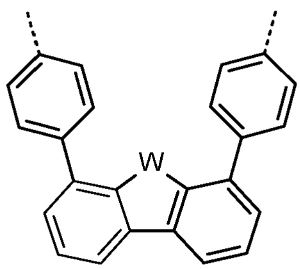

L-37

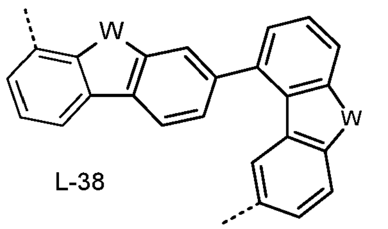

L-38

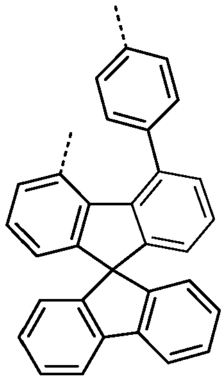

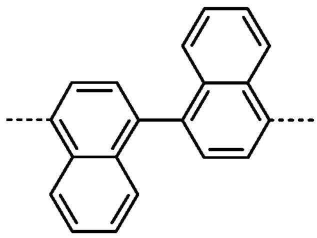

L-39 L-40 ,

wobei W O, S oder $C(R^0)_2$ bedeutet. W ist bevorzugt O oder S. W ist besonders bevorzugt $C(R^0)_2$, wobei $R^0$ Methyl oder Phenyl bedeutet.

Das aromatische Ringsystem mit 6 bis 18 C-Atomen und damit der Linker L wird besonders bevorzugt ausgewählt aus Phenylen, Naphthylen, Biphenylen, Phenanthrenylen oder Triphenylen, wobei die Anbindung an die weiteren Substituenten nicht eingeschränkt ist. Das aromatische Ringsystem mit 6 bis 18 C-Atomen ist bevorzugt Phenylen, wobei die Anbindung an die weiteren Substituenten nicht eingeschränkt ist. Phenylen kann hierbei in ortho, meta oder para-Stellung mit der Dibenzofuran/Dibenzothiophen-Einheit und der Carbazol-Einheit verknüpft sein. Bevorzugt wird L als Phenylen in meta-Stellung verknüpft.

Bevorzugt ist L oder einer der Linker L-1 bis L-40 unsubstituiert.

In Verbindungen der Formeln (1a), (1b), (1c), (1d) oder (1e) oder bevorzugt beschriebenen Verbindungen der Formeln(1a), (1b), (1c), (1d) oder (1e) kann L in Position 6, 7, 8 oder 9 des Dibenzofuran-Rings bzw. des Dibenzothiophen-Rings verknüpft sein. L, wie zuvor beschrieben oder als bevorzugt beschrieben, ist bevorzugt in Position 6 oder Position 8 des Dibenzofuran-Rings bzw. des Dibenzothiophen-Rings verknüpft. L, wie zuvor beschrieben oder als bevorzugt beschrieben, ist besonders bevorzugt in Position 8 des Dibenzofuran-Rings bzw. des Dibenzothiophen-Rings verknüpft.

In dieser Ausführungsform, wenn L an Position 8 des Dibenzofuran-Rings bzw. des Dibenzothiophen-Rings verknüpft ist, werden Verbindungen der Formel (1f) bevorzugt für die Zusammensetzung ausgewählt,

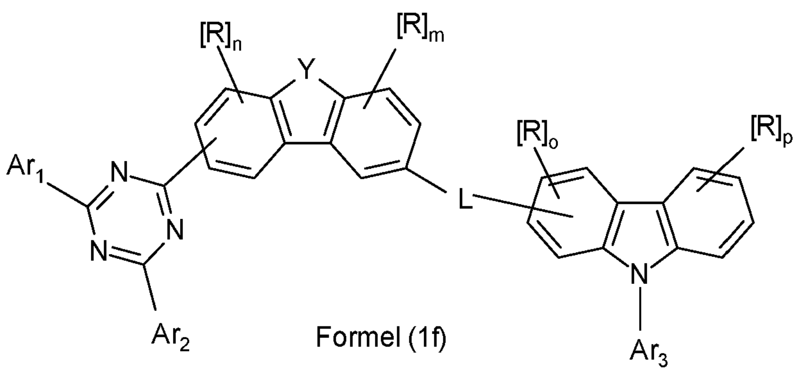

Formel (1f) ,

wobei Y, L, $Ar_1$, $Ar_2$, n und m eine zuvor angegebene oder bevorzugt angegebene Bedeutung haben, R eine zuvor oder nachfolgend angegebene Bedeutung hat, der Triazinsubstituent in 1, 2, 3 oder 4-Position angeknüpft ist, $Ar_3$ eine zuvor angegebene oder nachfolgend als bevorzugt beschriebene Bedeutung hat und p und o jeweils unabhängig voneinander

0, 1, 2 oder 3 bedeuten.

In Verbindungen der Formeln (1a), (1b), (1c), (1d), (1e) oder (1f) oder bevorzugt beschriebenen Verbindungen der Formeln (1a), (1b), (1c), (1d), (1e) oder (1f) kann L in beliebiger Position an das Heteroaryl, vorzugsweise das Carbazol gebunden sein. L, wie zuvor beschrieben oder als bevorzugt beschrieben, ist bevorzugt in Position 3 des Carbazols verknüpft.

In dieser Ausführungsform werden Verbindungen der Formel (1h) bevorzugt für die Zusammensetzung ausgewählt,

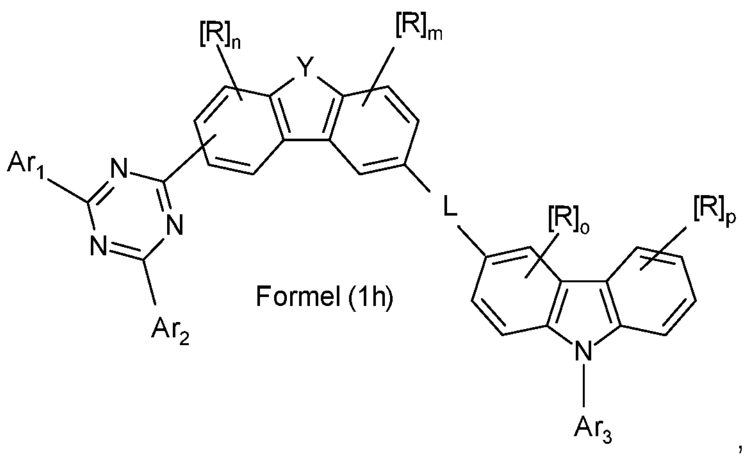

Formel (1h)

,

wobei Y, $Ar_1$, $Ar_2$, L, n und m eine zuvor angegebene oder bevorzugt angegebene Bedeutung haben, R eine zuvor oder nachfolgend angegebene Bedeutung hat, der Triazinsubstituent in 1, 2, 3 oder 4-Position angeknüpft ist, $Ar_3$ eine zuvor angegebene oder nachfolgend als bevorzugt beschriebene Bedeutung hat und p und o jeweils unabhängig voneinander 0, 1, 2 oder 3 bedeuten.

Besonders bevorzugt ausgewählte Verbindungen der Formel (1e), wie zuvor beschrieben oder als bevorzugt beschrieben, entsprechen der Formel (1i),

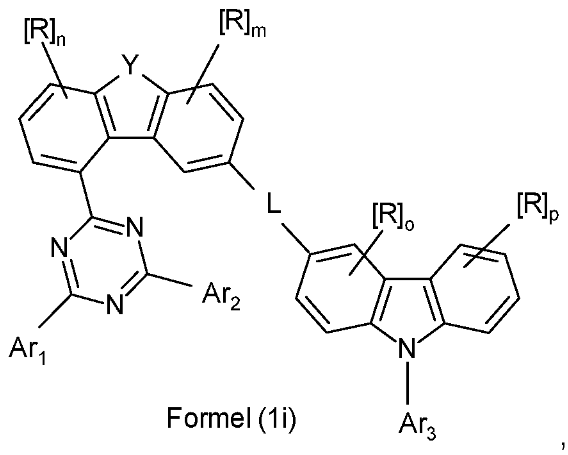

Formel (1i)

,

wobei Y, $Ar_1$, $Ar_2$, L, n und m eine zuvor angegebene oder bevorzugt angegebene Bedeutung haben, R eine zuvor oder nachfolgend angegebene Bedeutung hat, $Ar_3$ eine zuvor angegebene oder nachfolgend als bevorzugt beschriebene Bedeutung hat und p und o jeweils unabhängig voneinander 0, 1, 2 oder 3 bedeuten.

Besonders bevorzugt ausgewählte Verbindungen der Formel (1e), wie zuvor beschrieben oder als bevorzugt beschrieben, entsprechen der Formel (1j),

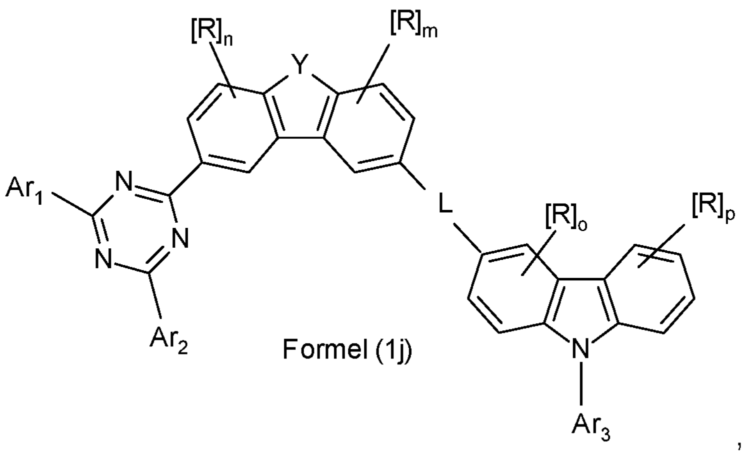

Formel (1j)

,

wobei Y, $Ar_1$, $Ar_2$, L, n und m eine zuvor angegebene oder bevorzugt angegebene Bedeutung haben, R eine zuvor oder nachfolgend angegebene Bedeutung hat, $Ar_3$ eine zuvor angegebene oder nachfolgend als bevorzugt beschriebene Bedeutung hat und p und o jeweils unabhängig voneinander 0, 1, 2 oder 3 bedeuten.

Besonders bevorzugt ausgewählte Verbindungen der Formel (1e), wie zuvor beschrieben oder als bevorzugt beschrieben, entsprechen der Formel (1k),

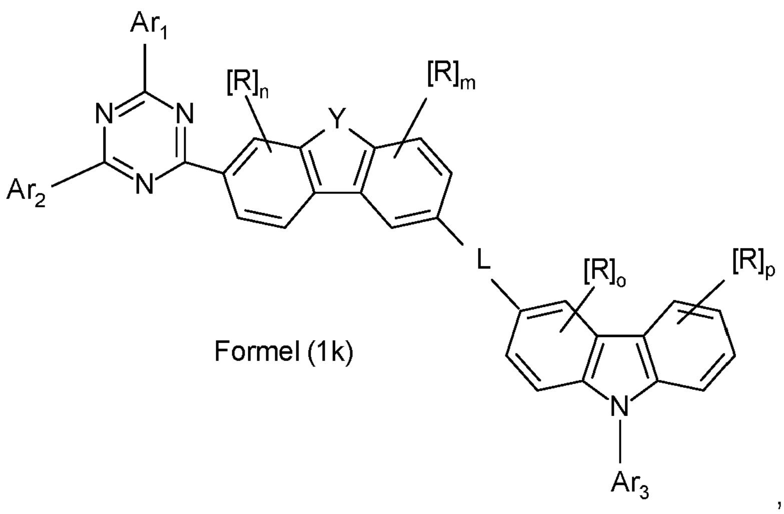

Formel (1k)

,

wobei Y, $Ar_1$, $Ar_2$, L, n und m eine zuvor angegebene oder bevorzugt angegebene Bedeutung haben, R eine zuvor oder nachfolgend angegebene Bedeutung hat, $Ar_3$ eine zuvor angegebene oder nachfolgend als bevorzugt beschriebene Bedeutung hat und p und o jeweils unabhängig voneinander 0, 1, 2 oder 3 bedeuten.

Besonders bevorzugt ausgewählte Verbindungen der Formel (1e), wie zuvor beschrieben oder als bevorzugt beschrieben, entsprechen der Formel (1l),

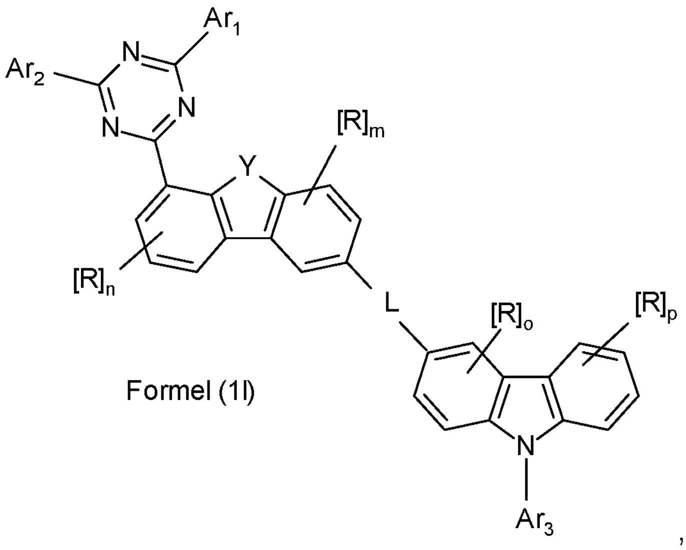

Formel (1l)

,

wobei Y, $Ar_1$, $Ar_2$, L, n und m eine zuvor angegebene oder bevorzugt angegebene Bedeutung haben, R eine zuvor oder nachfolgend angegebene Bedeutung hat, $Ar_3$ eine zuvor angegebene oder nachfolgend als bevorzugt beschriebene Bedeutung hat und p und o jeweils unabhängig voneinander 0, 1, 2 oder 3 bedeuten.

In Verbindungen der Formeln (1a), (1b), (1c), (1d), (1e), (1f), (1h), (1i), (1j), (1k) oder (1l) oder bevorzugt beschriebenen Verbindungen der Formeln (1a), (1b), (1c), (1d), (1e), (1f), (1h), (1i), (1j), (1k) oder (1l) ist o bevorzugt 0 oder 1, wobei R eine zuvor angegebene Bedeutung oder eine nachfolgend angegebene Bedeutung hat. Besonders bevorzugt ist o 0.

In Verbindungen der Formeln (1a), (1b), (1c), (1d), (1e), (1f), (1h), (1i), (1j), (1k) oder (1l) oder bevorzugt beschriebenen Verbindungen der Formeln (1a), (1b), (1c), (1d), (1e), (1f), (1h), (1i), (1j), (1k) oder (1l) ist p bevorzugt 0, 1 oder 2, wobei R jeweils unabhängig voneinander eine zuvor angegebene Bedeutung oder eine nachfolgend angegebene Bedeutung hat. Besonders bevorzugt ist p 0 oder 1. Ganz besonders bevorzugt ist p 0.

Wenn in Verbindungen der Formeln (1a), (1b), (1c), (1d), (1e), (1f), (1h), (1i), (1j), (1k) oder (1l) oder bevorzugt beschriebenen Verbindungen der Formeln (1a), (1b), (1c), (1d), (1e), (1f), (1h), (1i), (1j), (1k) oder (1l) p größer 0 ist, so wird der Substituent R bei jedem Auftreten gleich oder verschieden bevorzugt ausgewählt aus der Gruppe bestehend aus D, F, einer Alkylgruppe mit 1 bis 40 C-Atomen oder einem aromatischen oder heteroaromatischen Ringsystem mit 5 bis 40 aromatischen Ringatomen oder zwei Substituenten R, die an benachbarte Kohlenstoffatome gebunden sind, bilden ein aromatisches oder heteroaromatisches Ringsystem. Das aromatische oder heteroaromatische Ringsystem mit 5 bis 40 aromatischen Ringatomen in diesem R entspricht bevorzugt $Ar_3$. Bevorzugte Bedeutungen von $Ar_3$ werden nachfolgend beschrieben. Das durch zwei Substituenten R gebildete aromatische oder heteroaromatische Ringsystem entspricht besonders bevorzugt einem Spirobifluoren.

Wenn in Verbindungen der Formeln (1a), (1b), (1c), (1d), (1e), (1f), (1h), (1i), (1j), (1k) oder (1l) oder bevorzugt beschriebenen Verbindungen der Formeln (1a), (1b), (1c), (1d), (1e), (1f), (1h), (1i), (1j), (1k) oder (1l) p größer 0 ist, so wird der Substituent R bei jedem Auftreten gleich oder verschieden besonders bevorzugt von aromatischen oder heteroaromatischen Ringsystemen aus der Gruppe Carbazol, 9-Phenyl-carbazol, Dibenzofuran, Dibenzothiophen, Fluoren, Terphenyl oder Spirobifluoren abgeleitet, ganz besonders bevorzugt aus der Gruppe 9-Phenyl-carbazol und Spirobifluoren. Zwei Substituenten R am Carbazol, die zusammen ein aromatisches oder heteroaromatisches Ringsystem bilden, entsprechen bevorzugt der Formel (A),

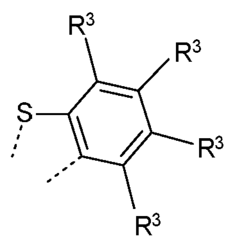

Formel (A)

.

In Verbindungen der Formeln (1a), (1b), (1c), (1d), (1e), (1f), (1h), (1i), (1j), (1k) oder (1l) oder bevorzugt beschriebenen Verbindungen der Formeln (1a), (1b), (1c), (1d), (1e), (1f), (1h), (1i), (1j), (1k) oder (1l) wird $Ar_3$ bevorzugt

aus einem aromatischen Ringsystem mit 6 bis 40 aromatischen Ringatomen oder einem heteroaromatischen Ringsystem mit 10 bis 40 aromatischen Ringatomen ausgewählt, das mit einem oder mehreren Resten $R^3$ substituiert sein kann, wobei heteroaromatische Ringsysteme mit 10 bis 40 aromatischen Ringatomen enthaltend N ausgenommen sind.

In Verbindungen der Formeln (1a), (1b), (1c), (1d), (1e), (1f), (1h), (1i), (1j), (1k) oder (1l) oder bevorzugt beschriebenen Verbindungen der Formeln (1a), (1b), (1c), (1d), (1e), (1f), (1h), (1i), (1j), (1k) oder (1l) wird $Ar_3$ bevorzugt aus den aromatischen oder heteroaromatischen Ringsystemen Ar-1 bis Ar-22 ausgewählt,

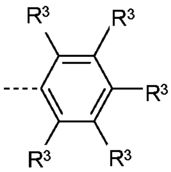
Ar-1

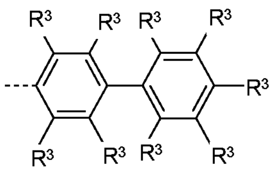
Ar-2

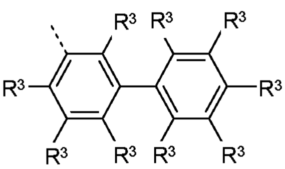
Ar-3

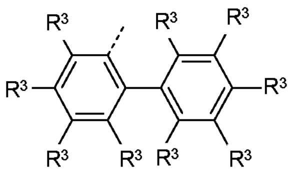
Ar-4

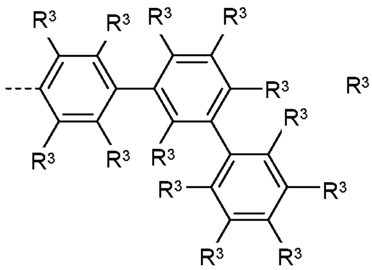
Ar-5

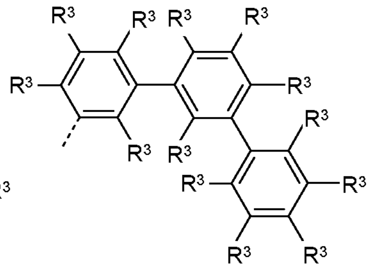
Ar-6

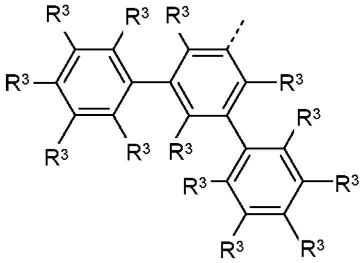
Ar-7

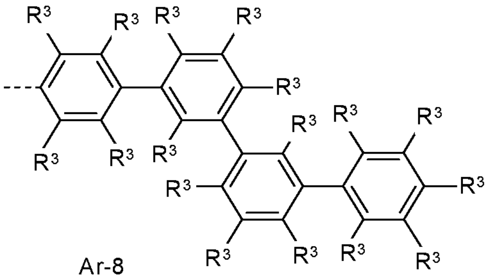
Ar-8

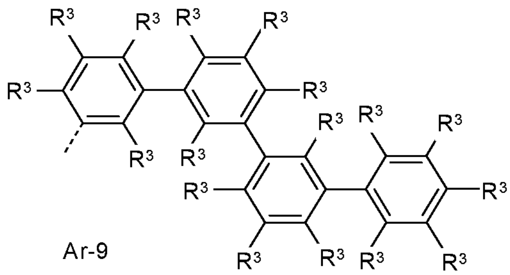
Ar-9

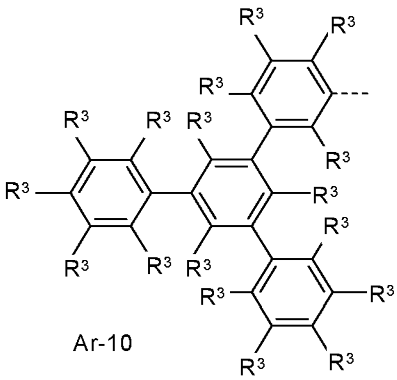
Ar-10

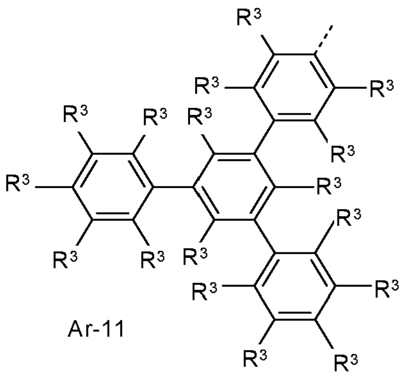
Ar-11

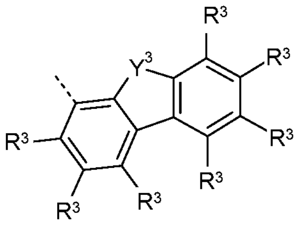
Ar-12

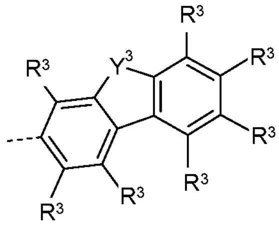
Ar-13

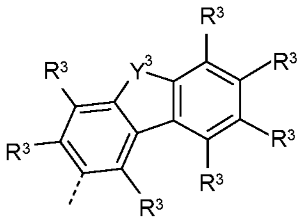
Ar-14

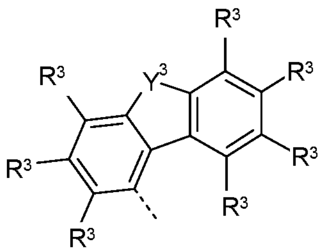
Ar-15

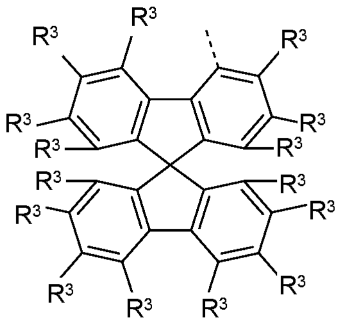
Ar-16

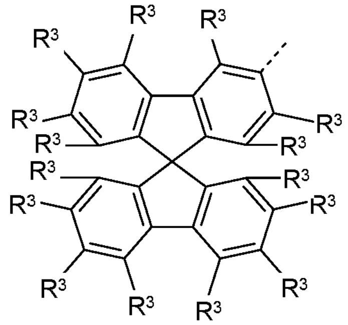
Ar-17

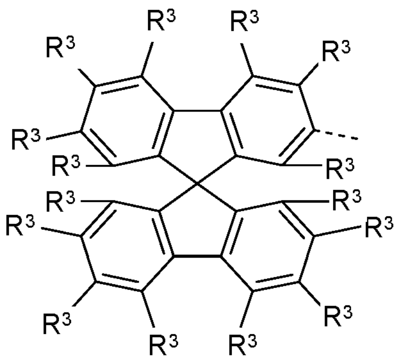
Ar-18

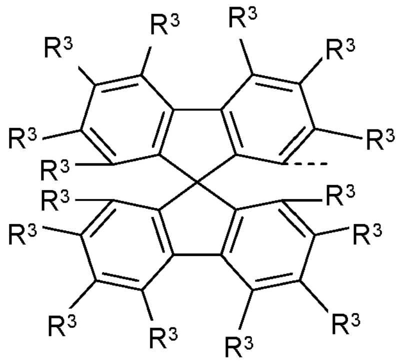
Ar-19

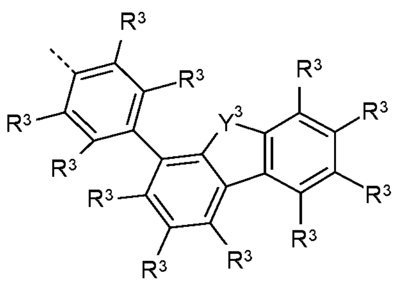
Ar-20

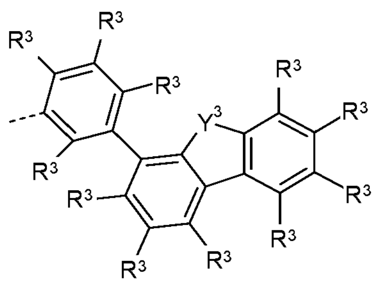
Ar-21

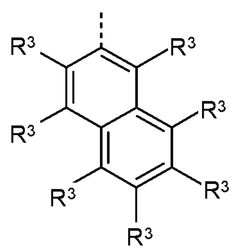
Ar-22

,

wobei $Y^3$ bei jedem Auftreten gleich oder verschieden O, S oder $C(R^{\#})_2$ bedeutet, wobei $R^3$ die zuvor genannte oder eine nachfolgend bevorzugte Bedeutung hat und die gestrichelte Bindung die Bindung an das N-Atom darstellt und wobei $R^3$ als Substituent für $Ar_3$ kein heteroaromatisches Ringsystem mit 5 bis 30 aromatischen Ringatomen umfasst.

Der Rest $R^{\#}$ ist bei jedem Auftreten gleich oder verschieden H, D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine

Alkenylgruppe mit 2 bis 20 C-Atomen, die jeweils mit einem oder mehreren Resten $R^2$ substituiert sein kann, wobei eine oder mehrere nicht-benachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 40 aromatischen Ringatomen, das jeweils mit einem oder mehreren Resten $R^2$ substituiert sein kann, eine Aryloxy- oder Heteroaryloxygruppe mit 5 bis 40 aromatischen Ringatomen, die mit einem oder mehreren Resten $R^2$ substituiert sein kann, oder eine Aralkyl- oder Heteroaralkylgruppe mit 5 bis 40 aromatischen Ringatomen, die mit einem oder mehreren Resten $R^2$ substituiert sein kann; dabei können optional zwei Substituenten $R^{\#}$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^2$ substituiert sein kann.

$Y^3$ ist bevorzugt O, S oder $C(CH_3)_2$. $Y^3$ ist ganz besonders bevorzugt O. $Y^3$ ist ganz besonders bevorzugt $C(CH_3)_2$.

In den Strukturen Ar-1 bis Ar-22 ist der Substituent $R^3$ bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen Ringsystem mit 6 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden. In den Strukturen Ar-1 bis Ar-22 ist der Substituent $R^3$ bevorzugt bei jedem Auftreten gleich oder verschieden ausgewählt aus der Gruppe bestehend aus H, F, CN, einem aliphatischen Kohlenwasserstoffrest mit 1 bis 10 C-Atomen oder einem aromatischen Ringsystem mit 6 bis 30 aromatischen Ringatomen. In den Strukturen Ar-1 bis Ar-22 ist der Substituent $R^3$ bevorzugt bei jedem Auftreten gleich oder verschieden ausgewählt aus der Gruppe bestehend aus H oder einem aromatischen Ringsystem mit 6 bis 30 aromatischen Ringatomen, wie zuvor beschrieben, bevorzugt jedoch Dibenzofuran, Dibenzothiophen oder Spirobifluoren.

Zwei Substituenten R und $R^3$, R als Substituent am Carbazol und $R^3$ als Substituent an $Ar_3$, können ebenfalls zusammen ein aromatisches oder heteroaromatisches Ringsystem bilden, wobei sie entsprechend über einen Linker miteinander verbunden sind, beispielsweise über -O-, -S- oder $--C(R^0)_2-$, wobei $R^0$ eine zuvor angegebene oder bevorzugte Bedeutung hat, bevorzugt über -O- oder $-C(CH_3)_2-$.

In den Strukturen Ar-1 bis Ar-22 ist der Substituent $R^3$ besonders bevorzugt bei jedem Auftreten H.

In Verbindungen der Formeln (1a), (1b), (1c), (1d), (1e), (1f), (1h), (1i), (1j), (1k) oder (1l) oder bevorzugt beschriebenen Verbindungen der Formeln (1a), (1b), (1c), (1d), (1e), (1f), (1h), (1i), (1j), (1k) oder (1l) wird $Ar_3$ besonders bevorzugt aus den aromatischen oder heteroaromatischen Ringsystemen Ar-1, Ar-2, Ar-3, Ar-7, Ar-10, Ar-11, Ar-14, Ar-15, Ar-20, Ar-21 und Ar-22 ausgewählt, wobei die Substituenten $R^3$ und $Y^3$ eine zuvor genannte oder als bevorzugt beschriebene Bedeutung haben.

Beispiele für besonders geeignete Verbindungen, die erfindungsgemäß ausgewählt werden, sind Verbindungen der Formeln (1f), (1h) oder (1i), wobei L eine bevorzugt oder besonders bevorzugt angegebene Bedeutung hat.

Beispiele für besonders geeignete Verbindungen, die erfindungsgemäß ausgewählt werden, sind Verbindungen der Formel (1i), wobei L eine bevorzugt oder besonders bevorzugt angegebene Bedeutung hat.

Beispiele für geeignete Verbindungen der Formeln (1a), (1b), (1c), (1d), (1e), (1f), (1h), (1i), (1j), (1k) oder (1l), die erfindungsgemäß ausgewählt werden, sind die nachstehend genannten Strukturen der Tabellen 1, 2, 3 und 4.

Tabelle 1:

| | | |
|---|---|---|
| 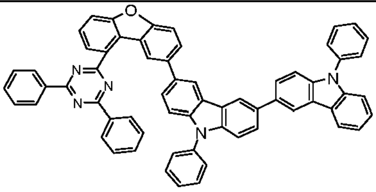 6 | 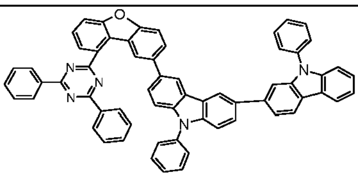 | 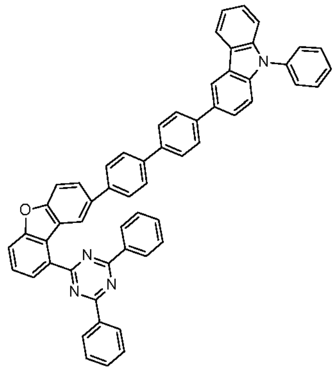 |
| 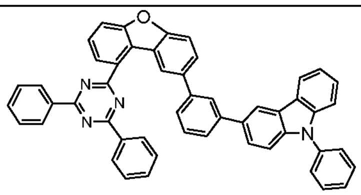 9 | 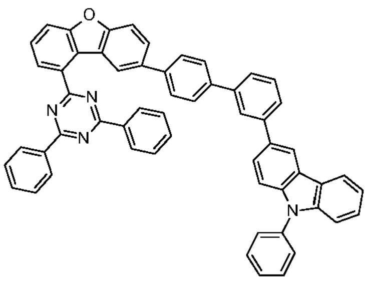 | 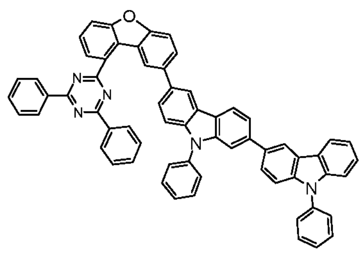 |
| | 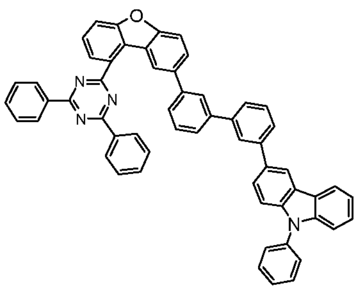 19 | 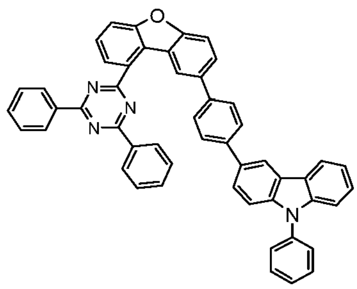 |

| | | |
|---|---|---|
| 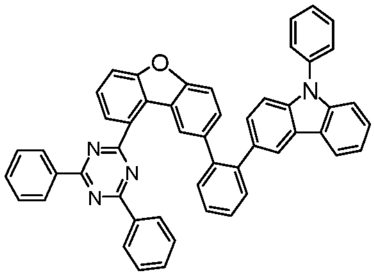 | 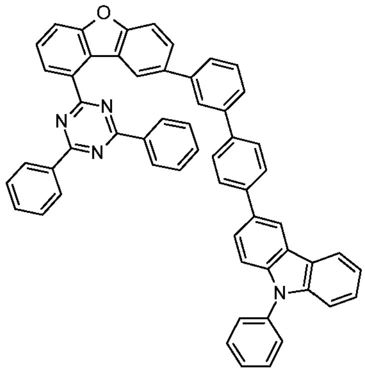 | 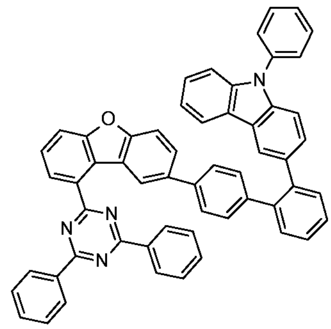 |
| 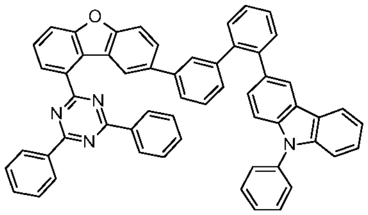 | 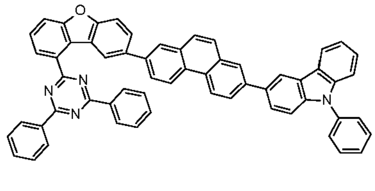 | 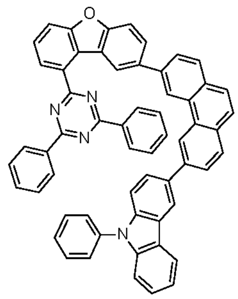 |
| 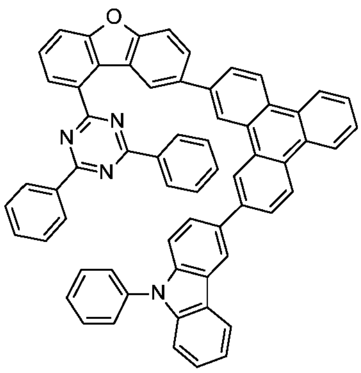 | 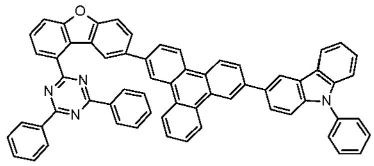 | 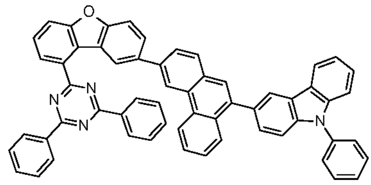 |
| 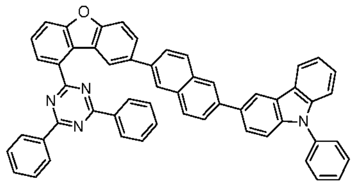 | 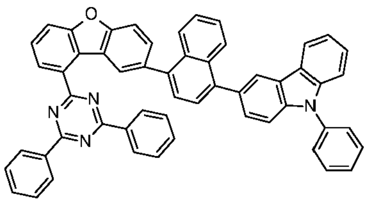 | 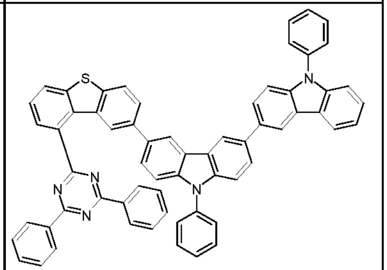 |
| 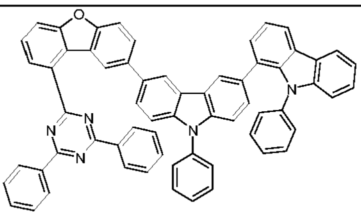 | 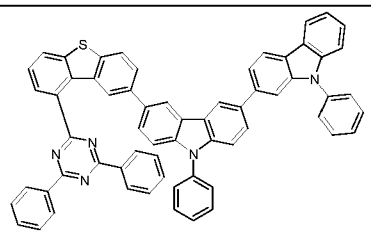 | 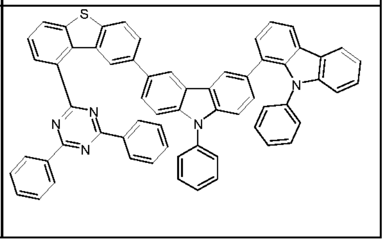 |

| | | |
|---|---|---|
| 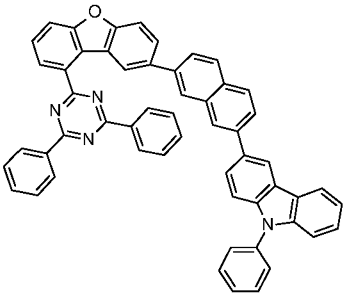 | 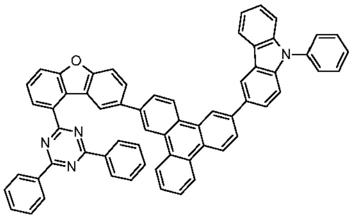 | 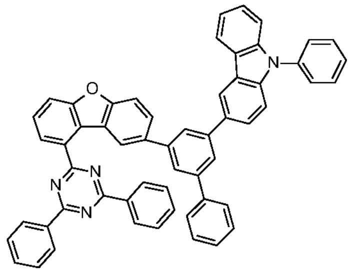 |
| 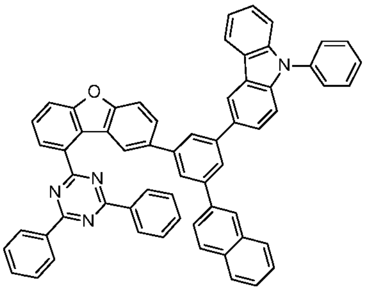 | 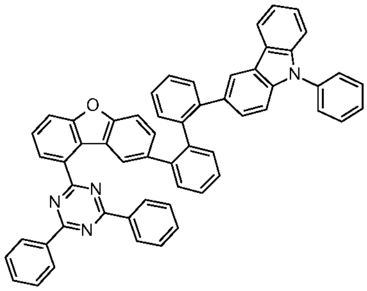 | 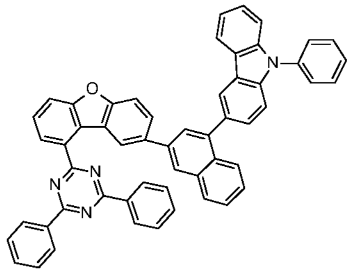 |
| 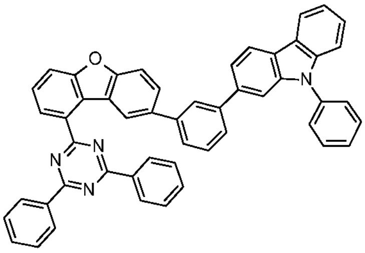 | 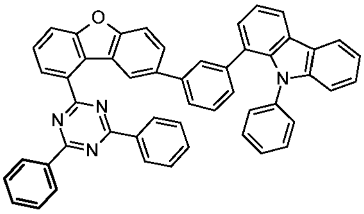 | 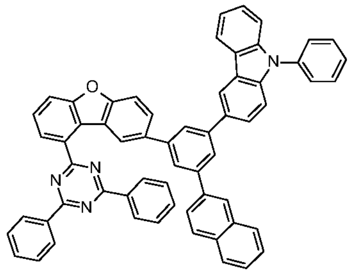 |
| 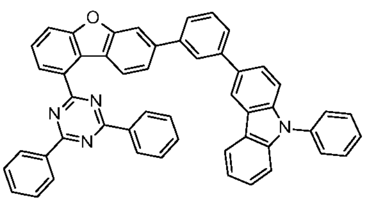 | 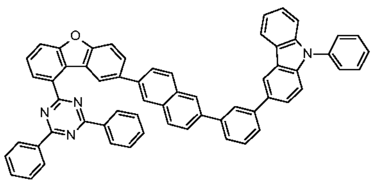 | |
| 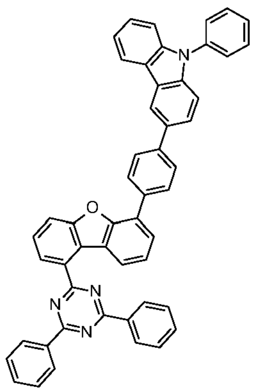 | 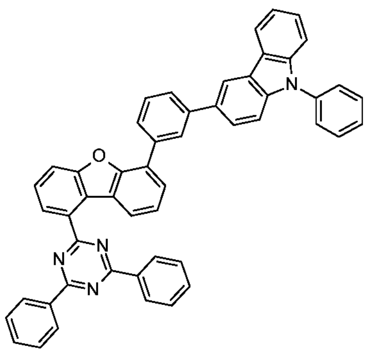 | 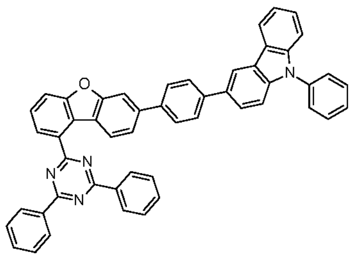 |

| | | |
|---|---|---|
| 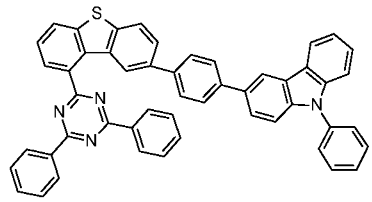 | 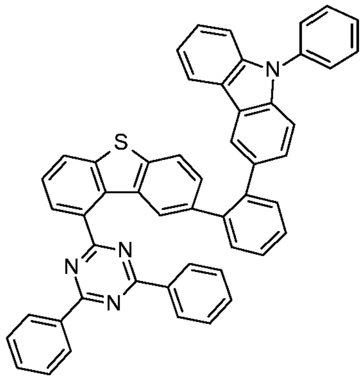 | 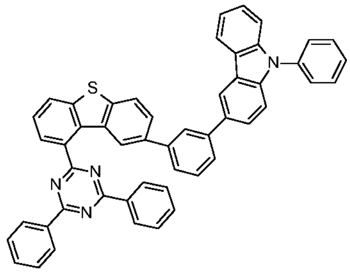 |

Tabelle 2:

| | | |
|---|---|---|
| 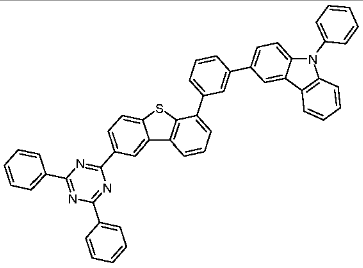 | 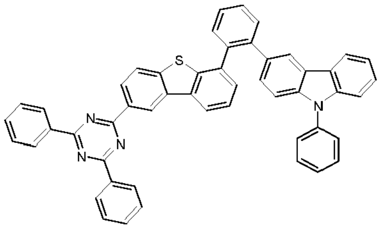 | 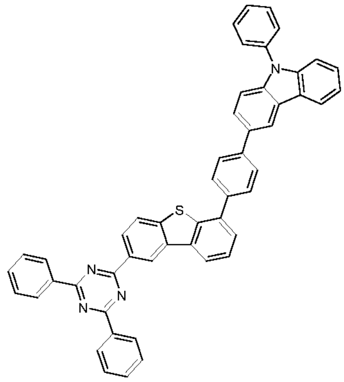 |
| 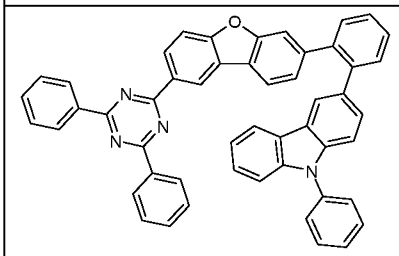 | 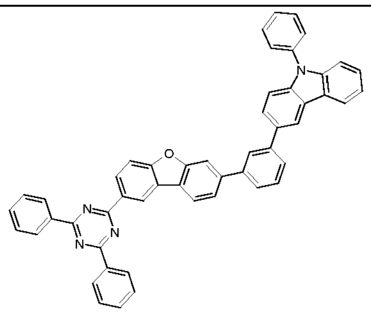 | 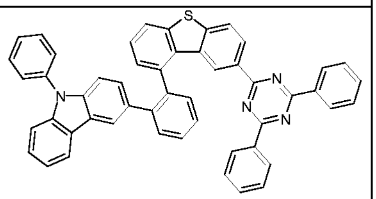 |
| 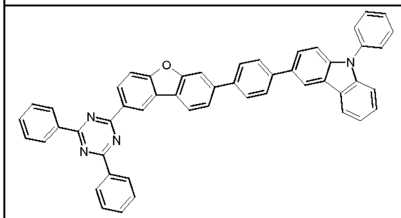 | 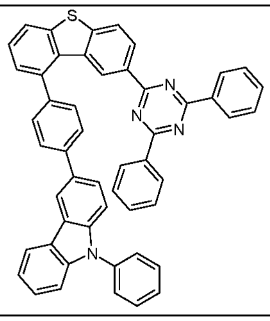 | 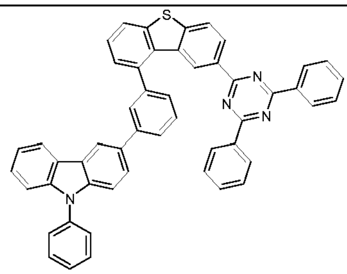 |

| | | |
|---|---|---|
| 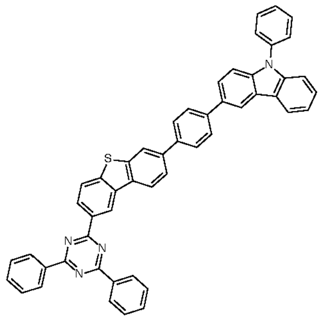 | 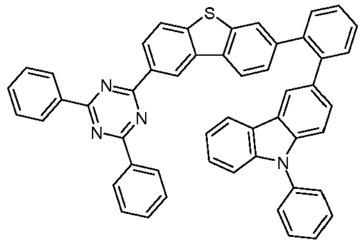 | 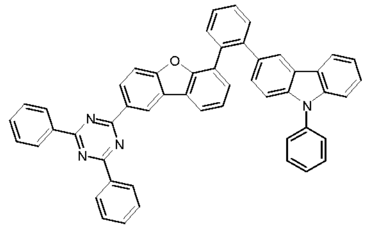 |
| 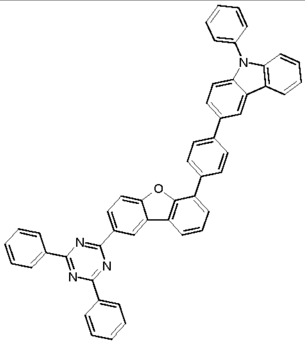 | 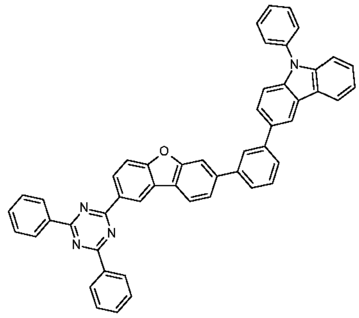 | 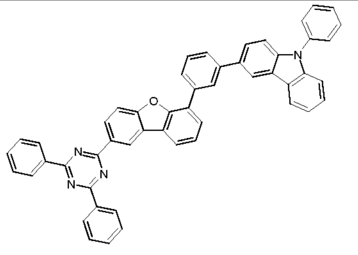 |
| 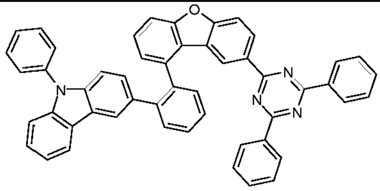 | 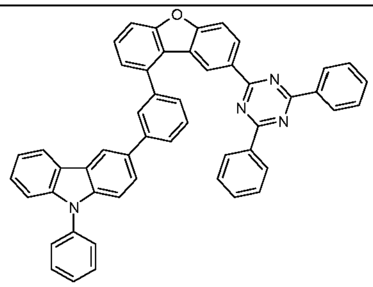 | 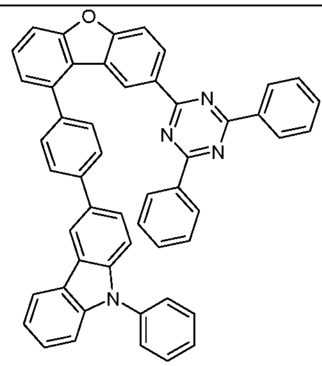 |
| 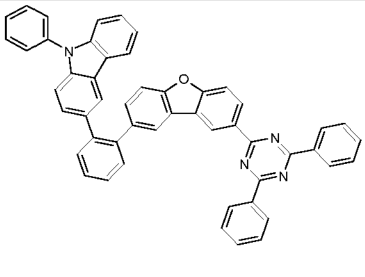 | 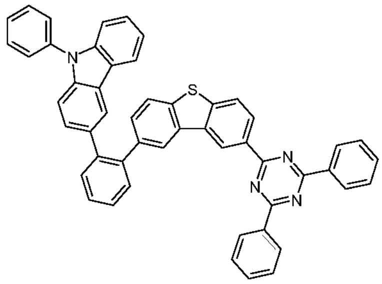 | 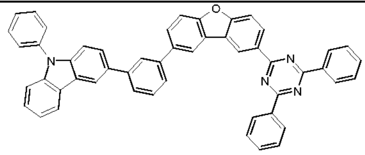 26 |
| 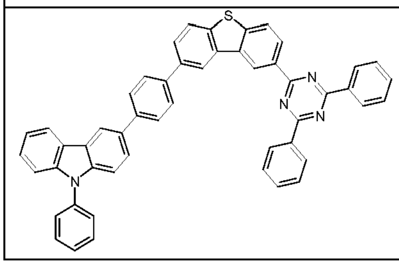 | 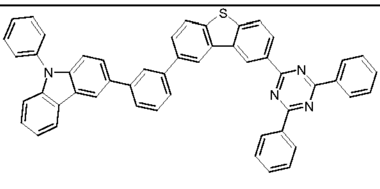 34 | 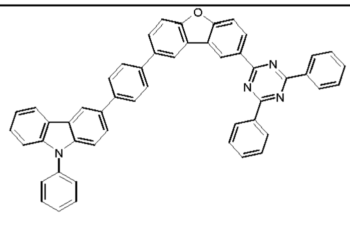 |

| | | |
|---|---|---|
| | 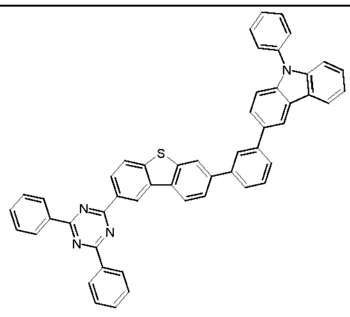 42 | |

Tabelle 3:

| | | |
|---|---|---|
| 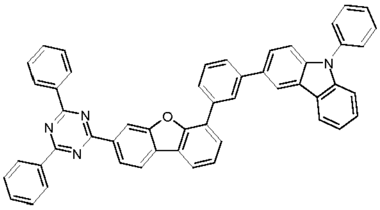 | 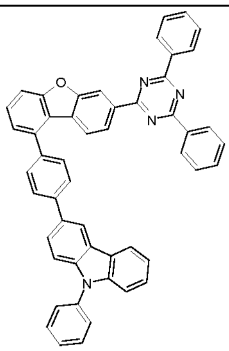 | 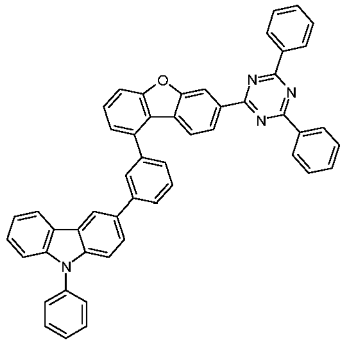 |
| 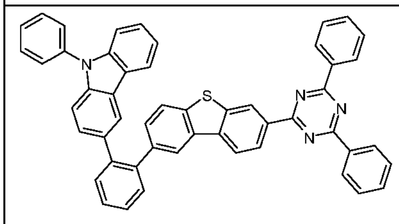 | 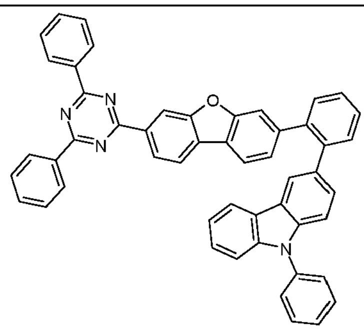 | 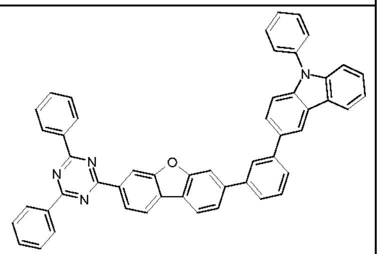 |
| 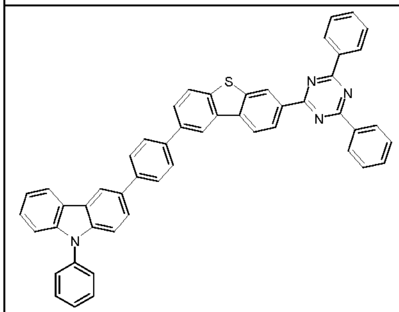 | 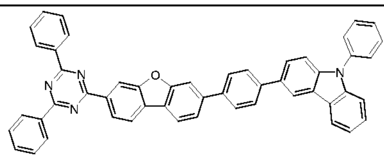 | 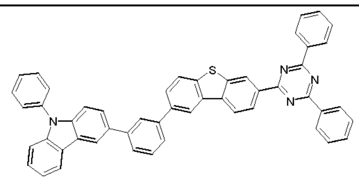 |
| 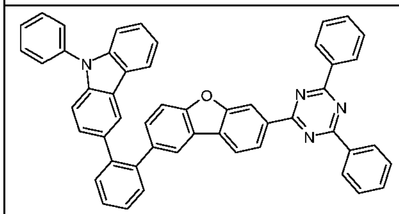 | 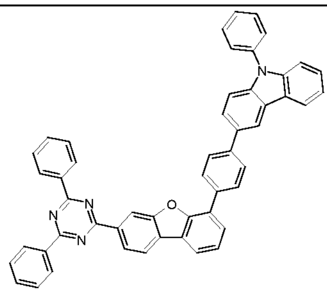 | 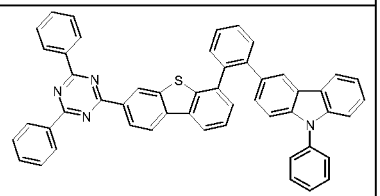 |

| | | |
|---|---|---|
| 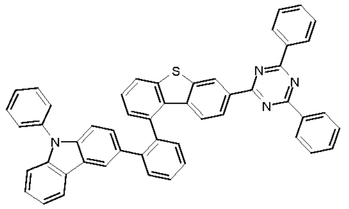 | 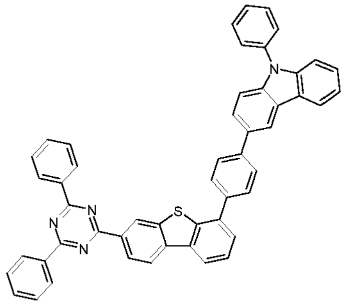 | 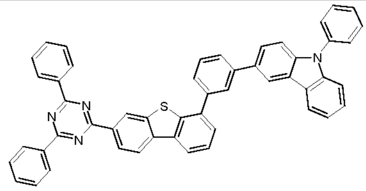 |
| 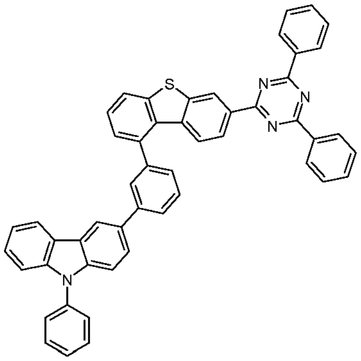 | 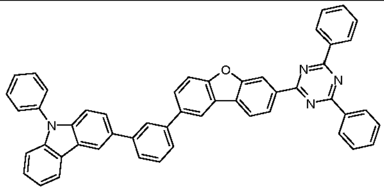<br>**48** | 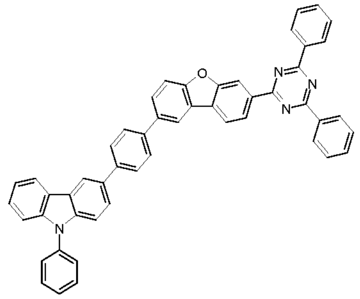 |
| 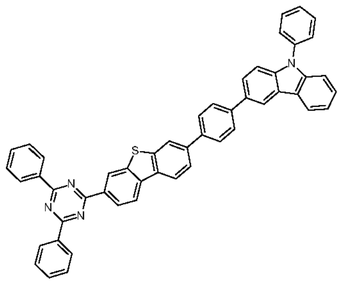 | 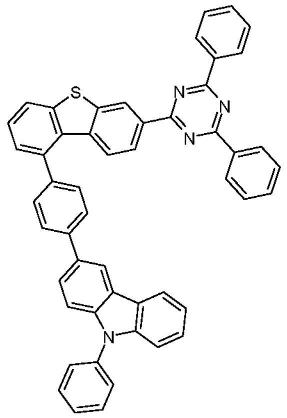 | 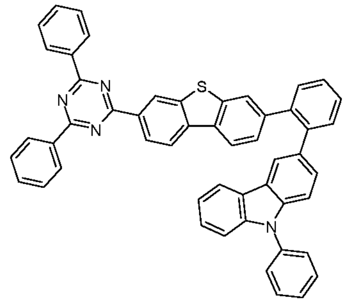 |
| 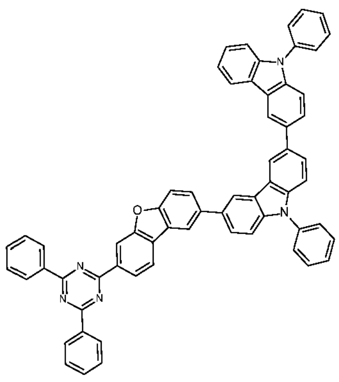<br>**59** | 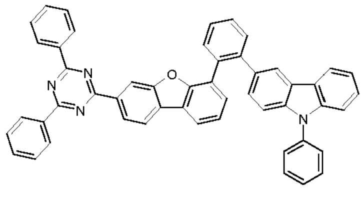 | 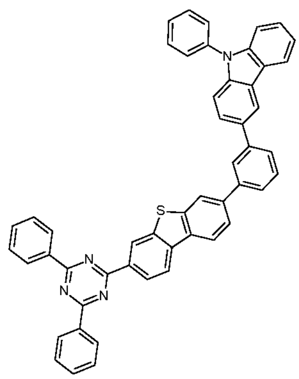<br>**50** |

Tabelle 4:

| | | |
|---|---|---|
| 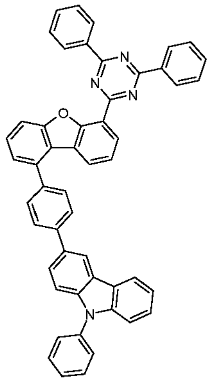 | 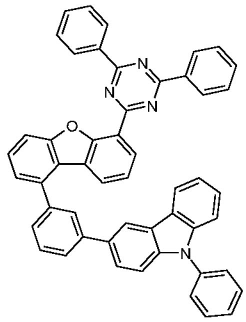 | 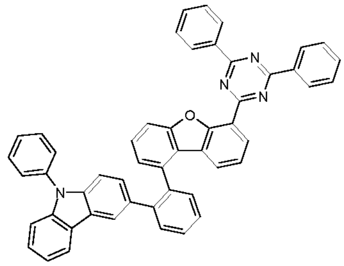 |
| 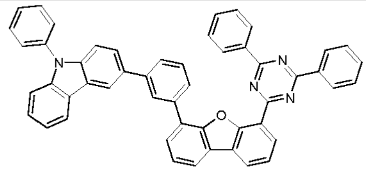 | 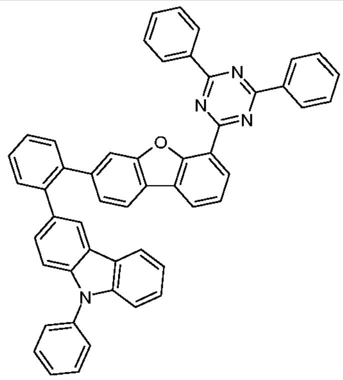 | 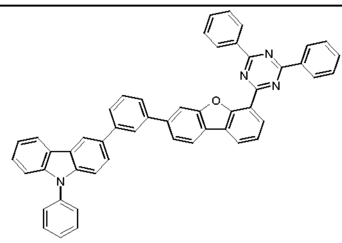 |
| 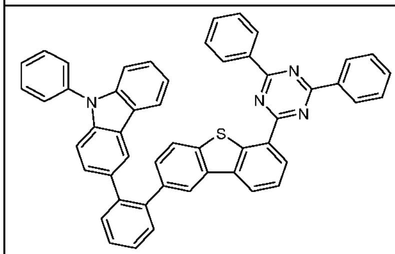 | 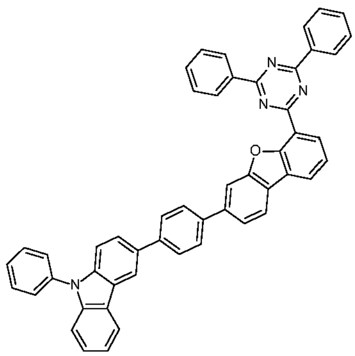 | 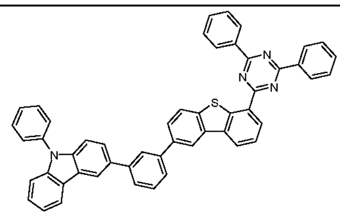 |
| 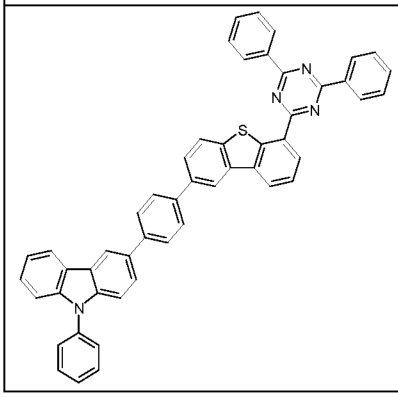 | 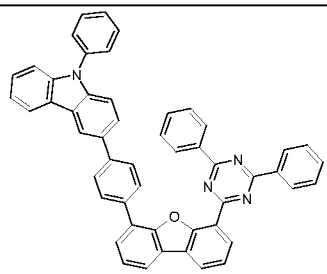 | 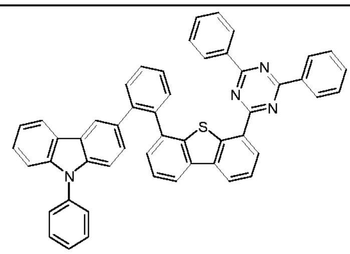 |

| | | |
|---|---|---|
| 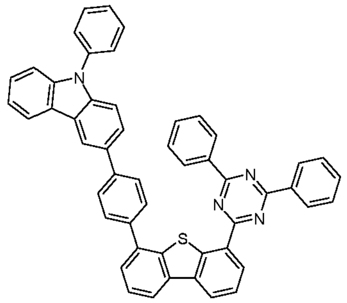 | 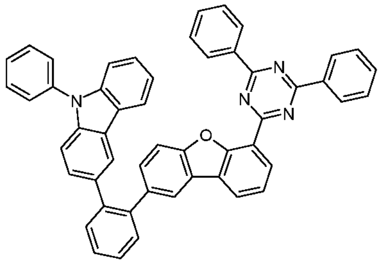 | 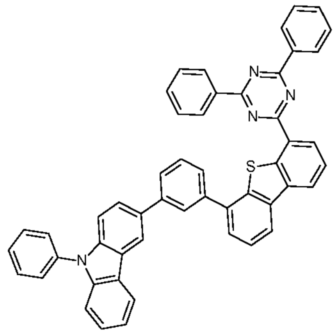 |
| 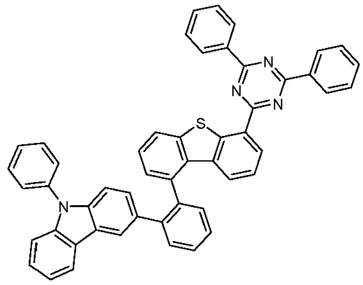 | 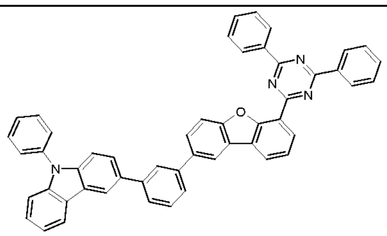 70 | 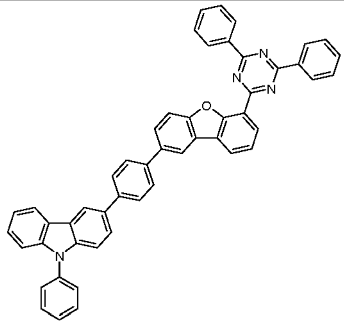 |
| 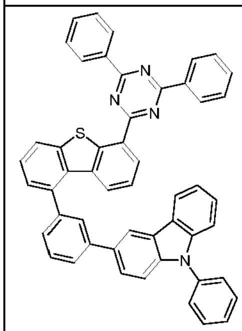 | 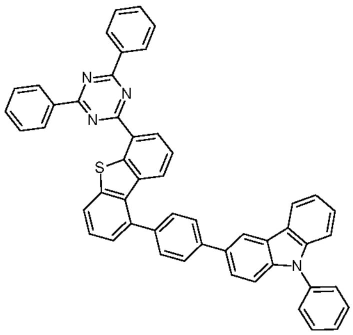 | 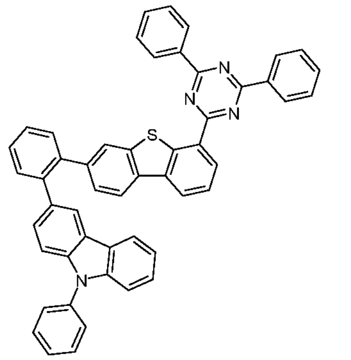 |
| | 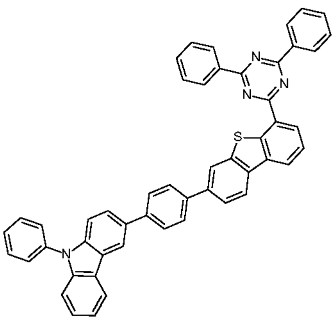 | 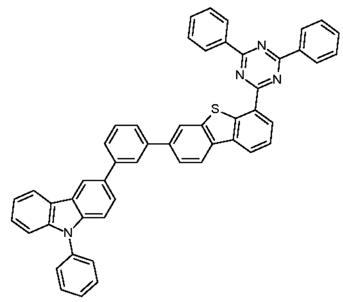 |

| | | |
|---|---|---|
| | 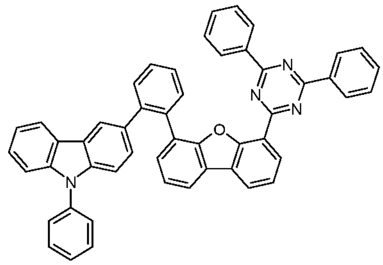 86 | 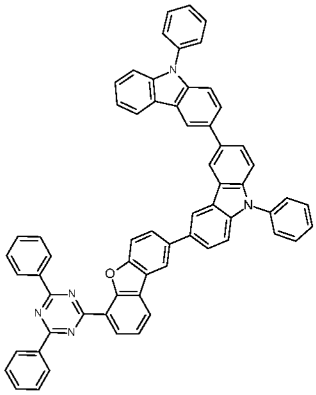 78 |

Besonders geeignete Verbindungen der Formeln (1b), (1e), (1f), (1h) oder (1i), die erfindungsgemäß ausgewählt werden, sind die Verbindungen der Tabelle 5. Ganz besonders geeignete Verbindungen für die erfindungsgemäße Zusammensetzung sind Verbindungen der Formel (1b) oder (1i), wobei L eine der bevorzugt genannten oder besonders bevorzugt genannten Bedeutungen hat.

Tabelle 5:

| | | |
|---|---|---|
| 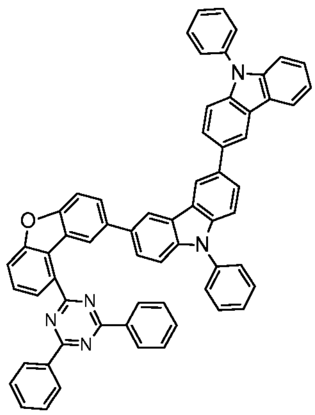 6 | 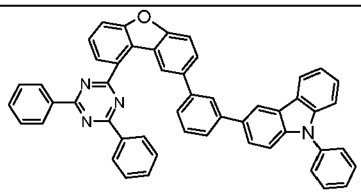 9 | 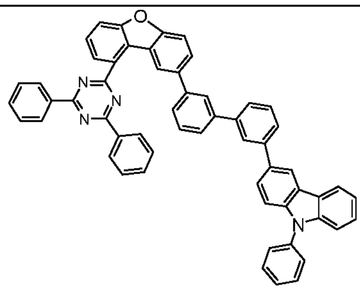 19 |

Besonders geeignete Verbindungen der Formeln (1a), (1e), (1f), (1h) oder (1j), die erfindungsgemäß ausgewählt werden, sind die Verbindungen der Tabelle 6.

Tabelle 6:

| | | |
|---|---|---|
| 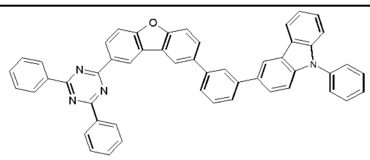 26 | 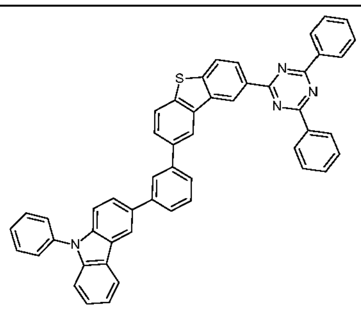 34 | 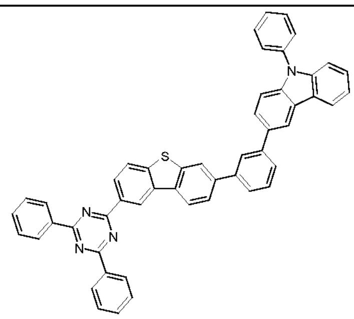 42 |

Besonders geeignete Verbindungen der Formeln (1d), (1e), (1f), (1h) oder (1k), die erfindungsgemäß ausgewählt werden, sind die Verbindungen der Tabelle 7.

Tabelle 7:

| | | |
|---|---|---|
| 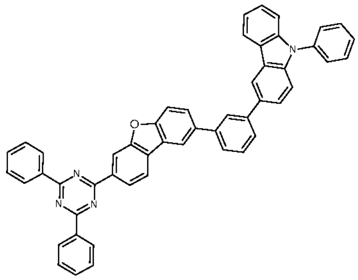 **48** | 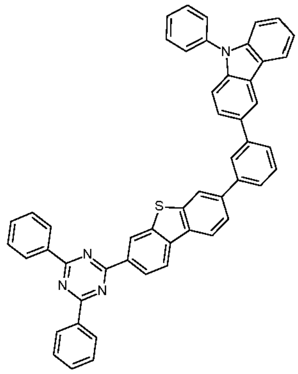 **50** | 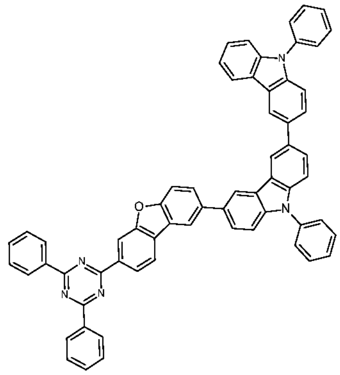 **59** |

Besonders geeignete Verbindungen der Formeln (1c), (1e), (1f), (1h) oder (1l), die erfindungsgemäß ausgewählt werden, sind die Verbindungen der Tabelle 8.

Tabelle 8:

| | | |
|---|---|---|
| 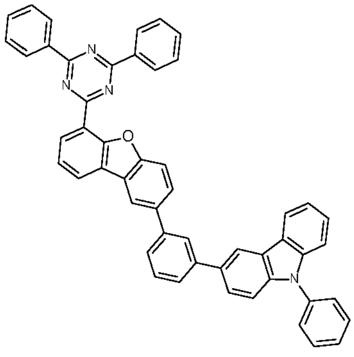 **70** | 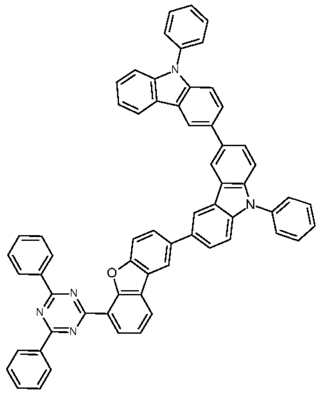 **78** | 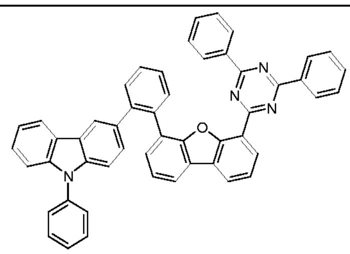 **86** |

Die Herstellung der Verbindungen der Formeln (1a) bis (1f) und (1h) bis (1l) sowie der Verbindungen **6, 9, 19, 26, 34, 42, 48, 50, 59, 70, 78** und **86** ist dem Fachmann bekannt. Die Verbindungen können nach dem Fachmann bekannten Syntheseschritten, wie z.B. Halogenierung, bevorzugt Bromierung, und einer sich anschließenden metallorganischen Kupplungsreaktion, z.B. Suzuki-Kupplung, Heck-Kupplung oder Hartwig-Buchwald-Kupplung, hergestellt werden. Die Herstellung der Verbindungen der Formeln (1a) bis (1f) und (1h) bis (1l) sowie der Verbindungen **6, 9, 19, 26, 34, 42, 48, 50, 59, 70, 78** und **86** ist insbesondere aus WO 2015/169412, insbesondere Seite 63 sowie der Synthesebeispiele der Seiten 77 bis 114, und WO 2011/057706, insbesondere die Synthesebeispiele der Seiten 92-94, bekannt.

Die Herstellung der Verbindungen der Formel (1l) kann nach folgendem Schema 1 erfolgen, wobei

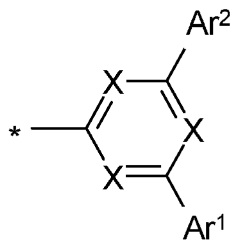

ein Triazin bedeutet und Y, $Ar_1$, $Ar_2$, $Ar_3$ eine der zuvor angegebenen Bedeutungen hat und R in Schema 1 eine Alkylgruppe mit 1 bis 4 C-Atomen bedeutet.

Schema 1:

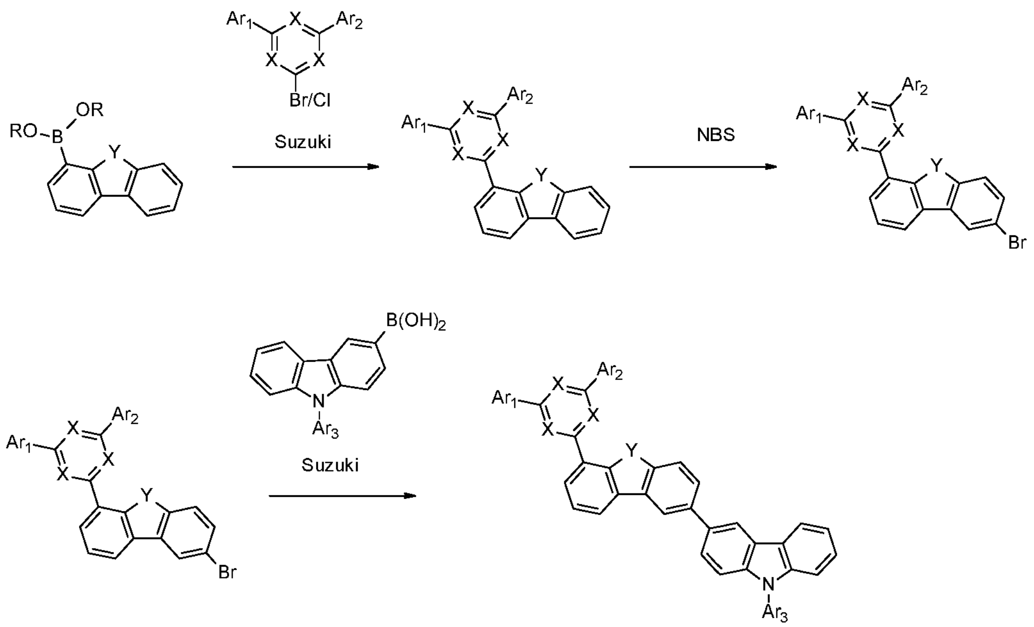


.

Die Herstellung der Verbindungen der Formel (1k) kann nach folgendem Schema 1 erfolgen, wobei

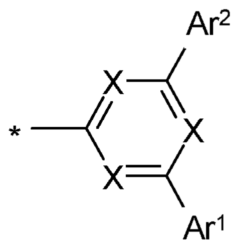

ein Triazin bedeutet und Y, $Ar_1$, $Ar_2$, $Ar_3$ eine der zuvor angegebenen Bedeutungen hat und R in Schema 2 eine Alkylgruppe mit 1 bis 4 C-Atomen bedeutet.

Schema 2:

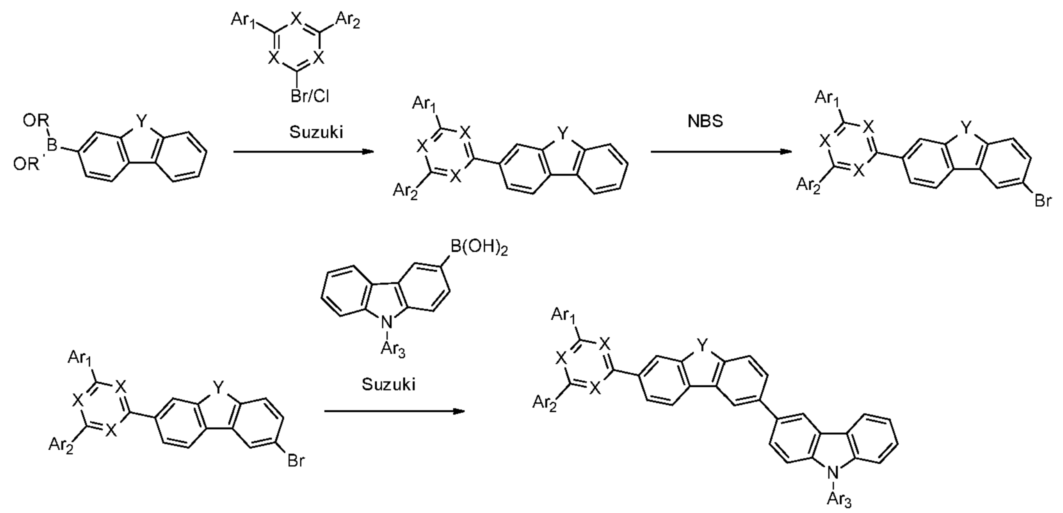


Die Herstellung der Verbindungen der Formel (1j) kann nach folgendem Schema 3 erfolgen, wobei

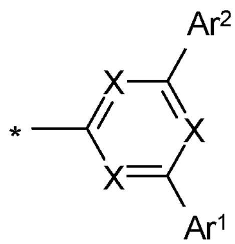

ein Triazin bedeutet und Y, $Ar_1$, $Ar_2$, $Ar_3$ eine der zuvor angegebenen Bedeutungen hat und R in Schema 3 eine Alkylgruppe mit 1 bis 4 C-Atomen bedeutet.

Schema 3:

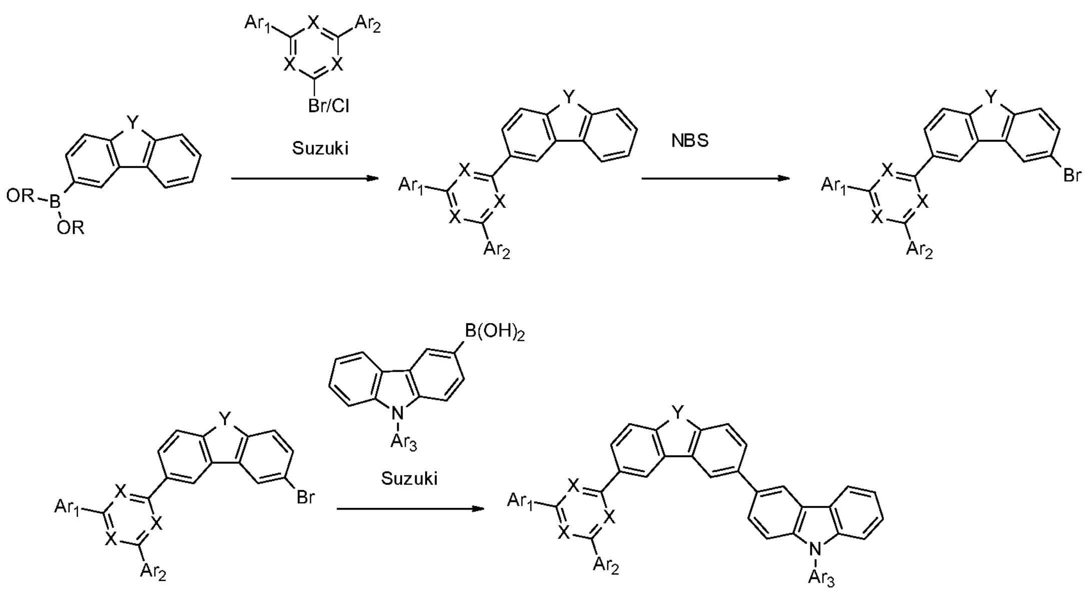


Die Herstellung der Verbindungen der Formel (1i) kann nach folgendem Schema 4 erfolgen, wobei

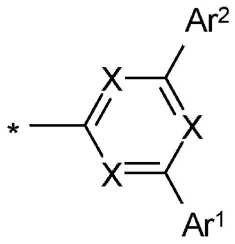

ein Triazin bedeutet und Y, $Ar_1$, $Ar_2$, $Ar_3$ eine der zuvor angegebenen Bedeutungen hat und R in Schema 4 eine Alkylgruppe mit 1 bis 4 C-Atomen bedeutet. Die Herstellung der Verbindungen der Formeln (1b) oder (1i) kann ebenfalls nach folgendem Schema 5 erfolgen, wobei

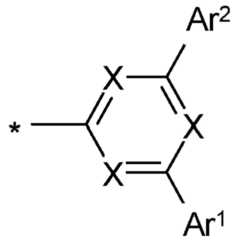

ein Triazin bedeutet und Y, $Ar_1$, $Ar_2$, $Ar_3$ eine der zuvor angegebenen Bedeutungen hat.

Schema 4:

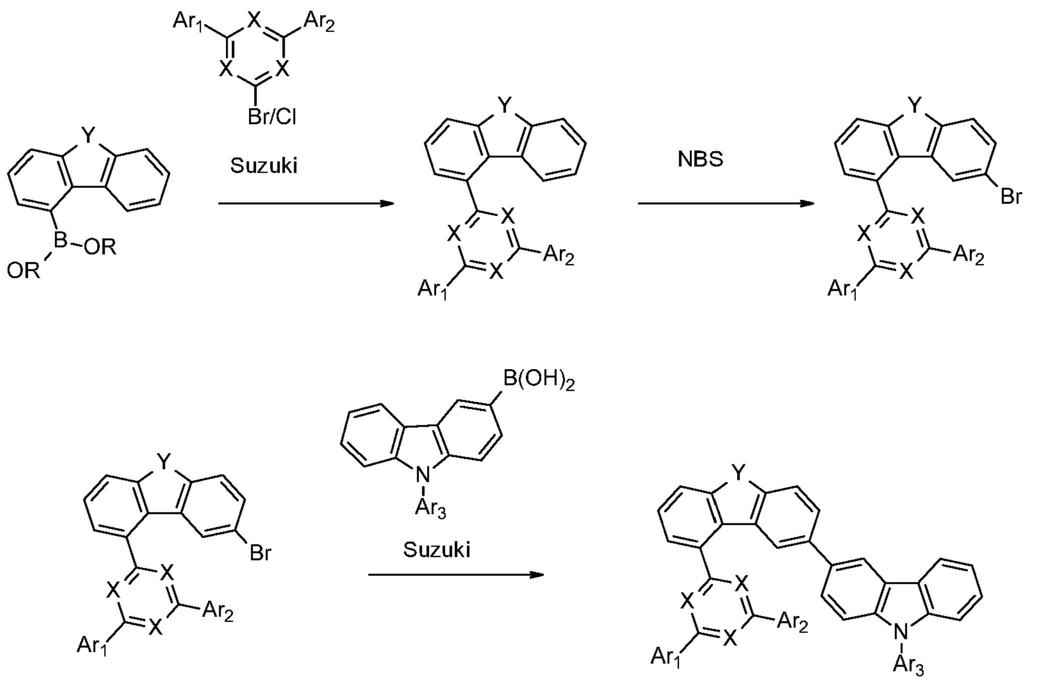

Schema 5

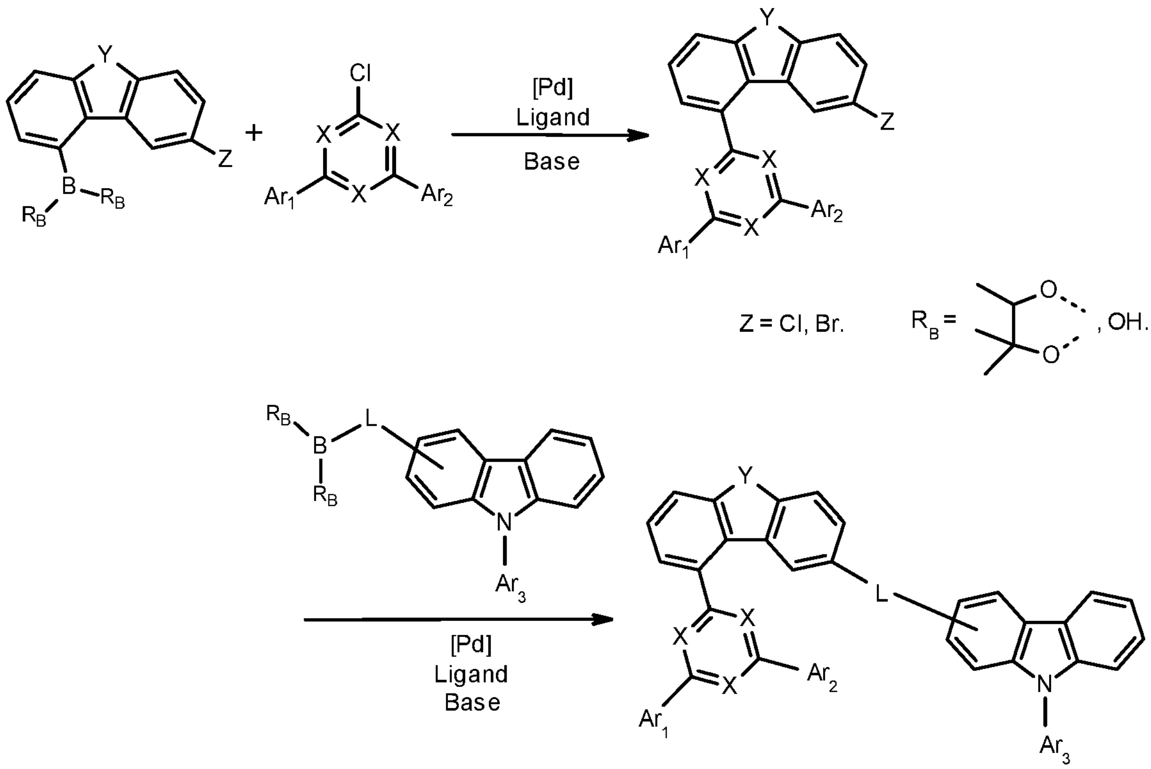


Lochtransportierende Hosts der Formel (2a):
In einer Ausführungsform der Erfindung werden Verbindungen der Formel (2a) ausgewählt, wie zuvor beschrieben, die mit Verbindungen der Formeln (1a), (1b), (1c), (1d), (1e), (1f), (1h), (1i), (1j), (1k) und (1l), wie zuvor beschrieben oder bevorzugt beschrieben, oder mit den Verbindungen **6, 9, 19, 26, 34, 42, 48, 50, 59, 70, 78** und **86** in der Zusammensetzung verwendet werden.

Verbindungen der Formel (2a) sind wie folgt dargestellt,

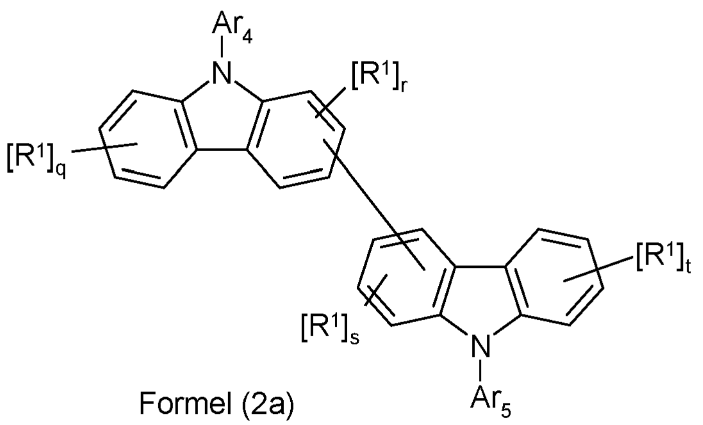

Formel (2a)

,

wobei $R^1$, $Ar_4$ und $Ar_5$ eine zuvor genannte Bedeutung haben oder eine nachfolgend beschriebene bevorzugte Bedeutung haben und q und t jeweils unabhängig voneinander 0, 1, 2, 3 oder 4 bedeuten und r und s jeweils unabhängig voneinander 0, 1, 2 oder 3 bedeuten.

In einer bevorzugten Ausführungsform der Verbindungen der Formel (2a) sind die beiden Carbazole jeweils in 3-Position miteinander verknüpft. Diese Ausführungsform wird durch die Verbindungen der Formel (2b) dargestellt,

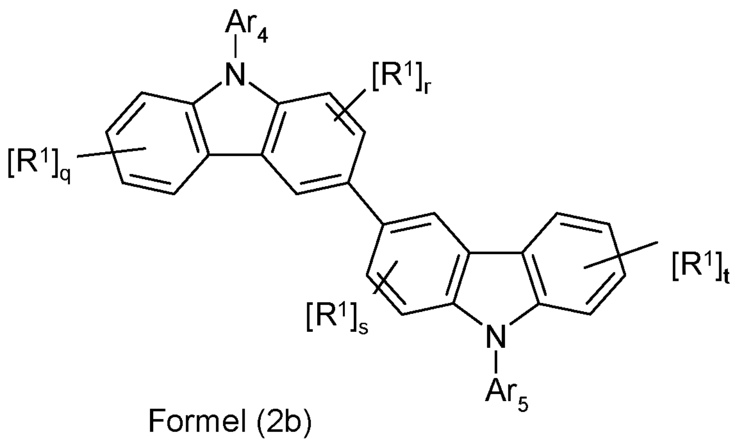

Formel (2b)

,

wobei $R^1$, $Ar_4$ und $Ar_5$ eine zuvor genannte Bedeutung haben oder eine nachfolgend beschriebene bevorzugte Bedeutung haben und q und t jeweils unabhängig voneinander 0, 1, 2, 3 oder 4 bedeuten und r und s jeweils unabhängig voneinander 0, 1, 2 oder 3 bedeuten.

In Verbindungen der Formeln (2a) oder (2b) ist q bevorzugt 0, 1 oder 2, wobei $R^1$ eine zuvor angegebene Bedeutung hat. Besonders bevorzugt ist q 0 oder 1. Ganz besonders bevorzugt ist q 0.

Wenn in Verbindungen der Formeln (2a) oder (2b) q größer 0 ist, so ist der Substituent $R^1$ bei jedem Auftreten gleich oder verschieden Phenyl oder Biphenyl. Die bevorzugte Position des/der Substituenten $[R^1]_q$ ist Position 1, 2, 3 oder 4 bzw. die Kombinationen der Positionen 1 und 4 und 1 und 3, besonders bevorzugt 1 und 3, 2 oder 3, ganz besonders bevorzugt 3, wobei $R^1$ eine der zuvor angegebenen bevorzugten Bedeutungen hat und q größer 0 ist.

In Verbindungen der Formeln (2a) oder (2b) ist r 0.

In Verbindungen der Formeln (2a) oder (2b) ist s 0.

In Verbindungen der Formeln (2a) oder (2b) ist t bevorzugt 0, 1 oder 2, wobei $R^1$ eine zuvor angegebene Bedeutung hat. Besonders bevorzugt ist t 0 oder 1. Ganz besonders bevorzugt ist t 0.

Wenn in Verbindungen der Formeln (2a) oder (2b) t größer 0 ist, so ist der Substituent $R^1$ bei jedem Auftreten gleich oder verschieden Phenyl, Biphenyl oder Terphenyl. Die bevorzugte Position des/der Substituenten $[R^1]_q$ ist Position 1, 2, 3 oder 4 bzw. die Kombinationen der Positionen 1 und 4, 1 und 3, 1 und 2 und 3 und 4, besonders bevorzugt 1 und 3, 2 oder 3, ganz besonders bevorzugt 2 oder 3, wobei $R^1$ eine der zuvor angegebenen bevorzugten Bedeutungen hat und t größer 0 ist.

Der Substituent $R^2$ ist bei jedem Auftreten gleich oder verschieden bevorzugt ausgewählt aus der Gruppe bestehend aus D, F, Cl, Br, I, CN, $NO_2$, $N(Ar)_2$, $NH_2$, $N(R^3)_2$, C(=O)Ar, C(=O)H, C(=O)$R^3$, P(=O)$(Ar)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, einem aromatischen oder heteroaromatischen Ringsystem mit 5 bis 60 aromatischen Ringatomen, das jeweils mit einem oder mehreren Resten $R^3$ substituiert sein kann, einer Aryloxy- oder Heteroaryloxygruppe mit 5 bis 60 aromatischen Ringatomen, die mit einem oder mehreren Resten $R^3$ substituiert sein kann. Der Substituent $R^2$ ist bei seinem Auftreten besonders bevorzugt ein aromatisches oder heteroaromatisches Ringsystem, wie zuvor beschrieben, bevorzugt ausgewählt aus der Gruppe Carbazol, 9-Phenyl-carbazol, Dibenzofuran, Dibenzothiophen, Fluoren, Terphenyl oder Spirobifluoren abgeleitet, ganz besonders bevorzugt von einem Dibenzofuran abgeleitet.

Im Fall der Substitution eines der Substituenten $R^2$, wie zuvor beschrieben, mit einem Substituenten $R^3$, gelten die Bedeutungen von $R^3$ wie zuvor beschrieben bzw. bevorzugt beschrieben.

In Verbindungen der Formeln (2a) oder (2b), wie zuvor beschrieben, sind $Ar_4$ und $Ar_5$ jeweils unabhängig voneinander ein aromatisches Ringsystem mit 6 bis 40 aromatischen Ringatomen oder ein heteroaromatisches Ringsystem mit 10 bis 40 aromatischen Ringatomen, das mit einem oder mehreren Resten $R^3$ substituiert sein kann, wobei das heteroaromatische Ringsystem, das mit einem oder mehreren $R^3$ substituiert sein kann, in der Gesamtheit nur ein N-Atom enthält oder in der Gesamtheit ein oder mehrere O- und/oder S-Atome enthält, mit der Maßgabe, dass $Ar_4$ und $Ar_5$ nicht gleichzeitig Phenyl sind. Durch die angegebene Bedingung unterscheidet sich die erfindungsgemäße Zusammensetzung von der Zusammensetzung der WO 2015/169412.

Bei den heteroaromatischen Ringsystemen mit 10 bis 40 aromatischen Ringatomen, das mit einem oder mehreren der Substituenten $R^3$ substituiert sei kann, sind elektronenreiche Ringsysteme besonders bevorzugt, wobei das durch $R^3$ substituierte Ringsystem in der Gesamtheit nur ein N-Atom enthält oder das gegebenenfalls durch $R^3$ substituierte Ringsystem in der Gesamtheit ein oder mehrere O- und/oder S-Atome enthält.

In Verbindungen der Formeln (2a) oder (2b) oder bevorzugt beschriebenen Verbindungen der Formeln (2a) oder (2b), werden $Ar_4$ und $Ar_5$ bevorzugt aus den aromatischen oder heteroaromatischen Ringsystemen Ar-1 bis Ar-22 ausgewählt, wie zuvor beschrieben, wobei auch die Ausführungen hinsichtlich der Gruppen $R^{\#}$, $Y^3$ und $R^3$ gelten, mit der Maßgabe, dass $Ar_4$ und $Ar_5$ nicht gleichzeitig Phenyl sind und mit der Bedingung, dass ein gegebenenfalls durch $R^3$ substituiertes heteroaromatisches Ringsystem repräsentiert durch Ar-12, Ar-13, Ar-14, Ar-15, Ar-20 und Ar-21 in der Gesamtheit nur ein N-Atom enthält.

In einer bevorzugten Ausführungsform der Erfindung werden Verbindungen der Formeln (2a) oder (2b) ausgewählt, in denen einer der Substituenten $Ar_4$ oder $Ar_5$ ein aromatisches Ringsystem mit 6 bis 40 aromatischen Ringatomen oder ein heteroaromatisches Ringsystem mit 10 bis 40 aromatischen Ringatomen, das mit einem oder mehreren Resten $R^3$ substituiert sein kann, bedeutet, wobei das heteroaromatische Ringsystem, das mit einem oder mehreren $R^3$ substituiert sein kann, in der Gesamtheit nur ein N-Atom enthält oder in der Gesamtheit ein oder mehrere O- und/oder S-Atome enthält, und der andere Substituent ein aromatisches Ringsystem mit 6 bis 40 aromatischen Ringatomen, das mit einem oder mehreren Resten $R^3$ substituiert sein kann, bedeutet, mit der Maßgabe, dass $Ar_4$ und $Ar_5$ nicht gleichzeitig Phenyl sind.

In dieser Ausführungsform ist es bevorzugt, wenn ein Substituent $Ar_4$ oder $Ar_5$ einer der Strukturen Ar-1 bis Ar-22 entspricht, wie zuvor beschrieben oder als bevorzugt beschrieben, und der andere Substituent einer der Strukturen Ar-1 bis Ar-11 oder Ar-16 bis Ar-19 oder Ar-22 entspricht, mit der Maßgabe, dass $Ar_4$ und $Ar_5$ nicht gleichzeitig Phenyl sind und bevorzugt mit der Bedingung, dass ein gegebenenfalls durch $R^3$ substituiertes heteroaromatisches Ringsystem repräsentiert durch Ar-12, Ar-13, Ar-14, Ar-15, Ar-20 und Ar-21 in der Gesamtheit nur ein N-Atom enthält.

In einer besonders bevorzugten Ausführungsform der Erfindung werden Verbindungen der Formeln (2a) oder (2b) ausgewählt, in denen die Substituenten $Ar_4$ oder $Ar_5$ jeweils unabhängig voneinander ein aromatisches Ringsystem mit 6 bis 40 aromatischen Ringatomen, das mit einem oder mehreren Resten $R^3$ substituiert sein kann, bedeuten, mit der Maßgabe, dass $Ar_4$ und $Ar_5$ nicht gleichzeitig Phenyl sind.

Die Substituenten $R^3$ sind bei ihrem Auftreten in dieser Ausführungsform, wenn $Ar_4$ und $Ar_5$ ein aromatisches Ringsystem mit 6 bis 40 Ringatomen bedeuten, bevorzugt aromatisch und enthalten kein Heteroatom.

Ein weiterer Gegenstand der Erfindung ist demzufolge eine Zusammensetzung, wie zuvor beschrieben oder als bevorzugt beschrieben, wobei in Verbindungen der Formeln (2a) oder (2b) die Substituenten $Ar_4$ oder $Ar_5$ jeweils unabhängig voneinander ein aromatisches Ringsystem mit 6 bis 40 aromatischen Ringatomen, das mit einem oder mehreren Resten $R^3$ substituiert sein kann, bedeuten, mit der Maßgabe, dass $Ar_4$ und $Ar_5$ nicht gleichzeitig Phenyl sind.

In dieser Ausführungsform ist es bevorzugt, wenn beide Substituenten $Ar_4$ und $Ar_5$ jeweils unabhängig voneinander einer der Strukturen Ar-1 bis Ar-11 oder Ar-16 bis Ar-19 oder Ar-22 entspricht, wie zuvor beschrieben oder als bevorzugt beschrieben, mit der Maßgabe, dass $Ar_4$ und $Ar_5$ nicht gleichzeitig Phenyl sind und bevorzugt mit der Bedingung, dass der Substituent $R^3$ in einem gegebenenfalls durch $R^3$ substituierten aromatischen Ringsystem so gewählt wird, dass er kein Heteroatom enthält.

Beispiele für geeignete Verbindungen der Formeln (2a) oder (2b), die erfindungsgemäß ausgewählt werden, sind die nachstehend genannten Strukturen der Tabelle 9.

Tabelle 9:

| **Struktur** | **CAS-Nummer** | **Struktur** | **CAS-Nummer** |
|---|---|---|---|
| 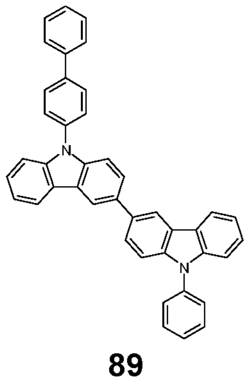 **89** | CAS-1454567-05-5 | 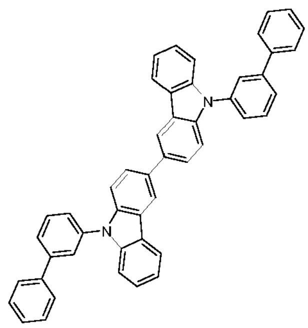 **90** | CAS-1352040-89-1 |

(fortgesetzt)

| Struktur | CAS-Nummer | Struktur | CAS-Nummer |
|---|---|---|---|
| 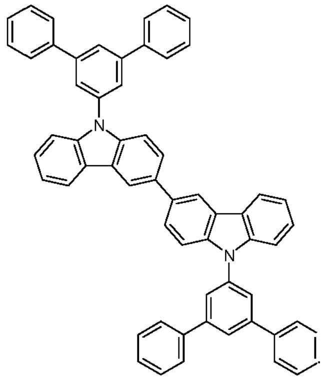 | CAS-1336889-25-8 | 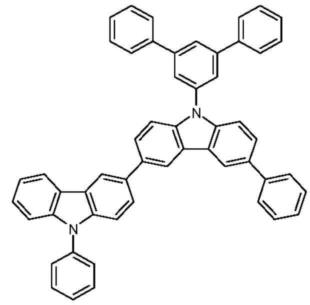 | CAS-1800544-05-1 |
| 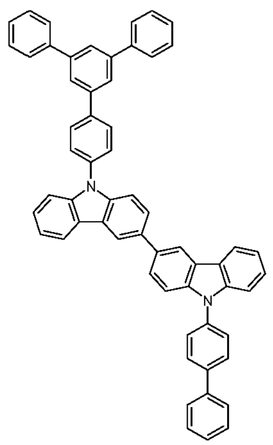 | CAS-1800544-08-4 | 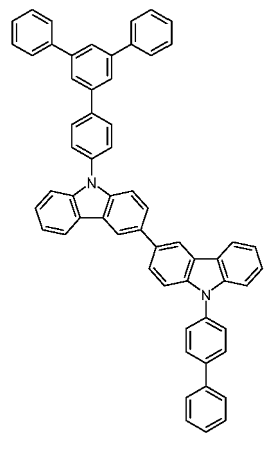 | CAS-1800544-08-4 |
| 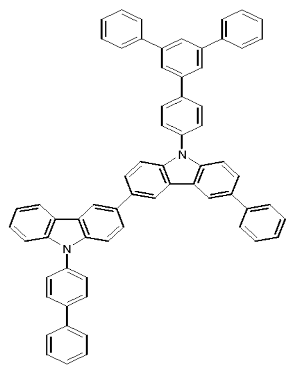 | CAS-1800544-09-5 | 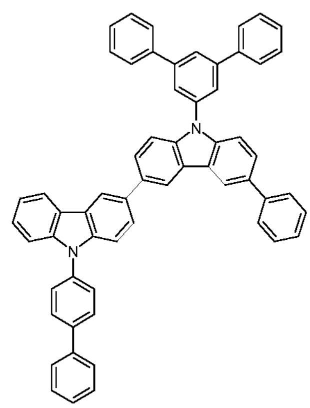 | CAS-1800544-10-8 |
| 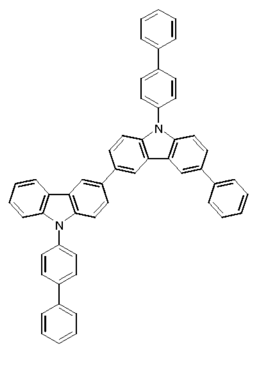 | CAS-1800544-11-9 | 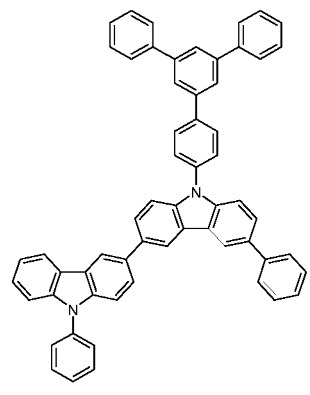 | CAS-1800544-04-0 |

(fortgesetzt)

| Struktur | CAS-Nummer | Struktur | CAS-Nummer |
|---|---|---|---|
| 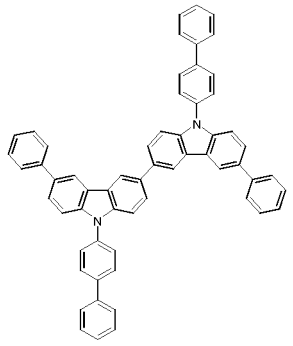 | CAS-1842320-52-8 | 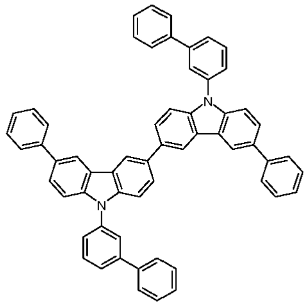 | CAS-1842320-53-9 |
| 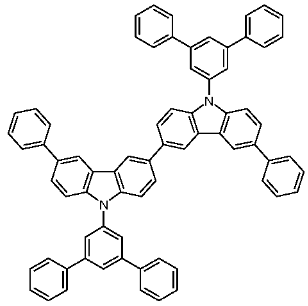 | CAS-1842320-54-0 | 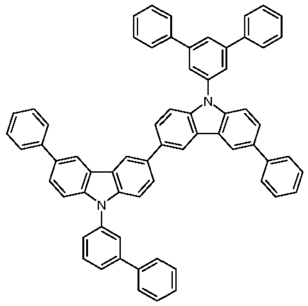 | CAS-1842320-55-1 |
| 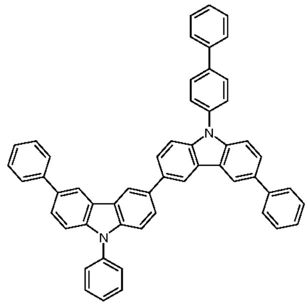 | CAS-1842320-56-2 | 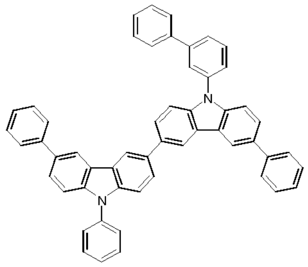 | CAS-1842320-57-3 |
| 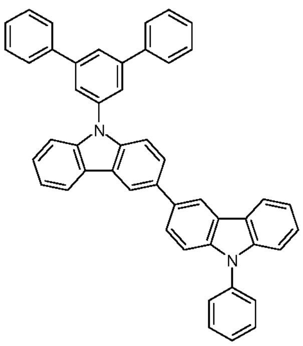 | CAS-1410876-33-3 | 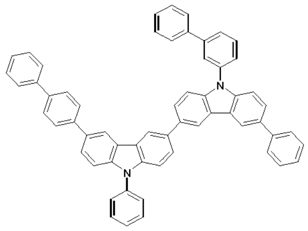 | CAS-1842320-58-4 |

(fortgesetzt)

| Struktur | CAS-Nummer | Struktur | CAS-Nummer |
|---|---|---|---|
| 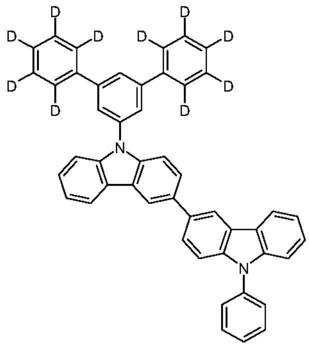 | CAS-1410876-47-9 | 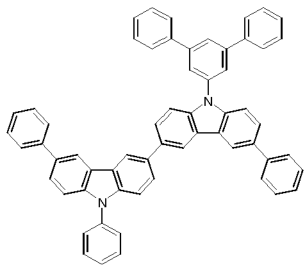 | CAS-1842320-59-5 |
| 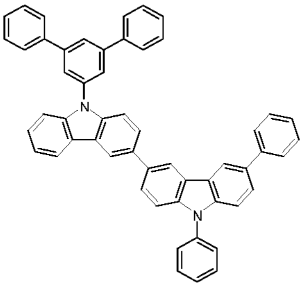 | CAS-1848256-38-1 | 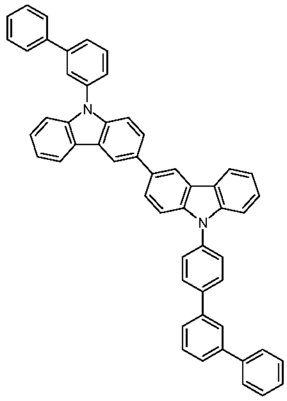 | CAS-1865661-14-8 |
| 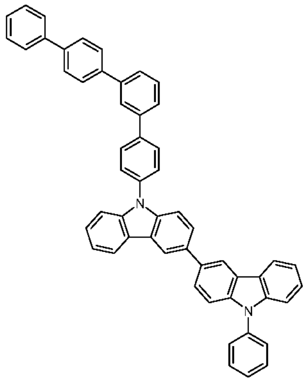 | CAS-1870867-25-6 | 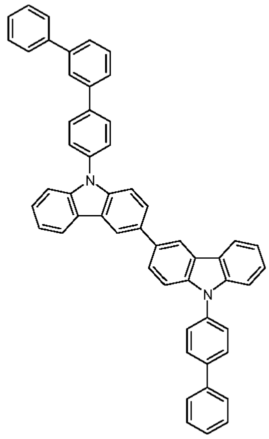 | CAS-1884707-32-7 |
| 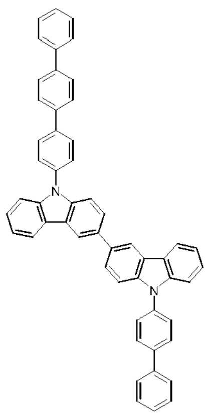 | CAS-1889262-88-7 | 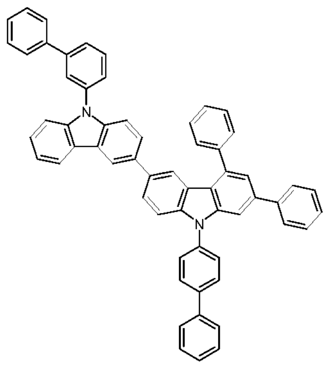 | CAS-2018307-89-4 |

(fortgesetzt)

| Struktur | CAS-Nummer | Struktur | CAS-Nummer |
|---|---|---|---|
| 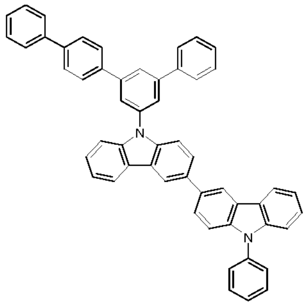 | CAS-1454655-29-8 | 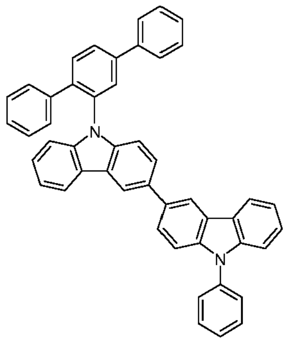 | CAS-1454655-33-4 |
| 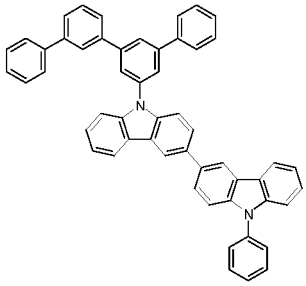 | CAS-1454660-22-0 | 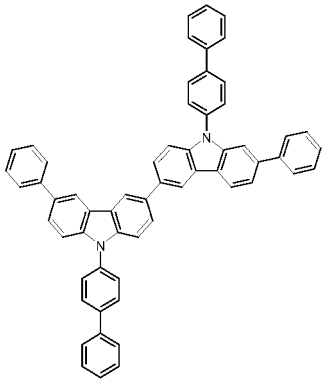 | CAS-1907663-27-7 |
| 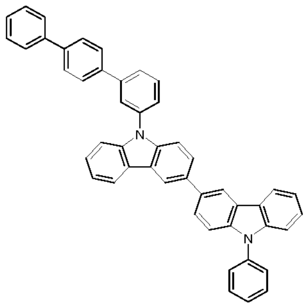 | CAS-1548581-24-3 | 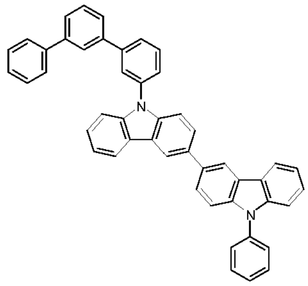 | CAS-1548581-27-6 |
| 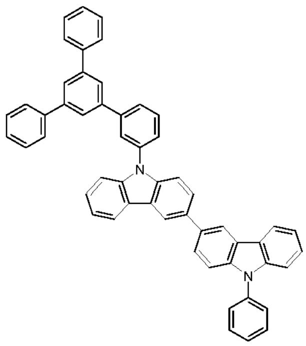 | CAS-1548581-29-8 | 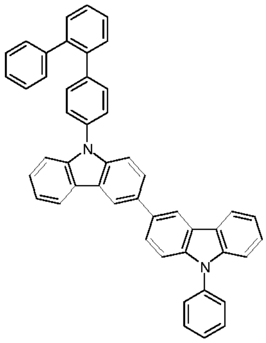 | CAS-1548581-37-8 |

(fortgesetzt)

| Struktur | CAS-Nummer | Struktur | CAS-Nummer |
|---|---|---|---|
| 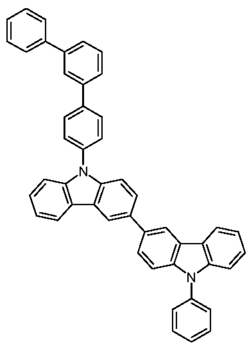 | CAS-1548581-40-3 | 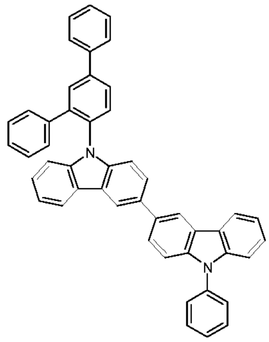 | CAS-1943719-62-7 |
| 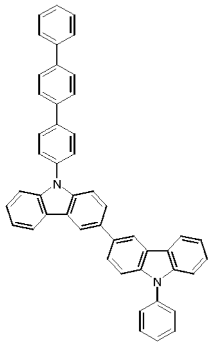 | CAS-1548581-42-5 | 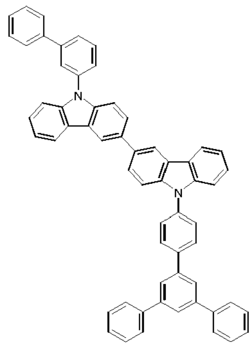 | CAS-1942079-50-6 |
| 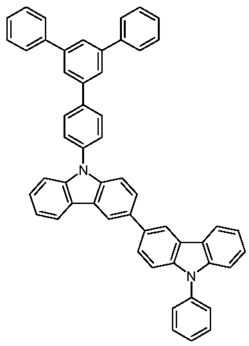 | CAS-1548581-44-7 | 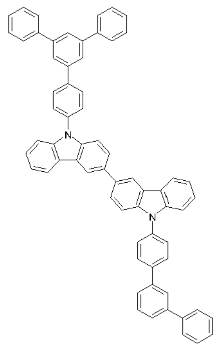 | CAS-1942079-51-7 |
| 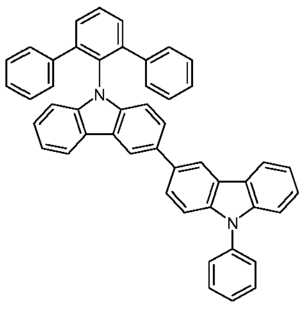 | CAS-1943719-63-8 | 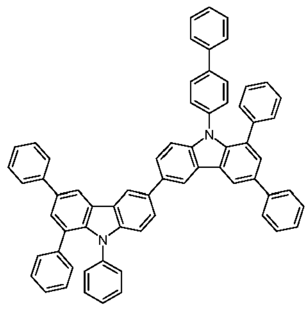 | CAS-1955476-12-6 |

(fortgesetzt)

| Struktur | CAS-Nummer | Struktur | CAS-Nummer |
|---|---|---|---|
| 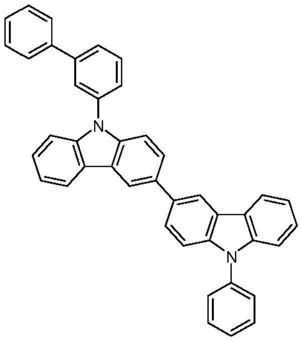<br>**96** | CAS-1619966-75-4 | 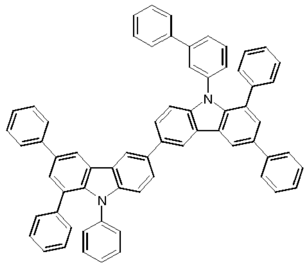 | CAS-1955476-13-7 |
| 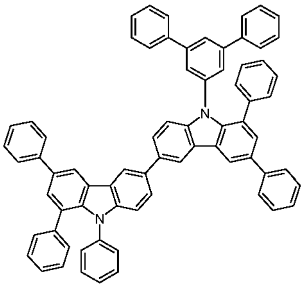 | CAS-1955476-15-9 | 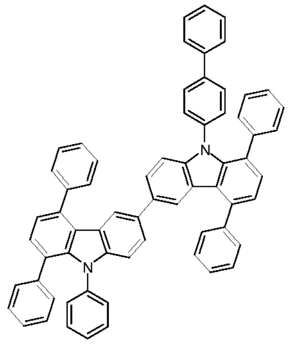 | CAS-1955476-28-4 |
| 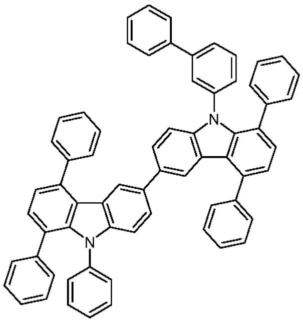 | CAS-1955476-30-8 | 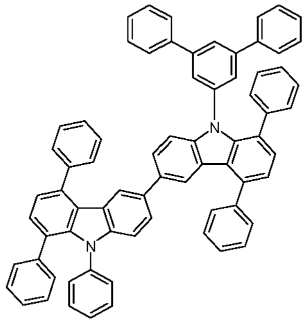 | CAS-1955476-32-0 |
| 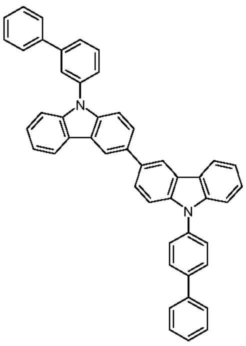<br>**91** | CAS-1643479-47-3 | 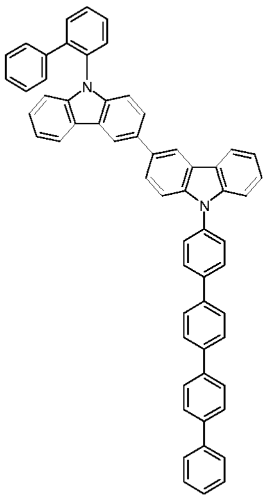 | CAS-1973498-04-2 |

(fortgesetzt)

| Struktur | CAS-Nummer | Struktur | CAS-Nummer |
|---|---|---|---|
| 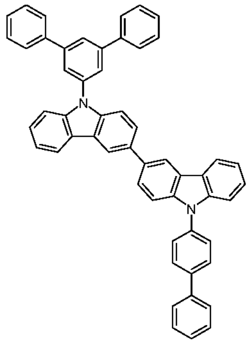 **92** | CAS-1643479-49-5 | 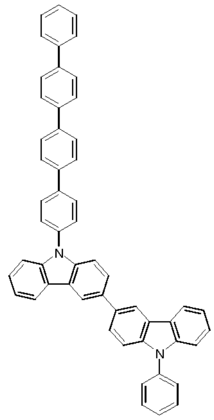 | CAS-1973498-03-1 |
| 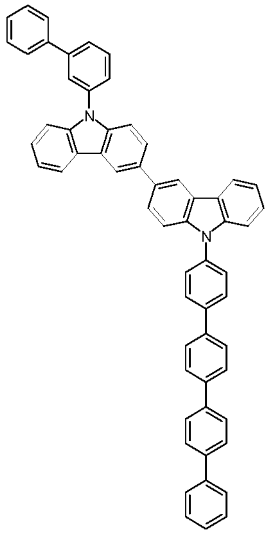 | CAS-1973498-05-3 | 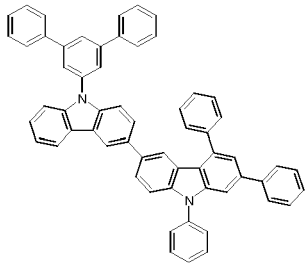 | CAS-2018307-36-1 |
| 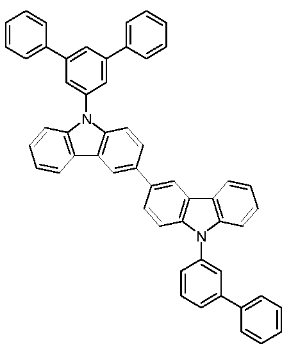 | CAS-1643479-56-4 | 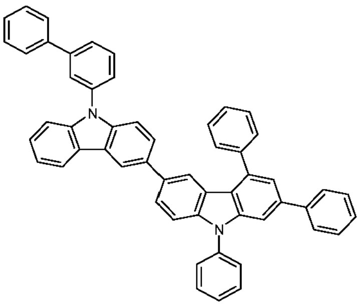 | CAS-2018307-35-0 |

(fortgesetzt)

| Struktur | CAS-Nummer | Struktur | CAS-Nummer |
|---|---|---|---|
| 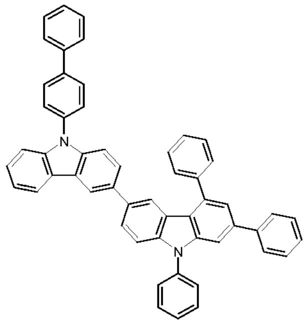 | CAS-2018307-37-2 | 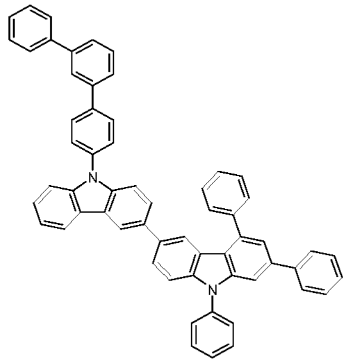 | CAS-2018307-38-3 |
| 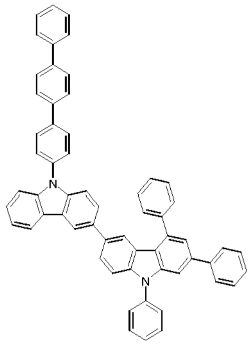 | CAS-2018307-39-4 | 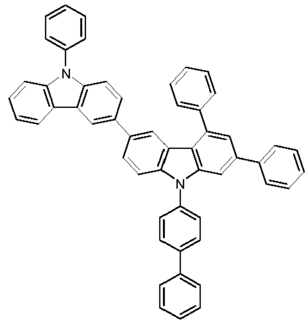 | CAS-2018307-77-0 |
| 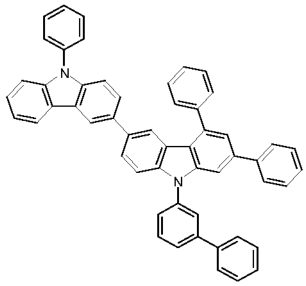 | CAS-2018307-78-1 | 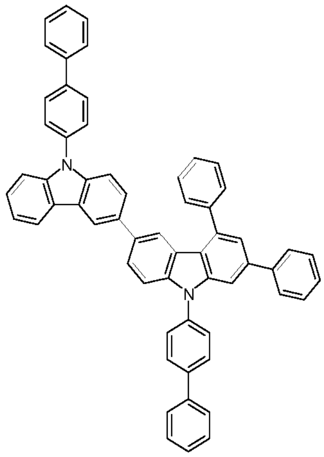 | CAS-2018307-90-7 |

(fortgesetzt)

| Struktur | CAS-Nummer | Struktur | CAS-Nummer |
|---|---|---|---|
| 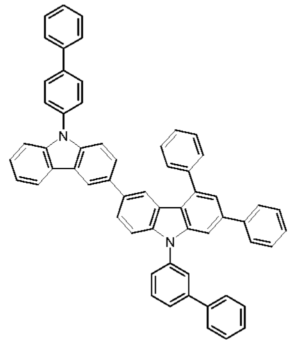 | CAS-2018307-91-8 | 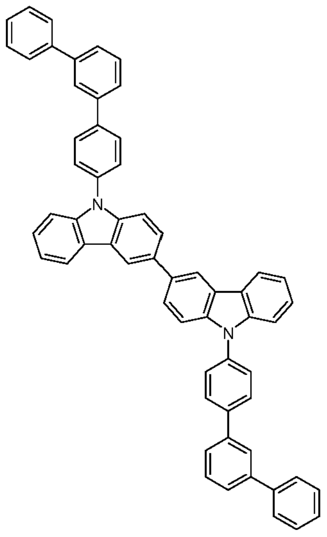 | CAS-1799958-74-9 |
| 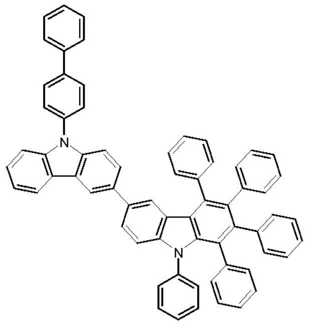 | CAS-2052160-86-6 | 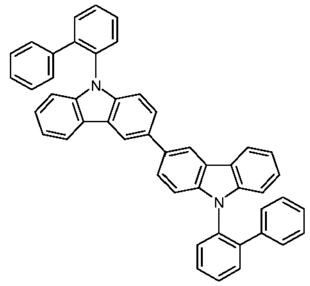 | CAS-1799958-79-4 |
| 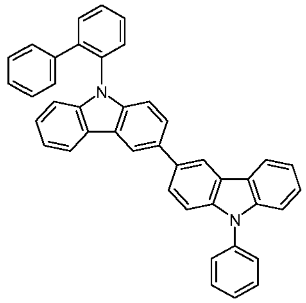 | CAS-1799958-76-1 | 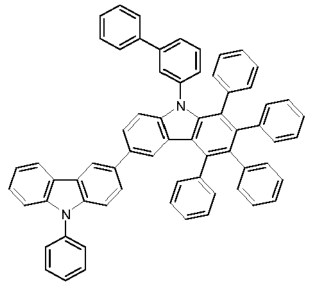 | CAS-2052160-91-3 |
| 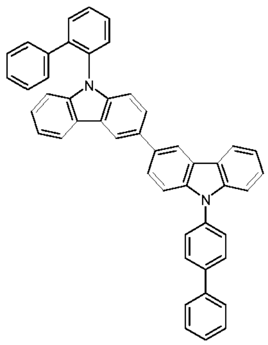 | CAS-1799958-77-2 | 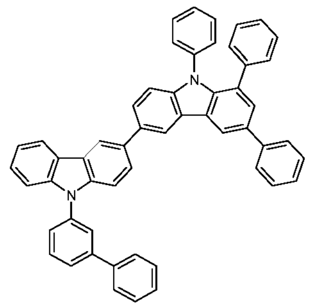 | CAS-2055848-40-1 |

(fortgesetzt)

| Struktur | CAS-Nummer | Struktur | CAS-Nummer |
|---|---|---|---|
| 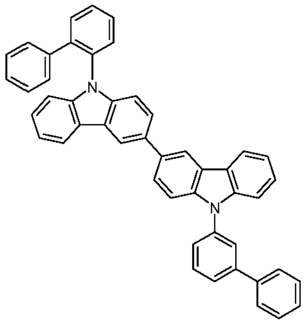<br>**93** | CAS-1799958-78-3 | 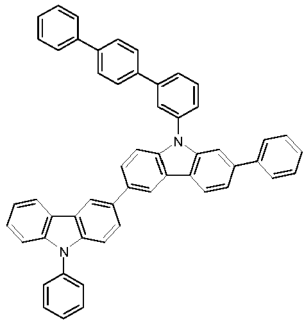 | CAS-2057418-19-4 |
| 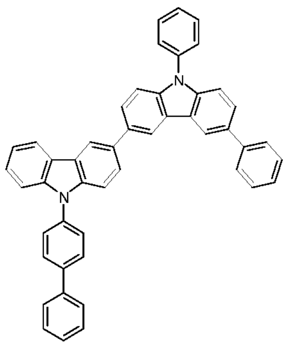 | CAS-1799958-99-8 | 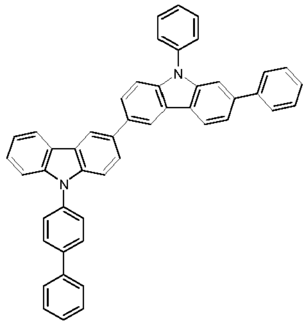 | CAS-1799959-01-5 |
| 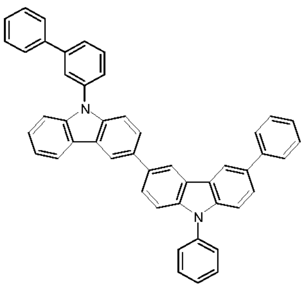 | CAS-1799959-03-7 | 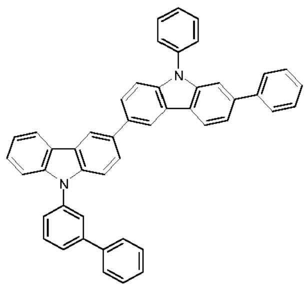 | CAS-1799959-05-9 |
| 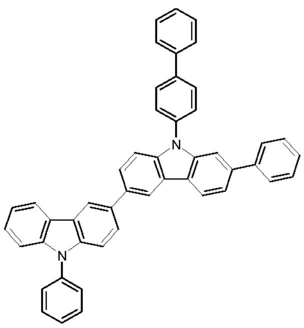 | CAS-1799959-07-1 | 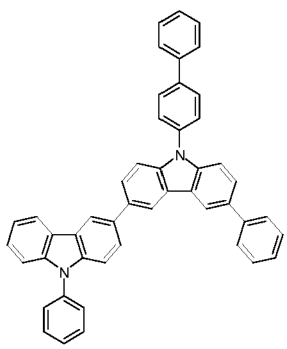 | CAS-1799959-09-3 |

(fortgesetzt)

| Struktur | CAS-Nummer | Struktur | CAS-Nummer |
|---|---|---|---|
| 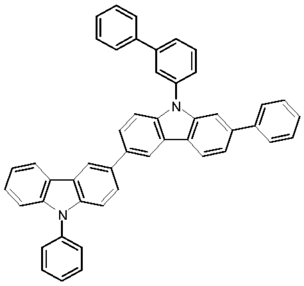 | CAS-1799959-11-7 | 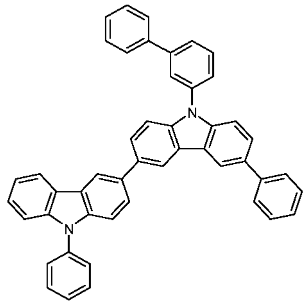 | CAS-1799959-13-9 |
| 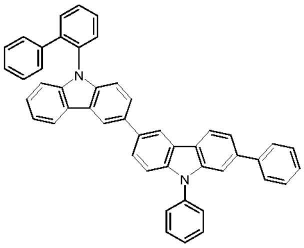 | CAS-2085318-61-0 | 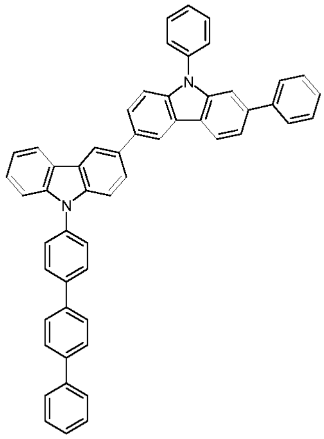 | CAS-2085318-62-1 |
| 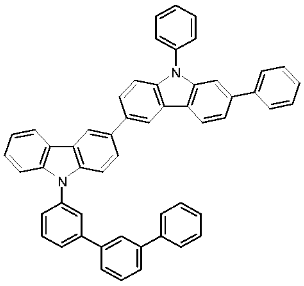 | CAS-2085318-64-3 | 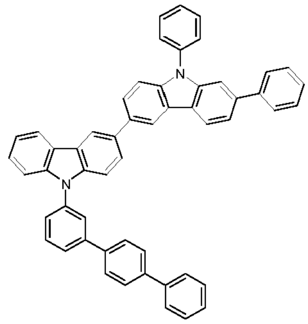 | CAS-2085318-63-2 |
| 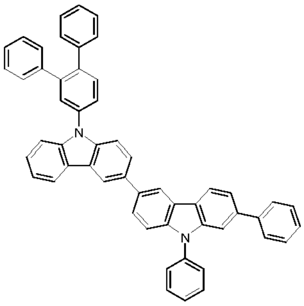 | CAS-2085318-66-5 | 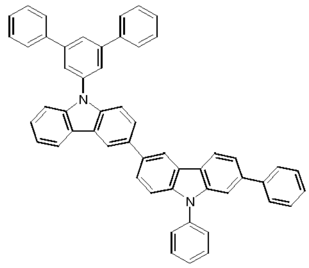 | CAS-2085318-65-4 |

(fortgesetzt)

| Struktur | CAS-Nummer | Struktur | CAS-Nummer |
|---|---|---|---|
| 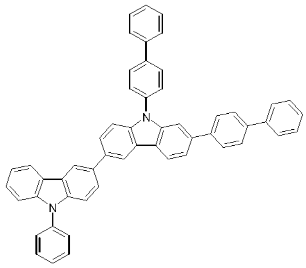 | CAS-2085318-77-8 | 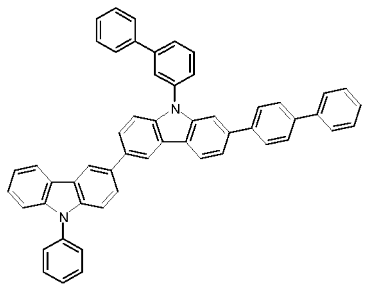 | CAS-2085318-78-9 |
| 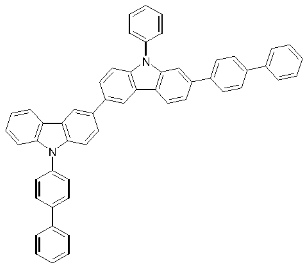 | CAS-2085318-79-0 | 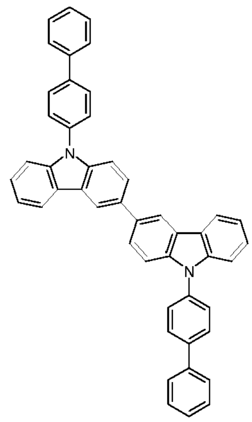 94 | CAS-57102-51-9 |
| 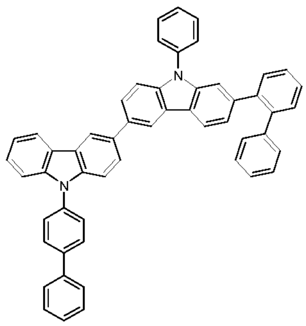 | CAS-2085318-81-4 | 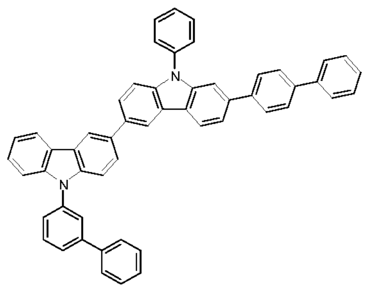 | CAS-2085318-80-3 |
| 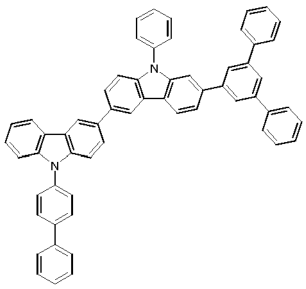 | CAS-2085318-83-6 | 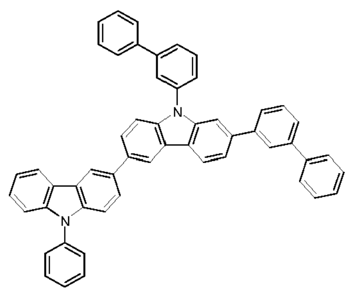 | CAS-2085318-82-5 |

(fortgesetzt)

| Struktur | CAS-Nummer | Struktur | CAS-Nummer |
|---|---|---|---|
| 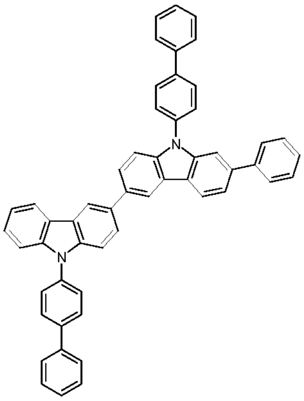 | CAS-2085318-88-1 | 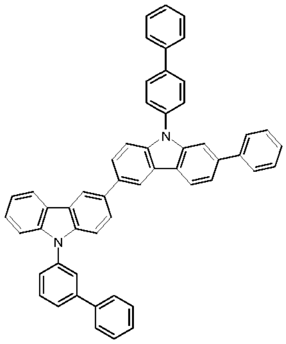 | CAS-2085318-87-0 |
| 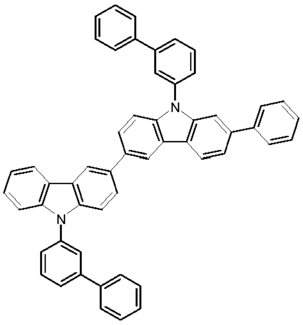 | CAS-2085318-92-7 | 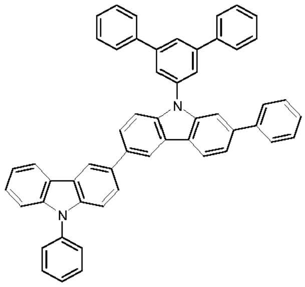 | CAS-2085318-89-2 |
| 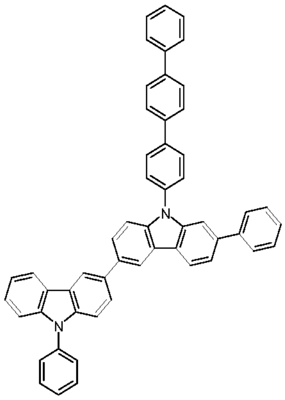 | CAS-2085318-94-9 | 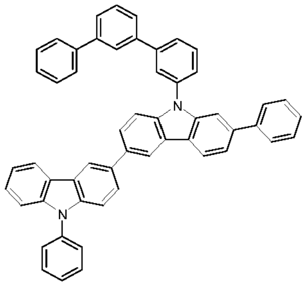 | CAS-2085318-93-8 |
| 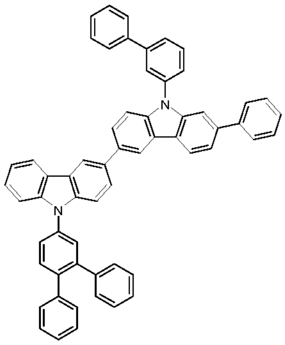 | CAS-2085318-98-3 | 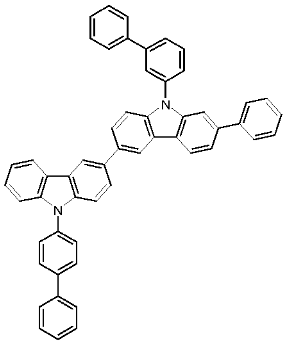 | CAS-2085318-97-2 |

(fortgesetzt)

| Struktur | CAS-Nummer | Struktur | CAS-Nummer |
|---|---|---|---|
| 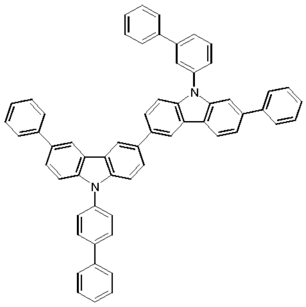 | CAS-2085319-00-0 | 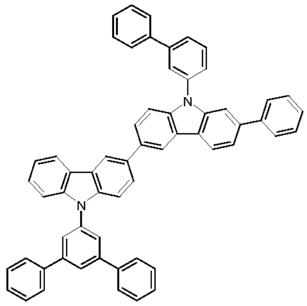 | CAS-2085318-99-4 |
| 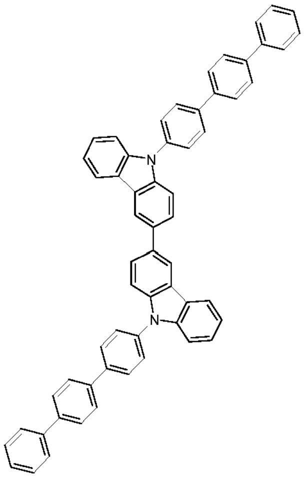 | CAS-251316-80-0 | | |
| 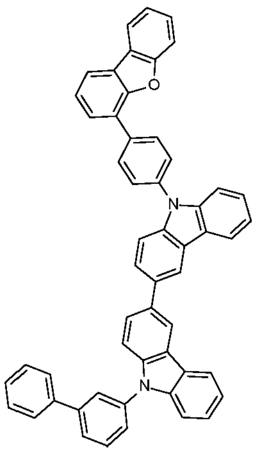 **95** | | 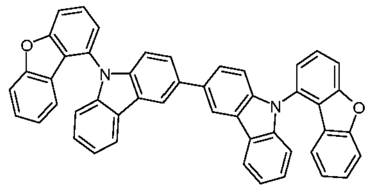 | CAS-1427160-09-5 |

(fortgesetzt)

| Struktur | CAS-Nummer | Struktur | CAS-Nummer |
|---|---|---|---|
| 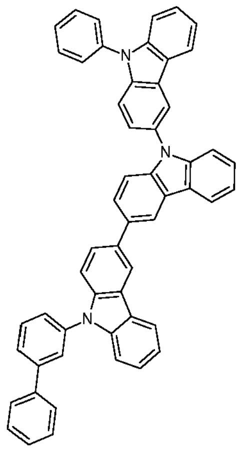 97 | CAS-1643479-72-4 | 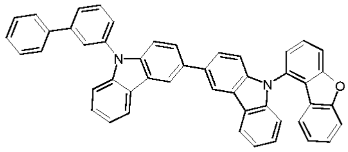 98 | |
| 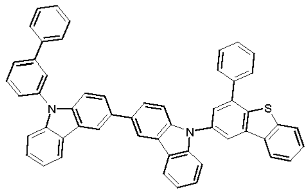 99 | | | |
| 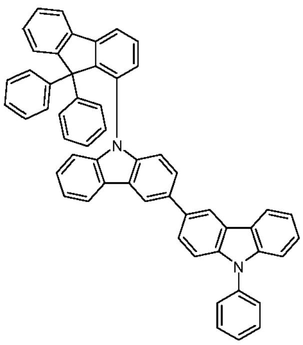 | CAS-1799960-24-9 | 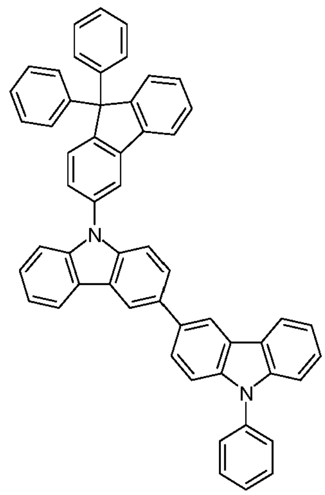 | CAS-1799960-25-0 |
| 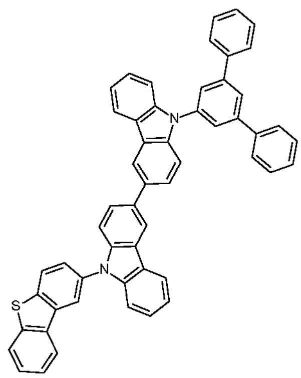 | CAS-1340668-17-8 | 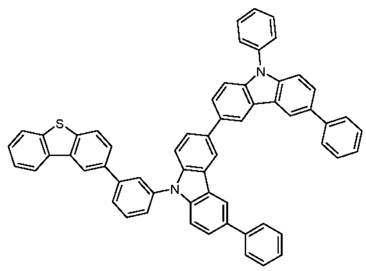 | CAS-1340668-19-0 |

(fortgesetzt)

| Struktur | CAS-Nummer | Struktur | CAS-Nummer |
|---|---|---|---|
| 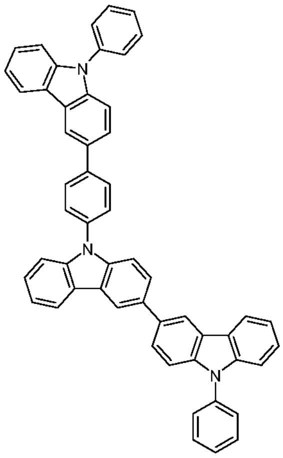 | CAS-1289556-24-6 | 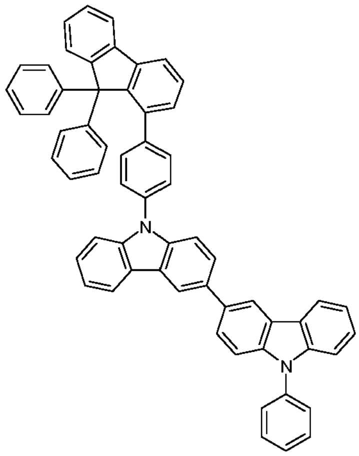 | CAS-1799960-56-7 |
| 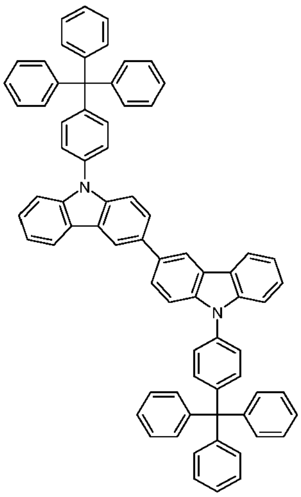 | CAS-1336889-27-0 | 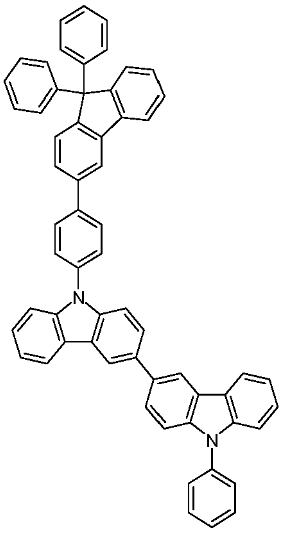 | CAS-1799960-58-9 |
| 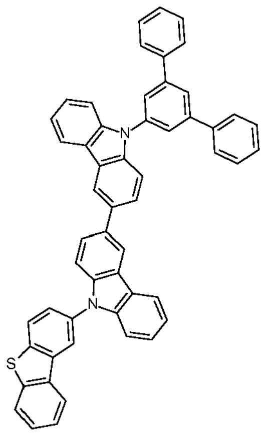 | CAS-1340668-17-8 | 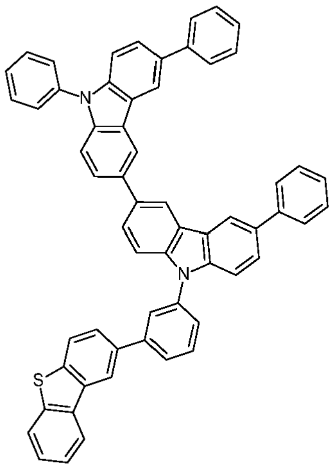 | CAS-1340668-19-0 |

(fortgesetzt)

| Struktur | CAS-Nummer | Struktur | CAS-Nummer |
|---|---|---|---|
| 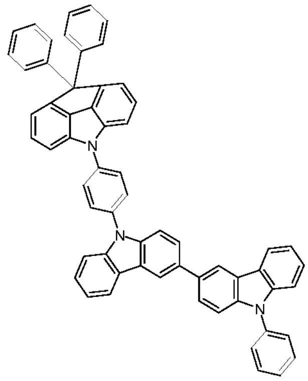 | CAS-1812208-18-6 | 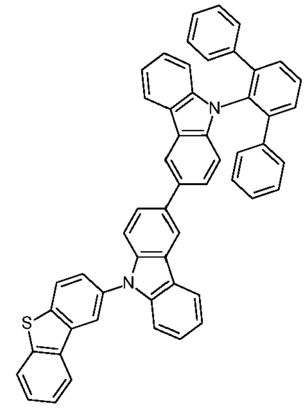 | CAS-1340668-35-0 |
| 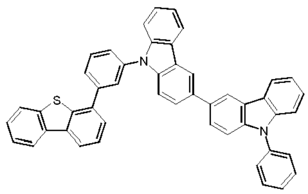 | CAS-1340668-37-2 | 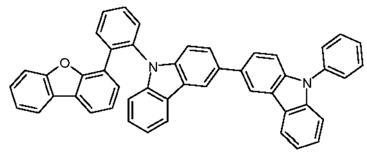 | CAS-1830334-82-1 |
| 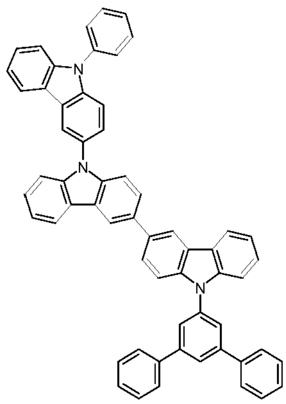 | CAS-1340669-19-3 | 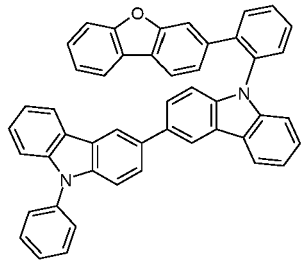 | CAS-1830334-85-4 |
| 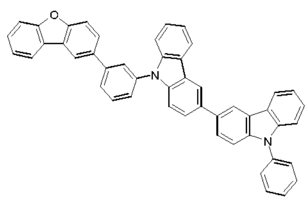 | CAS-1830334-94-5 | 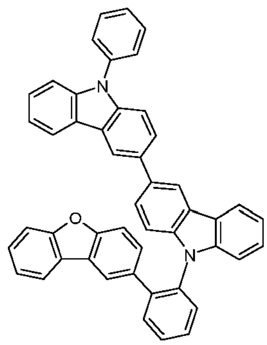 | CAS-1830334-88-7 |

(fortgesetzt)

| Struktur | CAS-Nummer | Struktur | CAS-Nummer |
|---|---|---|---|
| 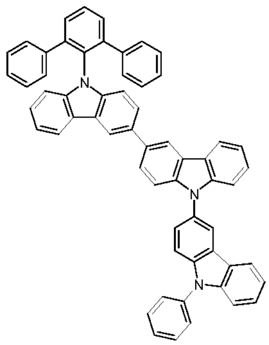 | CAS-1340669-32-0 | 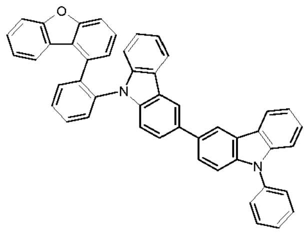 | CAS-1830334-90-1 |
| 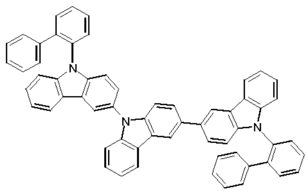 | CAS-1340669-33-1 | 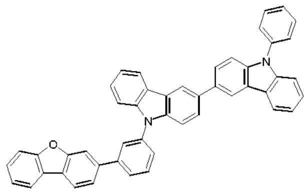 | CAS-1830334-91-2 |
| 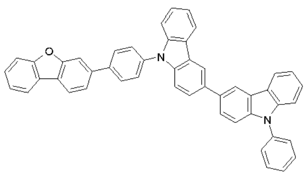 | CAS-1830335-02-8 | 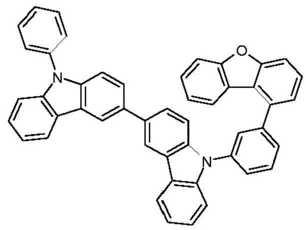 | CAS-1830334-97-8 |
| 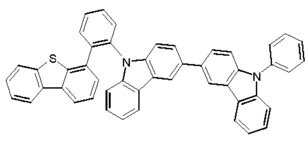 | CAS-1830335-71-1 | 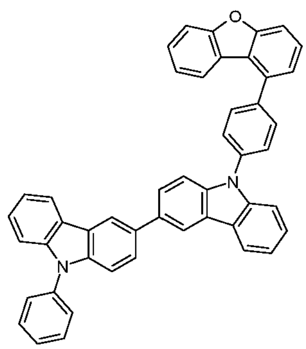 | CAS-1830335-07-3 |
| 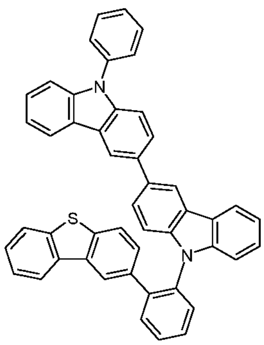 | CAS-1830335-76-6 | 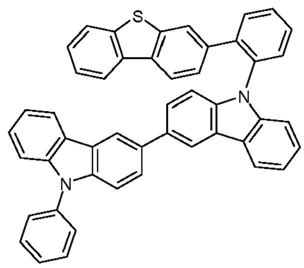 | CAS-1830335-72-2 |

(fortgesetzt)

| Struktur | CAS-Nummer | Struktur | CAS-Nummer |
|---|---|---|---|
| | | 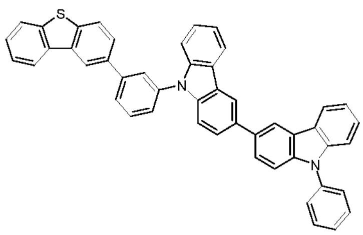 | CAS-1830335-85-7 |
| 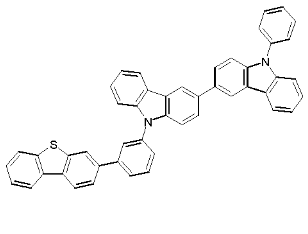 | CAS-1830335-82-4 | 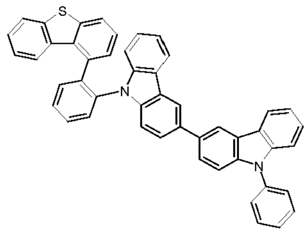 | CAS-1830335-79-9 |
| 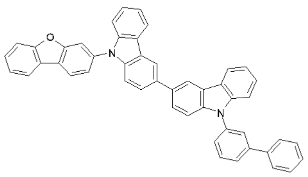 | CAS-1830339-40-6 | 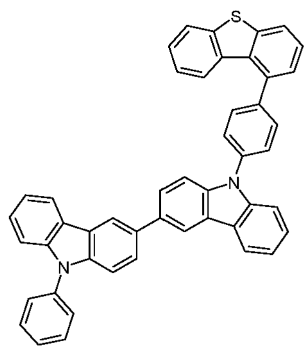 | CAS-1830335-95-9 |
| 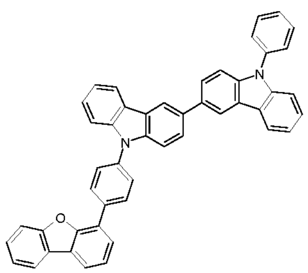 | CAS-1377150-35-0 | 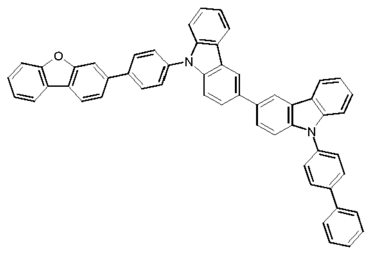 | CAS-1830339-41-7 |
| 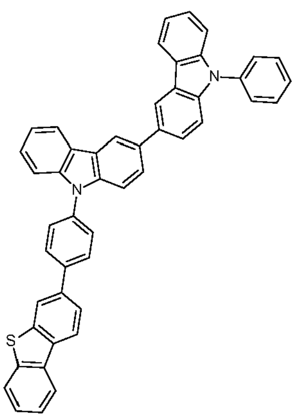 | CAS-1830335-90-4 | 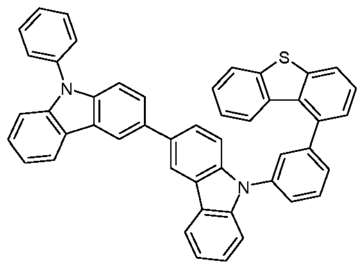 | CAS-1830335-87-9 |

(fortgesetzt)

| Struktur | CAS-Nummer | Struktur | CAS-Nummer |
|---|---|---|---|
| 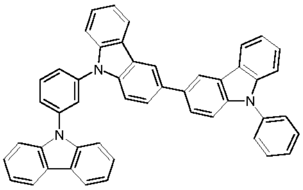 | CAS-1399855-37-8 | 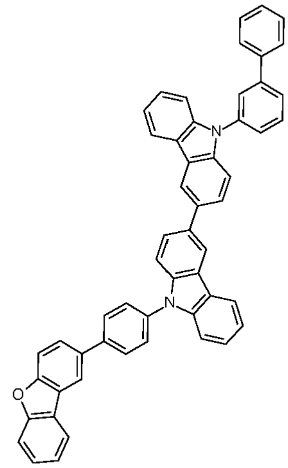 | CAS-1830339-42-8 |
| 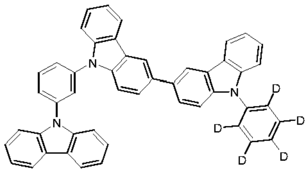 | CAS-1399855-38-9 | 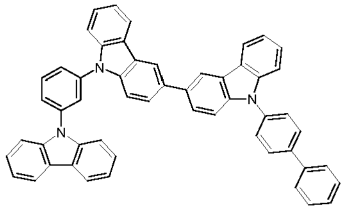 | CAS-1399855-39-0 |
| 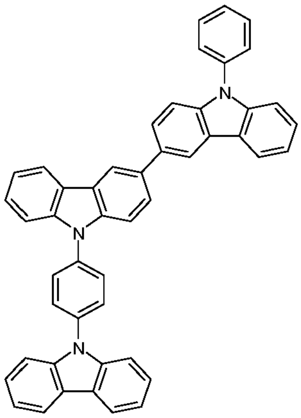 | CAS-1399855-46-9 | 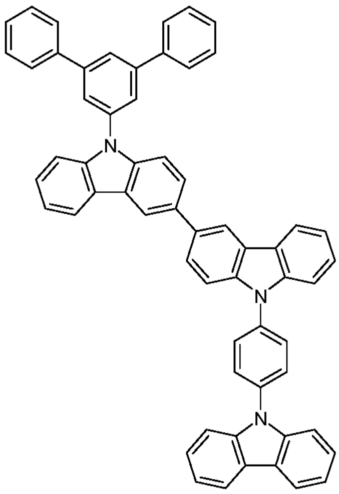 | CAS-1399855-47-0 |

(fortgesetzt)

| Struktur | CAS-Nummer | Struktur | CAS-Nummer |
|---|---|---|---|
| 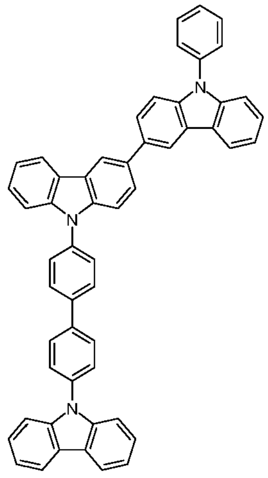 | CAS-1413936-92-1 | 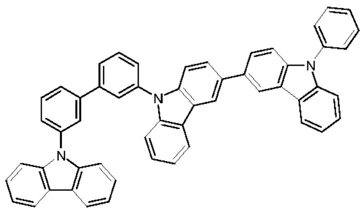 | CAS-1413936-95-4 |
| 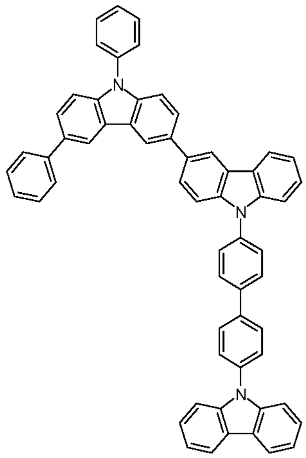 | CAS-1413936-96-5 | 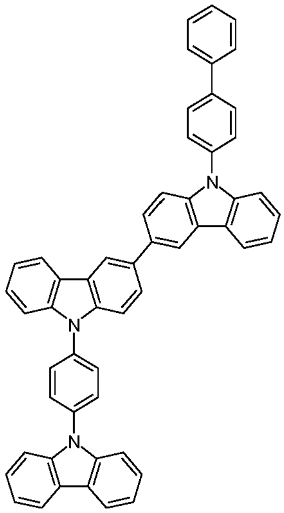 | CAS-1413936-97-6 |
| 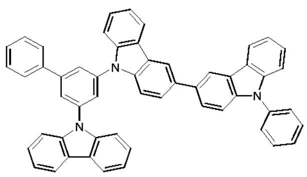 | CAS-1413937-08-2 | 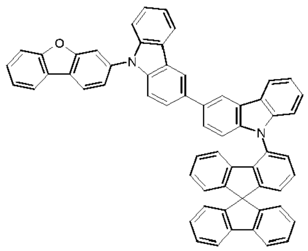 | CAS-1890157-92-2 |
| 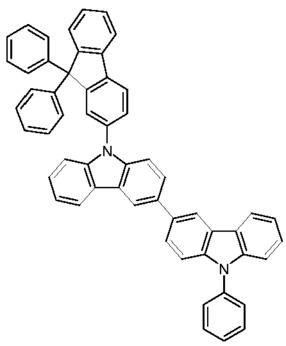 | CAS-1415348-93-4 | 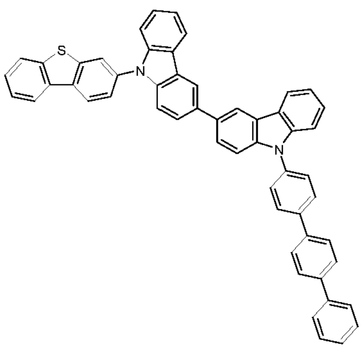 | CAS-1889262-89-8 |

(fortgesetzt)

| Struktur | CAS-Nummer | Struktur | CAS-Nummer |
|---|---|---|---|
| 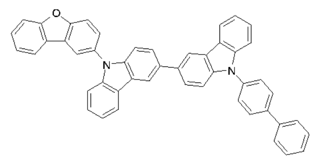 | CAS-1415348-99-0 | 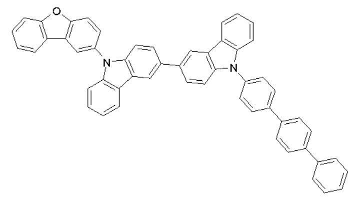 | CAS-1890156-90-7 |
| 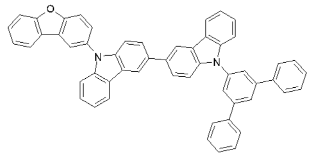 | CAS-1415349-00-6 | 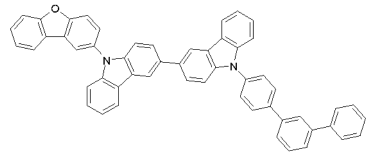 | CAS-1890156-91-8 |
| 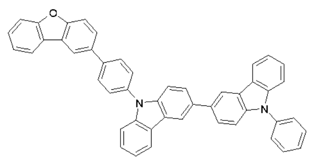 | CAS-1415349-01-7 | 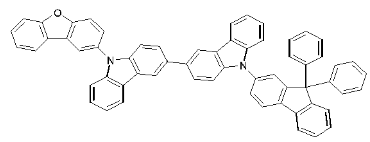 | CAS-1890157-12-6 |
| 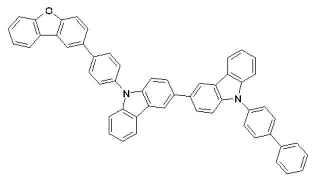 | CAS-1415349-02-8 | 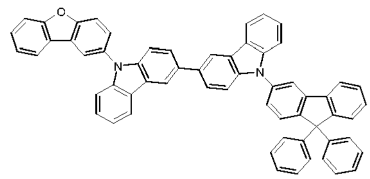 | CAS-1890157-13-7 |
| 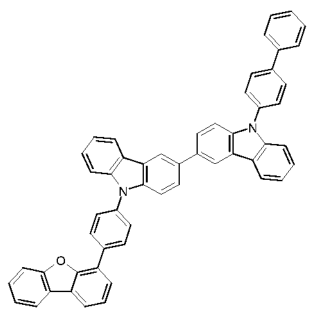 | CAS-1415349-03-9 | 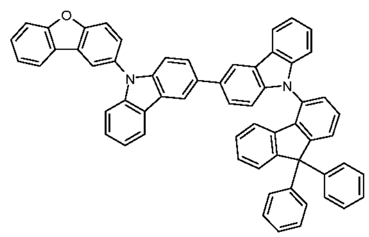 | CAS-1890157-14-8 |
| 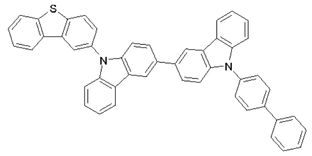 | CAS-1415349-04-0 | 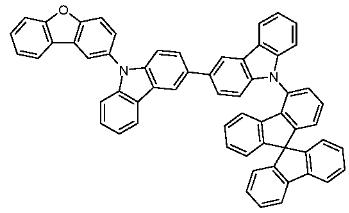 | CAS-1890157-37-5 |
| 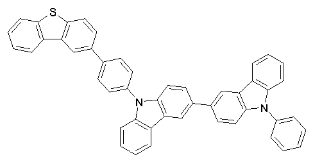 | CAS-1415349-05-1 | 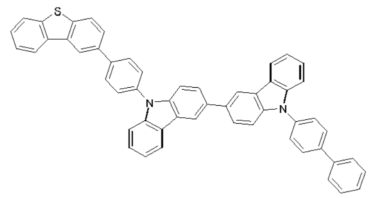 | CAS-1415349-06-2 |

(fortgesetzt)

| Struktur | CAS-Nummer | Struktur | CAS-Nummer |
|---|---|---|---|
| 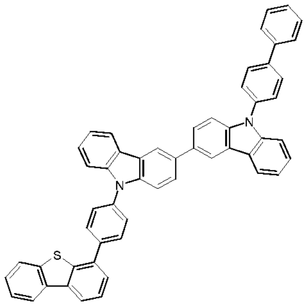 | CAS-1415349-07-3 | 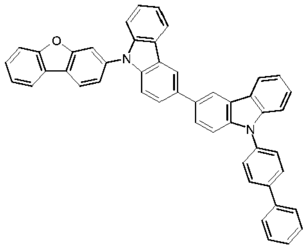 | CAS-1890157-41-1 |
| | | 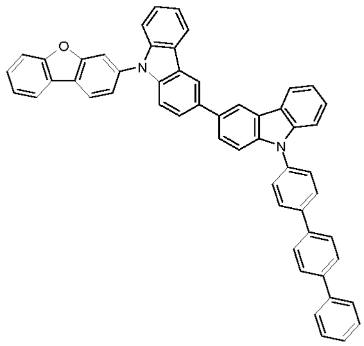 | CAS-1890157-42-2 |
| | | 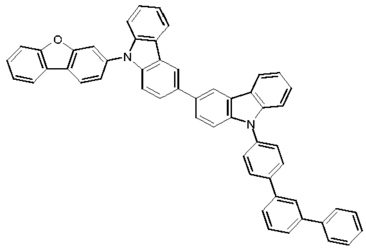 | CAS-1890157-43-3 |
| 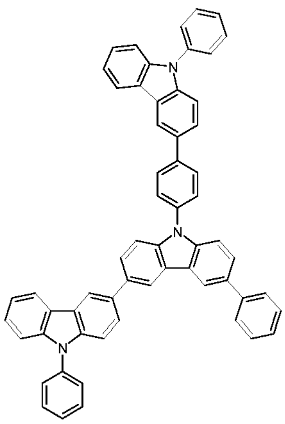 | CAS-1422451-48-6 | 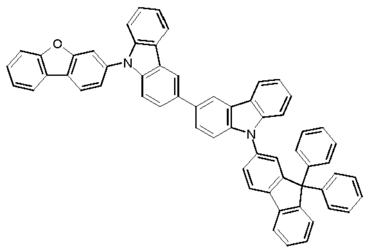 | CAS-1890157-64-8 |

(fortgesetzt)

| Struktur | CAS-Nummer | Struktur | CAS-Nummer |
|---|---|---|---|
| 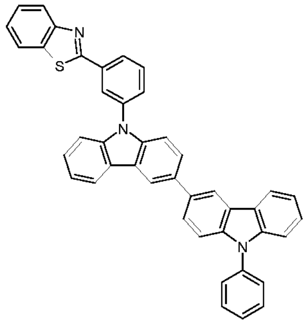 | CAS-1445952-53-3 | 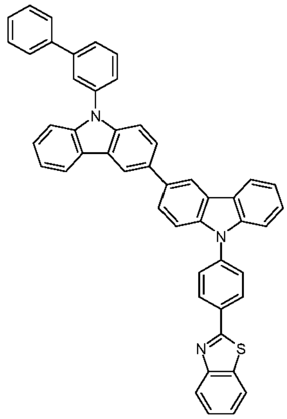 | CAS-1445952-58-8 |
| 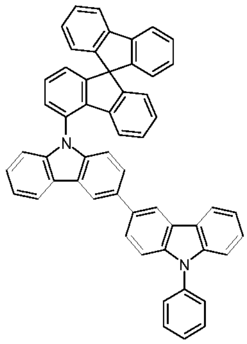 | CAS-1450933-86-4 | | |
| 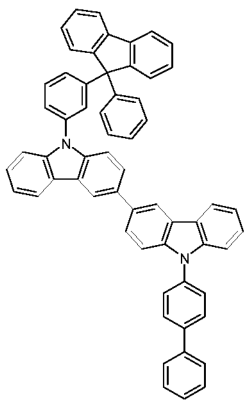 | CAS-1919031-93-8 | 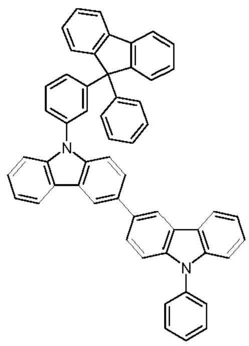 | CAS-1919031-92-7 |
| 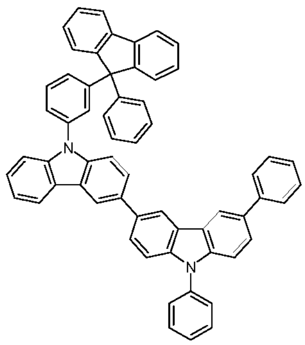 | CAS-1919031-95-0 | 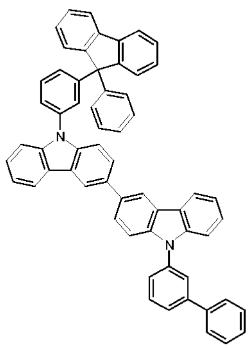 | CAS-1919031-94-9 |

(fortgesetzt)

| Struktur | CAS-Nummer | Struktur | CAS-Nummer |
|---|---|---|---|
| 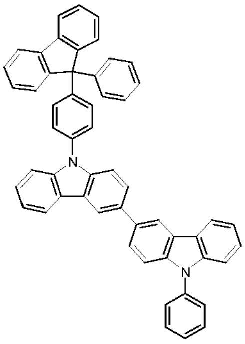 | CAS-1919031-97-2 | 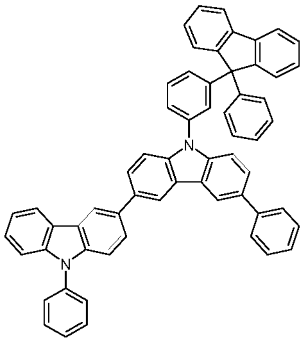 | CAS-1919031-96-1 |
| 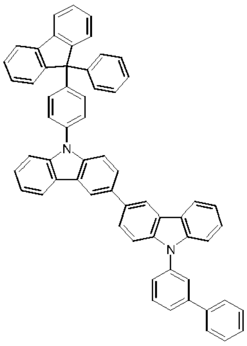 | CAS-1919031-99-4 | 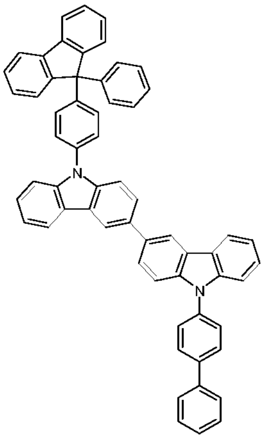 | CAS-1919031-98-3 |
| 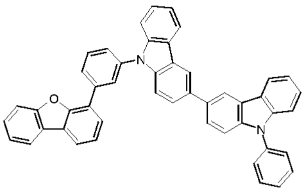 | CAS-1598389-98-0 | 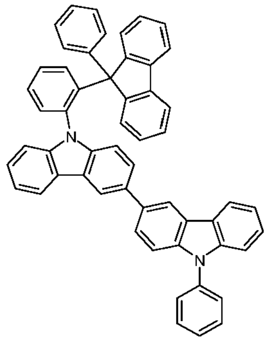 | CAS-1919032-02-2 |
| 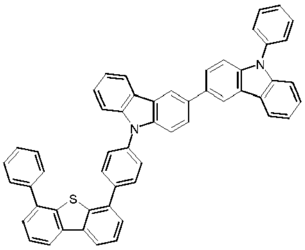 | CAS-1604034-14-1 | | |

(fortgesetzt)

| Struktur | CAS-Nummer | Struktur | CAS-Nummer |
|---|---|---|---|
| 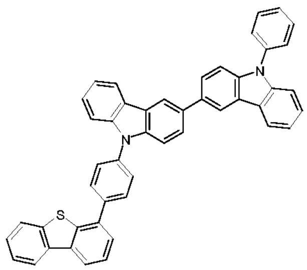 | CAS-1604034-02-7 | | |
| 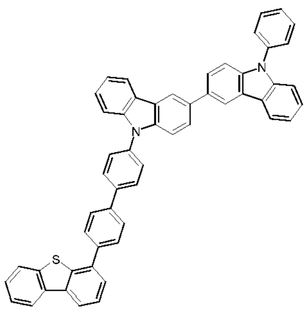 | CAS-1604034-07-2 | | |
| 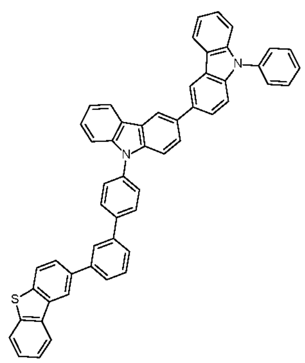 | CAS-1604034-12-9 | | |
| 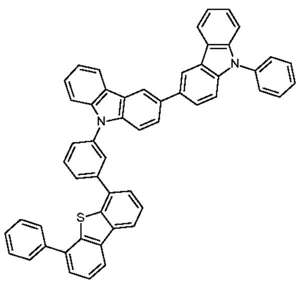 | CAS-1622931-00-3 | 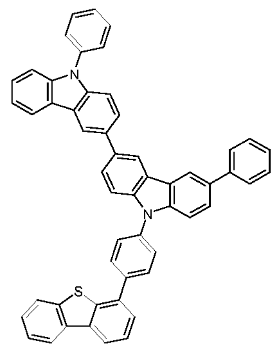 | CAS-1604034-15-2 |
| 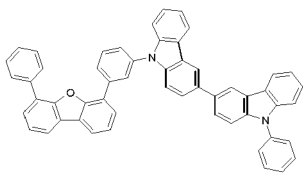 | CAS-1622931-01-4 | 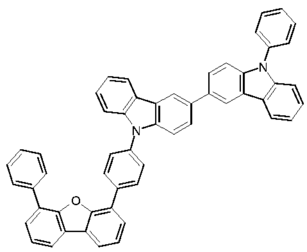 | CAS-1622931-04-7 |

(fortgesetzt)

| Struktur | CAS-Nummer | Struktur | CAS-Nummer |
|---|---|---|---|
| 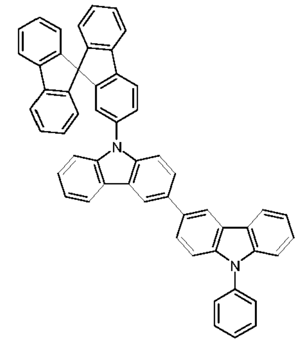 | CAS-1630029-28-5 | 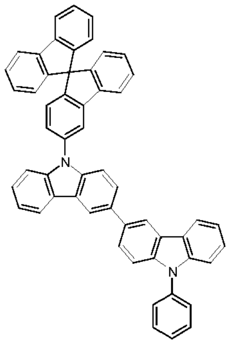 | CAS-1630029-29-6 |
| 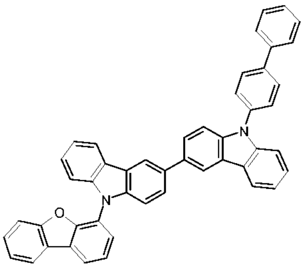 | CAS-1643479-51-9 | 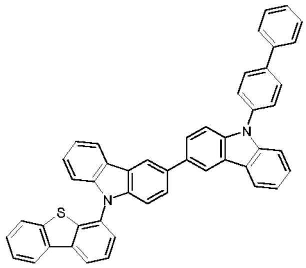 | CAS-1643479-52-0 |
| 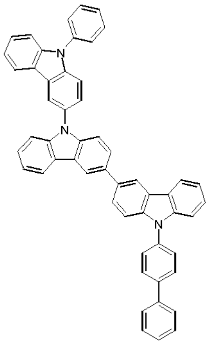 | CAS-1643479-54-2 | 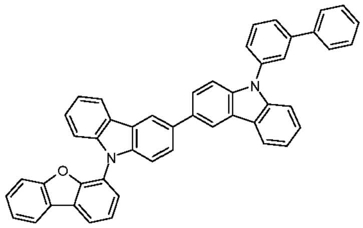 **100** | CAS-1643479-59-7 |
| 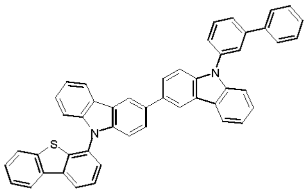 | CAS-1643479-62-2 | 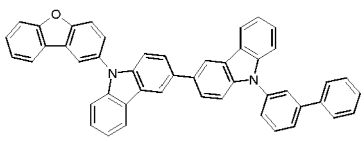 **101** | CAS-1643479-68-8 |
| 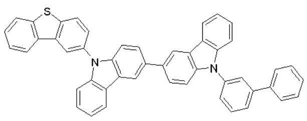 | CAS-1643479-69-9 | 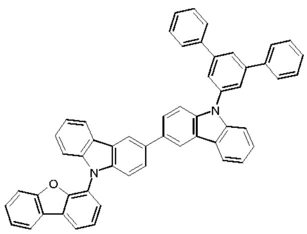 | CAS-1643479-74-6 |

(fortgesetzt)

| Struktur | CAS-Nummer | Struktur | CAS-Nummer |
|---|---|---|---|
| 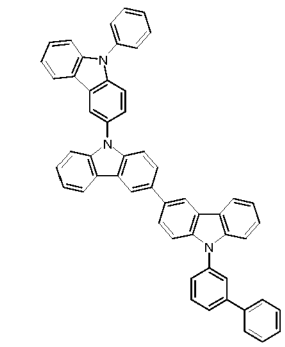 | CAS-1643479-72-4 | 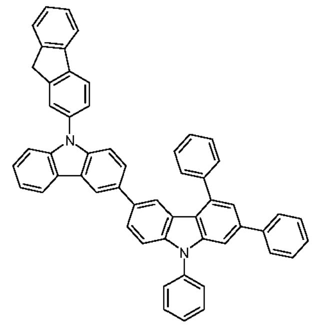 | CAS-2018307-43-0 |
| 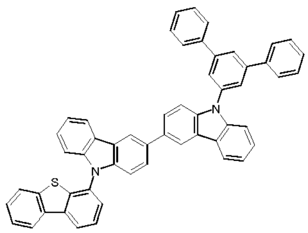 | CAS-1643479-75-7 | 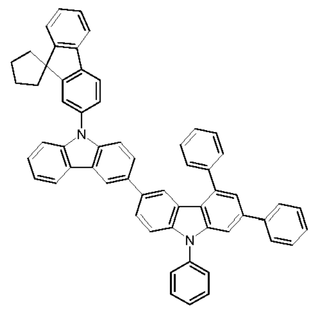 | CAS-2018307-47-4 |
| 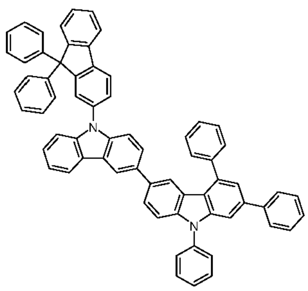 | CAS-2018307-50-9 | 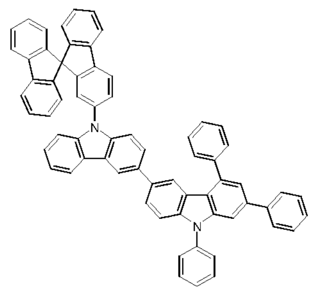 | CAS-2018307-49-6 |
| 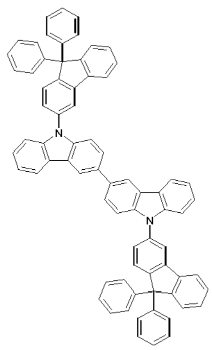 | CAS-1656982-30-7 | 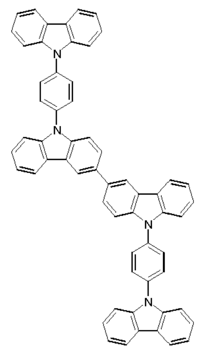 | CAS-1680184-58-0 |
| | | 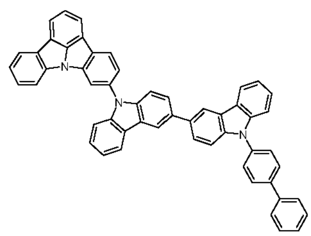 | CAS-1799483-56-9 |

(fortgesetzt)

| Struktur | CAS-Nummer | Struktur | CAS-Nummer |
|---|---|---|---|
| 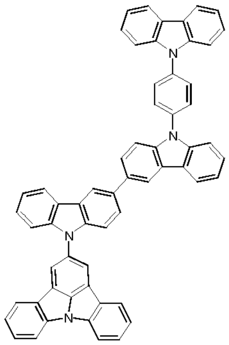 | CAS-1799519-35-9 | 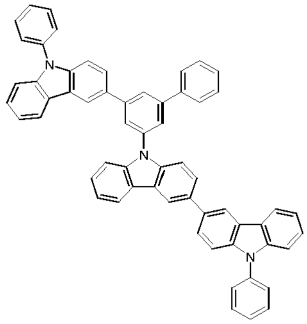 | CAS-1799678-37-7 |
| 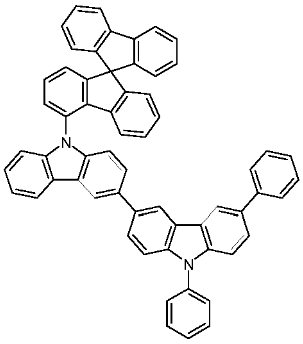 | CAS-2073116-97-7 | 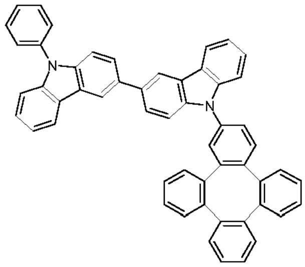 | CAS-2048236-10-6 |
| | | 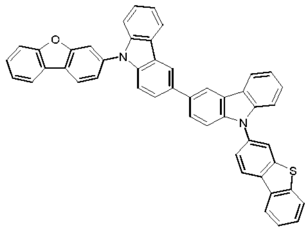 | CAS-1890157-93-3 |
| | | 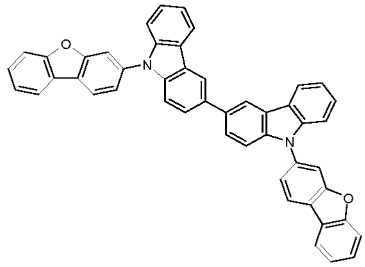 | CAS-1890157-94-4 |
| 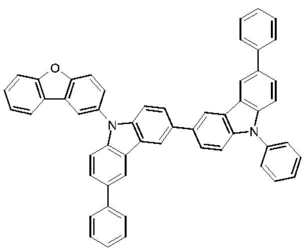 | CAS-1431151-34-6 | 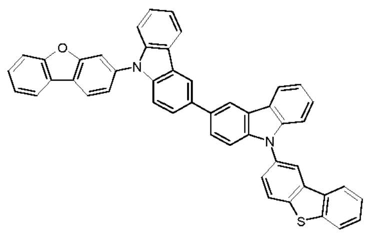 | CAS-1890157-95-5 |

(fortgesetzt)

| Struktur | CAS-Nummer | Struktur | CAS-Nummer |
|---|---|---|---|
| 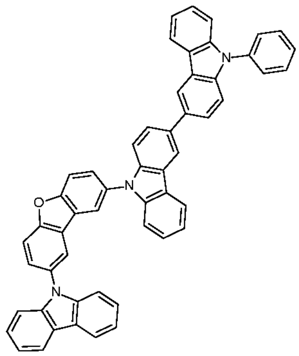 | CAS-1446411-07-9 | 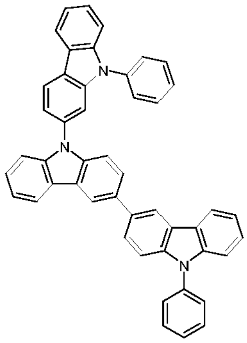 | CAS-1894193-97-5 |
| 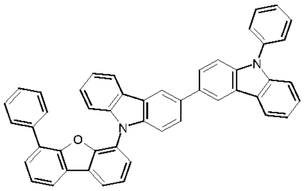 | CAS-1894194-10-5 | 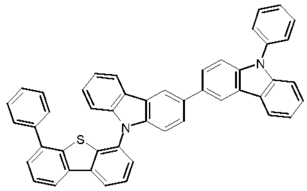 | CAS-1894194-11-6 |
| 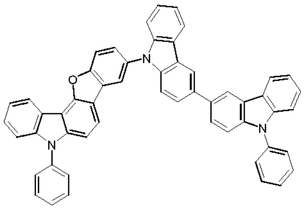 | CAS-1497337-43-5 | 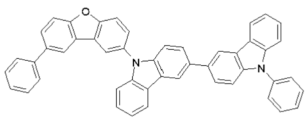 | CAS-1499917-70-2 |
| 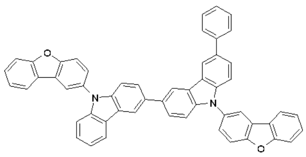 | CAS-1588866-10-7 | 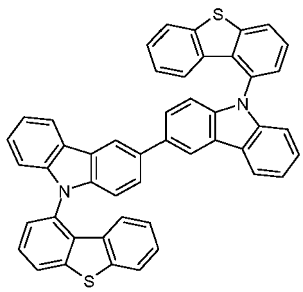 | CAS-1934252-94-4 |
| 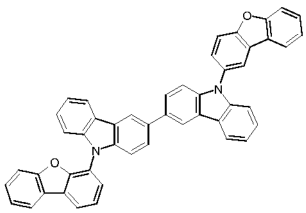 | CAS-1598389-99-1 | 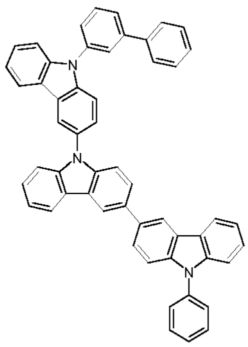 | CAS-1943719-77-4 |

(fortgesetzt)

| Struktur | CAS-Nummer | Struktur | CAS-Nummer |
|---|---|---|---|
| 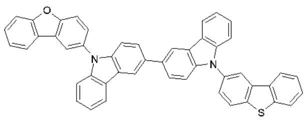 | CAS-1613752-14-9 | 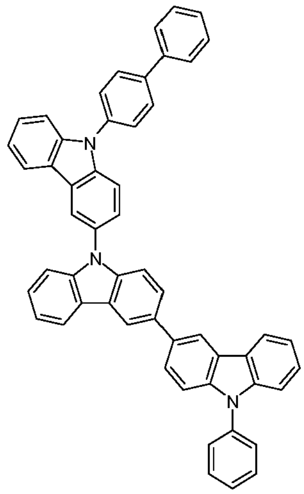 | CAS-1943719-78-5 |
| 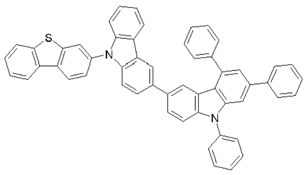 | CAS-2018307-45-2 | 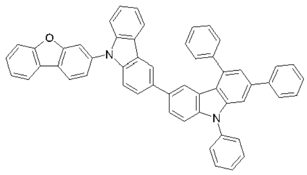 | CAS-2018307-44-1 |
| 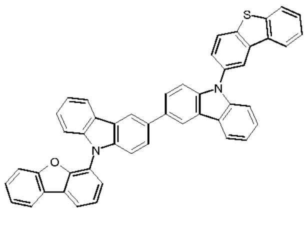 | CAS-1643479-80-4 | 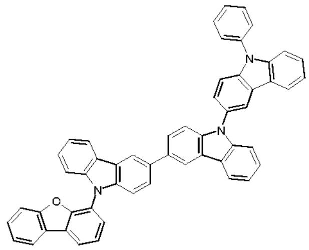 | CAS-1643479-84-8 |
| 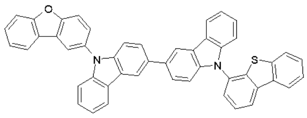 | CAS-1643479-88-2 | 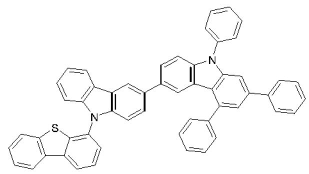 | CAS-2018307-52-1 |
| 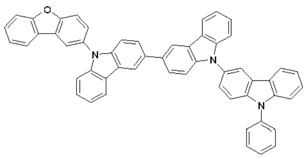 | CAS-1643480-02-7 | 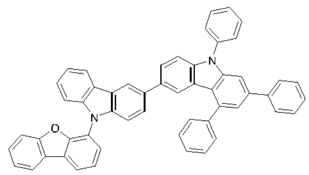 | CAS-2018307-51-0 |
| 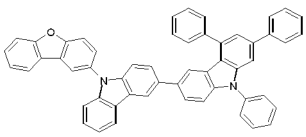 | CAS-2018307-53-2 | 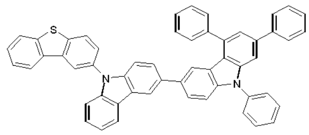 | CAS-2018307-54-3 |

(fortgesetzt)

| Struktur | CAS-Nummer | Struktur | CAS-Nummer |
|---|---|---|---|
| 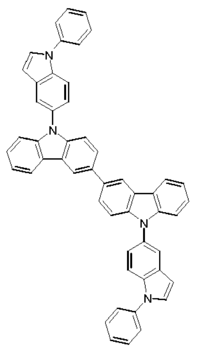 | CAS-1656982-32-9 | 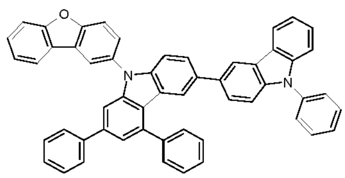 | CAS-2018307-80-5 |
| 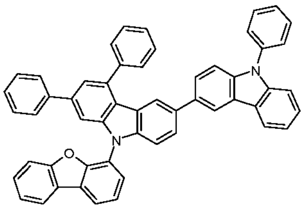 | CAS-2018307-79-2 | 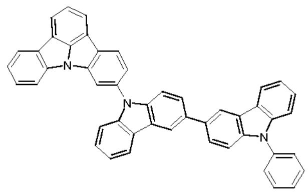 | CAS-1799483-31-0 |
| | | 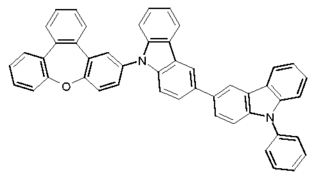 | CAS-1792238-01-7 |
| 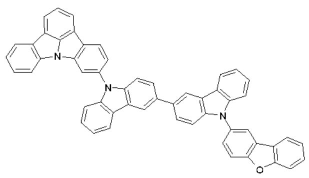 | CAS-1799483-43-4 | 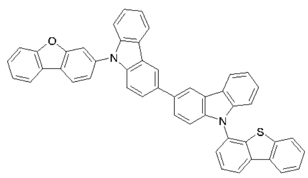 | CAS-2020391-63-1 |
| 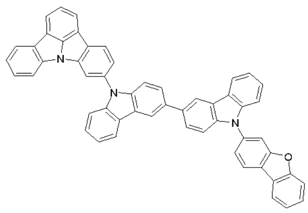 | CAS-1799483-44-5 | 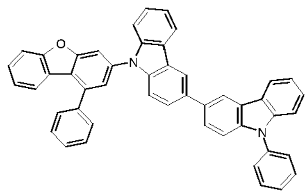 | CAS-2020391-71-1 |
| 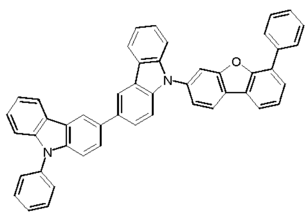 | CAS-2020391-73-3 | 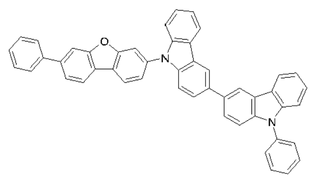 | CAS-2020391-72-2 |

(fortgesetzt)

| Struktur | CAS-Nummer | Struktur | CAS-Nummer |
|---|---|---|---|
| 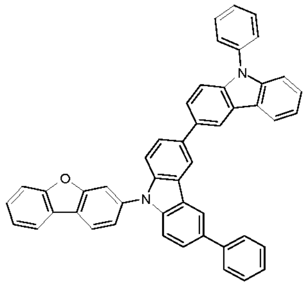 | CAS-2020391-75-5 | 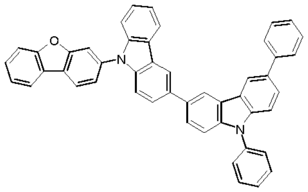 | CAS-2020391-74-4 |
| 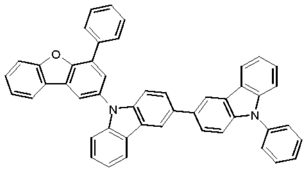 | CAS-2079874-13-6 | 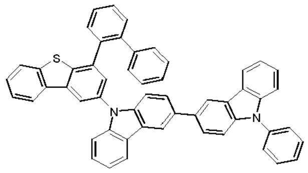 | CAS-2075738-96-2 |
| 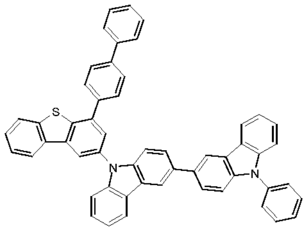 | CAS-2075738-98-4 | 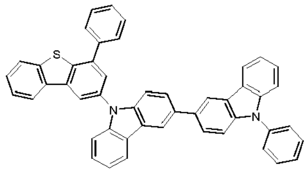 | CAS-2075738-97-3 |
| 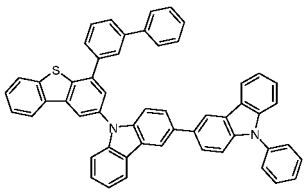 | CAS-2075738-99-5 | 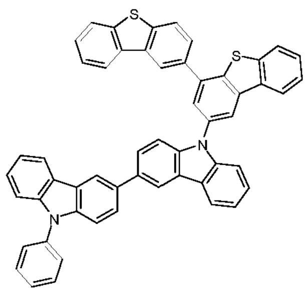 | CAS-2075739-04-5 |
| 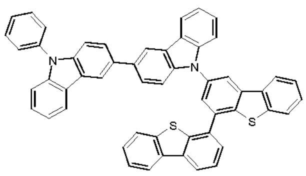 | CAS-2075739-05-6 | 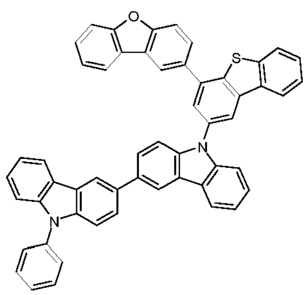 | CAS-2075739-06-7 |

(fortgesetzt)

| Struktur | CAS-Nummer | Struktur | CAS-Nummer |
|---|---|---|---|
| 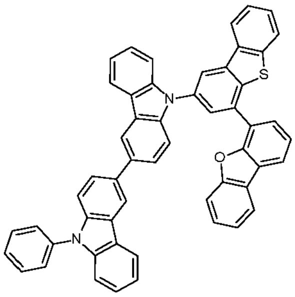 | CAS-2075739-07-8. | 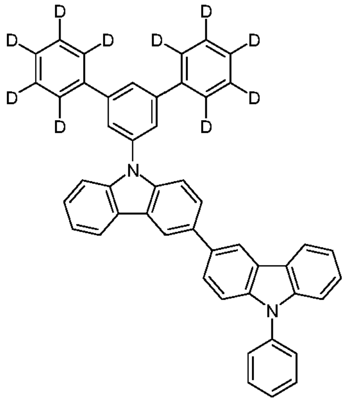 | |
| 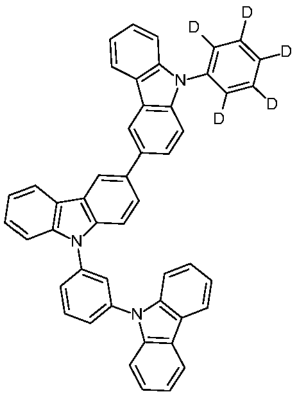 | | 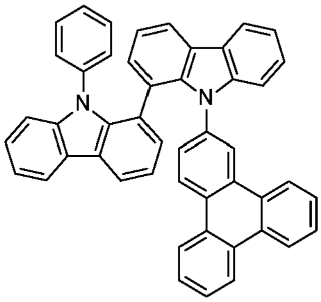 | |
| 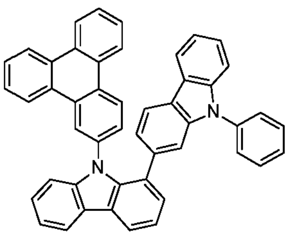 | | 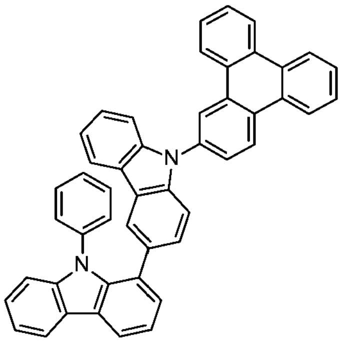 | |
| 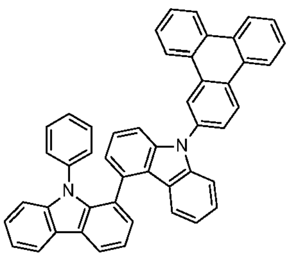 | | 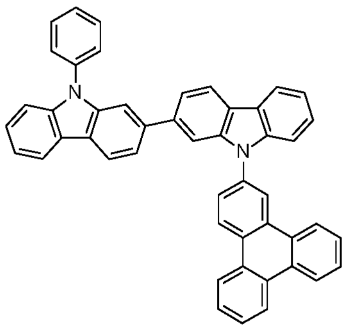 | |

(fortgesetzt)

| Struktur | CAS-Nummer | Struktur | CAS-Nummer |
|---|---|---|---|
| 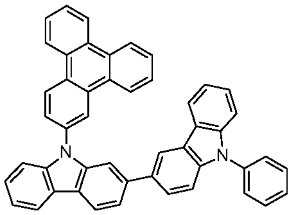 | | 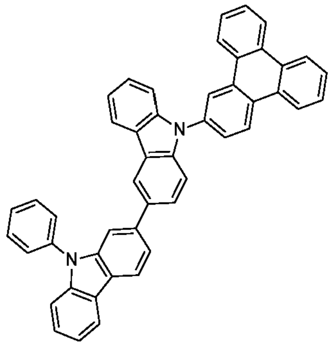 | |
| 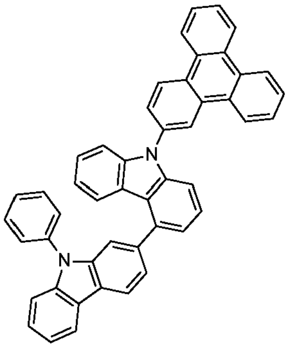 | | 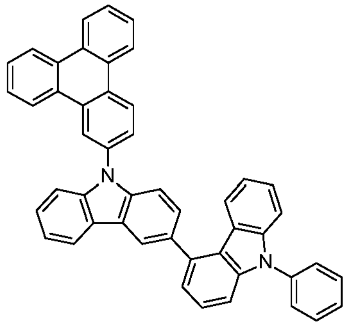 | |
| 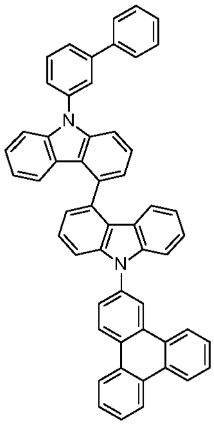 | | 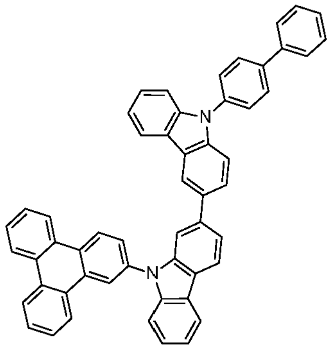 | |
| 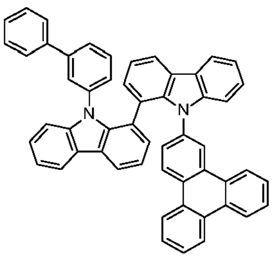 | | | |

Besonders geeignete Beispiele für Verbindungen der Formeln (2a) oder (2b), die erfindungsgemäß ausgewählt werden, sind die Verbindungen **89** bis **101,** wie zuvor beschrieben.

Die Herstellung der Verbindungen der Formeln (2a) und (2b) sowie der Verbindungen der Tabelle 9 ist dem Fachmann bekannt. Die Verbindungen können nach dem Fachmann bekannten Syntheseschritten, wie z.B. Halogenierung, bevorzugt Bromierung, und einer sich anschließenden metallorganischen Kupplungsreaktion, z.B. Suzuki-Kupplung, Heck-Kupplung oder Hartwig-Buchwald-Kupplung, hergestellt werden. Zum Teil sind die Biscarbazole der Formel

(2a) kommerziell erhältlich.

Die Herstellung der Verbindungen der Formeln (2a) und (2b) kann beispielsweise nach Schema 6 oder Schema 7 erfolgen.

Schema 6, für die Herstellung von unsymmetrischen Biscarbazolen der Formeln (2a) oder (2b):

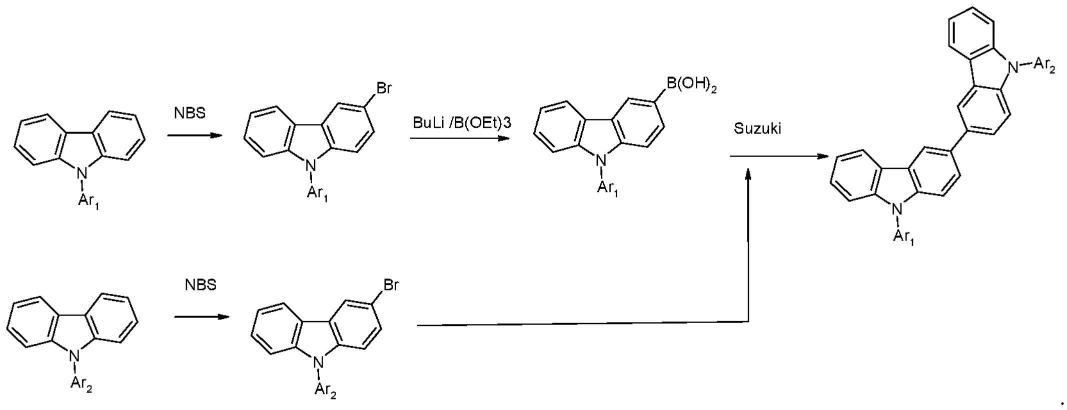


Schema 7, für die Herstellung von symmetrischen Biscarbazolen der Formeln (2a) oder (2b) (Ar1 und $Ar_2$ sind gleich und im Schema als Ar1 abgekürzt):

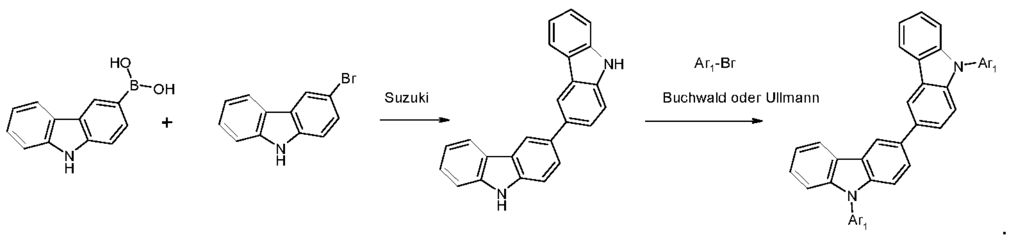


Weitere Details zu den Synthesen bzw. weitere Literaturzitate sind im Ausführungsteil beschrieben.

Die vorstehend genannten Hostmaterialien der Formel (1e) sowie deren bevorzugt beschriebene Ausführungsformen oder die Verbindungen der Tabellen 1 bis 8 können erfindungsgemäß beliebig mit den genannten Hostmaterialien der Formeln (2a) und (2b) sowie deren bevorzugt beschriebene Ausführungsformen oder den Verbindungen der Tabelle 9 kombiniert werden.

Besonders bevorzugte Mischungen der Hostmaterialien der Formel (1e) mit den Hostmaterialien der Formel (2a) für die erfindungsgemäße Zusammensetzung erhält man durch Kombination der Verbindungen **6, 9, 19, 26, 34, 42, 48, 50, 59, 70, 78** und **86** der Tabellen 5 bis 8 mit den Verbindungen der Tabelle 9.

Ganz besonders bevorzugte Mischungen der Hostmaterialien der Formel (1e) mit den Hostmaterialien der Formel (2a) erhält man durch Kombination der Verbindungen **6, 9 und 19** der Tabelle 5 mit den Verbindungen **89** bis **101** der Tabelle 9, wie im Folgenden in Tabelle 10 gezeigt.

Tabelle 10:

| | | | | | |
|---|---|---|---|---|---|
| M67 | **6** | **89** (CAS-1454567-05-5) | M68 | **6** | **90** (CAS-1352040-89-1) |
| M69 | **6** | **91** (CAS-1643479-47-3) | M70 | **6** | **92** (CAS-1643479-49-5) |
| M71 | **6** | **93** (CAS-1799958-78-3) | M72 | **6** | **94** (CAS-57102-51-9) |
| M73 | **6** | **95** | M74 | **6** | **96** (CAS-1427160-09-5) |
| M75 | **6** | **97** (CAS-1643479-72-4) | M76 | **6** | **98** |
| M77 | **6** | **99** | M78 | **6** | **100** (CAS-1643479-59-7) |
| M79 | **6** | **101** (CAS-1643479-68-8) | | | |
| M106 | **9** | **89** (CAS-1454567-05-5) | M107 | **9** | **90** (CAS-1352040-89-1) |

(fortgesetzt)

| | | | | | |
|---|---|---|---|---|---|
| M108 | **9** | **91** (CAS-1643479-47-3) | M109 | **9** | **92** (CAS-1643479-49-5) |
| M110 | **9** | **93** (CAS-1799958-78-3) | M111 | **9** | **94** (CAS-57102-51-9) |
| M112 | **9** | **95** | M113 | **9** | **96** (CAS-1427160-09-5) |
| M114 | **9** | **97** (CAS -1643479-72-4) | M115 | **9** | **98** |
| M116 | **9** | **99** | M117 | **9** | **100** (CAS-1643479-59-7) |
| M118 | **9** | **101** (CAS-1643479-68-8) | | | |
| M241 | **19** | **89** (CAS-1454567-05-5) | M242 | **19** | **90** (CAS-1352040-89-1) |
| M243 | **19** | **91** (CAS-1643479-47-3) | M244 | **19** | **92** (CAS-1643479-49-5) |
| M245 | **19** | **93** (CAS-1799958-78-3) | M246 | **19** | **94** (CAS-57102-51-9) |
| M247 | **19** | **95** | M248 | **19** | **96** (CAS-1427160-09-5) |
| M249 | **19** | **97** (CAS-1643479-72-4) | M250 | **19** | **98** |
| M251 | **19** | **99** | M252 | **19** | **100** (CAS-1643479-59-7) |
| M253 | **19** | **101** (CAS-1643479-68-8) | | | |

Ganz besonders bevorzugte Mischungen der Hostmaterialien der Formel (1e) mit den Hostmaterialien der Formel (2a) erhält man durch Kombination der Verbindungen **26, 34** und **42** der Tabelle 6 mit den Verbindungen **89** bis **101** der Tabelle 9, wie im Folgenden in Tabelle 11 gezeigt.

Tabelle 11:

| | | | | | |
|---|---|---|---|---|---|
| M319 | **26** | **89** (CAS-1454567-05-5) | M320 | **26** | **90** (CAS-1352040-89-1) |
| M321 | **26** | **91** (CAS-1643479-47-3) | M322 | **26** | **92** (CAS-1643479-49-5) |
| M323 | **26** | **93** (CAS-1799958-78-3) | M324 | **26** | **94** (CAS-57102-51-9) |
| M325 | **26** | **95** | M326 | **26** | **96** (CAS-1427160-09-5) |
| M327 | **26** | **97** (CAS-1643479-72-4) | M328 | **26** | **98** |
| M329 | **26** | **99** | M330 | **26** | **100** (CAS-1643479-59-7) |
| M331 | **26** | **101** (CAS-1643479-68-8) | | | |
| M423 | **34** | **89** (CAS-1454567-05-5) | M424 | **34** | **90** (CAS-1352040-89-1) |
| M425 | **34** | **91** (CAS-1643479-47-3) | M426 | **34** | **92** (CAS-1643479-49-5) |
| M427 | **34** | **93** (CAS-1799958-78-3) | M428 | **34** | **94** (CAS-57102-51-9) |
| M429 | **34** | **95** | M430 | **34** | **96** (CAS-1427160-09-5) |
| M431 | **34** | **97** (CAS-1643479-72-4) | M432 | **34** | 98 |
| M433 | **34** | **99** | M434 | **34** | **100** (CAS-1643479-59-7) |
| M435 | **34** | **101** (CAS-1643479-68-8) | | | |
| M527 | **42** | **89** (CAS-1454567-05-5) | M528 | **42** | **90** (CAS-1352040-89-1) |
| M529 | **42** | **91** (CAS-1643479-47-3) | M530 | **42** | **92** (CAS-1643479-49-5) |
| M531 | **42** | **93** (CAS-1799958-78-3) | M532 | **42** | **94** (CAS-57102-51-9) |
| M533 | **42** | **95** | M534 | **42** | **96** (CAS-1427160-09-5) |
| M535 | **42** | **97** (CAS-1643479-72-4) | M536 | **42** | 98 |
| M537 | **42** | **99** | M538 | **42** | **100** (CAS-1643479-59-7) |
| M539 | **42** | **101** (CAS-1643479-68-8) | | | |

Ganz besonders bevorzugte Mischungen der Hostmaterialien der Formel (1e) mit den Hostmaterialien der Formel (2a) erhält man durch Kombination der Verbindungen **48, 50** und **59** der Tabelle 7 mit den Verbindungen **89** bis **101** der Tabelle 9, wie im Folgenden in Tabelle 12 gezeigt.

Tabelle 12:

| | | | | | |
|---|---|---|---|---|---|
| M605 | **48** | **89** (CAS-1454567-05-5) | M606 | **48** | **90** (CAS-1352040-89-1) |
| M607 | **48** | **91** (CAS-1643479-47-3) | M608 | **48** | **92** (CAS-1643479-49-5) |
| M609 | **48** | **93** (CAS-1799958-78-3) | M610 | **48** | **94** (CAS-57102-51-9) |
| M611 | **48** | **95** | M612 | **48** | **96** (CAS-1427160-09-5) |
| M613 | **48** | **97** (CAS-1643479-72-4) | M614 | **48** | **98** |
| M615 | **48** | **99** | M616 | **48** | **100** (CAS-1643479-59-7) |
| M617 | **48** | **101** (CAS-1643479-68-8) | | | |
| M631 | **50** | **89** (CAS-1454567-05-5) | M632 | **50** | **90** (CAS-1352040-89-1) |
| M633 | **50** | **91** (CAS-1643479-47-3) | M634 | **50** | **92** (CAS-1643479-49-5) |
| M635 | **50** | **93** (CAS-1799958-78-3) | M636 | **50** | **94** (CAS-57102-51-9) |
| M637 | **50** | **95** | M638 | **50** | **96** (CAS-1427160-09-5) |
| M639 | **50** | **97** (CAS-1643479-72-4) | M640 | **50** | 98 |
| M641 | **50** | **99** | M642 | **50** | **100** (CAS-1643479-59-7) |
| M643 | **50** | **101** (CAS-1643479-68-8) | | | |
| M748 | **59** | **89** (CAS-1454567-05-5) | M749 | **59** | **90** (CAS-1352040-89-1) |
| M750 | **59** | **91** (CAS-1643479-47-3) | M751 | **59** | **92** (CAS-1643479-49-5) |
| M752 | **59** | **93** (CAS-1799958-78-3) | M753 | **59** | **94** (CAS-57102-51-9) |
| M754 | **59** | **95** | M755 | **59** | **96** (CAS-1427160-09-5) |
| M756 | **59** | **97** (CAS-1643479-72-4) | M757 | **59** | **98** |
| M758 | **59** | **99** | M759 | **59** | **100** (CAS-1643479-59-7) |
| M760 | **59** | **101** (CAS-1643479-68-8) | | | |

Ganz besonders bevorzugte Mischungen der Hostmaterialien der Formel (1e) mit den Hostmaterialien der Formel (2a) erhält man durch Kombination der Verbindungen **70, 78** und **86** der Tabelle 8 mit den Verbindungen **89** bis **101** der Tabelle 9, wie im Folgenden in Tabelle 13 gezeigt.

Tabelle 13:

| | | | | | |
|---|---|---|---|---|---|
| M891 | **70** | **89** (CAS-1454567-05-5) | M892 | **70** | **90** (CAS-1352040-89-1) |
| M893 | **70** | **91** (CAS-1643479-47-3) | M894 | **70** | **92** (CAS-1643479-49-5) |
| M895 | **70** | **93** (CAS-1799958-78-3) | M896 | **70** | **94** (CAS-57102-51-9) |
| M897 | **70** | **95** | M898 | **70** | **96** (CAS-1427160-09-5) |
| M899 | **70** | **97** (CAS-1643479-72-4) | M900 | **70** | 98 |
| M901 | **70** | **99** | M902 | **70** | **100** (CAS-1643479-59-7) |
| M903 | **70** | **101** (CAS-1643479-68-8) | | | |
| M995 | **78** | **89** (CAS-1454567-05-5) | M996 | **78** | **90** (CAS-1352040-89-1) |
| M997 | **78** | **91** (CAS-1643479-47-3) | M998 | **78** | **92** (CAS-1643479-49-5) |
| M999 | **78** | **93** (CAS-1799958-78-3) | M100 0 | **78** | **94** (CAS-57102-51-9) |
| M1001 | **78** | **95** | M100 2 | **78** | **96** (CAS-1427160-09-5) |

(fortgesetzt)

| | | | | | |
|---|---|---|---|---|---|
| M1003 | **78** | **97** (CAS-1643479-72-4) | M100 4 | **78** | **98** |
| M1005 | **78** | **99** | M100 6 | **78** | **100** (CAS-1643479-59-7) |
| M1007 | **78** | **101** (CAS-1643479-68-8) | | | |
| M1099 | **86** | **89** (CAS-1454567-05-5) | M110 0 | **86** | **90** (CAS-1352040-89-1) |
| M1101 | **86** | **91** (CAS-1643479-47-3) | M110 2 | **86** | **92** (CAS-1643479-49-5) |
| M1103 | **86** | **93** (CAS-1799958-78-3) | M110 4 | **86** | **94** (CAS-57102-51-9) |
| M1105 | **86** | **95** | M110 6 | **86** | **96** (CAS-1427160-09-5) |
| M1107 | **86** | **97** (CAS-1643479-72-4) | M110 8 | **86** | **98** |
| M1109 | **86** | **99** | M111 0 | **86** | **100** (CAS-1643479-59-7) |
| M1111 | **86** | **101** (CAS-1643479-68-8) | | | |

Die Konzentration des elektronentransportierenden Hosts der Formel (1e), wie zuvor beschrieben oder bevorzugt beschrieben, in der erfindungsgemäßen Zusammensetzung liegt im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-%, bezogen auf die gesamte Zusammensetzung.

Die Konzentration des lochtransportierenden Hosts der Formel (2a), wie zuvor beschrieben oder als bevorzugt beschrieben, in der Zusammensetzung liegt im Bereich von 10 Gew.-% bis 95 Gew.-%, bevorzugt im Bereich von 15 Gew.-% bis 90 Gew.-%, mehr bevorzugt im Bereich von 15 Gew.-% bis 80 Gew.-%, noch mehr bevorzugt im Bereich von 20 Gew.-% bis 70 Gew.-%, ganz besonders bevorzugt im Bereich von 40 Gew.-% bis 80 Gew.-% und am meisten bevorzugt im Bereich von 50 Gew.-% bis 70 Gew.-%, bezogen auf die gesamte Zusammensetzung.

Die Konzentration des lochtransportierenden Hosts der Formel (2a), wie zuvor beschrieben oder als bevorzugt beschrieben, in der emittierenden Schicht liegt bevorzugt im Bereich von 40 % bis 45 % Volumenanteil bezogen auf alle Bestandteile der emittierenden Schicht; die Konzentration des elektronentransportierenden Hosts der Formel (1e), wie zuvor beschrieben oder als bevorzugt beschrieben, in der emittierenden Schicht liegt bevorzugt im Bereich von 40 % bis 45 % Volumenanteil bezogen auf alle Bestandteile der emittierenden Schicht.

Bei Emitterkonzentrationen von kleiner 10% Volumenanteil in der emittierenden Schicht ist der Volumenanteil der lochtransportierenden Verbindungen der Formel (2a) bevorzugt höher als der Volumenanteil der elektronentransportierenden Verbindungen der Formel (1e), wie zuvor beschrieben oder bevorzugt beschrieben, bezogen auf alle Bestandteile der emittierenden Schicht. Der Volumenanteil der lochtransportierenden Verbindungen der Formel (2a), wie zuvor beschrieben oder bevorzugt beschrieben, in dieser Ausführungsform ist bevorzugt bei einem Volumenanteil von 65 bis 75%, bezogen auf alle Bestandteile der emittierenden Schicht.

Die erfindungsgemäße Zusammensetzung kann in einer weiteren bevorzugten Ausführungsform neben mindestens einer Verbindung der Formel (1e), wie zuvor beschrieben oder als bevorzugt beschrieben, als elektronentransportierender Host bzw. elektronentransportierendes Matrixmaterial, und mindestens einer Verbindung der Formel (2a), wie zuvor beschrieben oder als bevorzugt beschrieben, als lochtransportierender Host bzw. als lochtransportierendes Matrixmaterial, noch weitere Verbindungen, insbesondere organische funktionelle Materialien, enthalten. Die Zusammensetzung bildet in dieser Ausführungsform bevorzugt eine organische Schicht in einer elektronischen Vorrichtung, wie nachfolgend beschrieben.

Die vorliegende Erfindung betrifft daher auch eine Zusammensetzung, die neben den vorstehend genannten Materialien mindestens noch eine weitere Verbindung enthält, die ausgewählt ist aus der Gruppe bestehend aus Lochinjektionsmaterialien, Lochtransportmaterialien, Lochblockiermaterialien, *wide-band-gap*-Materialien, fluoreszierenden Emittern, phosphoreszierenden Emittern, Hostmaterialien, Elektronenblockiermaterialien, Elektronentransportmaterialien und Elektroneninjektionsmaterialien, n-Dotanden und p-Dotanden. Es bereitet dem Fachmann dabei keinerlei Schwierigkeiten, diese aus einer Vielzahl ihm bekannter Materialien auszuwählen.

Unter n-Dotanden werden hierin Reduktionsmittel, d.h. Elektronendonatoren verstanden. Bevorzugte Beispiele für n-Dotanden sind $W(hpp)_4$ und weitere elektronenreiche Metallkomplexe gemäß WO 2005/086251 A2, P=N-Verbindungen (z.B. WO 2012/175535 A1, WO 2012/175219 A1), Naphthylencarbodiimide (z.B. WO 2012/168358 A1), Fluorene (z.B. WO 2012/031735 A1), Radikale und Diradikale (z.B. EP 1837926 A1, WO 2007/107306 A1), Pyridine (z.B. EP 2452946 A1, EP 2463927 A1), N-heterocyclische Verbindungen (z.B. WO 2009/000237 A1) und Acridine sowie Phenazine (z.B. US 2007/145355 A1).

Unter p-Dotanden werden hierin Oxidationsmittel, d.h. Elektronenakzeptoren verstanden. Bevorzugte Beispiele für p-Dotanden sind $F_4$-TCNQ, $F_6$-TNAP, NDP-2 (Fa. Novaled), NDP-9 (Fa. Novaled), Chinone (z.B. EP 1538684 A1, WO 2006/081780 A1, WO 2009/003455 A1, WO 2010/097433 A1), Radialene (z.B. EP 1988587 A1, US 2010/102709 A1, EP 2180029 A1, WO 2011/131185A1, WO 2011134458 A1, US 2012/223296 A1), S-haltige Übergangsmetallkomplexe (z.B. WO 2007/134873 A1, WO 2008/061517 A2, WO 2008/061518 A2, DE 102008051737 A1, WO 2009/089821 A1, US 2010/096600 A1), Bisimidazole (z.B. WO 2008/138580 A1), Phthalocyanine (z.B. WO 2008/058525 A2), Bora-Tetraazapentalene (z.B. WO 2007/115540 A1) Fullerene (z.B. DE 102010046040 A1) und Hauptgruppenhalogenide (z.B. WO 2008/128519 A2).

Unter *wide-band-gap*-Material wird hierin ein Material im Sinne der Offenbarung von US 7,294,849 verstanden, das durch eine Bandlücke von mindestens 3.5 eV charakterisiert ist, wobei unter Bandlücke der Abstand zwischen HOMO und LUMO-Energie eines Materials verstanden wird.

Es ist bevorzugt, wenn die erfindungsgemäße Zusammensetzung umfassend einen bipolaren Host und einen elektronentransportierenden Host zusätzlich wenigstens eine lichtemittierende Verbindung bzw. einen Emitter enthält, wobei phosphoreszierende Emitter besonders bevorzugt sind.

Vom Begriff phosphoreszierende Emitter sind typischerweise Verbindungen umfasst, bei denen die Lichtemission durch einen spin-verbotenen Übergang aus einem angeregten Zustand mit höherer Spinmultiplizität, also einem Spinzustand > 1, erfolgt, beispielsweise durch einen Übergang aus einem Triplett-Zustand oder einem Zustand mit einer noch höheren Spinquantenzahl, beispielsweise einem Quintett-Zustand. Bevorzugt wird hierbei ein Übergang aus einem Triplett-Zustand verstanden.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Emitter angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind.

Beispiele der oben beschriebenen Emitter können den Anmeldungen WO 2016/015815, WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2015/036074, WO 2015/117718 und WO 2016/015815 entnommen werden.

Bevorzugte Beispiele von phosphoreszierenden Emittern sind in der folgenden Tabelle 14 aufgeführt.

Tabelle 14:

| | | |
|---|---|---|
| 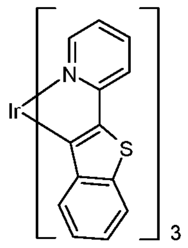 | 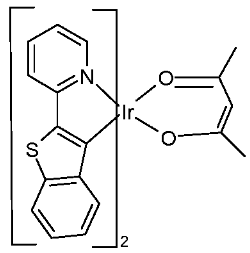 | 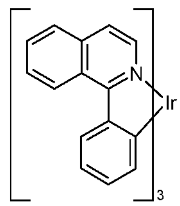 |
| 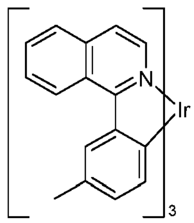 | 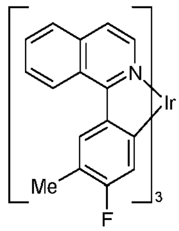 | 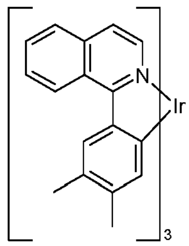 |
| 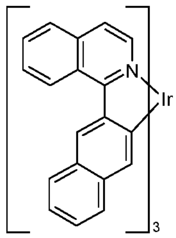 | 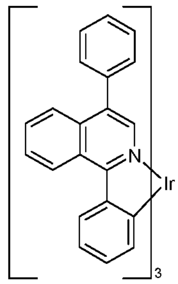 | 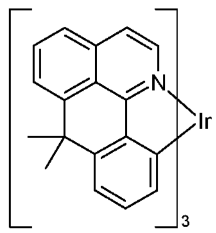 |

| | | |
|---|---|---|
| 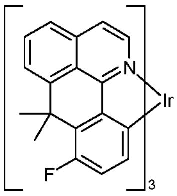 | 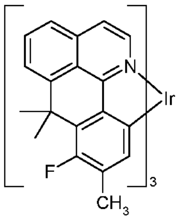 | 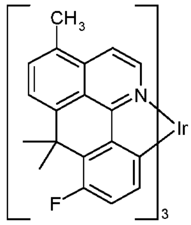 |
| 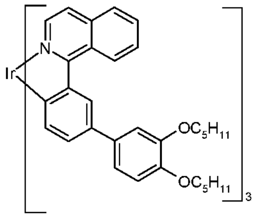 | 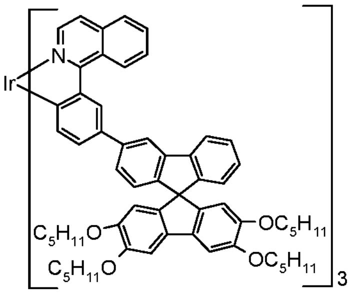 | 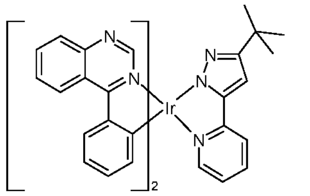 |
| 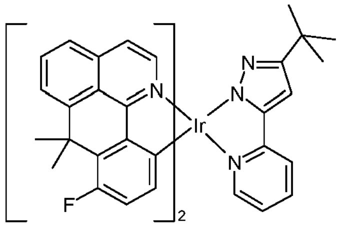 | 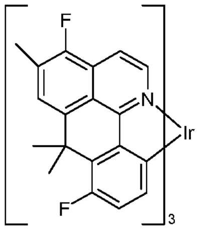 | 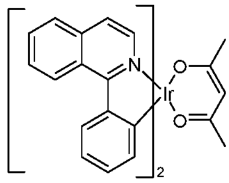 |
| 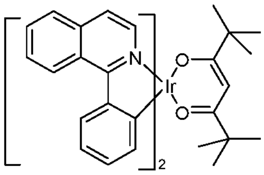 | 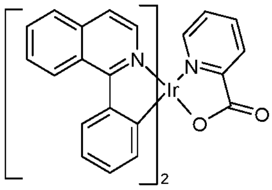 | 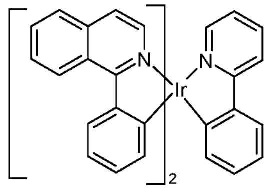 |
| 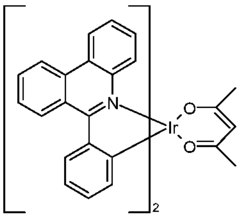 | 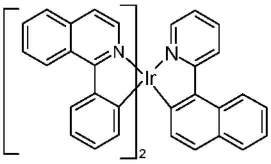 | 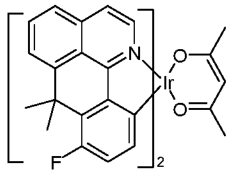 |
| 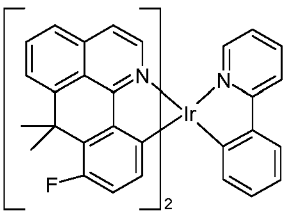 | 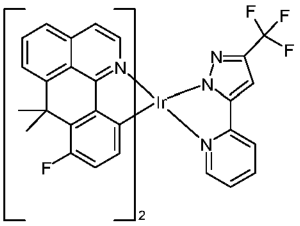 | 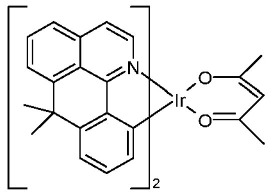 |
| 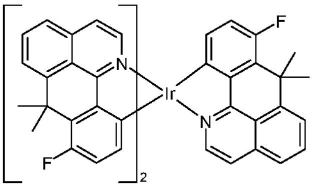 | 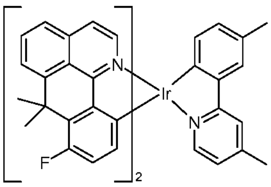 | 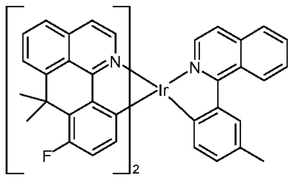 |

| | | |
|---|---|---|
| 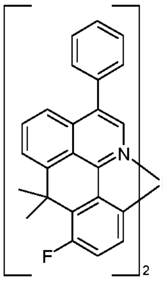 | 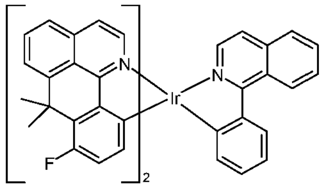 | 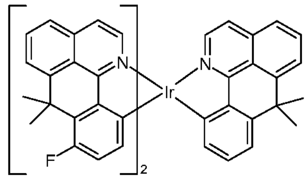 |
| 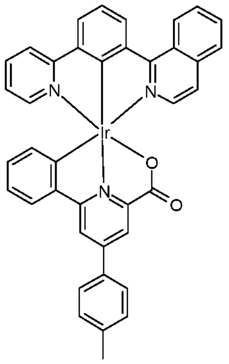 | 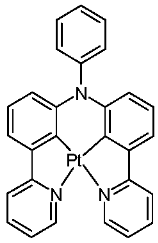 | 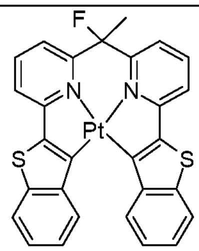 |
| 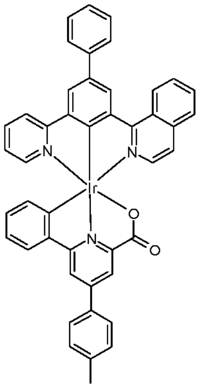 | 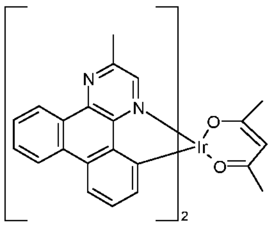 | 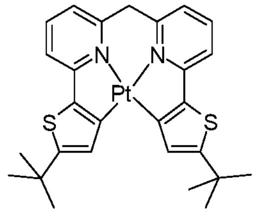 |
| 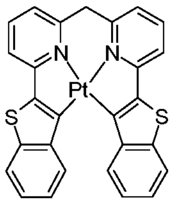 | 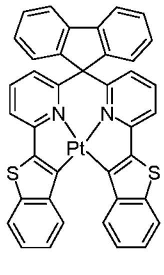 | 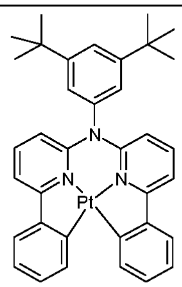 |
| 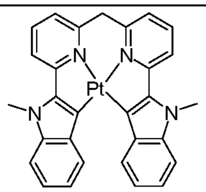 | 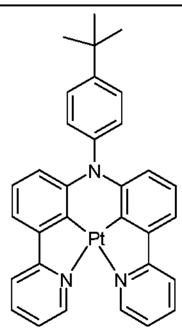 | 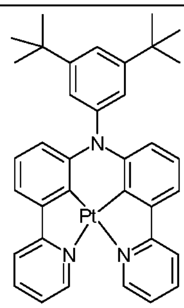 |

| | | |
|---|---|---|
| 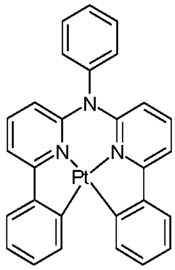 | 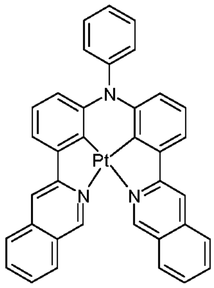 | |
| 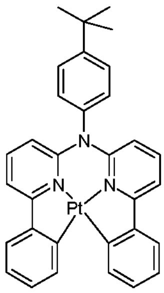 | 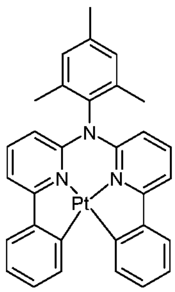 | 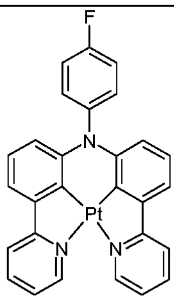 |
| 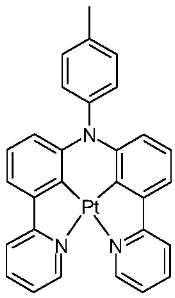 | 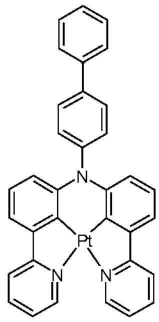 | 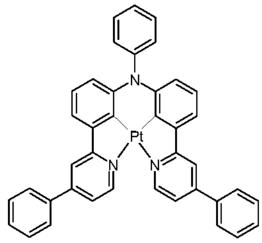 |
| 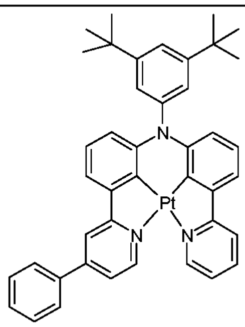 | 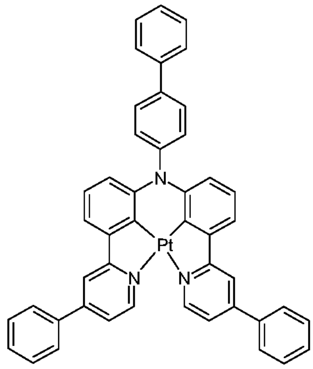 | 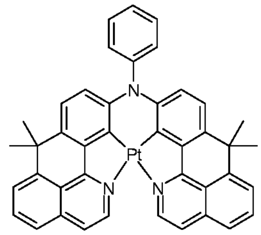 |
| 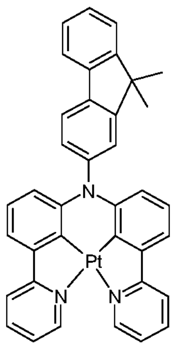 | 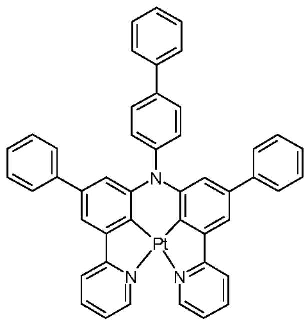 | 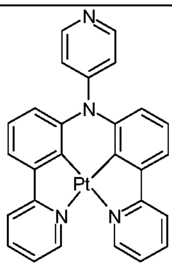 |

| | | |
|---|---|---|
| 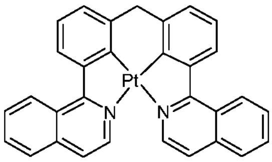 | 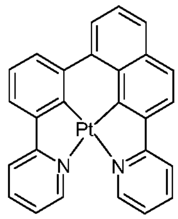 | 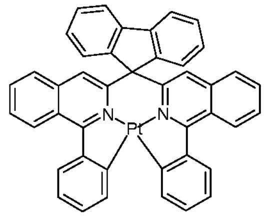 |
| 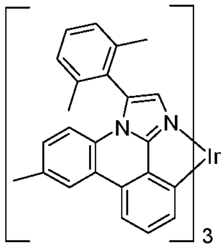 | 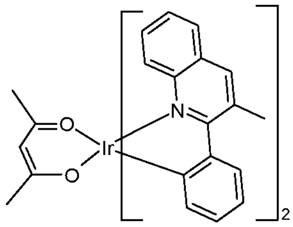 | 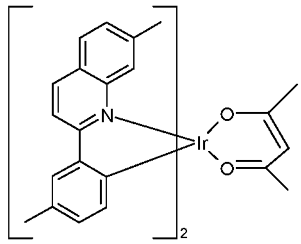 |
| 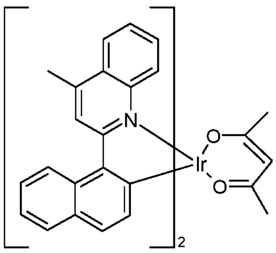 | 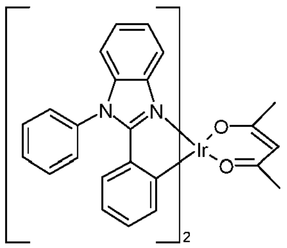 | 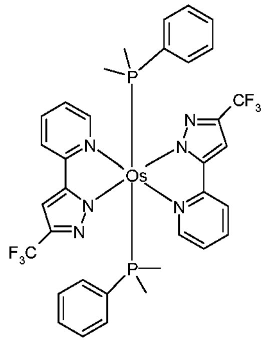 |
| 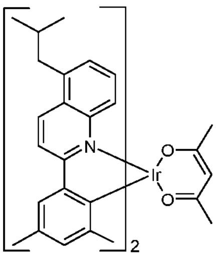 | 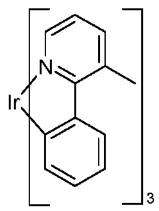 | 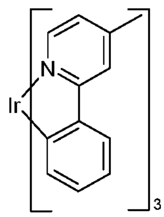 |
| 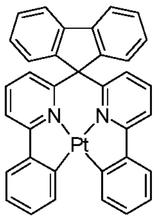 | 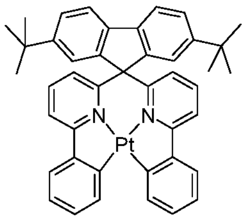 | 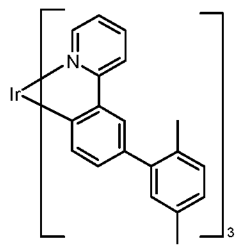 |
| 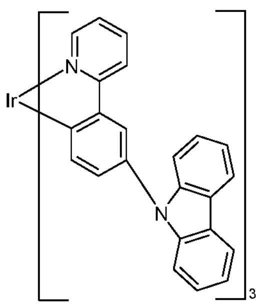 | 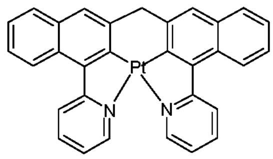 | 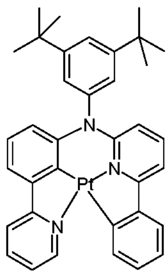 |

| | | |
|---|---|---|
| 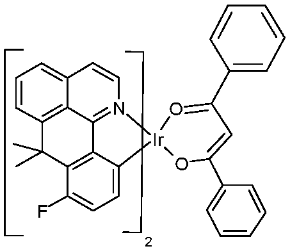 | 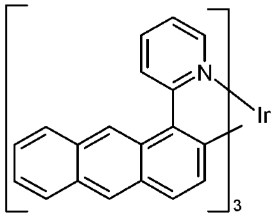 | 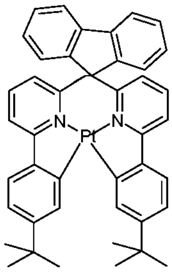 |
| 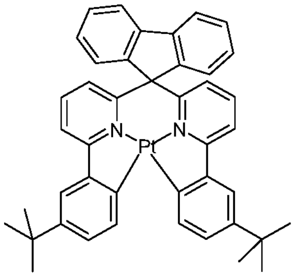 | 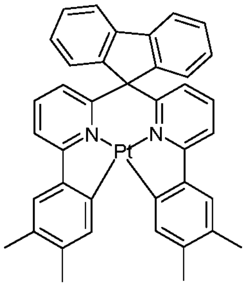 | 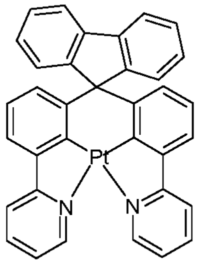 |
| 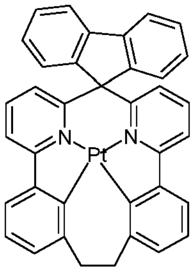 | 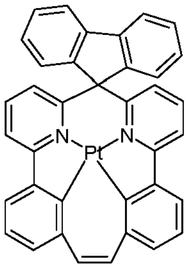 | 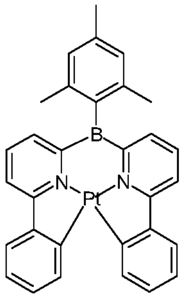 |
| 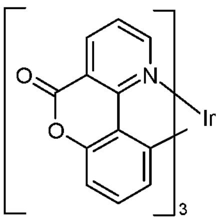 | 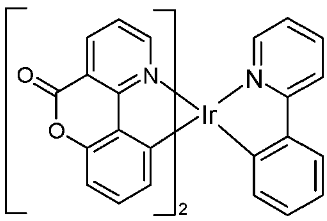 | 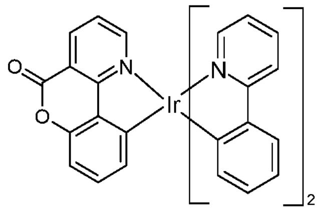 |
| 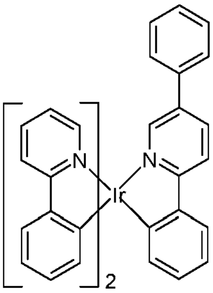 | 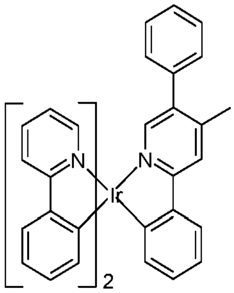 TEG1 | 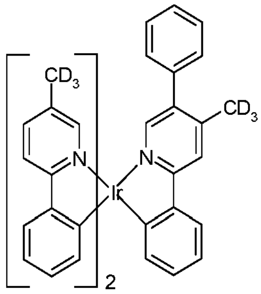 |
| 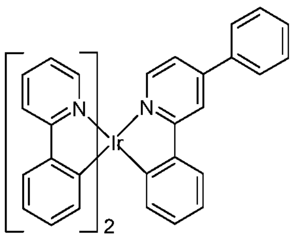 | 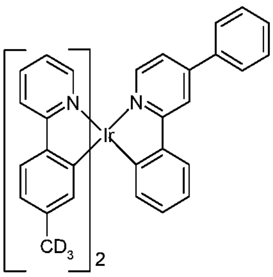 | 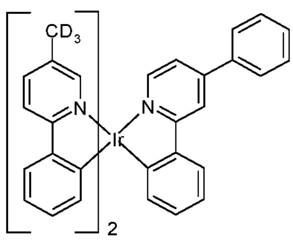 |

| | | |
|---|---|---|
| 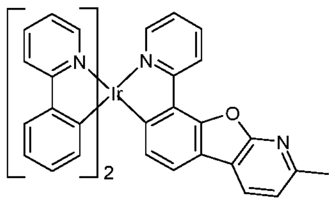 | 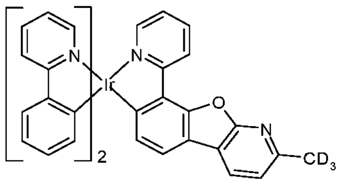 | 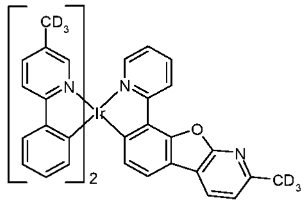 |
| 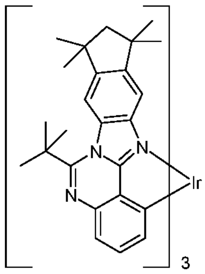 | 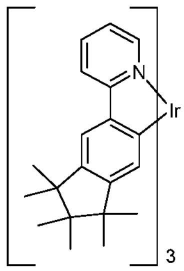 | 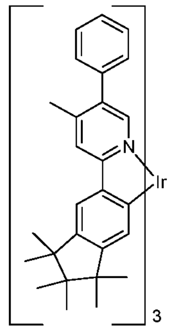 |
| 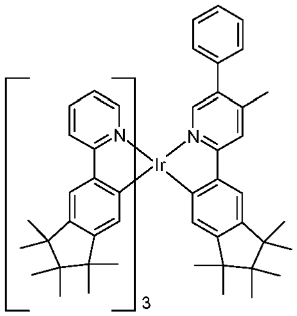 | 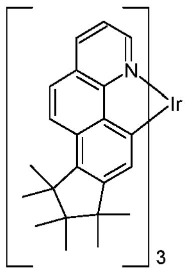 | 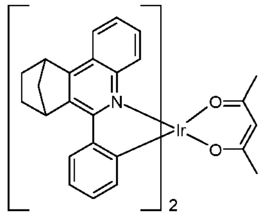 |

Bevorzugte Beispiele von phosphoreszierenden polypodalen Emittern sind in der folgenden Tabelle 15 aufgeführt.

Tabelle 15:

| | | | |
|---|---|---|---|
| CAS-1269508-30-6 | CAS-1989601-68-4 | CAS-1989602-19-8 | CAS-1989602-70-1 |
| CAS-1215692-34-4 | CAS-1989601-69-5 | CAS-1989602-20-1 | CAS-1989602-71-2 |
| CAS-1370364-40-1 | CAS-1989601-70-8 | CAS-1989602-21-2 | CAS-1989602-72-3 |
| CAS-1370364-42-3 | CAS-1989601-71-9 | CAS-1989602-22-3 | CAS-1989602-73-4 |
| CAS-1989600-74-9 | CAS-1989601-72-0 | CAS-1989602-23-4 | CAS-1989602-74-5 |
| CAS-1989600-75-0 | CAS-1989601-73-1 | CAS-1989602-24-5 | CAS-1989602-75-6 |
| CAS-1989600-77-2 | CAS-1989601-74-2 | CAS-1989602-25-6 | CAS-1989602-76-7 |
| CAS-1989600-78-3 | CAS-1989601-75-3 | CAS-1989602-26-7 | CAS-1989602-77-8 |
| CAS-1989600-79-4 | CAS-1989601-76-4 | CAS-1989602-27-8 | CAS-1989602-78-9 |
| CAS-1989600-82-9 | CAS-1989601-77-5 | CAS-1989602-28-9 | CAS-1989602-79-0 |
| CAS-1989600-83-0 | CAS-1989601-78-6 | CAS-1989602-29-0 | CAS-1989602-80-3 |
| CAS-1989600-84-1 | CAS-1989601-79-7 | CAS-1989602-30-3 | CAS-1989602-82-5 |
| CAS-1989600-85-2 | CAS-1989601-80-0 | CAS-1989602-31-4 | CAS-1989602-84-7 |
| CAS-1989600-86-3 | CAS-1989601-81-1 | CAS-1989602-32-5 | CAS-1989602-85-8 |
| CAS-1989600-87-4 | CAS-1989601-82-2 | CAS-1989602-33-6 | CAS-1989602-86-9 |
| CAS-1989600-88-5 | CAS-1989601-83-3 | CAS-1989602-34-7 | CAS-1989602-87-0 |

(fortgesetzt)

| | | | |
|---|---|---|---|
| CAS-1989600-89-6 | CAS-1989601-84-4 | CAS-1989602-35-8 | CAS-1989602-88-1 |
| CAS-1989601-11-7 | CAS-1989601-85-5 | CAS-1989602-36-9 | CAS-1989604-00-3 |
| CAS-1989601-23-1 | CAS-1989601-86-6 | CAS-1989602-37-0 | CAS-1989604-01-4 |
| CAS-1989601-26-4 | CAS-1989601-87-7 | CAS-1989602-38-1 | CAS-1989604-02-5 |
| CAS-1989601-28-6 | CAS-1989601-88-8 | CAS-1989602-39-2 | CAS-1989604-03-6 |
| CAS-1989601-29-7 | CAS-1989601-89-9 | CAS-1989602-40-5 | CAS-1989604-04-7 |
| CAS-1989601-33-3 | CAS-1989601-90-2 | CAS-1989602-41-6 | CAS-1989604-05-8 |
| CAS-1989601-40-2 | CAS-1989601-91-3 | CAS-1989602-42-7 | CAS-1989604-06-9 |
| CAS-1989601-41-3 | CAS-1989601-92-4 | CAS-1989602-43-8 | CAS-1989604-07-0 |
| CAS-1989601-42-4 | CAS-1989601-93-5 | CAS-1989602-44-9 | CAS-1989604-08-1 |
| CAS-1989601-43-5 | CAS-1989601-94-6 | CAS-1989602-45-0 | CAS-1989604-09-2 |
| CAS-1989601-44-6 | CAS-1989601-95-7 | CAS-1989602-46-1 | CAS-1989604-10-5 |
| CAS-1989601-45-7 | CAS-1989601-96-8 | CAS-1989602-47-2 | CAS-1989604-11-6 |
| CAS-1989601-46-8 | CAS-1989601-97-9 | CAS-1989602-48-3 | CAS-1989604-13-8 |
| CAS-1989601-47-9 | CAS-1989601-98-0 | CAS-1989602-49-4 | CAS-1989604-14-9 |
| CAS-1989601-48-0 | CAS-1989601-99-1 | CAS-1989602-50-7 | CAS-1989604-15-0 |
| CAS-1989601-49-1 | CAS-1989602-00-7 | CAS-1989602-51-8 | CAS-1989604-16-1 |
| CAS-1989601-50-4 | CAS-1989602-01-8 | CAS-1989602-52-9 | CAS-1989604-17-2 |
| CAS-1989601-51-5 | CAS-1989602-02-9 | CAS-1989602-53-0 | CAS-1989604-18-3 |
| CAS-1989601-52-6 | CAS-1989602-03-0 | CAS-1989602-54-1 | CAS-1989604-19-4 |
| CAS-1989601-53-7 | CAS-1989602-04-1 | CAS-1989602-55-2 | CAS-1989604-20-7 |
| CAS-1989601-54-8 | CAS-1989602-05-2 | CAS-1989602-56-3 | CAS-1989604-21-8 |
| CAS-1989601-55-9 | CAS-1989602-06-3 | CAS-1989602-57-4 | CAS-1989604-22-9 |
| CAS-1989601-56-0 | CAS-1989602-07-4 | CAS-1989602-58-5 | CAS-1989604-23-0 |
| CAS-1989601-57-1 | CAS-1989602-08-5 | CAS-1989602-59-6 | CAS-1989604-24-1 |
| CAS-1989601-58-2 | CAS-1989602-09-6 | CAS-1989602-60-9 | CAS-1989604-25-2 |
| CAS-1989601-59-3 | CAS-1989602-10-9 | CAS-1989602-61-0 | CAS-1989604-26-3 |
| CAS-1989601-60-6 | CAS-1989602-11-0 | CAS-1989602-62-1 | CAS-1989604-27-4 |
| CAS-1989601-61-7 | CAS-1989602-12-1 | CAS-1989602-63-2 | CAS-1989604-28-5 |
| CAS-1989601-62-8 | CAS-1989602-13-2 | CAS-1989602-64-3 | CAS-1989604-29-6 |
| CAS-1989601-63-9 | CAS-1989602-14-3 | CAS-1989602-65-4 | CAS-1989604-30-9 |
| CAS-1989601-64-0 | CAS-1989602-15-4 | CAS-1989602-66-5 | CAS-1989604-31-0 |
| CAS-1989601-65-1 | CAS-1989602-16-5 | CAS-1989602-67-6 | CAS-1989604-32-1 |
| CAS-1989601-66-2 | CAS-1989602-17-6 | CAS-1989602-68-7 | CAS-1989604-33-2 |
| CAS-1989601-67-3 | CAS-1989602-18-7 | CAS-1989602-69-8 | CAS-1989604-34-3 |
| CAS-1989604-35-4 | CAS-1989604-88-7 | CAS-1989605-52-8 | CAS-1989606-07-6 |
| CAS-1989604-36-5 | CAS-1989604-89-8 | CAS-1989605-53-9 | CAS-1989606-08-7 |
| CAS-1989604-37-6 | CAS-1989604-90-1 | CAS-1989605-54-0 | CAS-1989606-09-8 |
| CAS-1989604-38-7 | CAS-1989604-92-3 | CAS-1989605-55-1 | CAS-1989606-10-1 |

(fortgesetzt)

| | | | |
|---|---|---|---|
| CAS-1989604-39-8 | CAS-1989604-93-4 | CAS-1989605-56-2 | CAS-1989606-11-2 |
| CAS-1989604-40-1 | CAS-1989604-94-5 | CAS-1989605-57-3 | CAS-1989606-12-3 |
| CAS-1989604-41-2 | CAS-1989604-95-6 | CAS-1989605-58-4 | CAS-1989606-13-4 |
| CAS-1989604-42-3 | CAS-1989604-96-7 | CAS-1989605-59-5 | CAS-1989606-14-5 |
| CAS-1989604-43-4 | CAS-1989604-97-8 | CAS-1989605-61-9 | CAS-1989606-15-6 |
| CAS-1989604-45-6 | CAS-1989605-09-5 | CAS-1989605-62-0 | CAS-1989606-16-7 |
| CAS-1989604-46-7 | CAS-1989605-10-8 | CAS-1989605-63-1 | CAS-1989606-17-8 |
| CAS-1989604-47-8 | CAS-1989605-11-9 | CAS-1989605-64-2 | CAS-1989606-18-9 |
| CAS-1989604-48-9 | CAS-1989605-13-1 | CAS-1989605-65-3 | CAS-1989606-19-0 |
| CAS-1989604-49-0 | CAS-1989605-14-2 | CAS-1989605-66-4 | CAS-1989606-20-3 |
| CAS-1989604-50-3 | CAS-1989605-15-3 | CAS-1989605-67-5 | CAS-1989606-21-4 |
| CAS-1989604-52-5 | CAS-1989605-16-4 | CAS-1989605-68-6 | CAS-1989606-22-5 |
| CAS-1989604-53-6 | CAS-1989605-17-5 | CAS-1989605-69-7 | CAS-1989606-23-6 |
| CAS-1989604-54-7 | CAS-1989605-18-6 | CAS-1989605-70-0 | CAS-1989606-24-7 |
| CAS-1989604-55-8 | CAS-1989605-19-7 | CAS-1989605-71-1 | CAS-1989606-26-9 |
| CAS-1989604-56-9 | CAS-1989605-20-0 | CAS-1989605-72-2 | CAS-1989606-27-0 |
| CAS-1989604-57-0 | CAS-1989605-21-1 | CAS-1989605-73-3 | CAS-1989606-28-1 |
| CAS-1989604-58-1 | CAS-1989605-22-2 | CAS-1989605-74-4 | CAS-1989606-29-2 |
| CAS-1989604-59-2 | CAS-1989605-23-3 | CAS-1989605-75-5 | CAS-1989606-30-5 |
| CAS-1989604-60-5 | CAS-1989605-24-4 | CAS-1989605-76-6 | CAS-1989606-31-6 |
| CAS-1989604-61-6 | CAS-1989605-25-5 | CAS-1989605-77-7 | CAS-1989606-32-7 |
| CAS-1989604-62-7 | CAS-1989605-26-6 | CAS-1989605-78-8 | CAS-1989606-33-8 |
| CAS-1989604-63-8 | CAS-1989605-27-7 | CAS-1989605-79-9 | CAS-1989606-34-9 |
| CAS-1989604-64-9 | CAS-1989605-28-8 | CAS-1989605-81-3 | CAS-1989606-35-0 |
| CAS-1989604-65-0 | CAS-1989605-29-9 | CAS-1989605-82-4 | CAS-1989606-36-1 |
| CAS-1989604-66-1 | CAS-1989605-30-2 | CAS-1989605-83-5 | CAS-1989606-37-2 |
| CAS-1989604-67-2 | CAS-1989605-31-3 | CAS-1989605-84-6 | CAS-1989606-38-3 |
| CAS-1989604-68-3 | CAS-1989605-32-4 | CAS-1989605-85-7 | CAS-1989606-39-4 |
| CAS-1989604-69-4 | CAS-1989605-33-5 | CAS-1989605-86-8 | CAS-1989606-40-7 |
| CAS-1989604-70-7 | CAS-1989605-34-6 | CAS-1989605-87-9 | CAS-1989606-41-8 |
| CAS-1989604-71-8 | CAS-1989605-35-7 | CAS-1989605-88-0 | CAS-1989606-42-9 |
| CAS-1989604-72-9 | CAS-1989605-36-8 | CAS-1989605-89-1 | CAS-1989606-43-0 |
| CAS-1989604-73-0 | CAS-1989605-37-9 | CAS-1989605-90-4 | CAS-1989606-44-1 |
| CAS-1989604-74-1 | CAS-1989605-38-0 | CAS-1989605-91-5 | CAS-1989606-45-2 |
| CAS-1989604-75-2 | CAS-1989605-39-1 | CAS-1989605-92-6 | CAS-1989606-46-3 |
| CAS-1989604-76-3 | CAS-1989605-40-4 | CAS-1989605-93-7 | CAS-1989606-48-5 |
| CAS-1989604-77-4 | CAS-1989605-41-5 | CAS-1989605-94-8 | CAS-1989606-49-6 |
| CAS-1989604-78-5 | CAS-1989605-42-6 | CAS-1989605-95-9 | CAS-1989606-53-2 |
| CAS-1989604-79-6 | CAS-1989605-43-7 | CAS-1989605-96-0 | CAS-1989606-55-4 |

(fortgesetzt)

| | | | |
|---|---|---|---|
| CAS-1989604-80-9 | CAS-1989605-44-8 | CAS-1989605-97-1 | CAS-1989606-56-5 |
| CAS-1989604-81-0 | CAS-1989605-45-9 | CAS-1989605-98-2 | CAS-1989606-61-2 |
| CAS-1989604-82-1 | CAS-1989605-46-0 | CAS-1989605-99-3 | CAS-1989606-62-3 |
| CAS-1989604-83-2 | CAS-1989605-47-1 | CAS-1989606-00-9 | CAS-1989606-63-4 |
| CAS-1989604-84-3 | CAS-1989605-48-2 | CAS-1989606-01-0 | CAS-1989606-67-8 |
| CAS-1989604-85-4 | CAS-1989605-49-3 | CAS-1989606-04-3 | CAS-1989606-69-0 |
| CAS-1989604-86-5 | CAS-1989605-50-6 | CAS-1989606-05-4 | CAS-1989606-70-3 |
| CAS-1989604-87-6 | CAS-1989605-51-7 | CAS-1989606-06-5 | CAS-1989606-74-7 |
| CAS-1989658-39-0 | CAS-2088184-56-7 | CAS-2088185-07-1 | CAS-2088185-66-2 |
| CAS-1989658-41-4 | CAS-2088184-57-8 | CAS-2088185-08-2 | CAS-2088185-67-3 |
| CAS-1989658-43-6 | CAS-2088184-58-9 | CAS-2088185-09-3 | CAS-2088185-68-4 |
| CAS-1989658-47-0 | CAS-2088184-59-0 | CAS-2088185-10-6 | CAS-2088185-69-5 |
| CAS-1989658-49-2 | CAS-2088184-60-3 | CAS-2088185-11-7 | CAS-2088185-70-8 |
| CAS-2088184-07-8 | CAS-2088184-61-4 | CAS-2088185-12-8 | CAS-2088185-71-9 |
| CAS-2088184-08-9 | CAS-2088184-62-5 | CAS-2088185-13-9 | CAS-2088185-72-0 |
| CAS-2088184-09-0 | CAS-2088184-63-6 | CAS-2088185-14-0 | CAS-2088185-73-1 |
| CAS-2088184-10-3 | CAS-2088184-64-7 | CAS-2088185-15-1 | CAS-2088185-74-2 |
| CAS-2088184-11-4 | CAS-2088184-65-8 | CAS-2088185-16-2 | CAS-2088185-75-3 |
| CAS-2088184-13-6 | CAS-2088184-66-9 | CAS-2088185-17-3 | CAS-2088185-76-4 |
| CAS-2088184-14-7 | CAS-2088184-67-0 | CAS-2088185-18-4 | CAS-2088185-77-5 |
| CAS-2088184-15-8 | CAS-2088184-68-1 | CAS-2088185-19-5 | CAS-2088185-78-6 |
| CAS-2088184-16-9 | CAS-2088184-69-2 | CAS-2088185-20-8 | CAS-2088185-79-7 |
| CAS-2088184-17-0 | CAS-2088184-70-5 | CAS-2088185-21-9 | CAS-2088185-80-0 |
| CAS-2088184-18-1 | CAS-2088184-71-6 | CAS-2088185-22-0 | CAS-2088185-81-1 |
| CAS-2088184-19-2 | CAS-2088184-72-7 | CAS-2088185-23-1 | CAS-2088185-82-2 |
| CAS-2088184-20-5 | CAS-2088184-73-8 | CAS-2088185-32-2 | CAS-2088185-83-3 |
| CAS-2088184-21-6 | CAS-2088184-74-9 | CAS-2088185-33-3 | CAS-2088185-84-4 |
| CAS-2088184-22-7 | CAS-2088184-75-0 | CAS-2088185-34-4 | CAS-2088185-85-5 |
| CAS-2088184-23-8 | CAS-2088184-76-1 | CAS-2088185-35-5 | CAS-2088185-86-6 |
| CAS-2088184-24-9 | CAS-2088184-77-2 | CAS-2088185-36-6 | CAS-2088185-87-7 |
| CAS-2088184-25-0 | CAS-2088184-78-3 | CAS-2088185-37-7 | CAS-2088185-88-8 |
| CAS-2088184-26-1 | CAS-2088184-79-4 | CAS-2088185-38-8 | CAS-2088185-89-9 |
| CAS-2088184-27-2 | CAS-2088184-80-7 | CAS-2088185-39-9 | CAS-2088185-90-2 |
| CAS-2088184-28-3 | CAS-2088184-81-8 | CAS-2088185-40-2 | CAS-2088185-91-3 |
| CAS-2088184-29-4 | CAS-2088184-82-9 | CAS-2088185-41-3 | CAS-2088185-92-4 |
| CAS-2088184-30-7 | CAS-2088184-83-0 | CAS-2088185-42-4 | CAS-2088185-93-5 |
| CAS-2088184-32-9 | CAS-2088184-84-1 | CAS-2088185-43-5 | CAS-2088185-94-6 |
| CAS-2088184-34-1 | CAS-2088184-85-2 | CAS-2088185-44-6 | CAS-2088185-95-7 |
| CAS-2088184-35-2 | CAS-2088184-86-3 | CAS-2088185-45-7 | CAS-2088185-96-8 |

(fortgesetzt)

| | | | |
|---|---|---|---|
| CAS-2088184-36-3 | CAS-2088184-87-4 | CAS-2088185-46-8 | CAS-2088185-97-9 |
| CAS-2088184-37-4 | CAS-2088184-88-5 | CAS-2088185-47-9 | CAS-2088185-98-0 |
| CAS-2088184-38-5 | CAS-2088184-89-6 | CAS-2088185-48-0 | CAS-2088185-99-1 |
| CAS-2088184-39-6 | CAS-2088184-90-9 | CAS-2088185-49-1 | CAS-2088186-00-7 |
| CAS-2088184-40-9 | CAS-2088184-91-0 | CAS-2088185-50-4 | CAS-2088186-01-8 |
| CAS-2088184-41-0 | CAS-2088184-92-1 | CAS-2088185-51-5 | CAS-2088186-02-9 |
| CAS-2088184-42-1 | CAS-2088184-93-2 | CAS-2088185-52-6 | CAS-2088195-88-2 |
| CAS-2088184-43-2 | CAS-2088184-94-3 | CAS-2088185-53-7 | CAS-2088195-89-3 |
| CAS-2088184-44-3 | CAS-2088184-95-4 | CAS-2088185-54-8 | CAS-2088195-90-6 |
| CAS-2088184-45-4 | CAS-2088184-96-5 | CAS-2088185-55-9 | CAS-2088195-91-7 |
| CAS-2088184-46-5 | CAS-2088184-97-6 | CAS-2088185-56-0 | CAS-861806-70-4 |
| CAS-2088184-47-6 | CAS-2088184-98-7 | CAS-2088185-57-1 | CAS-1269508-30-6 |
| CAS-2088184-48-7 | CAS-2088184-99-8 | CAS-2088185-58-2 | |
| CAS-2088184-49-8 | CAS-2088185-00-4 | CAS-2088185-59-3 | |
| CAS-2088184-50-1 | CAS-2088185-01-5 | CAS-2088185-60-6 | |
| CAS-2088184-51-2 | CAS-2088185-02-6 | CAS-2088185-61-7 | |
| CAS-2088184-52-3 | CAS-2088185-03-7 | CAS-2088185-62-8 | |
| CAS-2088184-53-4 | CAS-2088185-04-8 | CAS-2088185-63-9 | |
| CAS-2088184-54-5 | CAS-2088185-05-9 | CAS-2088185-64-0 | |
| CAS-2088184-55-6 | CAS-2088185-06-0 | CAS-2088185-65-1 | |

In den erfindungsgemäßen Zusammensetzung wird bevorzugt jede Mischung M67, M68, M69, M70, M71, M72, M73, M74, M75, M76, M77, M78, M79, M106, M107, M108, M109, M110, M111, M112, M113, M114, M115, M116, M117, M118, M241, M242, M243, M244, M245, M246, M247, M248, M249, M250, M251, M252, M253, M319, M320, M321, M322, M323, M324, M325, M326, M327, M328, M329, M330, M331, M423, M424, M425, M426, M427, M428, M429, M430, M431, M432, M433, M434, M435, M527, M528, M529, M530, M531, M532, M533, M534, M535, M536, M537, M538, M539, M605, M606, M607, M608, M609, M610, M611, M612, M613, M614, M615, M616, M617, M748, M749, M750, M751, M752, M753, M754, M755, M756, M757, M758, M759, M760, M891, M892, M893, M894, M895, M896, M897, M898, M899, M900, M901, M902, M903, M995, M996, M997, M998, M999, M1000, M1001, M1002, M1003, M1004, M1005, M1006, M1007, M1099, M1100, M1101, M1102, M1103, M1104, M1105, M1106, M1107, M1108, M1109, M1110 oder M1111 mit einer Verbindung aus Tabelle 14 oder 15 kombiniert.

Die erfindungsgemäße Zusammensetzung enthaltend mindestens einen phosphoreszierenden Emitter bildet bevorzugt eine infrarot emittierende, gelb, orange, rot, grün, blau oder ultraviolett emittierende Schicht, besonders bevorzugt eine gelb oder grün emittierende Schicht und ganz besonders bevorzugt eine grün emittierende Schicht.

Dabei wird unter einer gelb emittierenden Schicht eine Schicht verstanden, deren Photolumineszenzmaximum im Bereich von 540 bis 570 nm liegt. Unter einer orange emittierenden Schicht wird eine Schicht verstanden, deren Photolumineszenzmaximum im Bereich von 570 bis 600 nm liegt. Unter einer rot emittierenden Schicht wird eine Schicht verstanden, deren Photolumineszenzmaximum im Bereich von 600 bis 750 nm liegt. Unter einer grün emittierenden Schicht wird eine Schicht verstanden, deren Photolumineszenzmaximum im Bereich von 490 bis 540 nm liegt. Unter einer blau emittierenden Schicht wird eine Schicht verstanden, deren Photolumineszenzmaximum im Bereich von 440 bis 490 nm liegt. Dabei wird das Photolumineszenzmaximum der Schicht durch Messung des Photolumineszenzspektrums der Schicht mit einer Schichtdicke von 50 nm bei Raumtemperatur bestimmt, wobei die Schicht die erfindungsgemäße Zusammensetzung aufweist, also Emitter und Matrix enthält.

Die Aufnahme des Photolumineszenzspektrums der Schicht erfolgt beispielsweise mit einem handelsüblichen Photolumineszenzspektrometer.

Das Photolumineszenzspektrum des gewählten Emitters wird in der Regel in Sauerstoff-freier Lösung, 10-5 molar, gemessen, wobei die Messung bei Raumtemperatur erfolgt und jedes Lösemittel geeignet ist, in dem sich der

gewählte Emitter in der genannten Konzentration löst. Besonders geeignete Lösemittel sind üblicherweise Toluol oder 2-Methyl-THF, aber auch Dichlormethan. Gemessen wird mit einem handelsüblichen Photolumineszenzspektrometer. Die Triplettenergie T1 in eV wird aus den Photolumineszenzspektren der Emitter bestimmt. Es wird zunächst das Peakmaximum Plmax. (in nm) des Photolumineszenzspektrums bestimmt. Das Peakmaximum Plmax. (in nm) wird dann in eV umgerechnet gemäß:

$$E(T1 \text{ in eV}) = 1240 / E(T1 \text{ in nm}) = 1240 / PLmax. (\text{in nm}).$$

Bevorzugte phosphoreszierende Emitter sind demzufolge infrarote Emitter, vorzugsweise aus Tabelle 14 oder 15, deren Triplettenergie $T_1$ bevorzugt bei ~1.9 eV bis ~1.0 eV liegt.

Bevorzugte phosphoreszierende Emitter sind demzufolge rote Emitter, vorzugsweise aus Tabelle 14 oder 15, deren Triplettenergie $T_1$ bevorzugt bei ~2.1 eV bis ~1.9 eV liegt.

Bevorzugte phosphoreszierende Emitter sind demzufolge gelbe Emitter, vorzugsweise aus Tabelle 14 oder 15, deren Triplettenergie $T_1$ bevorzugt bei ~2.3 eV bis ~2.1 eV liegt.

Bevorzugte phosphoreszierende Emitter sind demzufolge grüne Emitter, vorzugsweise aus Tabelle 14 oder 15, deren Triplettenergie $T_1$ bevorzugt bei ~2.5 eV bis ~2.3 eV liegt.

Bevorzugte phosphoreszierende Emitter sind demzufolge blaue Emitter, vorzugsweise aus Tabelle 14 oder 15, deren Triplettenergie $T_1$ bevorzugt bei ~3.1 eV bis ~2.5 eV liegt.

Bevorzugte phosphoreszierende Emitter sind demzufolge ultraviolette Emitter, vorzugsweise aus Tabelle 14 oder 15, deren Triplettenergie $T_1$ bevorzugt bei ~4.0 eV bis ~3.1 eV liegt.

Besonders bevorzugte phosphoreszierende Emitter sind demzufolge grüne oder gelbe Emitter, vorzugsweise aus Tabelle 14 oder 15, wie zuvor beschrieben.

Ganz besonders bevorzugte phosphoreszierende Emitter sind demzufolge grüne Emitter, vorzugsweise aus Tabelle 14 oder 15, deren Triplettenergie $T_1$ bevorzugt bei ~2.5 eV bis ~2.3 eV liegt.

Ganz besonders bevorzugt werden grüne Emitter, vorzugsweise aus Tabelle 14 oder 15, wie zuvor beschrieben, für die erfindungsgemäße Zusammensetzung bzw. erfindungsgemäße emittierende Schicht ausgewählt.

Bevorzugte fluoreszierende Emitter sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

In einer weiteren bevorzugten Ausführungsform der Erfindung wird die erfindungsgemäße Zusammensetzung als eine Komponente von Mixed-Matrix-Systemen verwendet. Die Mixed-Matrix-Systeme umfassen bevorzugt drei oder vier verschiedene Matrixmaterialien, besonders bevorzugt drei verschiedene Matrixmaterialien (das heißt, eine weitere Matrixkomponente zusätzlich zu der erfindungsgemäßen Zusammensetzung). Besonders geeignete Matrixmaterialien, welche in Kombination mit der erfindungsgemäßen Zusammensetzung als Matrixkomponenten eines Mixed-Matrix-Systems verwendet werden können, sind ausgewählt aus *wide-band-gap*-Materialien, Elektronentransportmaterialien (ETM) und Lochtransportmaterialien (HTM).

Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten. Besonders geeignete Matrixmaterialien, welche in Kombination mit der erfindungsgemäßen Zusammensetzung als Matrixkomponenten eines Mixed-Matrix-Systems in phosphoreszierenden oder fluoreszierenden organischen Elektrolumineszenzvorrichtungen eingesetzt werden können, sind ausgewählt aus den unten angegebenen bevorzugten Matrixmaterialien für phosphoreszierende Emitter oder den bevorzugten Matrixmaterialien für fluoreszierende Emitter, je nachdem welche Art von Emitter eingesetzt wird. Bevorzugt wird das Mixed-Matrix-System auf einen Emitter der Tabelle 14 oder 15 optimiert.

Als weitere Hostmaterialien, bevorzugt für fluoreszierende Emitter, kommen neben der erfindungsgemäßen

Zusammensetzung, wie zuvor beschrieben, umfassend besonders bevorzugt eine Mischung an Materialien ausgewählt aus M1 bis M1137, verschiedene Stoffklassen in Frage. Bevorzugte weitere Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Hostmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Als weitere Matrixmaterialien, bevorzugt für phosphoreszierende Emitter, kommen neben der erfindungsgemäßen Zusammensetzung, wie zuvor beschrieben, umfassend besonders bevorzugt eine Mischung an Materialien ausgewählt aus M67 bis M79, M106 bis M118, M241 bis M253, M319 bis M331, M423 bis M435, M527 bis M539, M605 bis M617, M748 bis M760, M891 bis M903, M995 bis M1007, M1099 bis M1111, verschiedene Stoffklassen in Frage. Bevorzugte weitere Matrixmaterialien sind ausgewählt aus den Klassen der aromatischen Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückten Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Gemäß einer alternativen Ausführungsform der vorliegenden Erfindung enthält die Zusammensetzung neben den Bestandteilen elektronentransportierender Host und lochtransportierender Host keine weiteren Bestandteile, das heißt, funktionellen Materialien. Es handelt sich in dieser Ausführungsform um Materialmischungen, die als solche zur Herstellung der organischen Schicht verwendet werden. Man bezeichnet diese Systeme auch als Premix-Systeme, die als einzige Materialquelle bei der Aufdampfung verwendet wird. Dadurch lässt sich auf einfache und schnelle Art und Weise das Aufdampfen einer Schicht mit gleichmäßiger Verteilung der Komponenten erreichen, ohne dass eine präzise Ansteuerung einer Vielzahl an Materialquellen notwendig ist.

Ein weiterer Gegenstand der Erfindung ist demzufolge eine Zusammensetzung bestehend aus einer Verbindung der Formeln (1a) bis (1f) oder (1h) bis (11) oder einer Verbindung ausgewählt aus Verbindungen **6, 9, 19, 26, 34, 42, 48, 50, 59, 70, 78** und **86** und einer Verbindung der Formeln(2a), (2b) oder einer Verbindung ausgewählt aus Verbindungen **89** bis **101.**

Die erfindungsgemäße Zusammensetzung, wie zuvor beschrieben oder bevorzugt beschrieben, eignet sich für die Verwendung in einer organischen elektronischen Vorrichtung. Dabei wird unter einer organischen elektronischen Vorrichtung eine Vorrichtung verstanden, welche mindestens eine Schicht enthält, die mindestens eine organische Verbindung enthält. Die Vorrichtung kann aber auch anorganische Materialien enthalten oder auch Schichten, welche vollständig aus anorganischen Materialien aufgebaut sind.

Ein weiterer Gegenstand der Erfindung ist demzufolge die Verwendung einer Zusammensetzung, wie zuvor beschrieben oder bevorzugt beschrieben, insbesondere einer Mischung ausgewählt aus M67 bis M79, M106 bis M118, M241 bis M253, M319 bis M331, M423 bis M435, M527 bis M539, M605 bis M617, M748 bis M760, M891 bis M903, M995 bis M1007, M1099 bis M1111, in einer organischen elektronischen Vorrichtung.

Die Komponenten bzw. Bestandteile der Zusammensetzungen können dabei durch Aufdampfen oder aus Lösung prozessiert werden. Sofern die Zusammensetzungen aus Lösung aufgebracht werden, sind Formulierungen der erfindungsgemäßen Zusammensetzung enthaltend wenigstens ein weiteres Lösungsmittel erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden.

Ein weiterer Gegenstand der vorliegenden Erfindung ist daher eine Formulierung enthaltend eine erfindungsgemäße Zusammensetzung und mindestens ein Lösemittel.

Geeignete und bevorzugte Lösemittel sind beispielsweise Toluol, Anisol, o-, m- oder p-Xylol, Methylbenzoat,

Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Die Formulierung kann dabei auch mindestens eine weitere organische oder anorganische Verbindung enthalten, die ebenfalls in der elektronischen Vorrichtung eingesetzt wird, insbesondere eine emittierende Verbindung, insbesondere ein phosphoreszierender Emitter und/oder ein weiteres Matrixmaterial. Geeignete emittierende Verbindungen und weitere Matrixmaterialien wurden vorstehend bereits aufgeführt.

Gegenstand der vorliegenden Erfindung ist auch die Verwendung der erfindungsgemäßen Zusammensetzung in einer organischen elektronischen Vorrichtung, bevorzugt in einer elektronentransportierenden und/oder in einer emittierenden Schicht.

Die organische elektronische Vorrichtung ist bevorzugt gewählt aus den organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren und organischen Photorezeptoren, wobei die organischen Elektrolumineszenzvorrichtungen besonders bevorzugt sind.

Ganz besonders bevorzugte organische Elektrolumineszenzvorrichtungen für die Verwendung der erfindungsgemäßen Zusammensetzung sind organische lichtemittierende Transistoren (OLETs), organische Feld-Quench-Devices (OFQDs), organische lichtemittierende elektrochemischen Zellen (OLECs, LECs, LEECs), organische Laserdioden (O-Laser) und organische lichtemittierende Dioden (OLEDs), insbesondere bevorzugt sind OLECs und OLEDs und am meisten bevorzugt sind OLEDs.

Bevorzugt wird die erfindungsgemäße Zusammensetzung, wie zuvor beschrieben oder als bevorzugt beschrieben, in einer elektronischen Vorrichtung in einer Schicht mit elektronentransportierender Funktion verwendet. Die Schicht ist bevorzugt eine Elektroneninjektions-schicht (EIL), eine Elektronentransportschicht (ETL), eine Lochblockier-schicht (HBL) und/oder eine Emissionsschicht (EML), besonders bevorzugt eine ETL, EIL und /oder eine EML. Ganz besonders bevorzugt wird die erfindungsgemäße Zusammensetzung in einer EML eingesetzt, insbesondere als Matrixmaterial.

Nochmals ein weiterer Gegenstand der vorliegenden Erfindung ist deshalb eine organische elektronische Vorrichtung, die insbesondere aus einer der vorstehend genannten elektronischen Vorrichtungen ausgewählt ist und die die erfindungsgemäße Zusammenfassung, wie zuvor beschrieben oder bevorzugt beschrieben, enthält, bevorzugt in einer Emissionsschicht (EML), in einer Elektronentransportschicht (ETL), in einer Elektroneninjektionsschicht (EIL) und/oder in einer Lochblockierschicht (HBL), ganz bevorzugt in einer EML, EIL und/oder ETL und ganz besonders bevorzugt in einer EML.

Wenn es sich um eine emittierende Schicht handelt, dann ist es insbesondere bevorzugt eine phosphoreszierende Schicht, die dadurch gekennzeichnet ist, dass sie zusätzlich zu der Zusammensetzung, wie zuvor beschrieben oder bevorzugt beschrieben, einen phosphoreszierenden Emitter enthält, insbesondere zusammen mit einem Emitter der Tabelle 14 oder 15 oder einem bevorzugten Emitter, wie zuvor beschrieben.

In einer besonders bevorzugten Ausführungsform der vorliegenden Erfindung handelt es sich daher bei der elektronischen Vorrichtung um eine organische Elektrolumineszenzvorrichtung, ganz besonders bevorzugt um eine organische lichtemittierende Diode (OLED), die die erfindungsgemäße Zusammensetzung, wie zuvor beschrieben oder bevorzugt beschrieben, zusammen mit einem phosphoreszierenden Emitter in der Emissionsschicht (EML) enthält.

Die erfindungsgemäße Zusammensetzung gemäß den bevorzugten Ausführungsformen und der emittierenden Verbindung enthält vorzugsweise zwischen 99,9 und 1 Vol.-%, weiter vorzugsweise zwischen 99 und 10 Vol.-%, besonders bevorzugt zwischen 98 und 60 Vol.-%, ganz besonders bevorzugt zwischen 97 und 80 Vol.-% an Matrixmaterial aus mindestens einer Verbindung der Formel (1e) und mindestens einer Verbindung der Formel (2a) gemäß den bevorzugten Ausführungsformen, bezogen auf die gesamte Zusammensetzung aus Emitter und Matrixmaterial. Entsprechend enthält die Zusammensetzung vorzugsweise zwischen 0,1 und 99 Vol.-%, weiter vorzugsweise zwischen 1 und 90 Vol.-%, besonders bevorzugt zwischen 2 und 40 Vol.-%, ganz besonders bevorzugt zwischen 3 und 20 Vol.-% des Emitters bezogen auf die gesamte Zusammensetzung aus Emitter und Matrixmaterial. Werden die Verbindungen aus Lösung verarbeitet, so werden statt der oben angegebenen Mengen in Vol.-% bevorzugt die entsprechenden Mengen in Gew.-% verwendet.

Außer Kathode, Anode und der Schicht enthaltend die erfindungsgemäße Zusammensetzung kann eine elektronische Vorrichtung noch weitere Schichten enthalten. Diese sind beispielsweise gewählt aus jeweils einer oder mehreren Lochinjektionsschichten, Lochtransportschichten, Lochblockierschichten, emittierenden Schichten, Elektronentransportschichten, Elektroneninjektionsschichten, Elektronenblockierschichten, Excitonenblockierschichten,

Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss.

Die Abfolge der Schichten in einer organischen Elektrolumineszenzvorrichtung ist bevorzugt die folgende: Anode / Lochinjektionsschicht / Lochtransportschicht / emittierende Schicht / Elektronentransportschicht / Elektroneninjektionsschicht / Kathode.

Diese Abfolge der Schichten ist eine bevorzugte Abfolge.

Dabei soll erneut darauf hingewiesen werden, dass nicht alle der genannten Schichten vorhanden sein müssen, und/oder dass zusätzlich weitere Schichten vorhanden sein können.

Eine organische Elektrolumineszenzvorrichtung, die die erfindungsgemäße Zusammensetzung enthält, kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Lochtransportmaterialien sind insbesondere Materialien bevorzugt, die in einer Lochtransport-, Lochinjektions- oder Elektronenblockierschicht verwendet werden können, wie Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. WO 2012/150001).

Weitere geeignete Lochtransportmaterialien sind die folgenden Verbindungen:

| | | |
|---|---|---|
| 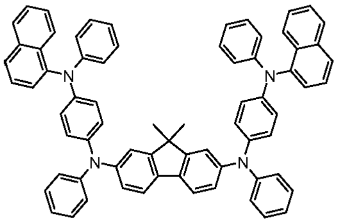 | 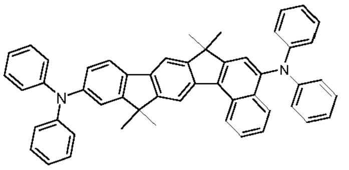 | 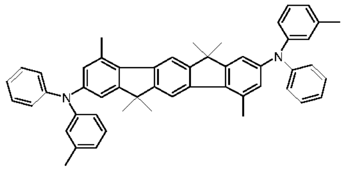 |
| 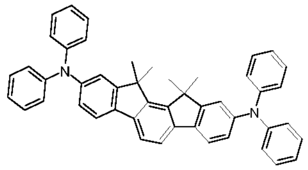 | 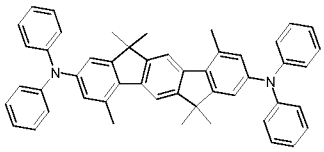 | 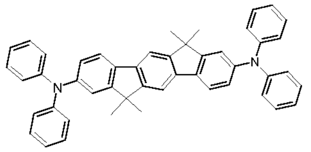 |
| 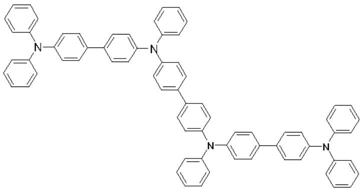 | 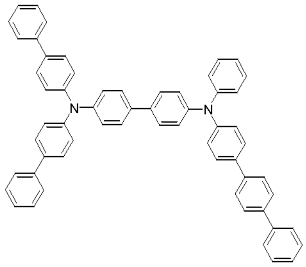 | 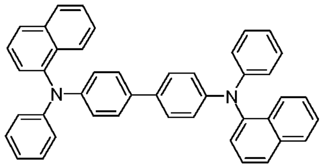 |

| | | |
|---|---|---|
| 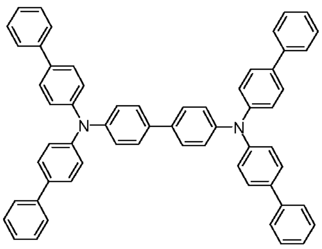 | 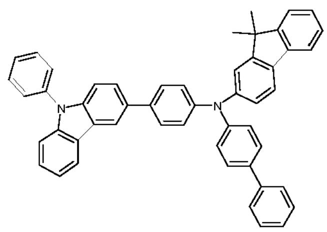 | 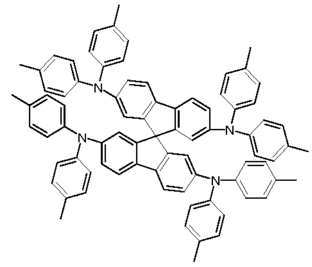 |
| 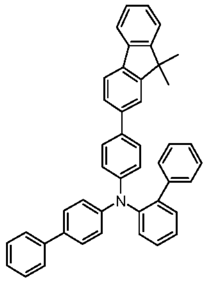 | 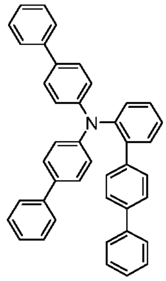 | 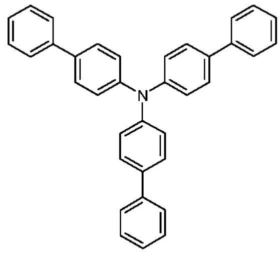 |
| 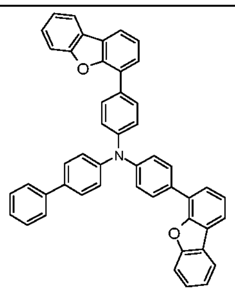 | 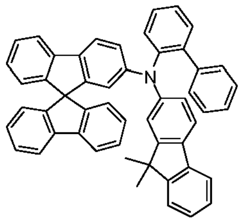 | 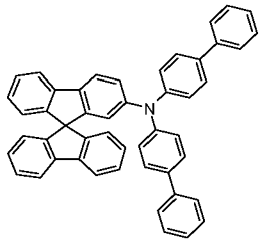 |
| 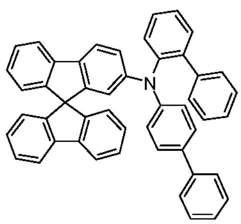 | 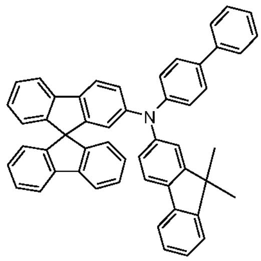 | 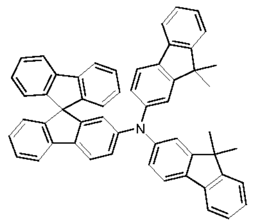 |
| 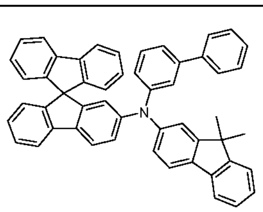 | 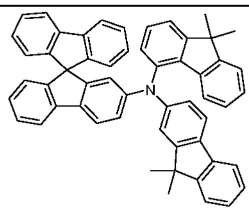 | 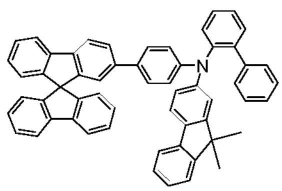 |
| 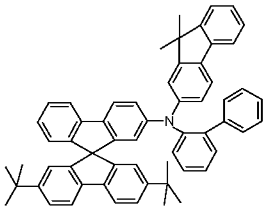 | 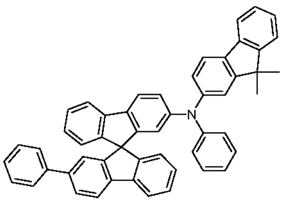 | 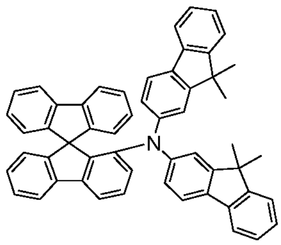 |

| | | |
|---|---|---|
| 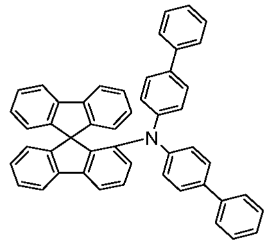 | 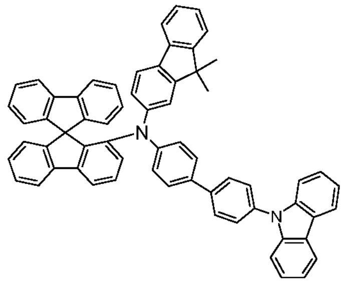 | 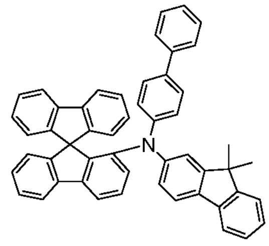 |
| 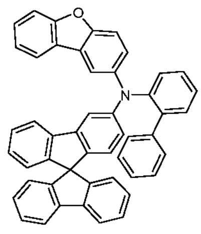 | 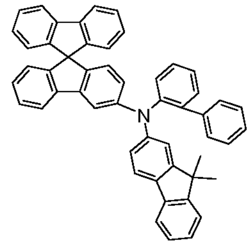 | 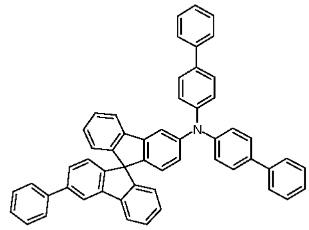 |
| 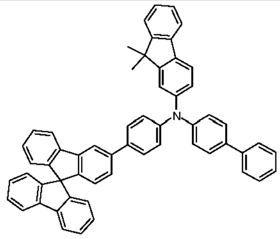 | 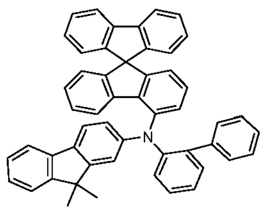 | 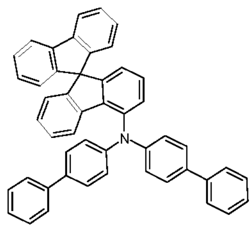 |
| 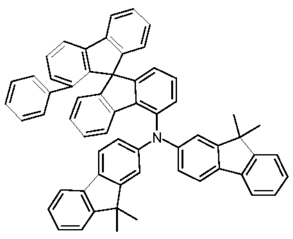 | 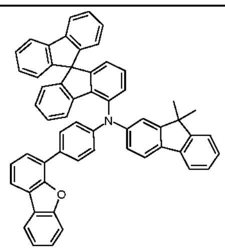 | 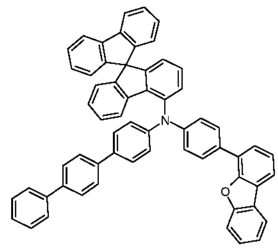 |
| 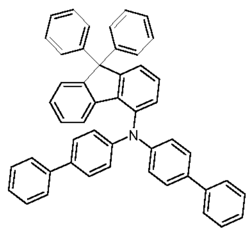 | 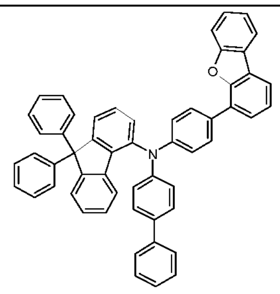 | 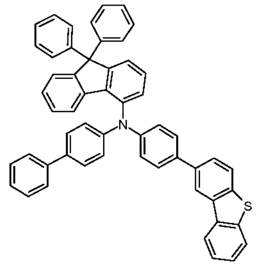 |
| 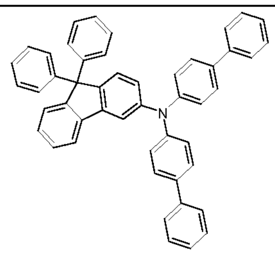 | 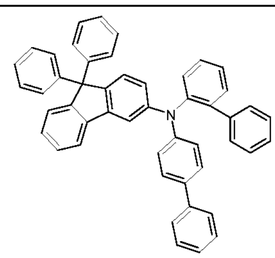 | 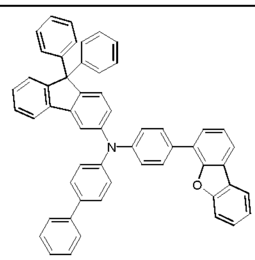 |

| | | |
|---|---|---|
| 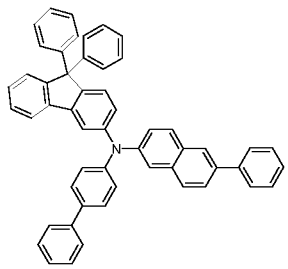 | 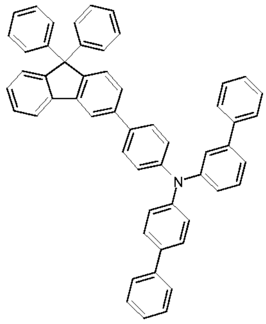 | 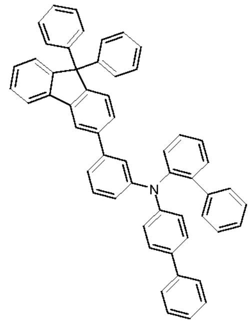 |
| 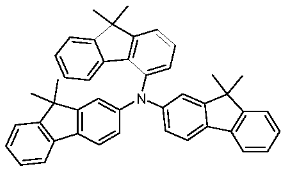 | 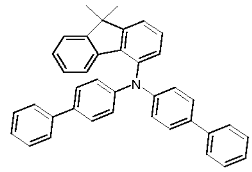 | 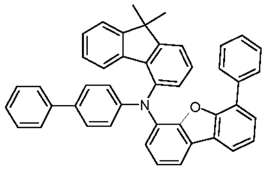 |
| 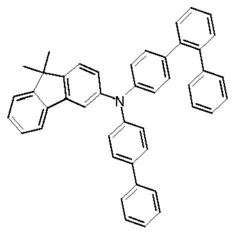 | 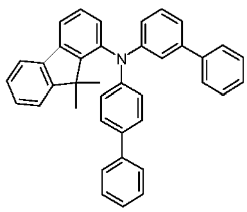 | 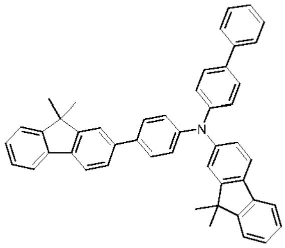 |
| 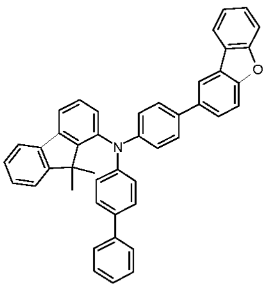 | 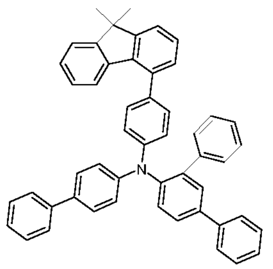 | 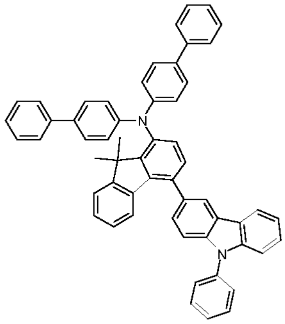 |
| 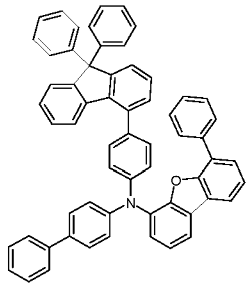 | 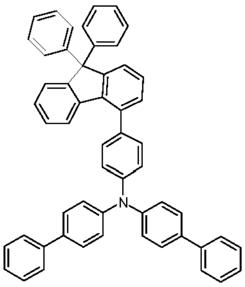 | 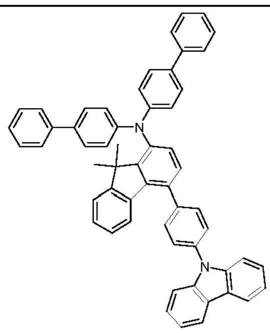 . |

Als Kathode elektronischer Vorrichtungen sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die organische elektronische Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer weiteren bevorzugten Ausführungsform ist die organische elektronische Vorrichtung, die die erfindungsgemäße Zusammensetzung enthält, dadurch gekennzeichnet, dass eine oder mehrere organische Schichten enthaltend die erfindungsgemäße Zusammensetzung mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere organische Schichten enthaltend die erfindungsgemäße Zusammensetzung aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen der Komponenten der erfindungsgemäßen Zusammensetzung nötig. Hohe Löslichkeit lässt sich durch geeignete Substitution der entsprechenden Verbindungen erreichen. Das Verarbeiten aus Lösung hat den Vorteil, dass die Schicht enthaltend die erfindungsgemäße Zusammensetzung sehr einfach und kostengünstig aufgebracht werden kann. Diese Technik eignet sich insbesondere für die Massenproduktion organischer elektronischer Vorrichtungen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können auf organische Elektrolumineszenzvorrichtungen angewandt werden.

Ein weiterer Gegenstand der Erfindung ist daher ein Verfahren zur Herstellung einer organischen elektronischen Vorrichtung enthaltend eine erfindungsgemäße Zusammensetzung, wie zuvor beschrieben oder bevorzugt beschrieben, dadurch gekennzeichnet, dass mindestens eine organische Schicht enthaltend eine erfindungsgemäße Zusammensetzung durch Gasphasenabscheidung, insbesondere mit einem Sublimationsverfahren und/oder mit einem OVPD (Organic Vapour Phase Deposition) Verfahren und/oder mit Hilfe einer Trägergassublimation, oder aus Lösung, insbesondere durch Spincoating oder mit einem Druckverfahren, aufgebracht wird.

Bei der Herstellung einer organischen elektronischen Vorrichtung mittels Gasphasenabscheidung bestehen grundsätzlich zwei Möglichkeiten, wie eine organische Schicht, die die erfindungsgemäße Zusammensetzung enthalten soll und die mehrere verschiedene Bestandteile umfassen kann, auf ein beliebiges Substrat aufgebracht bzw. aufgedampft werden kann. Zum einen können die verwendeten Materialien jeweils in einer Materialquelle vorgelegt und schließlich aus den verschiedenen Materialquellen verdampft werden ("co-evaporation"). Zum anderen können die verschiedenen Materialien vorgemischt ("premixed") und das Gemisch in einer einzigen Materialquelle vorgelegt werden, aus der es schließlich verdampft wird ("premix-evaporation"). Dadurch lässt sich auf einfache und schnelle Art und Weise das Aufdampfen einer Schicht mit gleichmäßiger Verteilung der Komponenten erreichen, ohne dass eine präzise Ansteuerung einer Vielzahl an Materialquellen notwendig ist.

Ein weiterer Gegenstand der Erfindung ist demzufolge ein Verfahren, dadurch gekennzeichnet, dass die mindestens eine Verbindung der Formel (1e), wie zuvor beschrieben oder als bevorzugt beschrieben, und die mindestens eine Verbindung der Formel (2a), wie zuvor beschrieben oder als bevorzugt beschrieben, nacheinander oder gleichzeitig

aus mindestens zwei Materialquellen, gegebenenfalls mit weiteren Materialien, wie zuvor beschrieben oder bevorzugt beschrieben, aus der Gasphase abgeschieden werden und die organische Schicht bilden.

In einer bevorzugten Ausführungsform der vorliegenden Erfindung wird die mindestens eine organische Schicht mittels Gasphasenabscheidung aufgebracht, wobei die Bestandeile der Zusammensetzung vorgemischt und aus einer einzigen Materialquelle verdampft werden.

Ein weiterer Gegenstand der Erfindung ist demzufolge ein Verfahren, dadurch gekennzeichnet, dass die erfindungsgemäße Zusammensetzung, wie zuvor beschrieben oder bevorzugt beschrieben, als Materialquelle zur Gasphasenabscheidung genutzt wird, und gegebenenfalls mit weiteren Materialien, die organische Schicht bildet.

Ein weiterer Gegenstand der Erfindung ist ein Verfahren zur Herstellung einer organischen elektronischen Vorrichtung enthaltend eine erfindungsgemäße Zusammensetzung, wie zuvor beschrieben oder bevorzugt beschrieben, dadurch gekennzeichnet, dass die erfindungsgemäße Formulierung, wie zuvor beschrieben, verwendet wird, um die organische Schicht aufzubringen.

Die erfindungsgemäßen Zusammensetzungen bzw. die erfindungsgemäßen Vorrichtungen zeichnen sich durch folgende überraschende Vorteile gegenüber dem Stand der Technik aus:
Die Verwendung der erfindungsgemäßen Zusammensetzungen in organischen elektronischen Vorrichtungen, insbesondere in einer organischen Elektrolumineszenzvorrichtungen, und insbesondere in einer OLED oder OLEC, führt zu deutlichen Steigerungen der Lebensdauer der Vorrichtungen.

Wie im nachfolgend angegebenen Beispiel 1 ersichtlich, lassen sich durch den Einsatz von Verbindungen gemäß Stand der Technik, beispielsweise der Verbindung V1, bei mittleren Emitterkonzentrationen in der EML von 10% gute Spannungen und Effizienzen erreichen. Die Lebensdauer der Bauteile ist jedoch gering.

Eine Verbesserung der Lebensdauer um einen Faktor größer 2 bei vergleichbarer Bauteilspannung und vergleichbarer oder verbesserter Bauteileffizienz kann durch die erfindungsgemäße Kombination der Verbindungen der Formel (1e), wie zuvor beschrieben, mit Verbindungen der Formel (2a), wie zuvor beschrieben, erreicht werden.

Diese Verbesserung der Lebensdauer um einen Faktor ca. größer 2 bei vergleichbarer Bauteilspannung und vergleichbarer oder verbesserter Bauteileffizienz kann bevorzugt durch die erfindungsgemäße Kombination der Verbindungen der Formel (1e), wie zuvor beschrieben, mit Verbindungen der Formel (2a), wie zuvor beschrieben, bei Emitterkonzentrationen von 2 bis 15 Gew.-% in der Emissionsschicht erreicht werden.

Selbst mit einer geringen Emitterkonzentration von lediglich 7% in der EML, bei der die Lebensdauer einer OLED typischerweise sinkt, sind die erzielten Lebensdauern der erfindungsgemäßen Kombinationen noch deutlich gegenüber dem Stand der Technik verbessert. Dies wird stellvertretend und repräsentativ für Verbindungen der Formel (1e) durch Verwendung der Verbindung **9** mit dem Biscarbazol **91** in dem Beispiel E5 bei einer Emitterkonzentration von 7% gezeigt.

Verbindung **9,** repräsentativ für Verbindungen der Formel (1e), (1f), (1h) und (1i) in Kombination mit erfindungsgemäßen Verbindungen der Formel (2a), wie zuvor beschrieben zeigt die besten Ergebnisse.

Der Unterschied zu dem Vergleichsbeispiel liegt in der elektronischen Struktur des Substituenten $Ar_4$ und $Ar_5$ im Biscarbazol der Formel (2a), die nicht gleichzeitig Phenyl sind. Für den Fachmann nicht vorhersehbar, bewirkt die höhere elektronische Dichte mindestens einer der Substituenten $Ar_4$ und $Ar_5$ als aromatisches Ringsystem mit 10 bis 40 Ringatomen, insbesondere von 12 bis 40 Ringatomen, bzw. als heteroaromatisches elektronenreiches Ringsystem mit 10 bis 40 Ringatomen ein verbessertes Aufdampfverhalten und führt in der Konsequenz zu einer Verbesserung der Lebensdauer von elektronischen Vorrichtungen, insbesondere von OLEDs. Die Verbesserung wird deutlich, da die Lebensdauer im Vergleich zum Stand der Technik erhöht wird, insbesondere um einen Faktor ca. größer 1,5, insbesondere um einen Faktor ca. größer 2, ganz besonders um einen Faktor von 2 bis 3.

Ohne an die Theorie gebunden zu sein wird vermutet, dass auch die Konjugation der ausgewählten Substituenten $Ar_4$ und $Ar_5$ Einfluss nimmt. Wechselt man von Phenyl zu Biphenyl, so wird nämlich auch die Konjugation verbessert und die Vorrichtung zeigt die vorteilhaften Eigenschaften, wie zuvor beschrieben. Wechselt man von Biphenyl zu einem heteroaromatischen Ringsystem, wie beispielsweise einem Dibenzofuran, Dibenzothiophen oder Carbazol, so planarisiert sich das System durch die Verbrückung über das O-Atom, S-Atom oder N-Atom und die Konjugation wird zusätzlich verbessert. Die Vorteile werden daher auch bei Verwendung von elektronenreichen heteroaromatischen Ringsystemen erzielt.

Der weitere Unterschied zum Stand der Technik liegt in der Auswahl spezieller Verbindungen der Formel (1e), in denen der Linker L ein aromatisches Ringsystem mit 6 bis 18 C-Atomen bedeutet, wobei die Lebensdauer überraschenderweise noch einmal verbessert wird.

Die erfindungsgemäßen Zusammensetzungen eignen sich sehr gut für den Einsatz in einer Emissionsschicht und zeigen verbesserte Leistungsdaten, insbesondere für die Lebensdauer, gegenüber Verbindungen aus dem Stand der Technik, wie zuvor beschrieben.

Die erfindungsgemäßen Zusammensetzungen können leicht prozessiert werden und eignen sich daher sehr gut für die Massenproduktion in der kommerziellen Anwendung.

Die erfindungsgemäßen Zusammensetzungen können vorgemischt und aus einer einzigen Materialquelle aufgedampft werden, so dass auf einfache und schnelle Art und Weise eine organische Schicht mit gleichmäßiger Verteilung der verwendeten Komponenten hergestellt werden kann.

Diese oben genannten Vorteile gehen nicht mit einer Verschlechterung der weiteren elektronischen Eigenschaften einer elektronischen Vorrichtung einher.

Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden.

Alle Merkmale der vorliegenden Erfindung können in jeder Art miteinander kombiniert werden, es sei denn, dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Allgemeine Methoden:

**Bestimmung von Orbitalenergien und elektronischer Zustände**

Die HOMO- und LUMO-Energien sowie das Triplettniveau und die Singulettniveaus der Materialien werden über quantenchemische Rechnungen bestimmt. Hierzu wird vorliegend das Programmpaket "Gaussian09, Revision D.01" (Gaussian Inc.) verwendet. Zur Berechnung organischer Substanzen ohne Metalle (mit Methode "org." bezeichnet) wird zuerst eine Geometrieoptimierung mit der semi-empirischen Methode AM1 (Gaussian-Eingabezeile "# AM1 opt") mit der Ladung (Charge) 0 und der Multiplizität (Multiplicity) 1 durchgeführt. Im Anschluss erfolgt auf Grundlage der optimierten Geometrie eine Energierechnung (single point) für den elektronischen Grundzustand und das Triplett-Niveau. Hierbei wird die TDDFT-Methode (time dependent density functional theory) B3PW91 mit dem Basissatz 6-31G(d) (Gaussian-Eingabezeile "# B3PW91/6-31G(d) td=(50-50, nstates=4)") verwendet (Ladung 0, Multiplizität 1). Für metallorganische Verbindungen (mit Methode "M-org." bezeichnet) wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt, wie oben beschrieben, analog zu der der organischen Substanzen, mit dem Unterschied, dass für das Metallatom der Basissatz "LanL2DZ" und für die Liganden der Basissatz "6-31G(d)" verwendet wird (Gaussian-Eingabezeile "#B3PW91/gen pseudo=lanl2 td=(50-50, nstates=4)"). Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Daraus wird der anhand von Cyclovoltammetriemessungen kalibrierte HOMO- und LUMO-Wert in Elektronenvolt wie folgt bestimmt:

$$\text{HOMO (eV)} = (\text{HEh}*27.212)*0.8308-1.118;$$

$$\text{LUMO (eV)} = (\text{LEh}*27.212)*1.0658-0.5049.$$

Das Triplett-Niveau T1 eines Materials ist definiert als die relative Anregungsenergie (in eV) des Triplettzustands mit der niedrigsten Energie, der sich aus der quantenchemischen Energierechnung ergibt.

Das Singulett-Niveau S1 eines Materials ist definiert als die relative Anregungsenergie (in eV) des Singulettzustands mit der zweitniedrigsten Energie, der sich aus der quantenchemischen Energierechnung ergibt. Der energetisch niedrigste Singulettzustand wird als S0 bezeichnet.

Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian09, Revision D.01" verwendet.

Beispiel 1: **Herstellung der OLEDs**

In den folgenden Beispielen E5, E5a, E10 und E10a (siehe Tabelle 16) wird der Einsatz der erfindungsgemäßen Materialkombinationen in OLEDs vorgestellt. Die OLEDs E1 bis E4, E6 bis E9 enthalten nicht erfindungsgemäße Materialkombinationen.

**Vorbehandlung für die Beispiele E1 - E10a:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs

aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 16 zu entnehmen. Die zur Herstellung der OLEDs benötigten Materialien sind in Tabelle 17 gezeigt. Die Daten der OLEDs sind in Tabelle 18 aufgelistet. Das Beispiele V1 ist ein Vergleichsbeispiel gemäß WO 2015/169412. Die Beispiele E5, E5a, E10 und E10a zeigen die erfindungsgemäßen OLEDs.

Alle Materialien werden in einer Vakuumkammer thermisch aufgedampft. Dabei besteht die Emissionsschicht immer aus mindestens einem Matrixmaterial (Hostmaterial, Wirtsmaterial), im Sinn der Erfindung mindestens zwei Matrixmaterialien, und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie CbzT1:BisC1:TEG1 (45%:45%:10%) bedeutet hierbei, dass das Material CbzT1 in einem Volumenanteil von 45%, BisC1 in einem Anteil von 45% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 × und y Farbkoordinaten berechnet. Die Angabe U1000 in Tabelle 18 bezeichnet die Spannung, die für eine Leuchtdichte von 1000 cd/$m^2$ benötigt wird. SE1000 und EQE1000 bezeichnen die Stromeffizienz bzw. die externe Quanteneffizienz, die bei 1000cd/$m^2$ erreicht werden.

Als Lebensdauer LD wird die Zeit definiert, nach der die Leuchtdichte bei Betrieb mit konstanter Stromdichte $j_0$ von der Startleuchtdichte auf einen gewissen Anteil L1 absinkt. Eine Angabe L1=80% in Tabelle 18 bedeutet, dass die in Spalte LD angegebene Lebensdauer der Zeit entspricht, nach der die Leuchtdichte auf 80% ihres Anfangswertes absinkt.

**Verwendung von erfindungsgemäßen Mischungen in OLEDs**

Die erfindungsgemäßen Materialkombinationen können in der Emissionsschicht in phosphoreszierenden OLEDs eingesetzt werden. Die erfindungsgemäße Kombination der Verbindung **9** mit der Verbindung **91** wird in den Beispielen E5, E5a, E10 und E10a als Matrixmaterial in der Emissionsschicht eingesetzt und ist entsprechend mit einem * in Tabellen 16 und 18 gekennzeichnet.

Tabelle 16: Aufbau der OLEDs; * erfindungsgemäß

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 230nm | SpMA2 20nm | CbzT1:BisC1:TEG1 (45%:45%:10%) 30nm | ST2 10nm | ST2:LiQ (50%:50%) 30nm | --- |
| E1 | HATCN 5nm | SpMA1 230nm | SpMA2 20nm | CbzT1:BisC2:TEG1 (44%:44%:12%) 30nm | ST2 10nm | ST2:LiQ (50%:50%) 30nm | LiQ 1nm |
| E2 | HATCN 5nm | SpMA1 230nm | SpMA2 20nm | CbzT1:BisC3:TEG1 (44%:44%:12%) 30nm | ST2 10nm | ST2:LiQ (50%:50%) 30nm | LiQ 1nm |
| E3 | HATCN 5nm | SpMA1 230nm | SpMA2 20nm | CbzT1:BisC2:TEG1 (46%:47%:7%) 30nm | ST2 10nm | ST2:LiQ (50%:50%) 30nm | LiQ 1nm |
| E4 | HATCN 5nm | SpMA1 230nm | SpMA2 20nm | CbzT1:BisC3:TEG1 (46%:47%:7%) 30nm | ST2 10nm | ST2:LiQ (50%:50%) 30nm | LiQ 1nm |
| E5* | HATCN 5nm | SpMA1 230nm | SpMA2 20nm | **9**:**91**:TEG1 (23%:70%:7%) 30nm | ST2 10nm | ST2:LiQ (50%:50%) 30nm | LiQ 1nm |
| E5*a | HATCN 5nm | SpMA1 230nm | SpMA2 20nm | **9**:**91**:TEG1 (46%:47%:7%) 30nm | ST2 10nm | ST2:LiQ (50%:50%) 30nm | LiQ 1nm |
| E6 | HATCN 5nm | SpMA1 230nm | SpMA2 20nm | **13**:**91**:TEG1 (46%:47%:7%) 30nm | ST2 10nm | ST2:LiQ (50%:50%) 30nm | LiQ 1nm |

(fortgesetzt)

| Bsp | HIL Dicke | HTL Dicke | EBL Dicke | EML Dicke | HBL Dicke | ETL Dicke | EIL Dicke |
|---|---|---|---|---|---|---|---|
| E7 | HATCN 5nm | SpMA1 230nm | SpMA2 20nm | **15:91:TEG1** (46%:47%:7%) 30nm | ST2 10nm | ST2:LiQ (50%:50%) 30nm | LiQ 1nm |
| E8 | HATCN 5nm | SpMA1 230nm | SpMA2 20nm | **69:91**:TEG1 (46%:47%:7%) 30nm | ST2 10nm | ST2:LiQ (50%:50%) 30nm | LiQ 1nm |
| E9 | HATCN 5nm | SpMA1 230nm | SpMA2 20nm | CbzT1:**91**:TEG1 (46%:47%:7%) 30nm | ST2 10nm | ST2:LiQ (50%:50%) 30nm | LiQ 1nm |
| E10* | HATCN 5nm | SpMA1 230nm | SpMA2 20nm | **9:91**:TEG1 (44%:44%:12%) 30nm | ST2 10nm | ST2:LiQ (50%:50%) 30nm | LiQ 1nm |
| E10a* | HATCN 5nm | SpMA1 230nm | SpMA2 20nm | **9:91**:TEG1 (22%:66%:12%) 30nm | ST2 10nm | ST2:LiQ (50%:50%) 30nm | LiQ 1nm |

Tabelle 17: Strukturformeln der Materialien für OLEDs

| | |
|---|---|
| 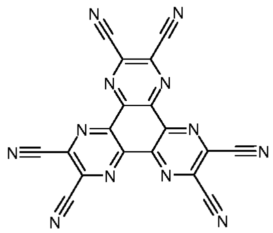 | 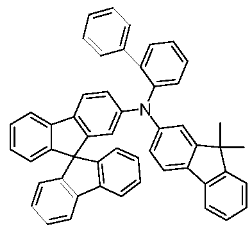 |
| HATCN | SpMA1 |
| 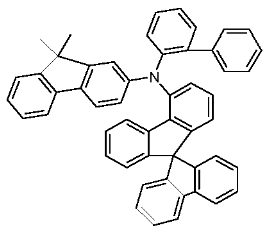 | 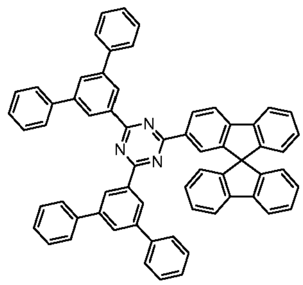 |
| SpMA2 | ST2 |
| 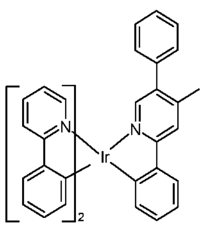 | 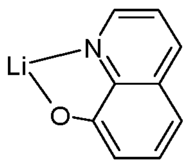 |
| TEG1 | LiQ |

(fortgesetzt)

| | |
|---|---|
| 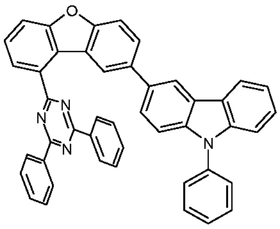 | 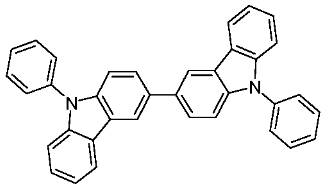 |
| CbzT1 | BisC1 |
| 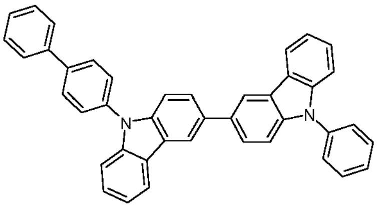 | 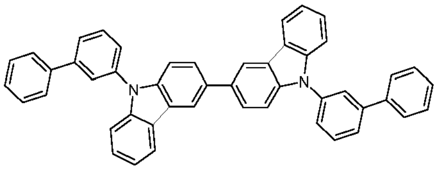 |
| BisC2 | BisC3 |
| 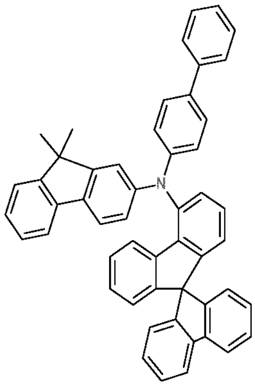 | 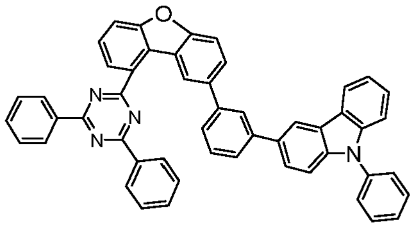 |
| SpMA3 | **9** |
| 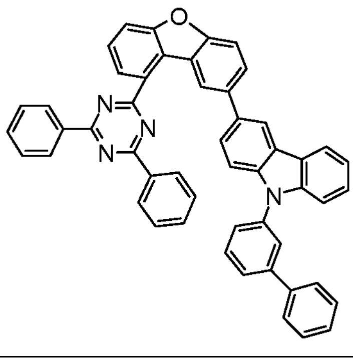 | 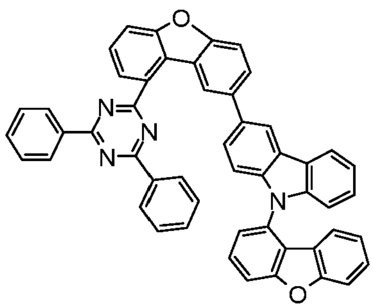 |
| **13** | **15** |

(fortgesetzt)

| | |
|---|---|
| 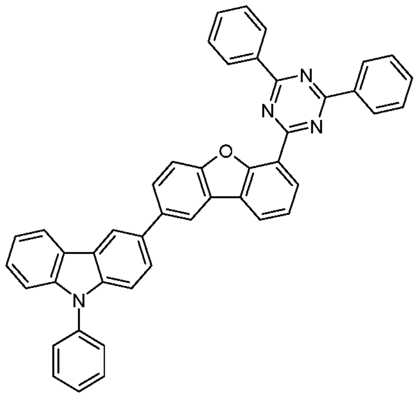 | 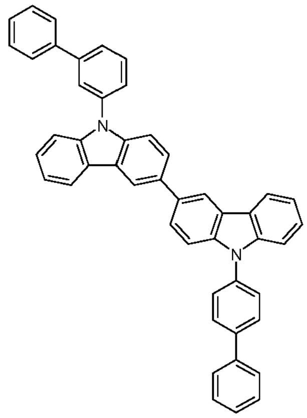 |
| **69** | **91** |

Tabelle 18: Daten der OLEDs; * erfindungsgemäß

| Bsp. | U10 00 (V) | SE1000 (cd/A) | EQE 1000 (%) | CIE x/y bei 1000 cd/m$^2$ | $j_0$ (mA/cm$^2$) | L1 (%) | LD (h) |
|---|---|---|---|---|---|---|---|
| V1 | 3.5 | 70 | 18.4 | 0.33/0.63 | 20 | 80 | 370 |
| E1 | 3.2 | 67 | 18.0 | 0.33/0.63 | 20 | 80 | 930 |
| E2 | 3.1 | 69 | 18.8 | 0.32/0.64 | 20 | 80 | 980 |
| E3 | 3.2 | 74 | 20.1 | 0.32/0.63 | 20 | 80 | 650 |
| E4 | 3.2 | 73 | 19.8 | 0.33/0.63 | 20 | 80 | 608 |
| E5* | 3.4 | 69 | 19.0 | 0.31/0.64 | 20 | 80 | 1030 |
| E5a* | 3.2 | 75 | 20.5 | 0.31/0.64 | 20 | 80 | 645 |
| E6 | 3.2 | 75 | 20.4 | 0.32/0.64 | 20 | 80 | 850 |
| E7 | 3.2 | 77 | 20.9 | 0.31/0.64 | 20 | 80 | 480 |
| E8 | 3.4 | 79 | 21.5 | 0.31/0.64 | 20 | 80 | 520 |
| E9 | 3.4 | 72 | 19.9 | 0.31/0.64 | 20 | 80 | 620 |
| E10* | 3.2 | 66 | 18.3 | 0.32/0.64 | 20 | 80 | 990 |
| E10a* | 3.4 | 60 | 16.5 | 0.31/0.64 | 20 | 80 | 1125 |

Beispiel 2: Synthese der Verbindung **1** (CbzT1)

**a) 6-Brom-2-fluor-2'-methoxy-biphenyl**

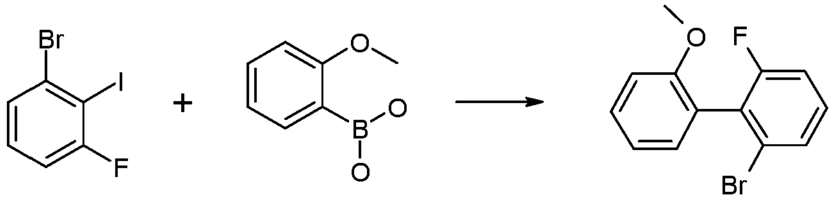

200 g (664 mmol) 1-Brom-3-fluor-2-iodbenzol, 101 g (664 mmol) 2-Methoxyphenylboronsäure und 137.5 g (997 mmol) Natriumtetraborat werden in 1000 mL THF und 600 ml Wasser gelöst und entgast. Es wird mit 9.3 g (13.3

mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 155 g (553 mmol), 83 % der Theorie.

**b) 6'-Brom-2'-fluor-biphenyl-2-ol**

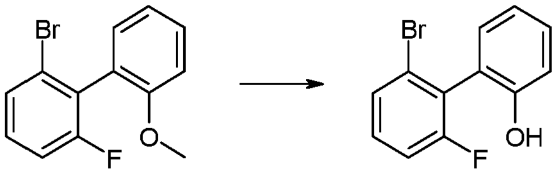

112 g (418 mmol) 6-Brom-2-fluor-2'-methoxy-biphenyl werden in 2 L Dichloromethan gelöst und auf 5 °C gekühlt. Zu dieser Lösung wird innerhalb von 90 min. 41.01 ml (431 mmol) Bortribromid zugetropft und über Nacht weiter gerührt. Das Gemisch wird im Anschluss langsam mit Wasser versetzt, die organische Phase dreimal mit Wasser gewaschen, über $Na_2SO_4$ getrocknet, einrotiert und chromatographisch gereinigt. Ausbeute: 104 g (397 mmol), 98 % der Theorie.

**c) 1-Brom-dibenzofuran**

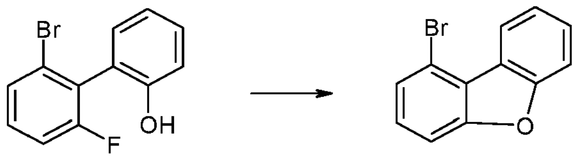

111 g (416 mmol) 6'-Brom-2'-fluor-biphenyl-2-ol werden in 2 L DMF (max. 0.003 % $H_2O$) SeccoSolv® gelöst und auf 5 °C gekühlt. Zu dieser Lösung wird portionsweise 20 g (449 mmol) Natriumhydrid (60% Suspension in Paraffinöl) zugegeben, nach beendeter Zugabe 20 min. nachgerührt und dann für 45 min. auf 100 °C erhitzt. Das Gemisch wird nach dem Abkühlen langsam mit 500 ml Ethanol versetzt, einrotiert und dann chromatographisch gereinigt. Ausbeute: 90 g (367 mmol), 88.5 % der Theorie.

**d) Dibenzofuran-1-boronsäure**

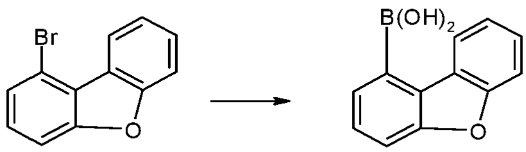

180 g (728 mmol) 1-Brom-dibenzofuran werden in 1500 mL trockenem THF gelöst und auf -78 °C gekühlt. Bei dieser Temperatur wird mit 305 mL (764 mmol / 2.5 M in Hexan) n-Butyllithium innerhalb von ca. 5 min. versetzt und anschließend für 2.5 h bei -78 °C nachgerührt. Bei dieser Temperatur wird mit 151 g (1456 mmol) Borsäuretrimethylester möglichst zügig versetzt und die Reaktion langsam auf Raumtemperatur kommen gelassen (ca. 18 h). Die Reaktionslösung wird mit Wasser gewaschen und der ausgefallene Feststoff und die organische Phase mit Toluol azeotrop getrocknet. Das Rohprodukt wird aus Toluol/Methylenchlorid bei ca. 40°C ausgerührt und abgesaugt. Ausbeute: 146 g (690 mmol), 95 % der Theorie.

**e) 2-Dibenzofuran-1-yl-4,6-diphenyl-[1,3,5]triazin**

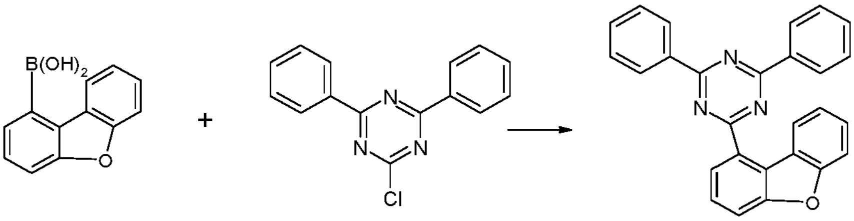

[3842-55-5]

23 g (110.0 mmol) Dibenzofuran-1-boronsäure, 29.5 g (110.0 mmol) 2-Chlor-4,6-diphenyl-1,3,5-triazin und 21 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan /Heptan umkristallisiert. Die Ausbeute beträgt 37 g (94 mmol), entsprechend 87 % der Theorie.

**f) 2-(8-Brom-dibenzofuran-1-yl)-4,6-diphenyl-[1,3,5]triazin**

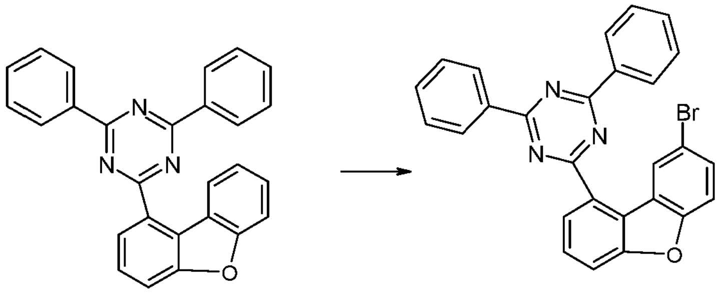

70 g (190.0 mmol) 2-Dibenzofuran-1-yl-4,6-diphenyl-[1,3,5]triazin werden in 2000 mL Essigsäure(100%) und 2000 mL Schwefelsäure (95-98%) suspendiert. Zu dieser Suspension werden portionsweise 34 g (190 mmol) NBS zugegeben und 2 h in Dunkelheit gerührt. Danach mit Wasser/Eis versetzt, der Feststoff abgetrennt und mit Ethanol nachgewaschen. Der Rückstand wird aus Toluol umkristallisiert. Die Ausbeute beträgt 80 g (167 mmol), entsprechend 87 % der Theorie.

**g) 3-[9-(4,6-Diphenyl-[1,3,5]triazin-2-yl)-dibenzofuran-2-yl]-9-phenyl-9H-carbazol**

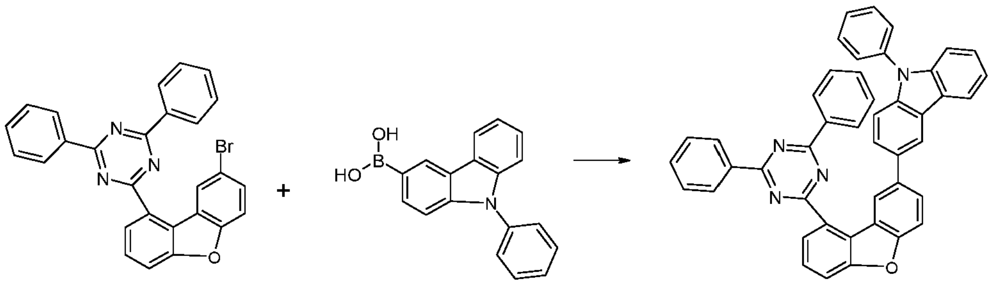

[854952-58-2] 1

75 g (156 mmol) 2-(8-Brom-dibenzofuran-1-yl)-4,6-diphenyl-[1,3,5]-triazin, 50 g (172 mmol) N-Phenyl-carbazol-3-boronsäure [854952-58-2] und 36 g (340 mmol) Natriumcarbonat werden in 1000 mL Ethylenglycoldiaminether und 280 mL Wasser suspendiert. Zu dieser Suspension werden 1.8 g (1.5 mmol) Tetrakis-(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Das Produkt wird via Säulenchromatographie an Kieselgel mit Toluol/Heptan (1:2) gereinigt und abschließend im Hochvakuum ($p = 5 \times 10^{-7}$ mbar) sublimiert (Reinheit 99,9%). Die Ausbeute beträgt 50 g (78mmol), entsprechend 50 % der Theorie.

Analog können die folgenden Verbindungen dargestellt werden. Hierbei kann zur Aufreinigung auch Säulenchromatographie verwendet werden, oder andere gängige Lösungsmittel wie n Heptan, Butanol, Aceton, Ethylacetat, Acetonitril, Toluol, Xylol, Dichlormethan, Methanol, N,N Dimethylformamid, Tetrahydrofuran, Ethylacetat, n Butylacetat 1,4 Dioxan können zur Umkristallisation oder Heißextraktion verwendet werden.

| | Edukt 1 | Edukt 2 | Produkt | Ausbeute |
|---|---|---|---|---|
| G1 | 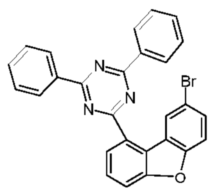 | 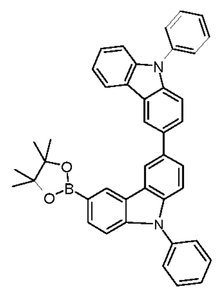 [1572537-61-1] | 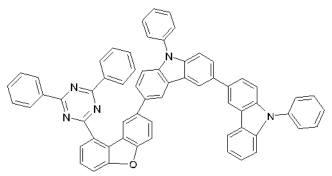 6 | 61% |
| G2 | 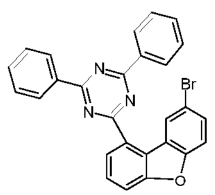 | 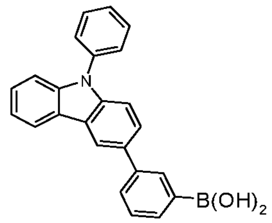 854952-60-6 | 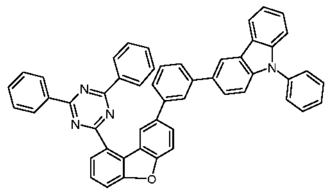 9 | 56% |
| G3 | 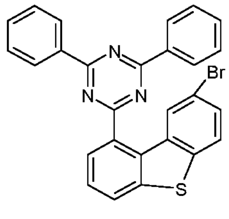 | 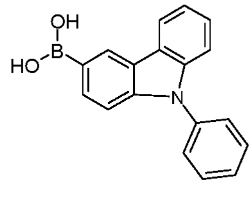 [854952-58-2] | 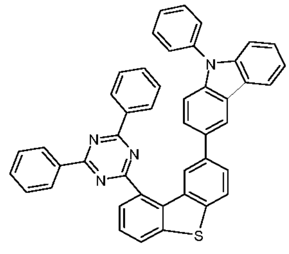 | 63% |

(fortgesetzt)

| | Edukt 1 | Edukt 2 | Produkt | Ausbeute |
|---|---|---|---|---|
| G4 | 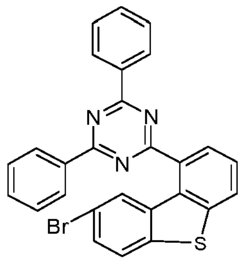 | 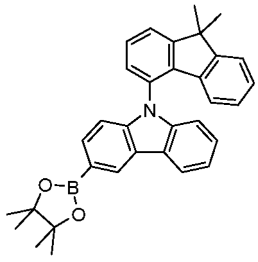 [1802588-7] | 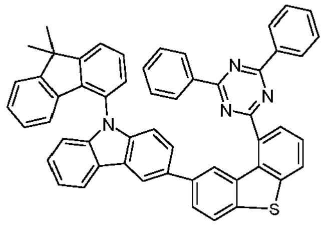 | 60% |
| G5 | 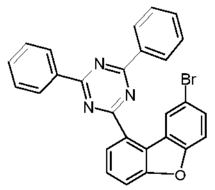 | 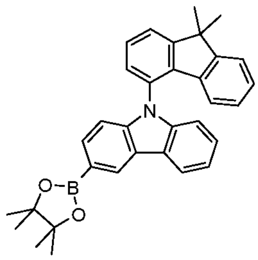 [1802588-7] | 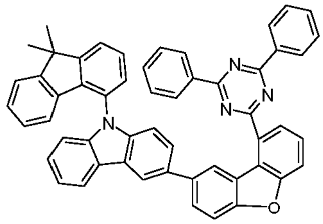 **17** | 65% |
| G6 | 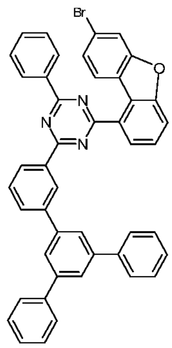 | 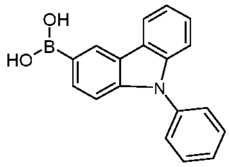 [854952-58-2] | 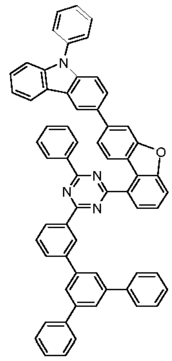 **8** | 54% |
| G7 | 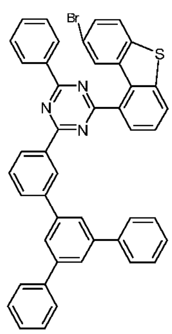 | 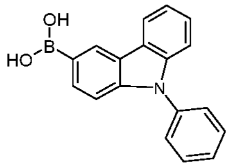 [854952-58-2] | 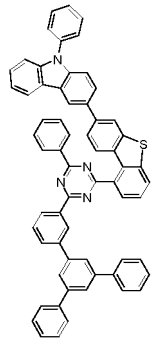 | 59% |

(fortgesetzt)

| | Edukt 1 | Edukt 2 | Produkt | Ausbeute |
|---|---|---|---|---|
| G8 | 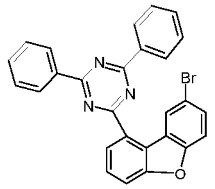 | 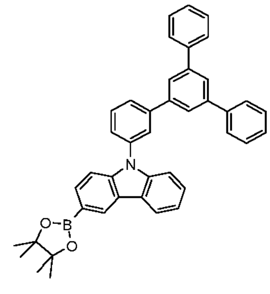 [1846559-20-3] | 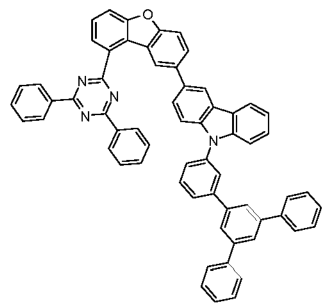 **16** | 60% |
| G9 | 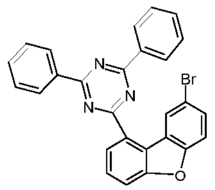 | 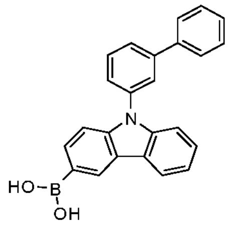 [1416814-68-0] | 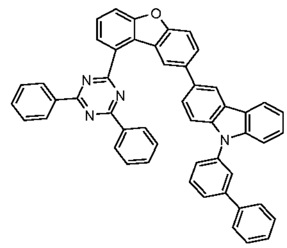 **13** | 62% |
| G10 | 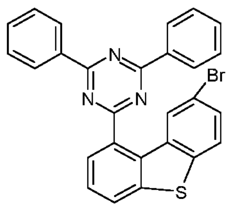 | 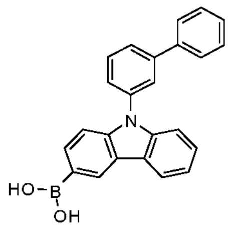 [1416814-68-0] | 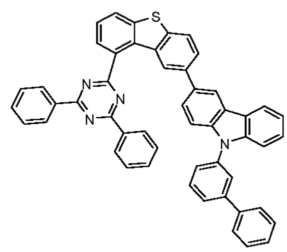 | 54% |
| G11 | 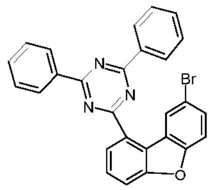 | 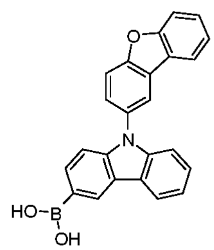 [1338488-91-7] | 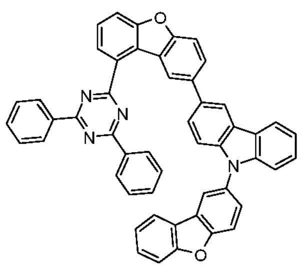 **14** | 52% |
| G12 | 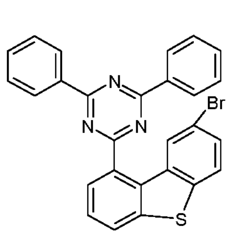 | 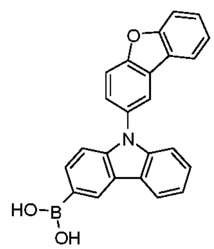 | 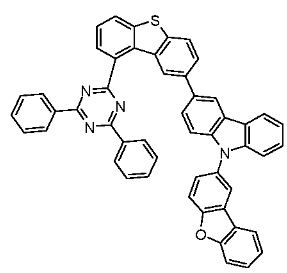 | 50% |

(fortgesetzt)

| | Edukt 1 | Edukt 2 | Produkt | Ausbeute |
|---|---|---|---|---|
| G13 | 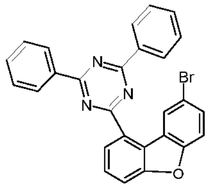 | 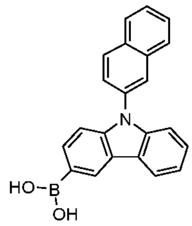 [1133057-98-3] | 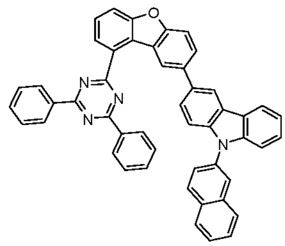 **12** | 62% |
| G14 | 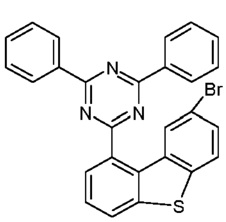 | 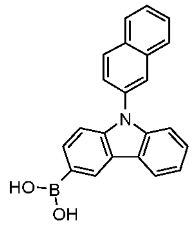 [1133057-98-3] | 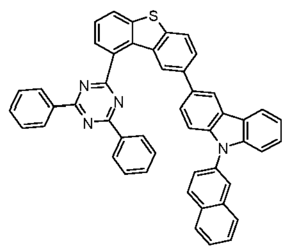 | 57% |
| G15 | 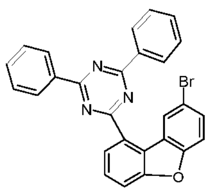 | 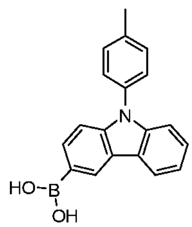 [731016-45-8] | 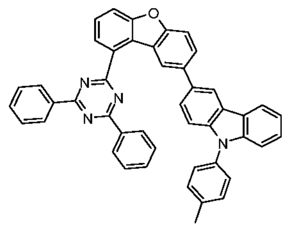 **18** | 62% |
| G16 | 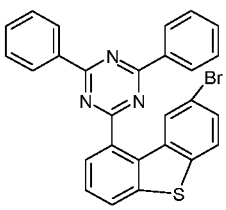 | 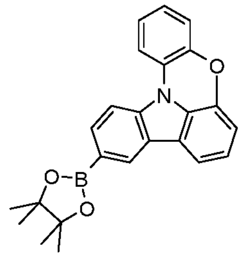 [1380485-64-2] | 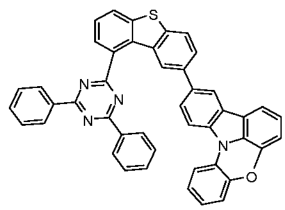 | 56% |
| G17 | 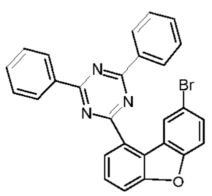 | 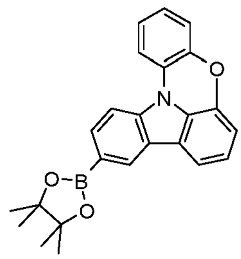 [1380485-64-2] | 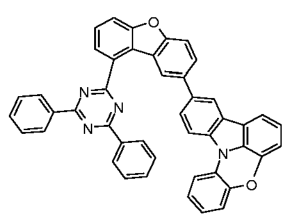 **21** | 52% |

(fortgesetzt)

| | Edukt 1 | Edukt 2 | Produkt | Ausbeute |
|---|---|---|---|---|
| G18 | 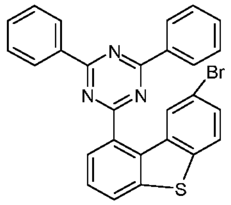 | 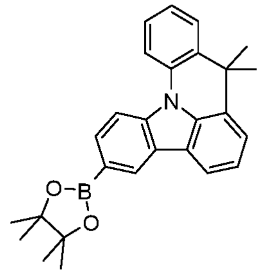 [1456606-40-8] | 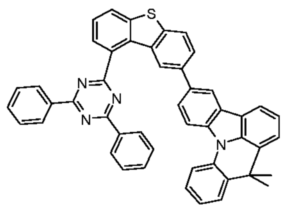 | 55% |
| G19 | 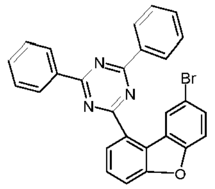 | 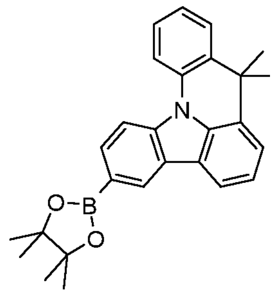 [1456606-40-8] | 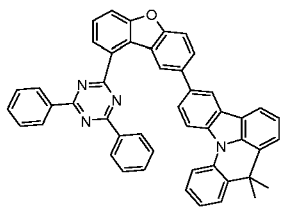 **20** | 60% |
| G20 | 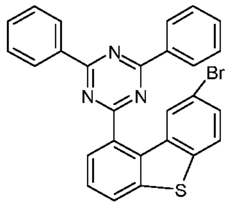 | 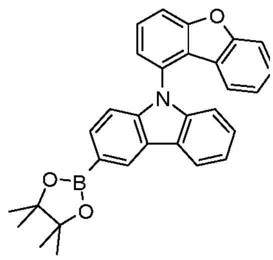 [1427160-10-8] | 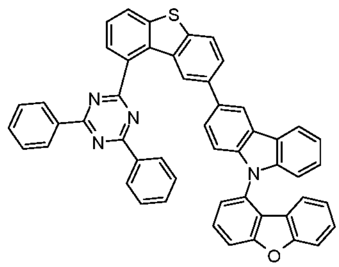 | 54% |
| G21 | 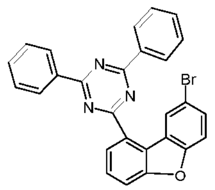 | 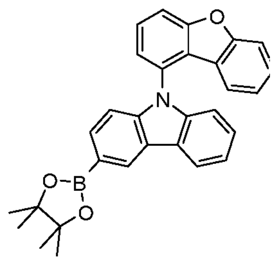 [1427160-10-8] | 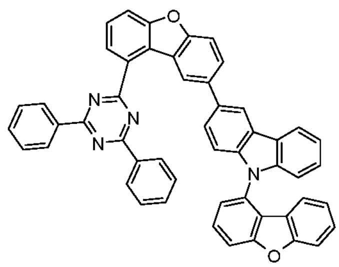 **15** | 56% |
| G50 | 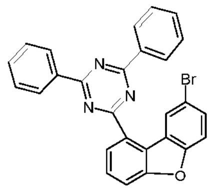 | 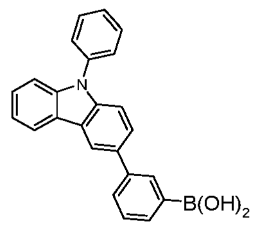 [854952-60-6] | 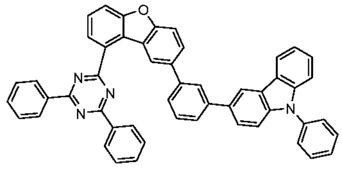 **9** | 62% |

(fortgesetzt)

| | Edukt 1 | Edukt 2 | Produkt | Ausbeute |
|---|---|---|---|---|
| G51 | 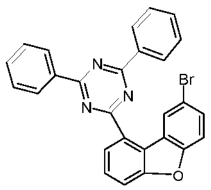 | 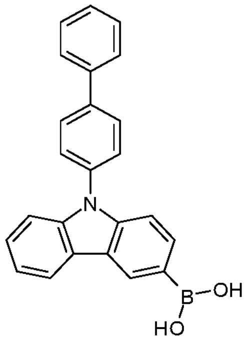 [1028648-22-7] | 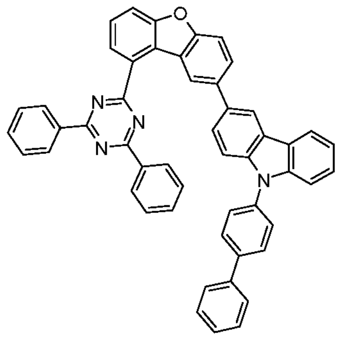 **11** | 66% |
| G52 | 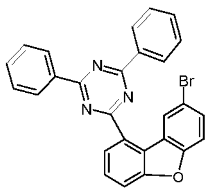 | 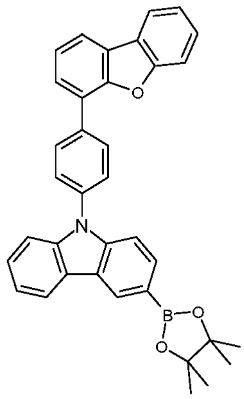 | 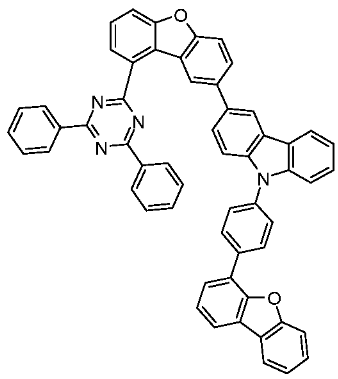 **4** | 55% |

Beispiel 3: Synthese der Verbindungen **89** (BisC2) und **90** (BisC3)

Verbindung **89** ist literaturbekannt und wird analog zu US 20150001488 hergestellt.

Verbindung **90** ist literaturbekannt und wird analog zu Physical Chemistry Chemical Physics, 17(37), 2015, 24468-24474 hergestellt.

Beispiel 4:

Analog zu Beispiel 2g) können die folgenden Verbindungen dargestellt werden. Hierbei kann zur Aufreinigung auch Säulenchromatographie verwendet werden, oder andere gängige Lösungsmittel wie n Heptan, Butanol, Aceton, Ethylacetat, Acetonitril, Toluol, Xylol, Dichlormethan, Methanol, N,N Dimethylformamid, Tetrahydrofuran, Ethylacetat, n Butylacetat 1,4 Dioxan können zur Umkristallisation oder Heißextraktion verwendet werden.

| | Edukt 1 | Edukt 2 | Produkt | Ausbeute |
|---|---|---|---|---|
| G23 | 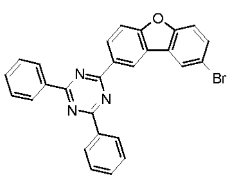 [2102445-25-8] | 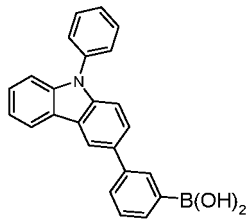 [854952-60-6] | 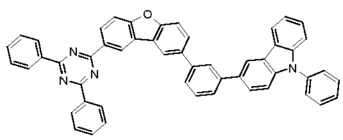 **26** | 62% |

(fortgesetzt)

| | Edukt 1 | Edukt 2 | Produkt | Ausbeute |
|---|---|---|---|---|
| G24 | 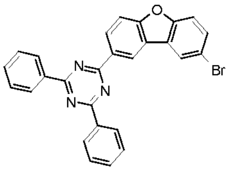 [2102445-25-8] | 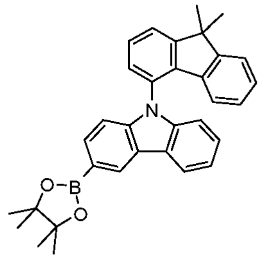 [1802588-7] | 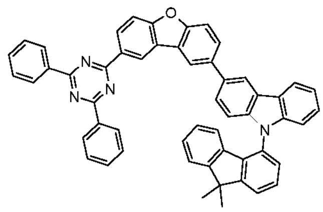 **32** | 67% |
| G25 | 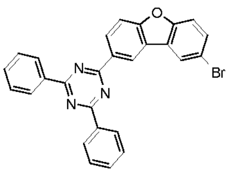 [2102445-25-8] | 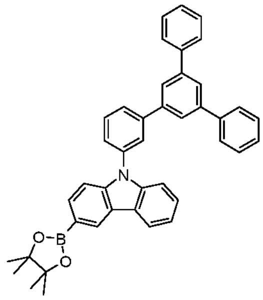 [1846559-20-3] | 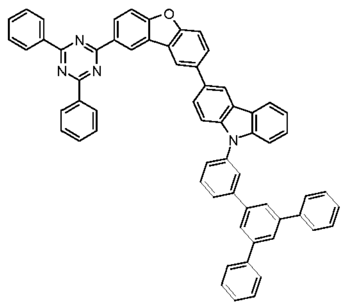 **29** | 64% |
| G26 | 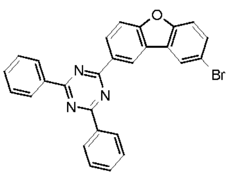 [2102445-25-8] | 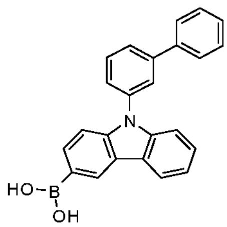 [1416814-68-0] | 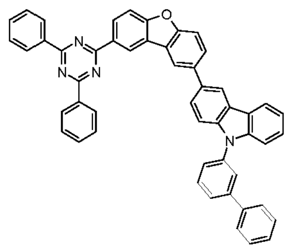 **30** | 58% |
| G27 | 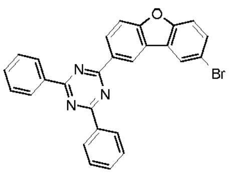 [2102445-25-8] | 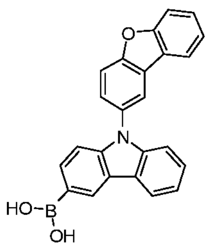 [1338488-91-7] | 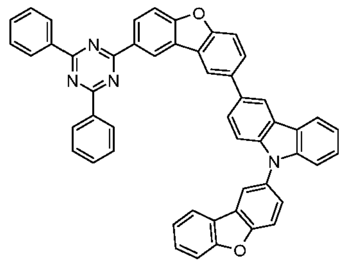 **41** | 53% |
| G28 | 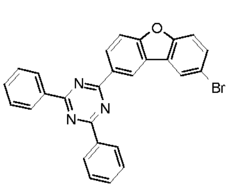 [2102445-25-8] | 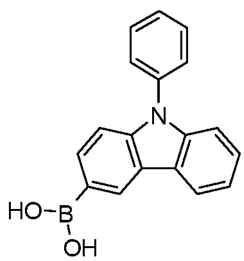 [854952-58-2] | 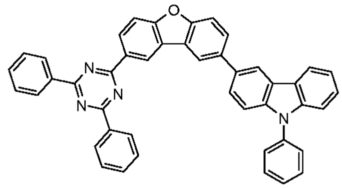 **25** | 70% |

(fortgesetzt)

| | Edukt 1 | Edukt 2 | Produkt | Ausbeute |
|---|---|---|---|---|
| G29 | 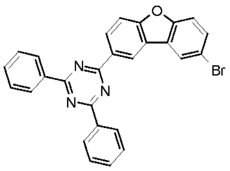 [2102445-25-8] | 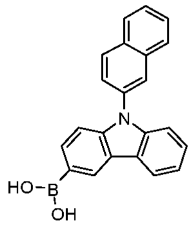 [1133057-98-3] | 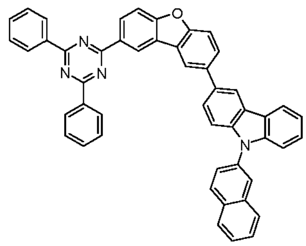 **44** | 59% |
| G30 | 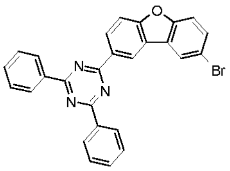 [2102445-25-8] | 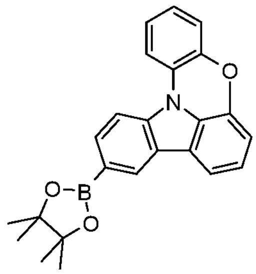 [1380485-64-2] | 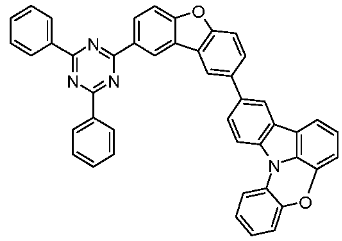 **24** | 68% |
| G31 | 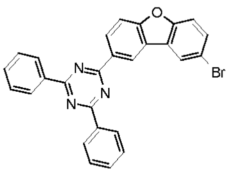 [2102445-25-8] | 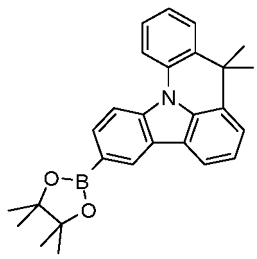 [1456606-40-8] | 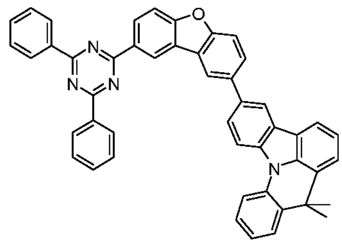 **38** | 53% |
| G32 | 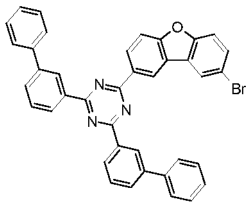 | 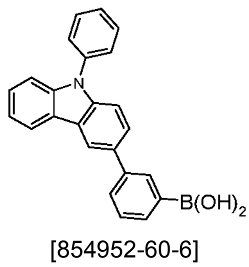 [854952-60-6] | 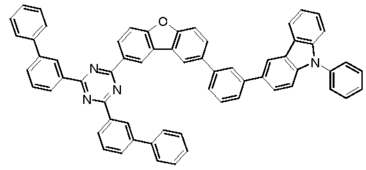 **43** | 50% |

Beispiel 5:

A)
Darstellung der Brom-Zwischenstufe analog zu Beispiel 2f) ausgehend von 2-(Dibenzo[b,d]furan-3-yl)-4,6-diphenyl-1,3,5-triazin [1651203-47-2]. Ausbeute 83%.

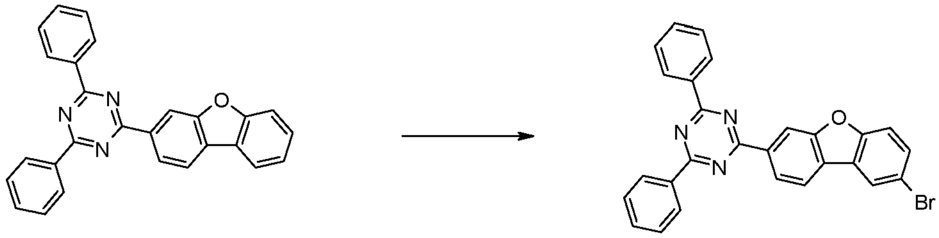

B)

Analog zu Beispiel 2g) können die folgenden Verbindungen dargestellt werden. Hierbei kann zur Aufreinigung auch Säulenchromatographie verwendet werden, oder andere gängige Lösungsmittel wie n Heptan, Butanol, Aceton, Ethylacetat, Acetonitril, Toluol, Xylol, Dichlormethan, Methanol, N,N Dimethylformamid, Tetrahydrofuran, Ethylacetat, n Butylacetat 1,4 Dioxan können zur Umkristallisation oder Heißextraktion verwendet werden.

| | Edukt 1 | Edukt 2 | Produkt | Ausbeute |
|---|---|---|---|---|
| G34 | 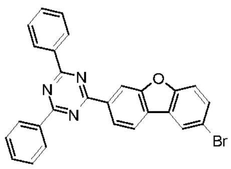 | 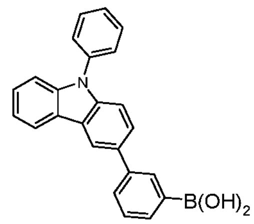 [854952-60-6] | 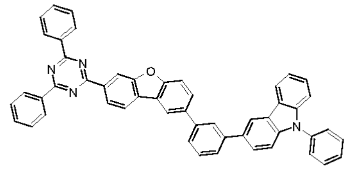 **48** | 63% |
| G35 | 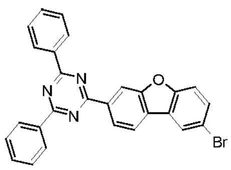 | 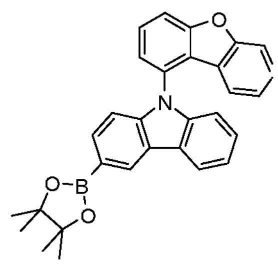 [1427160-10-8] | 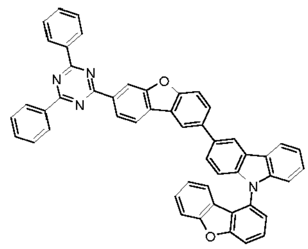 **53** | 56% |
| G36 | 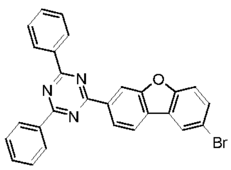 | 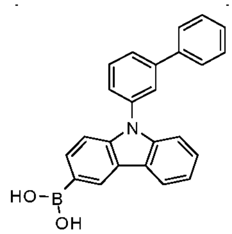 [1416814-68-0] | 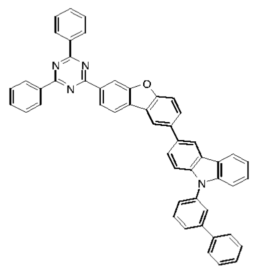 **55** | 66% |
| G37 | 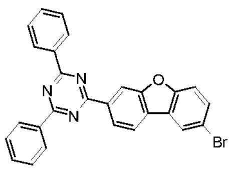 | 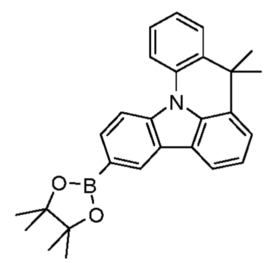 [1456606-40-8] | 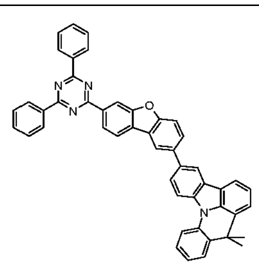 **63** | 58% |

(fortgesetzt)

| | Edukt 1 | Edukt 2 | Produkt | Ausbeute |
|---|---|---|---|---|
| G38 | 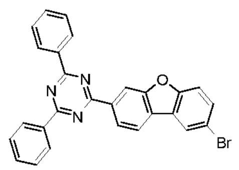 | 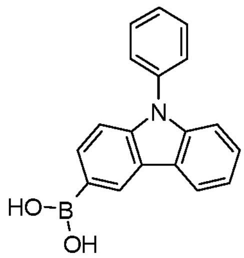 [854952-58-2] | 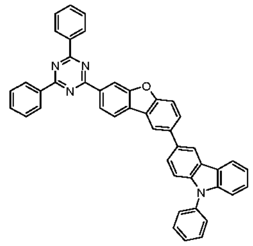 **47** | 65% |

Beispiel 6:

Analog zu Beispiel 2g) können die folgenden Verbindungen dargestellt werden. Hierbei kann zur Aufreinigung auch Säulenchromatographie verwendet werden, oder andere gängige Lösungsmittel wie n Heptan, Butanol, Aceton, Ethylacetat, Acetonitril, Toluol, Xylol, Dichlormethan, Methanol, N,N Dimethylformamid, Tetrahydrofuran, Ethylacetat, n Butylacetat 1,4 Dioxan können zur Umkristallisation oder Heißextraktion verwendet werden.

| | Edukt 1 | Edukt 2 | Produkt | Ausbeute |
|---|---|---|---|---|
| G40 | 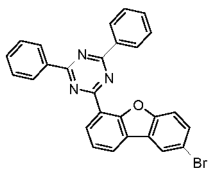 [1821221-55-9] | 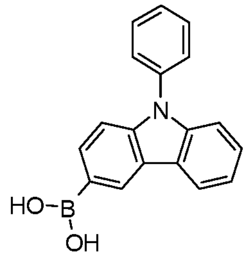 [854952-58-2] | 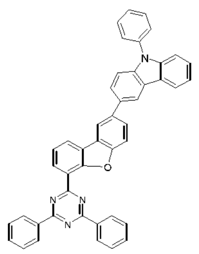 **69** | 58% |
| G41 | 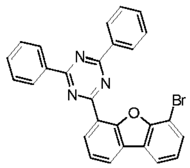 [1651196-06-3] | 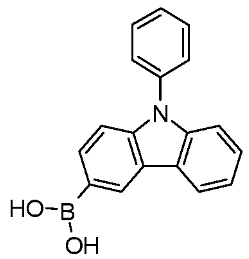 [854952-58-2] | 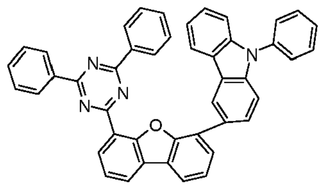 **67** | 49% |
| G42 | 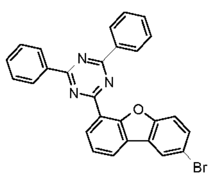 [1821221-55-9] | 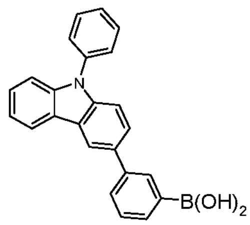 [854952-60-6] | 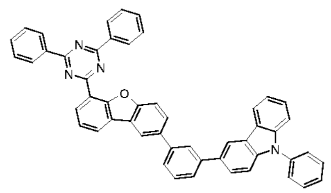 **70** | 66% |

(fortgesetzt)

| | Edukt 1 | Edukt 2 | Produkt | Ausbeute |
|---|---|---|---|---|
| G43 | 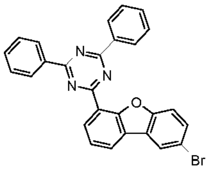 [1821221-55-9] | 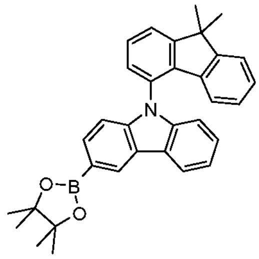 [1802588-7] | 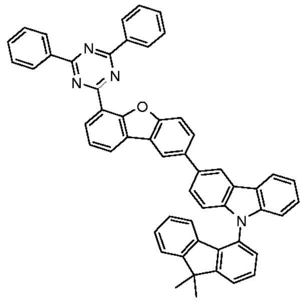 **76** | 47% |
| G44 | 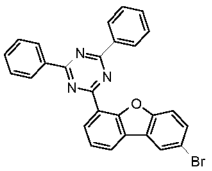 [1821221-55-9] | 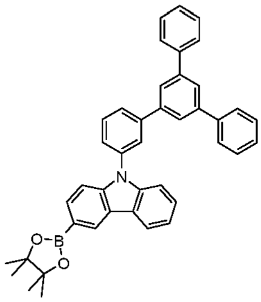 [1846559-20-3] | 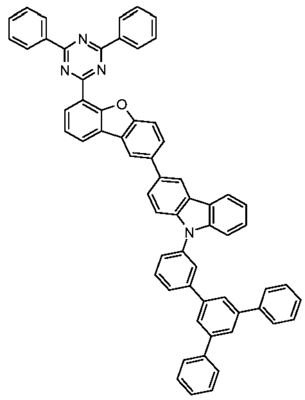 **88** | 51% |
| G45 | 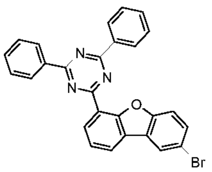 [1821221-55-9] | 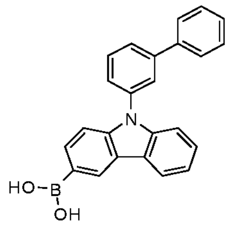 [1416814-68-0] | 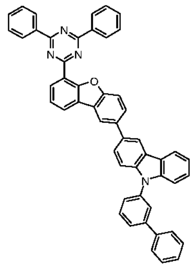 **74** | 60% |
| G46 | 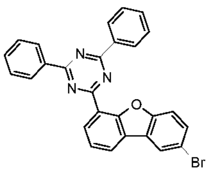 [1821221-55-9] | 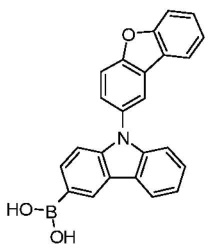 [1338488-91-7] | 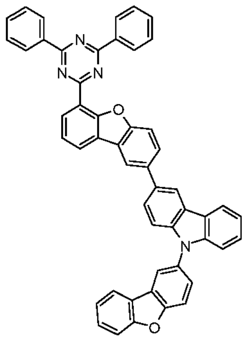 **73** | 49% |

(fortgesetzt)

| | Edukt 1 | Edukt 2 | Produkt | Ausbeute |
|---|---|---|---|---|
| G47 | 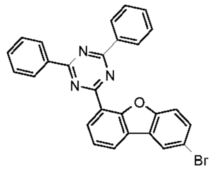 [1821221-55-9] | 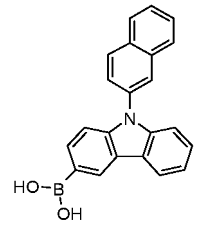 [1133057-98-3] | 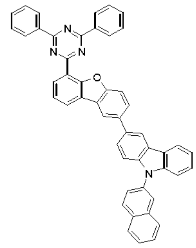 **72** | 57% |
| G48 | 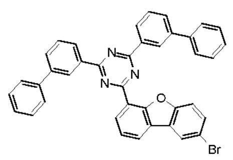 | 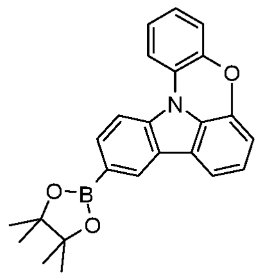 [1380485-64-2] | 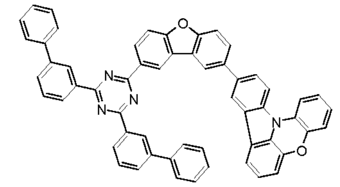 **68** | 41% |
| G49 | 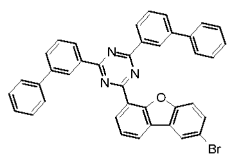 | 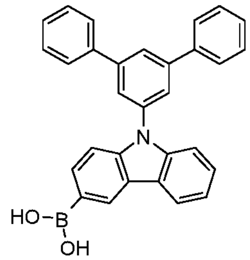 [1609267-51-7] | 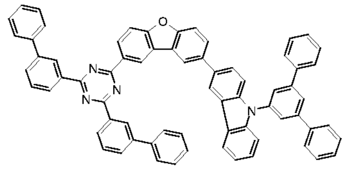 **77** | 53% |

## Patentansprüche

1. Zusammensetzung umfassend mindestens eine Verbindung der Formel (1e) und mindestens eine Verbindung der Formel (2a),

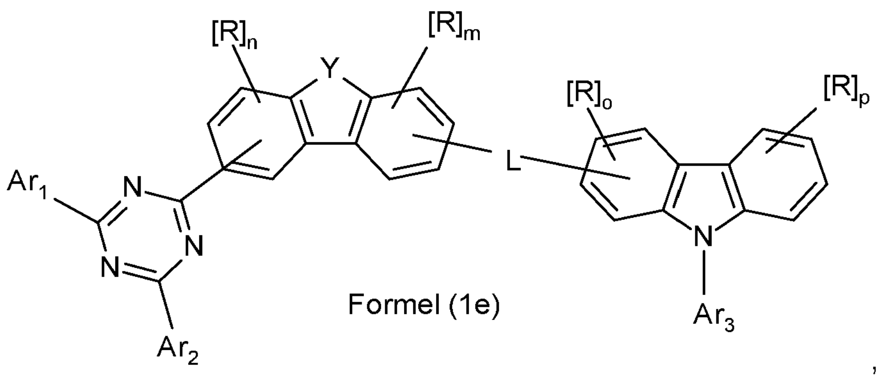

Formel (1e)

,

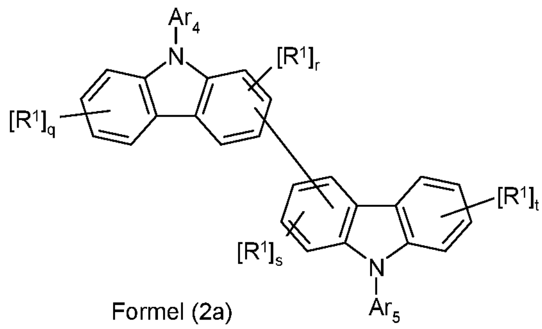

Formel (2a)

,

wobei für die verwendeten Symbole und Indizes gilt:

Y ist ausgewählt aus O oder S;

L ist bei jedem Auftreten gleich oder verschieden ein aromatisches oder heteroaromatisches Ringsystem mit 5 bis 30 aromatischen Ringatomen, das durch einen oder mehrere Reste $R^3$ substituiert sein kann, bevorzugt ein aromatisches oder heteroaromatisches Ringsystem mit 6 bis 18 C-Atomen, das durch einen oder mehrere Reste $R^3$ substituiert sein kann;

$Ar_1$, $Ar_2$ sind jeweils unabhängig voneinander bei jedem Auftreten eine Aryl- oder Heteroarylgruppe mit 5 bis 40 aromatischen Ringatomen, die mit einem oder mehreren Resten $R^3$ substituiert sein können;

$Ar_3$ ist ein aromatisches Ringsystem mit 6 bis 40 aromatischen Ringatomen oder ein heteroaromatisches Ringsystem mit 10 bis 40 aromatischen Ringatomen, das mit einem oder mehreren Resten $R^3$ substituiert sein kann;

$Ar_4$ und $Ar_5$ sind jeweils unabhängig voneinander ein aromatisches Ringsystem mit 6 bis 40 aromatischen Ringatomen oder ein heteroaromatisches Ringsystem mit 10 bis 40 aromatischen Ringatomen, das mit einem oder mehreren Resten $R^3$ substituiert sein kann, wobei das heteroaromatische Ringsystem, das mit einem oder mehreren $R^3$ substituiert sein kann, in der Gesamtheit nur ein N-Atom enthält oder in der Gesamtheit ein oder mehrere O- und/oder S-Atome enthält, mit der Maßgabe, dass $Ar_4$ und $Ar_5$ nicht gleichzeitig Phenyl sein können;

R ist bei jedem Auftreten gleich oder verschieden ausgewählt aus der Gruppe bestehend aus H, D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 aromatischen Ringatomen, das jeweils mit einem oder mehreren Resten $R^2$ substituiert sein kann, einer Aryloxy- oder Heteroaryloxygruppe mit 5 bis 40 aromatischen Ringatomen, die mit einem oder mehreren Resten $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^2$ substituiert sein kann;

$R^1$ in $[R^1]_q$ ist bei jedem Auftreten unabhängig Phenyl oder Biphenyl, wenn q größer 0 bedeutet;

$R^1$ in $[R^1]_t$ ist bei jedem Auftreten unabhängig Phenyl, Biphenyl oder Terphenyl, wenn t größer 0 bedeutet;

$R^2$ ist bei jedem Auftreten gleich oder verschieden ausgewählt aus der Gruppe bestehend aus H, D, F, Cl, Br, I, CN, $NO_2$, $N(Ar)_2$, $NH_2$, $N(R^3)_2$, C(=O)Ar, C(=O)H, $C(=O)R^3$, $P(=O)(Ar)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nicht-benachbarte $CH_2$-Gruppen durch HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH oder $CONR^3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 aromatischen Ringatomen, das jeweils mit einem oder mehreren Resten $R^3$ substituiert sein kann, einer Aryloxy- oder Heteroaryloxygruppe mit 5 bis 60 aromatischen Ringatomen, die mit einem oder mehreren Resten $R^3$ substituiert sein kann, oder einer Kombination dieser Systeme, wobei optional zwei oder mehr benachbarte Substituenten $R^2$ ein monocyclisches oder polycyclisches, aliphatisches, aromatisches oder heteroaromatisches Ringsystem bilden können, das mit einem oder mehreren Resten $R^3$ substituiert sein kann;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden;
Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^3$ substituiert sein kann; dabei können zwei Reste Ar, welche an dasselbe N-Atom, P-Atom oder B-Atom binden, auch durch eine Einfachbindung oder eine Brücke, ausgewählt aus $N(R^3)$, $C(R^3)_2$, O oder S, miteinander verbrückt sein und
n und m bedeuten unabhängig voneinander 0, 1, 2 oder 3;
p und o bedeuten jeweils unabhängig voneinander 0, 1, 2 oder 3;
q und t bedeuten jeweils unabhängig voneinander 0, 1, 2, 3 oder 4;
r ist 0 und
s ist 0.

2. Zusammensetzung nach Anspruch 1, **dadurch gekennzeichnet, dass** die Verbindung der Formel (1e) der Formel (1a), (1b), (1c) oder (1d) entspricht,

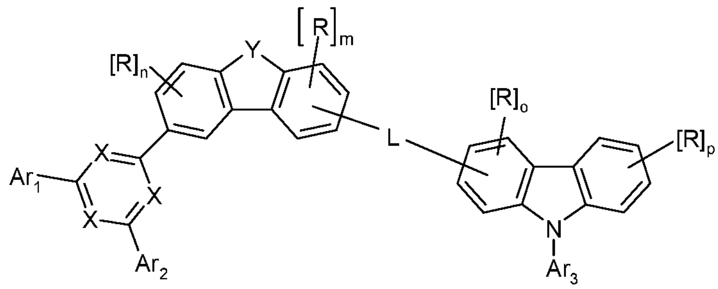

Formel (1a)

,

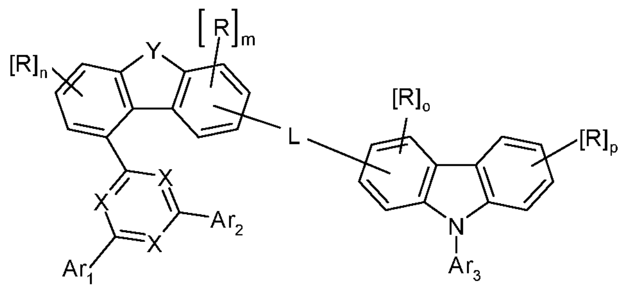

Formel (1b)

,

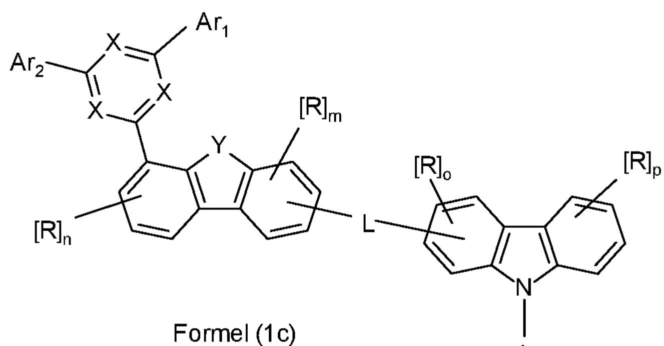

Formel (1c)

,

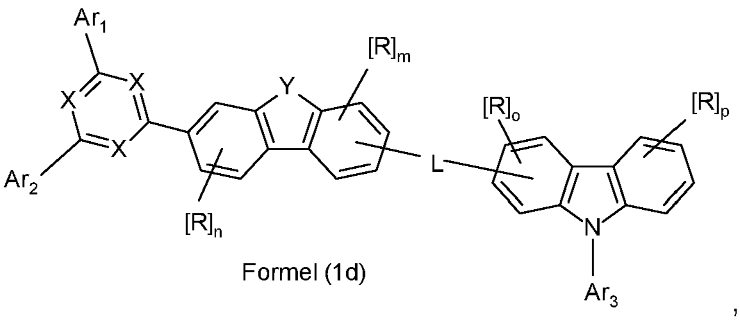

Formel (1d)

,

wobei der Substituent

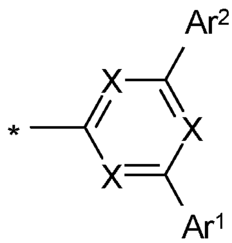

ein Triazin bedeutet.

**3.** Zusammensetzung nach Anspruch 1 oder 2, **dadurch gekennzeichnet, dass** L in den Verbindungen ausgewählt wird aus L-1 bis L-40, die unsubstituiert sind oder durch $R^3$ substituiert sein können,

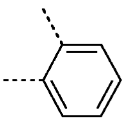
L-1

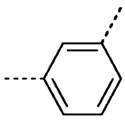
L-2

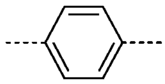
L-3

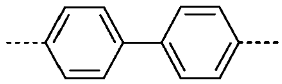
L-4

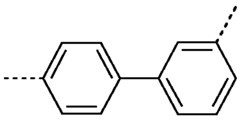
L-5

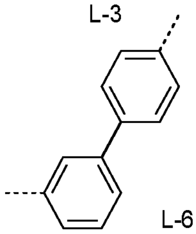
L-6

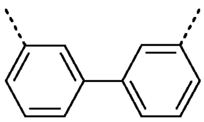
L-7

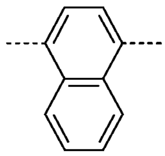
L-8

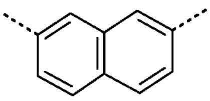
L-9

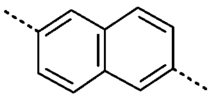
L-10

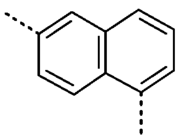
L-11

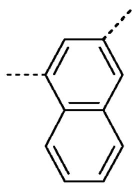
L-12

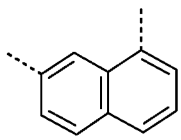
L-13

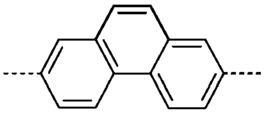
L-14

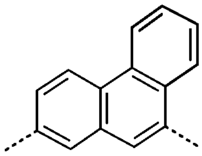
L-15

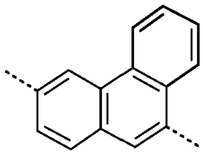
L-16

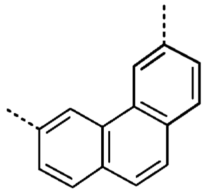
L-17

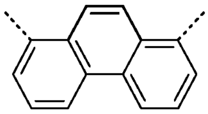
L-18

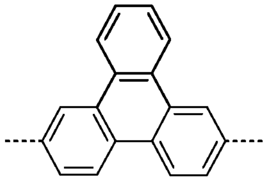
L-19

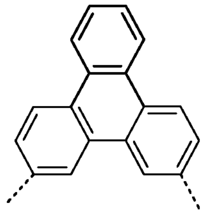
L-20

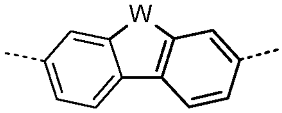
L-21

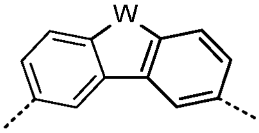
L-22

,

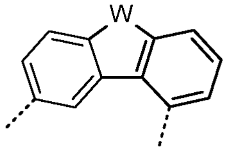
L-23

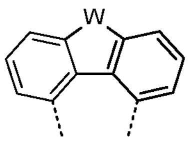
L-24

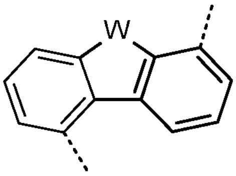
L-25

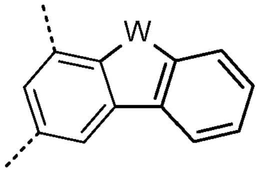
L-26

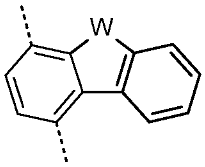
L-27

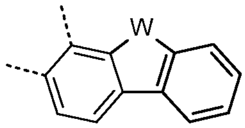
L-28

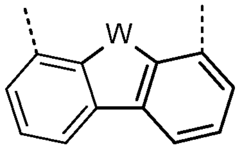
L-29

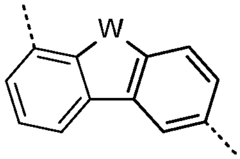
L-30

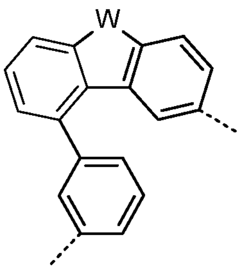
L-31

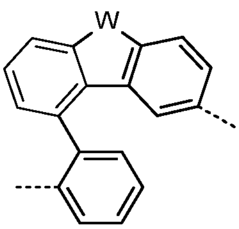
L-32

L-33

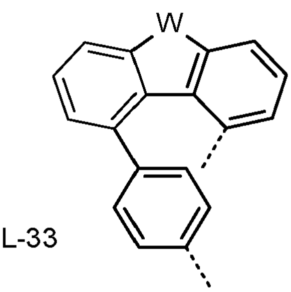

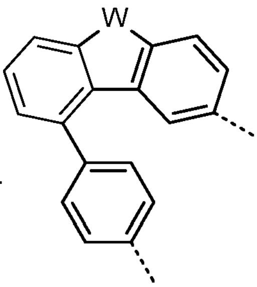

L-34

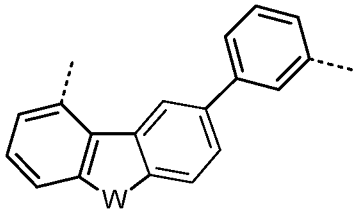

L-35

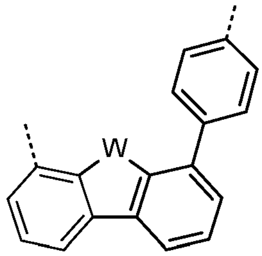

L-36

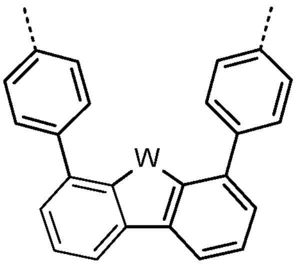

L-37

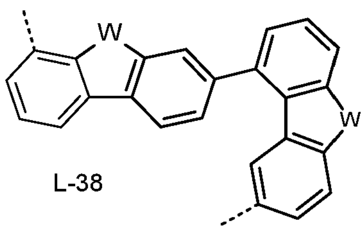

L-38

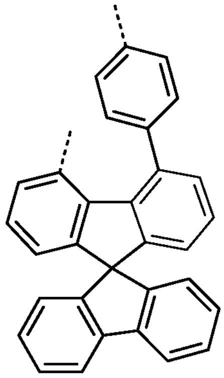

L-39

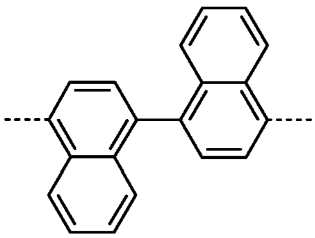

L-40 ,

wobei W O, S oder $C(R^0)_2$ bedeutet und
wobei R° in $C(R^0)_2$ Methyl oder Phenyl bedeutet.

**4.** Zusammensetzung nach einem oder mehreren der Ansprüche 1 bis 3, **dadurch gekennzeichnet, dass** einer der Substituenten $Ar_4$ oder $Ar_5$ ein aromatisches Ringsystem mit 6 bis 40 aromatischen Ringatomen oder ein heteroaromatisches Ringsystem mit 10 bis 40 aromatischen Ringatomen, das mit einem oder mehreren Resten $R^3$ substituiert sein kann, bedeutet, wobei das heteroaromatische Ringsystem, das mit einem oder mehreren $R^3$ substituiert sein kann, in der Gesamtheit nur ein N-Atom enthält oder in der Gesamtheit ein oder mehrere O- und/oder S-Atome enthält und der andere Substituent ein aromatisches Ringsystem mit 6 bis 40 aromatischen Ringatomen, das mit einem oder mehreren Resten $R^3$ substituiert sein kann, bedeutet, mit der Maßgabe, dass $Ar_4$ und $Ar_5$ nicht gleichzeitig Phenyl sein können.

**5.** Zusammensetzung nach einem oder mehreren der Ansprüche 1 bis 4, **dadurch gekennzeichnet, dass** die Substituenten $Ar_4$ oder $Ar_5$ jeweils unabhängig voneinander ein aromatisches Ringsystem mit 6 bis 40 aromatischen Ringatomen, das mit einem oder mehreren Resten $R^3$ substituiert sein kann, bedeuten, mit der Maßgabe, dass $Ar_4$ und $Ar_5$ nicht gleichzeitig Phenyl sind.

**6.** Zusammensetzung nach einem oder mehreren der Ansprüche 1 bis 5, **dadurch gekennzeichnet, dass** die Zusammensetzung mindestens eine weitere Verbindung enthält, die ausgewählt ist aus der Gruppe bestehend aus

Lochinjektionsmaterialien, Lochtransportmaterialien, Lochblockiermaterialien, *wide-band-gap*-Materialien, fluoreszierenden Emittern, phosphoreszierenden Emittern, Hostmaterialien, Elektronenblockiermaterialien, Elektronentransport-materialien und Elektroneninjektionsmaterialien, n-Dotanden und p-Dotanden.

**7.** Formulierung enthaltend eine Zusammensetzung nach einem oder mehreren der Ansprüche 1 bis 6 sowie mindestens ein Lösemittel.

**8.** Verwendung einer Zusammensetzung nach einem oder mehreren der Ansprüche 1 bis 6 in einer organischen elektronischen Vorrichtung.

**9.** Verwendung nach Anspruch 8, **dadurch gekennzeichnet, dass** die organische elektronische Vorrichtung aus der Gruppe von organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren und organischen Photorezeptoren ausgewählt wird.

**10.** Organische elektronische Vorrichtung enthaltend mindestens eine Zusammensetzung nach einem oder mehreren der Ansprüche 1 bis 6.

**11.** Vorrichtung nach Anspruch 10, **dadurch gekennzeichnet, dass** sie aus der Gruppe von organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren und organischen Photorezeptoren ausgewählt wird.

**12.** Vorrichtung nach Anspruch 10 oder 11, **dadurch gekennzeichnet, dass** es sich um eine Elektrolumineszenzvorrichtung handelt, die ausgewählt ist aus den organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und den organischen lichtemittierenden Dioden (OLEDs).

**13.** Vorrichtung nach einem oder mehreren der Ansprüche 10 bis 12, **dadurch gekennzeichnet, dass** diese die Zusammensetzung nach einem oder mehreren der Ansprüche 1 bis 6 in einer Emissionsschicht (EML), in einer Elektronentransportschicht (ETL), in einer Elektroneninjektionsschicht (EIL) und/oder in einer Lochblockierschicht (HBL) enthält.

**14.** Vorrichtung nach einem oder mehreren der Ansprüche 10 bis 13, **dadurch gekennzeichnet, dass** diese die Zusammensetzung nach einem oder mehreren der Ansprüche 1 bis 6 in der Emissionsschicht zusammen mit einem phosphoreszierenden Emitter enthält.

**15.** Verfahren zur Herstellung einer Vorrichtung nach einem oder mehreren der Ansprüche 10 bis 14, **dadurch gekennzeichnet, dass** mindestens eine organische Schicht enthaltend eine Zusammensetzung nach einem oder mehreren der Ansprüche 1 bis 6 durch Gasphasenabscheidung oder aus Lösung aufgebracht wird.

**16.** Verfahren nach Anspruch 15 **dadurch gekennzeichnet, dass** mindestens eine Verbindung der Formel (1e) und mindestens eine Verbindung der Formel (2a), wie in einem der Ansprüche 1 bis 6 beschrieben, nacheinander oder gleichzeitig aus mindestens zwei Materialquellen, gegebenenfalls mit weiteren Materialien, aus der Gasphase abgeschieden werden und die organische Schicht bilden.

**17.** Verfahren nach Anspruch 15, **dadurch gekennzeichnet, dass** die Zusammensetzung nach einem oder mehreren der Ansprüche 1 bis 6 als Materialquelle zur Gasphasenabscheidung genutzt wird und die organische Schicht bildet.

**18.** Verfahren nach Anspruch 15, **dadurch gekennzeichnet, dass** die Formulierung nach Anspruch 7 verwendet wird, um die organische Schicht aufzubringen.

## Claims

**1.** Composition comprising at least one compound of the formula (1e) and at least one compound of the formula (2a)

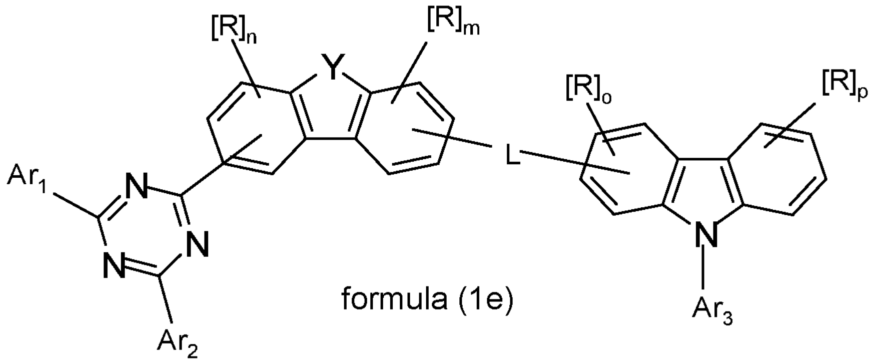

formula (1e)

,

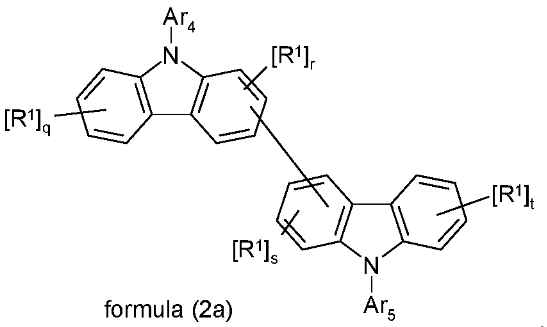

formula (2a)

,

where the following applies to the symbols and indices used:

Y is selected from O or S;
L is on each occurrence, identically or differently, an aromatic or heteroaromatic ring system having 5 to 30 aromatic ring atoms, which may be substituted by one or more radicals $R^3$, preferably an aromatic or heteroaromatic ring system having 6 to 18 C atoms, which may be substituted by one or more radicals $R^3$;
$Ar_1$, $Ar_2$ are in each case, independently of one another on each occurrence, an aryl or heteroaryl group having 5 to 40 aromatic ring atoms, which may be substituted by one or more radicals $R^3$;
$Ar_3$ is an aromatic ring system having 6 to 40 aromatic ring atoms or a heteroaromatic ring system having 10 to 40 aromatic ring atoms, which may be substituted by one or more radicals $R^3$;
$Ar_4$ and $Ar_5$ are in each case, independently of one another, an aromatic ring system having 6 to 40 aromatic ring atoms or a heteroaromatic ring system having 10 to 40 aromatic ring atoms, which may be substituted by one or more radicals $R^3$, where the heteroaromatic ring system, which may be substituted by one or more $R^3$, contains in totality only one N atom or contains in totality one or more O and/or S atoms, with the proviso that $Ar_4$ and $Ar_5$ cannot simultaneously be phenyl;
R is selected on each occurrence, identically or differently, from the group consisting of H, D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, a straight-chain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case be substituted by one or more radicals $R^2$, where one or more non-adjacent $CH_2$ groups may be replaced by $R^2C=CR^2$, $Si(R^2)_2$, C=O, C=S, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, an aromatic or heteroaromatic ring system having 5 to 40 aromatic ring atoms, which may in each case be substituted by one or more radicals $R^2$, an aryloxy or heteroaryloxy group having 5 to 40 aromatic ring atoms, which may be substituted by one or more radicals $R^2$, or an aralkyl or heteroaralkyl group having 5 to 40 aromatic ring atoms, which may be substituted by one or more radicals $R^2$; two substituents R, which are bonded to the same carbon atom or to adjacent carbon atoms, may optionally form a monocyclic or polycyclic, aliphatic, aromatic or heteroaromatic ring system, which may be substituted by one or more radicals $R^2$;
$R^1$ in $[R^1]_q$ is on each occurrence, independently, phenyl or biphenyl if q is greater than 0;
$R^1$ in $[R^1]_t$ is on each occurrence, independently, phenyl, biphenyl or terphenyl if t is greater than 0;
$R^2$ is selected on each occurrence, identically or differently, from the group consisting of H, D, F, Cl, Br, I, CN, $NO_2$, $N(Ar)_2$, $NH_2$, $N(R^3)_2$, C(=O)Ar, C(=O)H, $C(=O)R^3$, $P(=O)(Ar)_2$, a straight-chain alkyl, alkoxy or thioalkyl group having 1 to 40 C atoms or a branched or cyclic alkyl, alkoxy or thioalkyl group having 3 to 40 C atoms or

an alkenyl or alkynyl group having 2 to 40 C atoms, which may in each case be substituted by one or more radicals $R^3$, where one or more non-adjacent $CH_2$ groups may be replaced by HC=CH, $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH or $CONR^3$ and where one or more H atoms may be replaced by D, F, Cl, Br, I, CN or $NO_2$, an aromatic or heteroaromatic ring system having 5 to 60 aromatic ring atoms, which may in each case be substituted by one or more radicals $R^3$, an aryloxy or heteroaryloxy group having 5 to 60 aromatic ring atoms, which may be substituted by one or more radicals $R^3$, or a combination of these systems, where two or more adjacent substituents $R^2$ may optionally form a monocyclic or polycyclic, aliphatic, aromatic or heteroaromatic ring system, which may be substituted by one or more radicals $R^3$;

$R^3$ is selected on each occurrence, identically or differently, from the group consisting of H, D, F, CN, an aliphatic hydrocarbon radical having 1 to 20 C atoms or an aromatic or heteroaromatic ring system having 5 to 30 aromatic ring atoms, in which one or more H atoms may be replaced by D, F, Cl, Br, I or CN and which may be substituted by one or more alkyl groups, in each case having 1 to 4 carbon atoms; two or more adjacent substituents $R^3$ may form a mono- or polycyclic, aliphatic ring system with one another;

Ar is on each occurrence, identically or differently, an aromatic or heteroaromatic ring system having 5 to 30 aromatic ring atoms, which may be substituted by one or more non-aromatic radicals $R^3$; two radicals Ar which are bonded to the same N atom, P atom or B atom may also be bridged to one another by a single bond or a bridge selected from $N(R^3)$, $C(R^3)_2$, O or S, and

n and m, independently of one another, denote 0, 1, 2 or 3;

p and o in each case, independently of one another, denote 0, 1, 2 or 3;

q and t in each case, independently of one another, denote 0, 1, 2, 3 or 4;

r is 0 and

s is 0.

2. Composition according to Claim 1, **characterised in that** the compound of the formula (1e) corresponds to the formula (1a), (1b), (1c) or (1d),

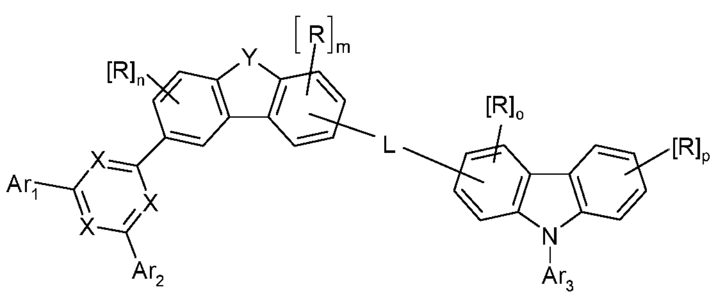

formula (1a) ,

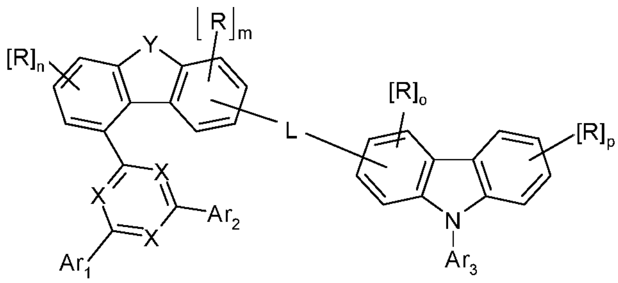

formula (1b) ,

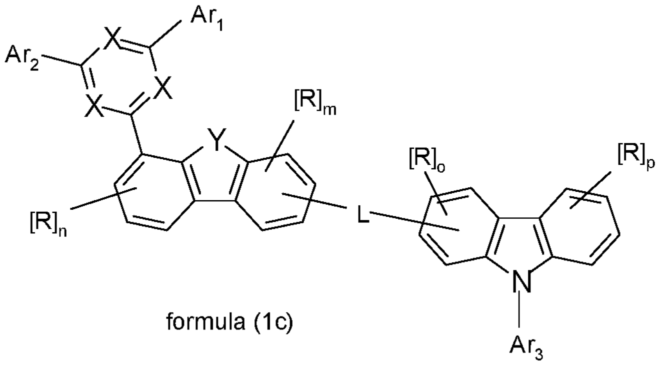

formula (1c)

,

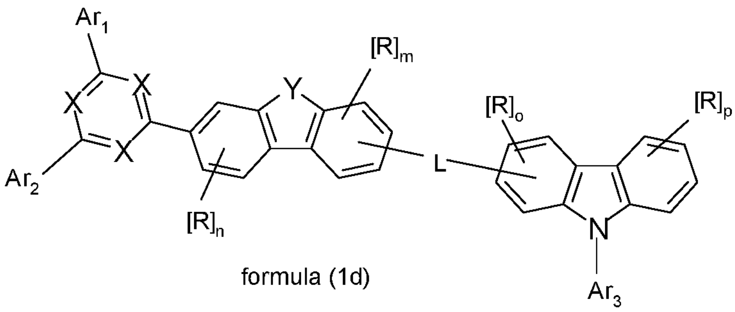

formula (1d)

,

where the substituent

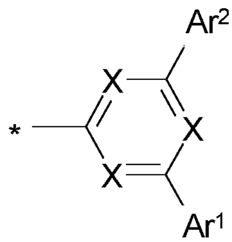

denotes a triazine.

**3.** Composition according to Claim 1 or 2, **characterised in that** L in the compounds is selected from L-1 to L-40, which are unsubstituted or may be substituted by $R^3$,

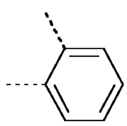

L-1

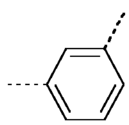

L-2

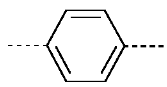

L-3

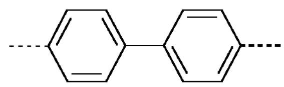

L-4

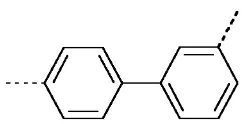

L-5

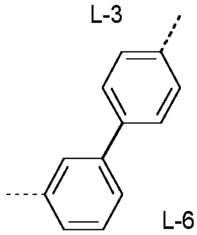

L-6

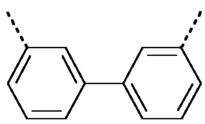

L-7

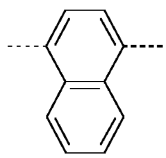

L-8

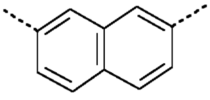
L-9

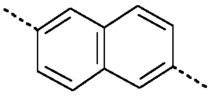
L-10

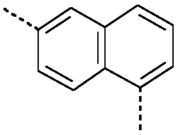
L-11

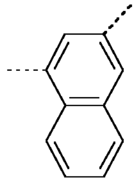
L-12

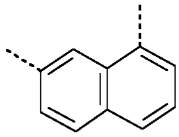
L-13

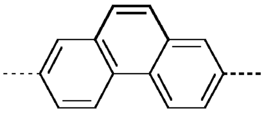
L-14

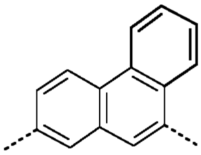
L-15

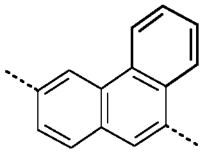
L-16

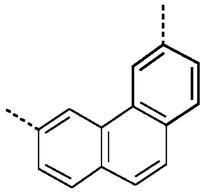
L-17

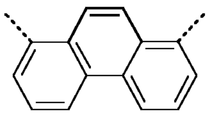
L-18

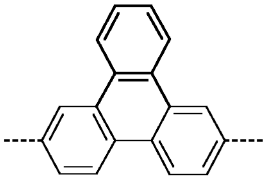
L-19

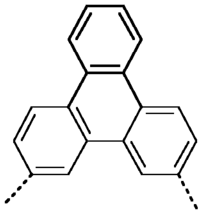
L-20

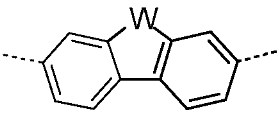
L-21

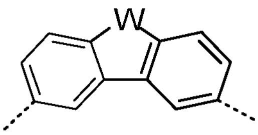
L-22

,

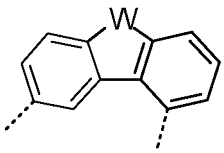
L-23

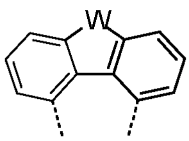
L-24

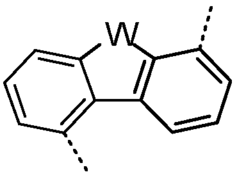
L-25

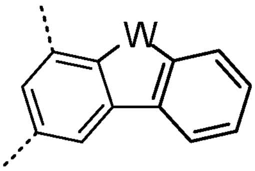
L-26

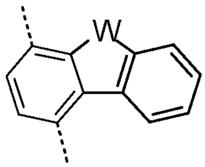
L-27

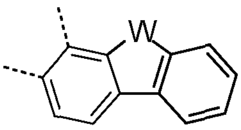
L-28

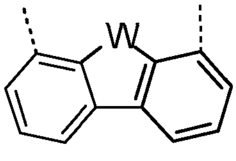
L-29

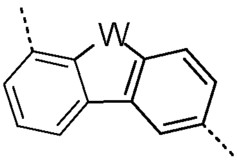
L-30

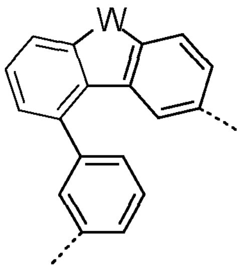

L-31

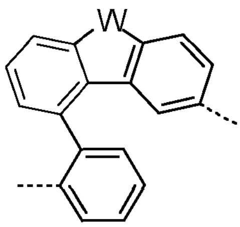

L-32

L-33

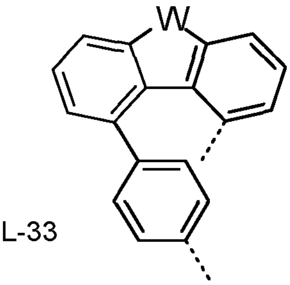

L-34

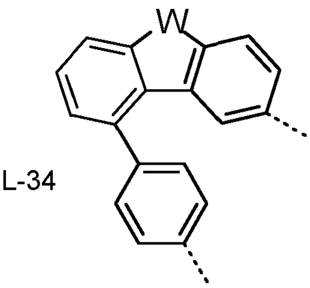

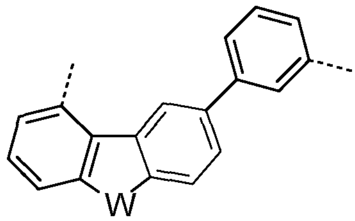

L-35

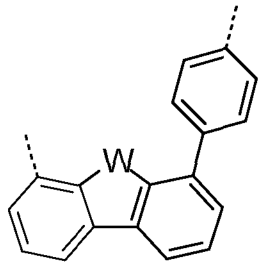

L-36

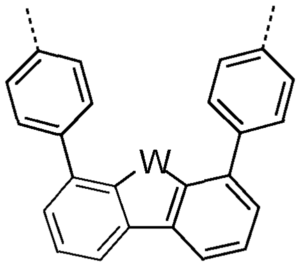

L-37

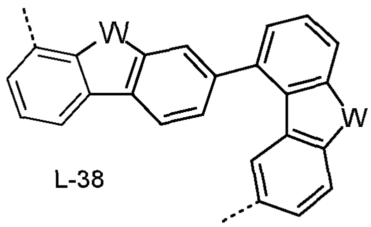

L-38

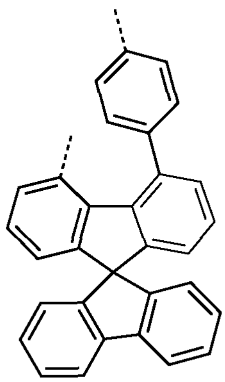

L-39

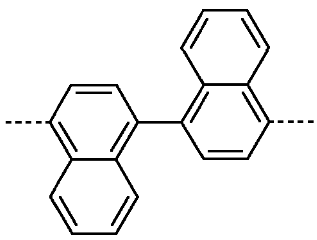

L-40 ,

where W denotes O, S or $C(R^0)_2$ and
where $R^0$ in $C(R^0)_2$ denotes methyl or phenyl.

**4.** Composition according to one or more of Claims 1 to 3, **characterised in that** one of the substituents $Ar_4$ or $Ar_5$ denotes an aromatic ring system having 6 to 40 aromatic ring atoms or a heteroaromatic ring system having 10 to 40 aromatic ring atoms, which may be substituted by one or more radicals $R^3$, where the heteroaromatic ring system, which may be substituted by one or more $R^3$, contains in totality only one N atom or contains in totality one or more O and/or S atoms, and the other substituent denotes an aromatic ring system having 6 to 40 aromatic ring atoms,

which may be substituted by one or more radicals $R^3$, with the proviso that $Ar_4$ and $Ar_5$ cannot simultaneously be phenyl.

5. Composition according to one or more of Claims 1 to 4, **characterised in that** the substituents $Ar_4$ and $Ar_5$ in each case, independently of one another, denote an aromatic ring system having 6 to 40 aromatic ring atoms, which may be substituted by one or more radicals $R^3$, with the proviso that $Ar_4$ and $Ar_5$ are not simultaneously phenyl.

6. Composition according to one or more of Claims 1 to 5, **characterised in that** the composition comprises at least one further compound selected from the group consisting of hole-injection materials, hole-transport materials, hole-blocking materials, wide band-gap materials, fluorescent emitters, phosphorescent emitters, host materials, electron-blocking materials, electron-transport materials and electron-injection materials, n-dopants and p-dopants.

7. Formulation comprising a composition according to one or more of Claims 1 to 6 and at least one solvent.

8. Use of a composition according to one or more of Claims 1 to 6 in an organic electronic device.

9. Use according to Claim 8, **characterised in that** the organic electronic device is selected from the group of organic integrated circuits (OICs), organic field-effect transistors (OFETs), organic thin-film transistors (OTFTs), organic electroluminescent devices, organic solar cells (OSCs), organic optical detectors and organic photoreceptors.

10. Organic electronic device containing at least one composition according to one or more of Claims 1 to 6.

11. Device according to Claim 10, **characterised in that** it is selected from the group of organic integrated circuits (OICs), organic field-effect transistors (OFETs), organic thin-film transistors (OTFTs), organic electroluminescent devices, organic solar cells (OSCs), organic optical detectors and organic photoreceptors.

12. Device according to Claim 10 or 11, **characterised in that** it is an electroluminescent device selected from organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs).

13. Device according to one or more of Claims 10 to 12, **characterised in that** it contains the composition according to one or more of Claims 1 to 6 in an emission layer (EML), in an electron-transport layer (ETL), in an electron-injection layer (EIL) and/or in a hole-blocking layer (HBL).

14. Device according to one or more of Claims 10 to 13, **characterised in that** it contains the composition according to one or more of Claims 1 to 6 in the emission layer together with a phosphorescent emitter.

15. Process for the production of a device according to one or more of Claims 10 to 14, **characterised in that** at least one organic layer comprising a composition according to one or more of Claims 1 to 6 is applied by gas-phase deposition or from solution.

16. Process according to Claim 15, **characterised in that** at least one compound of the formula (1e) and at least one compound of the formula (2a), as described in one of Claims 1 to 6, are deposited from the gas phase successively or simultaneously from at least two material sources, optionally with further materials, and form the organic layer.

17. Process according to Claim 15, **characterised in that** the composition according to one or more of Claims 1 to 6 is utilised as material source for the gas-phase deposition and forms the organic layer.

18. Process according to Claim 15, **characterised in that** the formulation according to Claim 7 is used in order to apply the organic layer.

## Revendications

1. Composition comprenant au moins un composé de formule (1e) et au moins un composé de formule (2a)

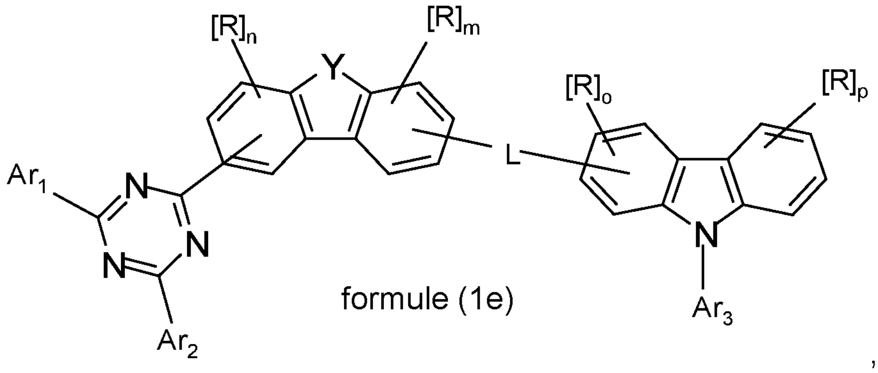

formule (1e)

,

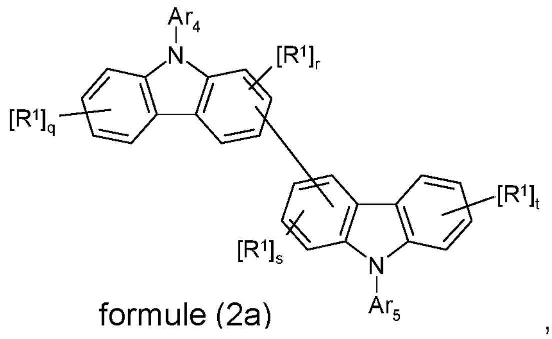

formule (2a)

,

dans laquelle ce qui suit s'applique aux symboles et indices utilisés :

Y est choisi parmi O ou S ;

L est à chaque occurrence, de manière identique ou différente, un noyau aromatique ou hétéroaromatique ayant de 5 à 30 atomes de cycle aromatique, qui peut être substitué par un ou plusieurs radicaux $R^3$, préférablement un noyau aromatique ou hétéroaromatique ayant de 6 à 18 atomes de C, qui peut être substitué par un ou plusieurs radicaux $R^3$ ;

$Ar_1$, $Ar_2$ sont dans chaque cas, indépendamment l'un de l'autre à chaque occurrence, un groupement aryle ou hétéroaryle ayant de 5 à 40 atomes de cycle aromatique, qui peut être substitué par un ou plusieurs radicaux $R^3$ ;

$Ar_3$ est un noyau aromatique ayant de 6 à 40 atomes de cycle aromatique ou un noyau hétéroaromatique ayant de 10 à 40 atomes de cycle aromatique, qui peut être substitué par un ou plusieurs radicaux $R^3$ ;

$Ar_4$ et $Ar_5$ sont dans chaque cas, indépendamment l'un de l'autre, un noyau aromatique ayant de 6 à 40 atomes de cycle aroma-tique ou un noyau hétéroaromatique ayant de 10 à 40 atomes de cycle aromatique, qui peut être substitué par un ou plusieurs radicaux $R^3$, où le noyau hétéroaromatique, qui peut être substitué par un ou plusieurs $R^3$, ne contient au total qu'un seul atome de N ou contient au total un ou plusieurs atomes de O et/ou S, à condition que $Ar_4$ et $Ar_5$ ne puissent être simultanément phényle ;

R est choisi à chaque occurrence, de manière identique ou différente, dans le groupe constitué par H, D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ayant de 2 à 20 atomes de C, qui peut dans chaque cas être substitué par un ou plusieurs radicaux $R^2$, où un ou plusieurs groupements $CH_2$ non adjacents peuvent être remplacés par $R^2C=CR^2$, $Si(R^2)_2$, C=O, C=S, $C=NR^2$, $P(=O)(R^2)$, SO, $SO_2$, $NR^2$, O, S ou $CONR^2$ et où un ou plusieurs atomes de H peuvent être remplacés par D, F, Cl, Br, I, CN ou $NO_2$, un noyau aromatique ou hétéroaromatique ayant de 5 à 40 atomes de cycle aromatique, qui peut dans chaque cas être substitué par un ou plusieurs radicaux $R^2$, un groupement aryloxy ou hétéroaryloxy ayant de 5 à 40 atomes de cycle aromatique, qui peut être substitué par un ou plusieurs radicaux $R^2$, ou un groupement aralkyle ou hétéroaralkyle ayant de 5 à 40 atomes de cycle aromatique, qui peut être substitué par un ou plusieurs radicaux $R^2$ ; deux substituants R, qui sont liés au même atome de carbone ou à des atomes de carbone adjacents, peuvent éventuellement former un noyau aliphatique, aromatique ou hétéroaromatique, monocyclique ou polycyclique, qui peut être substitué par un ou plusieurs radicaux $R^2$ ;

$R^1$ dans $[R^1]_q$ est à chaque occurrence, indépendamment, phényle ou biphényle si q est supérieur à 0 ;

$R^1$ dans $[R^1]_t$ est à chaque occurrence, indépendamment, phényle, biphényle ou terphényle si t est supérieur à 0 ;

$R^2$ est choisi à chaque occurrence, de manière identique ou différente, dans le groupe constitué par H, D, F,

Cl, Br, I, CN, $NO_2$, $N(Ar)_2$, $NH_2$, $N(R^3)_2$, C(=O)Ar, C(=O)H, $C(=O)R^3$, $P(=O)(Ar)_2$, un groupement alkyle, alcoxy ou thioalkyle à chaîne linéaire ayant de 1 à 40 atomes de C ou un groupement alkyle, alcoxy ou thioalkyle ramifié ou cyclique ayant de 3 à 40 atomes de C ou un groupement alcényle ou alcynyle ayant de 2 à 40 atomes de C, qui peut dans chaque cas être substitué par un ou plusieurs radicaux $R^3$, où un ou plusieurs groupements $CH_2$ non adjacents peuvent être remplacés par HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH ou $CONR^3$ et où un ou plusieurs atomes de H peuvent être remplacés par D, F, Cl, Br, I, CN ou $NO_2$, un noyau aromatique ou hétéroaromatique ayant de 5 à 60 atomes de cycle aromatique, qui peut dans chaque cas être substitué par un ou plusieurs radicaux $R^3$, un groupement aryloxy ou hétéroaryloxy ayant de 5 à 60 atomes de cycle aromatique, qui peut être substitué par un ou plusieurs radicaux $R^3$, ou une combinaison de ces systèmes, où deux, ou plus, substituants $R^2$ adjacents peuvent éventuellement former un noyau aliphatique, aromatique ou hétéroaromatique, monocyclique ou polycy-clique, qui peut être substitué par un ou plusieurs radicaux $R^3$ ;

$R^3$ est choisi à chaque occurrence, de manière identique ou diffé-rente, dans le groupe constitué par H, D, F, CN, un radical hydrocarboné aliphatique ayant de 1 à 20 atomes de C ou un noyau aromatique ou hétéroaromatique ayant de 5 à 30 atomes de cycle aromatique, dans lequel un ou plusieurs atomes de H peuvent être remplacés par D, F, Cl, Br, I ou CN et qui peut être substitué par un ou plusieurs groupements alkyle, dans chaque cas ayant de 1 à 4 atomes de carbone ; deux, ou plus, substituants $R^3$ adjacents peuvent former un noyau aliphatique mono- ou polycyclique, les uns avec les autres ;

Ar est à chaque occurrence, de manière identique ou différente, un noyau aromatique ou hétéroaromatique ayant de 5 à 30 atomes de cycle aromatique, qui peut être substitué par un ou plusieurs radicaux $R^3$ non aromatiques ; deux radicaux Ar qui sont liés au même atome de N, atome de P ou atome de B peuvent également être pontés l'un avec l'autre par une liaison simple ou un pont choisi parmi $N(R^3)$, $C(R^3)_2$, O ou S, et

n et m, indépendamment l'un de l'autre, désignent 0, 1, 2 ou 3 ;

p et o dans chaque cas, indépendamment l'un de l'autre, désignent 0, 1, 2 ou 3 ;

q et t dans chaque cas, indépendamment l'un de l'autre, désignent 0, 1, 2, 3 ou 4 ;

r vaut 0 et

s vaut 0.

2. Composition selon la revendication 1, **caractérisée en ce que** le composé de formule (1e) correspond à la formule (1a), (1b), (1c) ou (1d),

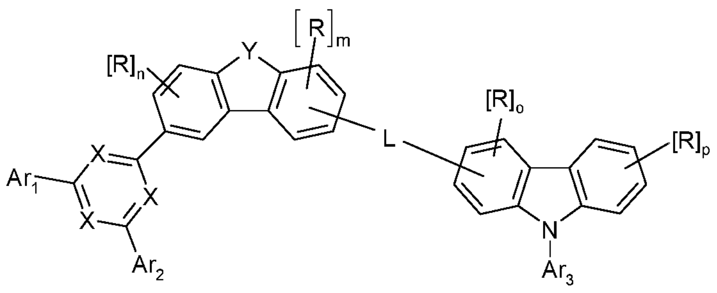

formule (1a)

,

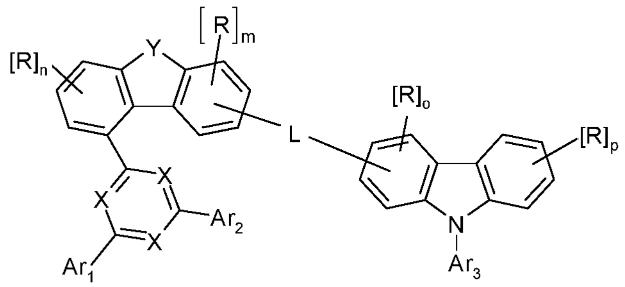

formule (1b)

,

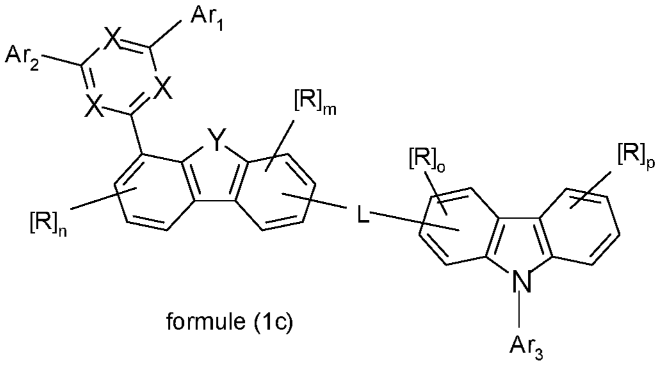

formule (1c)

,

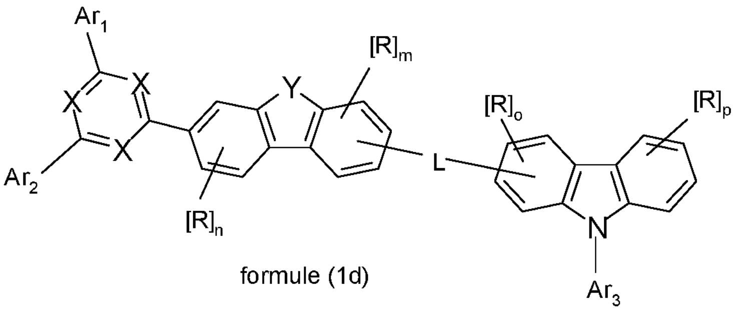

formule (1d)

,

dans laquelle le substituant

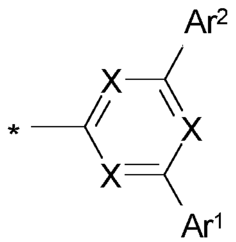

désigne une triazine.

**3.** Composition selon la revendication 1 ou 2, **caractérisée en ce que** L dans les composés est choisi parmi L-1 à L-40, qui sont non substitués ou peuvent être substitués par $R^3$,

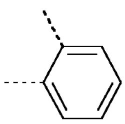

L-1

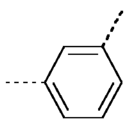

L-2

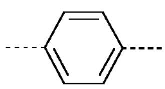

L-3

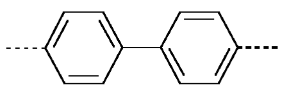

L-4

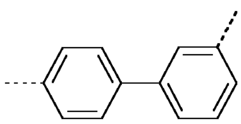

L-5

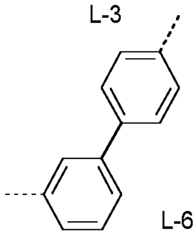

L-6

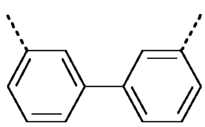

L-7

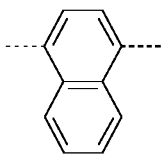

L-8

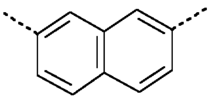
L-9

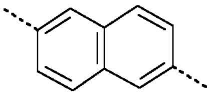
L-10

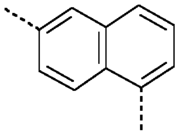
L-11

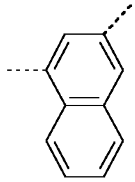
L-12

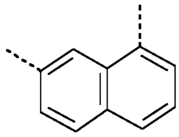
L-13

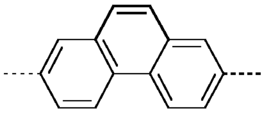
L-14

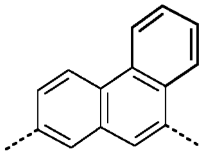
L-15

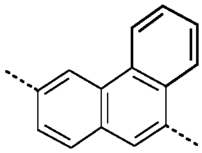
L-16

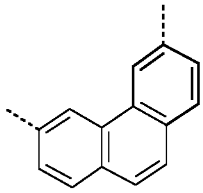
L-17

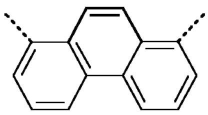
L-18

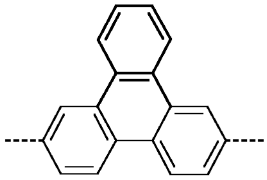
L-19

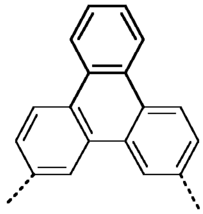
L-20

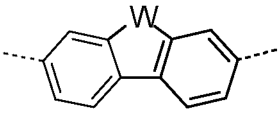
L-21

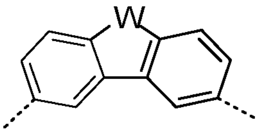
L-22 ,

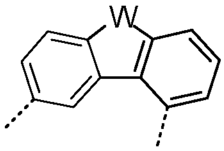
L-23

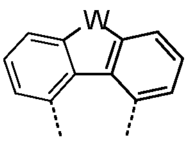
L-24

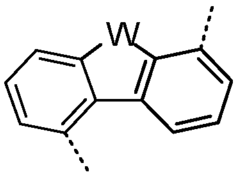
L-25

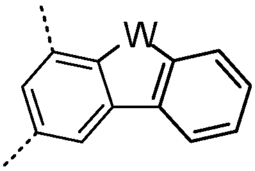
L-26

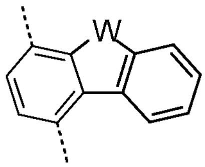
L-27

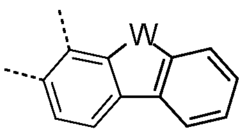
L-28

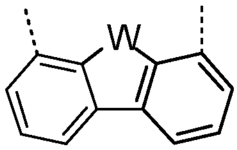
L-29

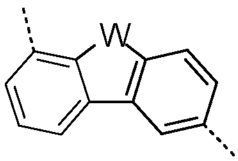
L-30

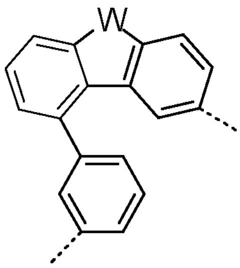

L-31

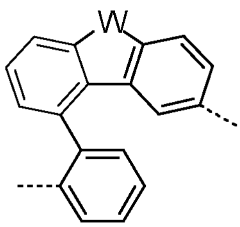

L-32

L-33

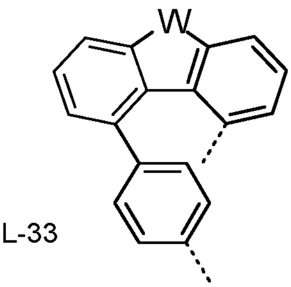

L-34

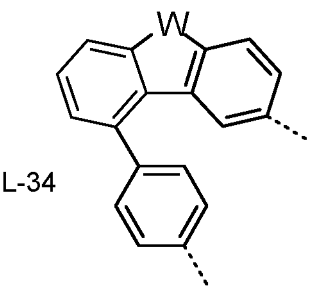

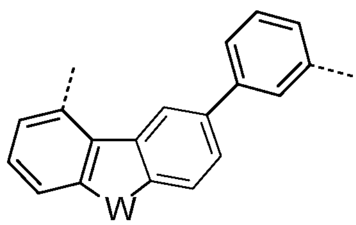

L-35

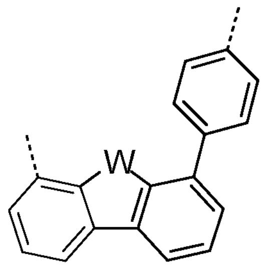

L-36

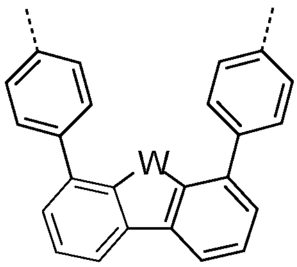

L-37

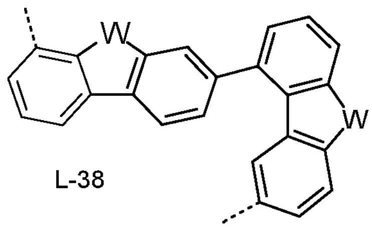

L-38

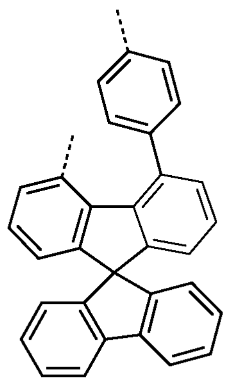

L-39

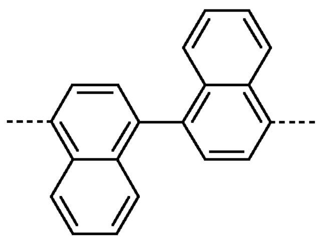

L-40 ,

où W désigne O, S ou $C(R^0)_2$ et
où $R^0$ dans $C(R^0)_2$ désigne méthyle ou phényle.

**4.** Composition selon l'une ou plusieurs parmi les revendications 1 à 3, **caractérisée en ce que** l'un parmi les substituants $Ar_4$ ou $Ar_5$ désigne un noyau aromatique ayant de 6 à 40 atomes de cycle aromatique ou un noyau hétéroaromatique ayant de 10 à 40 atomes de cycle aromatique, qui peut être substitué par un ou plusieurs radicaux $R^3$, où le noyau hétéroaromatique, qui peut être substitué par un ou plusieurs $R^3$, ne contient au total qu'un seul atome de N ou contient au total un ou plusieurs atomes de O et/ou S, et l'autre substituant désigne un noyau

aromatique ayant de 6 à 40 atomes de cycle aromatique, qui peut être substitué par un ou plusieurs radicaux $R^3$, à condition que $Ar_4$ et $Ar_5$ ne puissent pas être simultanément phényle.

**5.** Composition selon l'une ou plusieurs parmi les revendications 1 à 4, **caractérisée en ce que** les substituants $Ar_4$ et $Ar_5$ dans chaque cas, indépendamment l'un de l'autre, désignent un noyau aromatique ayant de 6 à 40 atomes de cycle aromatique, qui peut être substitué par un ou plusieurs radicaux $R^3$, à condition que $Ar_4$ et $Ar_5$ ne soient pas simultanément phényle.

**6.** Composition selon l'une ou plusieurs parmi les revendications 1 à 5, **caractérisée en ce que** la composition comprend au moins un autre composé choisi dans le groupe constitué par les matériaux d'injection de trous, les matériaux de transport de trous, les matériaux de blocage de trous, les matériaux à large bande interdite, les émetteurs fluorescents, les émetteurs phosphorescents, les matériaux hôtes, les matériaux de blocage d'électrons, les matériaux de transport d'électrons et les matériaux d'injection d'électrons, les dopants de type N et les dopants de type P.

**7.** Formulation comprenant une composition selon l'une ou plusieurs parmi les revendications 1 à 6, et au moins un solvant.

**8.** Utilisation d'une composition selon l'une ou plusieurs parmi les revendications 1 à 6, dans un dispositif électronique organique.

**9.** Utilisation selon la revendication 8, **caractérisée en ce que** le dispositif électronique organique est choisi dans le groupe constitué par les circuits intégrés organiques (OIC), les transistors organiques à effet de champ (OFET), les transistors organiques à couche mince (OTFT), les dispositifs électroluminescents organiques, les cellules solaires organiques (OSC), les détecteurs optiques organiques et les photorécepteurs organiques.

**10.** Dispositif électronique organique contenant au moins une composition selon l'une ou plusieurs parmi les revendications 1 à 6.

**11.** Dispositif selon la revendication 10, **caractérisé en ce qu'**il est choisi dans le groupe constitué par les circuits intégrés organiques (OIC), les transistors organiques à effet de champ (OFET), les transistors organiques à couche mince (OTFT), les dispositifs électroluminescents organiques, les cellules solaires organiques (OSC), les détecteurs optiques organiques et les photorécepteurs organiques.

**12.** Dispositif selon la revendication 10 ou 11, **caractérisé en ce qu'**il s'agit d'un dispositif électroluminescent choisi parmi les transistors organiques émetteurs de lumière (OLET), les dispositifs organiques à extinction de champ (OFQD), les cellules électrochimiques organiques émettrices de lumière (OLEC, LEC, LEEC), les diodes laser organiques (O-lasers) et les diodes électroluminescentes organiques (OLED).

**13.** Dispositif selon l'une ou plusieurs parmi les revendications 10 à 12, **caractérisé en ce qu'**il contient la composition selon l'une ou plusieurs parmi les revendications 1 à 6 dans une couche d'émission (EML), dans une couche de transport d'électrons (ETL), dans une couche d'injection d'électrons (EIL) et/ou dans une couche de blocage de trous (HBL).

**14.** Dispositif selon l'une ou plusieurs parmi les revendications 10 à 13, **caractérisé en ce qu'**il contient la composition selon l'une ou plusieurs parmi les revendications 1 à 6 dans la couche d'émission conjointement avec un émetteur phosphorescent.

**15.** Procédé de production d'un dispositif selon l'une ou plusieurs parmi les revendications 10 à 14, **caractérisé en ce qu'**au moins une couche organique comprenant une composition selon l'une ou plusieurs parmi les revendications 1 à 6 est appliquée par dépôt en phase gazeuse ou à partir d'une solution.

**16.** Procédé selon la revendication 15, **caractérisé en ce qu'**au moins un composé de formule (1e) et au moins un composé de formule (2a), tels que décrits selon l'une des revendications 1 à 6, sont déposés à partir de la phase gazeuse, successivement ou simultanément à partir d'au moins deux sources de matériaux, éventuellement avec d'autres matériaux, et forment la couche organique.

**17.** Procédé selon la revendication 15, **caractérisé en ce que** la composition selon l'une ou plusieurs parmi les revendications 1 à 6 est utilisée comme source de matériau pour le dépôt en phase gazeuse et forme la couche organique.

**18.** Procédé selon la revendication 15, **caractérisé en ce que** la formulation selon la revendication 7 est utilisée pour appliquer la couche organique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2004093207 A
- WO 2010006680 A
- WO 2005003253 A
- WO 2008056746 A
- EP 0906947 A
- EP 0908787 A
- EP 0906948 A
- WO 2011116865 A
- WO 2011137951 A
- WO 2005039246 A
- US 20050069729 A
- WO 2014015931 A
- WO 2007063754 A
- WO 2010136109 A
- WO 2011000455 A
- WO 2011057706 A
- WO 2011046182 A
- WO 2009069442 A
- WO 2015014434 A
- WO 2015169412 A
- US 6392250 B1
- US 6803720 B1
- US 9601698 B
- WO 2015156587 A
- KR 101744248 B1
- KR 20170113320
- WO 2005086251 A2
- WO 2012175535 A1
- WO 2012175219 A1
- WO 2012168358 A1
- WO 2012031735 A1
- EP 1837926 A1
- WO 2007107306 A1
- EP 2452946 A1
- EP 2463927 A1
- WO 2009000237 A1
- US 2007145355 A1
- EP 1538684 A1
- WO 2006081780 A1
- WO 2009003455 A1
- WO 2010097433 A1
- EP 1988587 A1
- US 2010102709 A1
- EP 2180029 A1
- WO 2011131185 A1
- WO 2011134458 A1
- US 2012223296 A1
- WO 2007134873 A1
- WO 2008061517 A2
- WO 2008061518 A2
- DE 102008051737 A1
- WO 2009089821 A1
- US 2010096600 A1
- WO 2008138580 A1
- WO 2008058525 A2
- WO 2007115540 A1
- DE 102010046040 A1
- WO 2008128519 A2
- US 7294849 B
- WO 2016015815 A
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016124304 A
- WO 2017032439 A
- WO 2006108497 A
- WO 2006122630 A

- WO 2008006449 A
- WO 2007140847 A
- WO 2010012328 A
- WO 2010108579 A
- EP 676461 A
- WO 2004081017 A
- WO 2004058911 A
- WO 2005084081 A
- WO 2005084082 A
- WO 2006048268 A
- WO 2006117052 A
- WO 2008145239 A
- JP 2004288381 A
- EP 1205527 A
- WO 2008086851 A
- WO 2011088877 A
- WO 2011128017 A
- EP 1617710 A
- EP 1617711 A
- EP 1731584 A
- JP 2005347160 A
- WO 2007137725 A
- WO 2005111172 A
- WO 2010015306 A
- EP 652273 A
- WO 2009062578 A
- WO 2010054729 A
- WO 2010054730 A
- WO 2005011013 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A
- EP 12000929
- WO 2014015937 A
- WO 2014015938 A
- WO 2014015935 A
- WO 2013083216 A
- WO 2012150001 A
- US 20150001488 A


**In der Beschreibung aufgeführte Nicht-Patentliteratur**


- **M. A. BALDO et al.** *Appl. Phys. Lett.*, 1999, vol. 75, 4-6
- *CHEMICAL ABSTRACTS*, 1454567-05-5
- *CHEMICAL ABSTRACTS*, 1352040-89-1
- *CHEMICAL ABSTRACTS*, 1336889-25-8
- *CHEMICAL ABSTRACTS*, 1800544-05-1
- *CHEMICAL ABSTRACTS*, 1800544-08-4
- *CHEMICAL ABSTRACTS*, 1800544-09-5
- *CHEMICAL ABSTRACTS*, 1800544-10-8
- *CHEMICAL ABSTRACTS*, 1800544-11-9
- *CHEMICAL ABSTRACTS*, 1800544-04-0
- *CHEMICAL ABSTRACTS*, 1842320-52-8
- *CHEMICAL ABSTRACTS*, 1842320-53-9
- *CHEMICAL ABSTRACTS*, 1842320-54-0
- *CHEMICAL ABSTRACTS*, 1842320-55-1
- *CHEMICAL ABSTRACTS*, 1842320-56-2
- *CHEMICAL ABSTRACTS*, 1842320-57-3
- *CHEMICAL ABSTRACTS*, 1410876-33-3
- *CHEMICAL ABSTRACTS*, 1842320-58-4
- *CHEMICAL ABSTRACTS*, 1410876-47-9
- *CHEMICAL ABSTRACTS*, 1842320-59-5
- *CHEMICAL ABSTRACTS*, 1848256-38-1
- *CHEMICAL ABSTRACTS*, 1865661-14-8
- *CHEMICAL ABSTRACTS*, 1870867-25-6
- *CHEMICAL ABSTRACTS*, 1884707-32-7
- *CHEMICAL ABSTRACTS*, 1889262-88-7
- *CHEMICAL ABSTRACTS*, 2018307-89-4
- *CHEMICAL ABSTRACTS*, 1454655-29-8
- *CHEMICAL ABSTRACTS*, 1454655-33-4
- *CHEMICAL ABSTRACTS*, 1454660-22-0
- *CHEMICAL ABSTRACTS*, 1907663-27-7
- *CHEMICAL ABSTRACTS*, 1548581-24-3
- *CHEMICAL ABSTRACTS*, 1548581-27-6
- *CHEMICAL ABSTRACTS*, 1548581-29-8
- *CHEMICAL ABSTRACTS*, 1548581-37-8
- *CHEMICAL ABSTRACTS*, 1548581-40-3
- *CHEMICAL ABSTRACTS*, 1943719-62-7
- *CHEMICAL ABSTRACTS*, 1548581-42-5
- *CHEMICAL ABSTRACTS*, 1942079-50-6
- *CHEMICAL ABSTRACTS*, 1548581-44-7
- *CHEMICAL ABSTRACTS*, 1942079-51-7
- *CHEMICAL ABSTRACTS*, 1943719-63-8
- *CHEMICAL ABSTRACTS*, 1955476-12-6
- *CHEMICAL ABSTRACTS*, 1619966-75-4
- *CHEMICAL ABSTRACTS*, 1955476-13-7
- *CHEMICAL ABSTRACTS*, 1955476-15-9
- *CHEMICAL ABSTRACTS*, 1955476-28-4
- *CHEMICAL ABSTRACTS*, 1955476-30-8
- *CHEMICAL ABSTRACTS*, 1955476-32-0
- *CHEMICAL ABSTRACTS*, 1643479-47-3
- *CHEMICAL ABSTRACTS*, 1973498-04-2
- *CHEMICAL ABSTRACTS*, 1643479-49-5
- *CHEMICAL ABSTRACTS*, 1973498-03-1
- *CHEMICAL ABSTRACTS*, 1973498-05-3

- *CHEMICAL ABSTRACTS,* 2018307-36-1
- *CHEMICAL ABSTRACTS,* 1643479-56-4
- *CHEMICAL ABSTRACTS,* 2018307-35-0
- *CHEMICAL ABSTRACTS,* 2018307-37-2
- *CHEMICAL ABSTRACTS,* 2018307-38-3
- *CHEMICAL ABSTRACTS,* 2018307-39-4
- *CHEMICAL ABSTRACTS,* 2018307-77-0
- *CHEMICAL ABSTRACTS,* 2018307-78-1
- *CHEMICAL ABSTRACTS,* 2018307-90-7
- *CHEMICAL ABSTRACTS,* 2018307-91-8
- *CHEMICAL ABSTRACTS,* 1799958-74-9
- *CHEMICAL ABSTRACTS,* 2052160-86-6
- *CHEMICAL ABSTRACTS,* 1799958-79-4
- *CHEMICAL ABSTRACTS,* 1799958-76-1
- *CHEMICAL ABSTRACTS,* 2052160-91-3
- *CHEMICAL ABSTRACTS,* 1799958-77-2
- *CHEMICAL ABSTRACTS,* 2055848-40-1
- *CHEMICAL ABSTRACTS,* 1799958-78-3
- *CHEMICAL ABSTRACTS,* 2057418-19-4
- *CHEMICAL ABSTRACTS,* 1799958-99-8
- *CHEMICAL ABSTRACTS,* 1799959-01-5
- *CHEMICAL ABSTRACTS,* 1799959-03-7
- *CHEMICAL ABSTRACTS,* 1799959-05-9
- *CHEMICAL ABSTRACTS,* 1799959-07-1
- *CHEMICAL ABSTRACTS,* 1799959-09-3
- *CHEMICAL ABSTRACTS,* 1799959-11-7
- *CHEMICAL ABSTRACTS,* 1799959-13-9
- *CHEMICAL ABSTRACTS,* 2085318-61-0
- *CHEMICAL ABSTRACTS,* 2085318-62-1
- *CHEMICAL ABSTRACTS,* 2085318-64-3
- *CHEMICAL ABSTRACTS,* 2085318-63-2
- *CHEMICAL ABSTRACTS,* 2085318-66-5
- *CHEMICAL ABSTRACTS,* 2085318-65-4
- *CHEMICAL ABSTRACTS,* 2085318-77-8
- *CHEMICAL ABSTRACTS,* 2085318-78-9
- *CHEMICAL ABSTRACTS,* 2085318-79-0
- *CHEMICAL ABSTRACTS,* 57102-51-9
- *CHEMICAL ABSTRACTS,* 2085318-81-4
- *CHEMICAL ABSTRACTS,* 2085318-80-3
- *CHEMICAL ABSTRACTS,* 2085318-83-6
- *CHEMICAL ABSTRACTS,* 2085318-82-5
- *CHEMICAL ABSTRACTS,* 2085318-88-1
- *CHEMICAL ABSTRACTS,* 2085318-87-0
- *CHEMICAL ABSTRACTS,* 2085318-92-7
- *CHEMICAL ABSTRACTS,* 2085318-89-2
- *CHEMICAL ABSTRACTS,* 2085318-94-9
- *CHEMICAL ABSTRACTS,* 2085318-93-8
- *CHEMICAL ABSTRACTS,* 2085318-98-3
- *CHEMICAL ABSTRACTS,* 2085318-97-2
- *CHEMICAL ABSTRACTS,* 2085319-00-0
- *CHEMICAL ABSTRACTS,* 2085318-99-4
- *CHEMICAL ABSTRACTS,* 251316-80-0
- *CHEMICAL ABSTRACTS,* 1427160-09-5
- *CHEMICAL ABSTRACTS,* 1643479-72-4
- *CHEMICAL ABSTRACTS,* 1799960-24-9
- *CHEMICAL ABSTRACTS,* 1799960-25-0
- *CHEMICAL ABSTRACTS,* 1340668-17-8
- *CHEMICAL ABSTRACTS,* 1340668-19-0
- *CHEMICAL ABSTRACTS,* 1289556-24-6
- *CHEMICAL ABSTRACTS,* 1799960-56-7
- *CHEMICAL ABSTRACTS,* 1336889-27-0
- *CHEMICAL ABSTRACTS,* 1799960-58-9
- *CHEMICAL ABSTRACTS,* 1812208-18-6
- *CHEMICAL ABSTRACTS,* 1340668-35-0
- *CHEMICAL ABSTRACTS,* 1340668-37-2
- *CHEMICAL ABSTRACTS,* 1830334-82-1
- *CHEMICAL ABSTRACTS,* 1340669-19-3
- *CHEMICAL ABSTRACTS,* 1830334-85-4
- *CHEMICAL ABSTRACTS,* 1830334-94-5
- *CHEMICAL ABSTRACTS,* 1830334-88-7
- *CHEMICAL ABSTRACTS,* 1340669-32-0
- *CHEMICAL ABSTRACTS,* 1830334-90-1
- *CHEMICAL ABSTRACTS,* 1340669-33-1
- *CHEMICAL ABSTRACTS,* 1830334-91-2
- *CHEMICAL ABSTRACTS,* 1830335-02-8
- *CHEMICAL ABSTRACTS,* 1830334-97-8
- *CHEMICAL ABSTRACTS,* 1830335-71-1
- *CHEMICAL ABSTRACTS,* 1830335-07-3
- *CHEMICAL ABSTRACTS,* 1830335-76-6
- *CHEMICAL ABSTRACTS,* 1830335-72-2
- *CHEMICAL ABSTRACTS,* 1830335-85-7
- *CHEMICAL ABSTRACTS,* 1830335-82-4
- *CHEMICAL ABSTRACTS,* 1830335-79-9
- *CHEMICAL ABSTRACTS,* 1830339-40-6
- *CHEMICAL ABSTRACTS,* 1830335-95-9
- *CHEMICAL ABSTRACTS,* 1377150-35-0
- *CHEMICAL ABSTRACTS,* 1830339-41-7
- *CHEMICAL ABSTRACTS,* 1830335-90-4
- *CHEMICAL ABSTRACTS,* 1830335-87-9
- *CHEMICAL ABSTRACTS,* 1399855-37-8
- *CHEMICAL ABSTRACTS,* 1830339-42-8
- *CHEMICAL ABSTRACTS,* 1399855-38-9
- *CHEMICAL ABSTRACTS,* 1399855-39-0
- *CHEMICAL ABSTRACTS,* 1399855-46-9
- *CHEMICAL ABSTRACTS,* 1399855-47-0
- *CHEMICAL ABSTRACTS,* 1413936-92-1
- *CHEMICAL ABSTRACTS,* 1413936-95-4
- *CHEMICAL ABSTRACTS,* 1413936-96-5
- *CHEMICAL ABSTRACTS,* 1413936-97-6
- *CHEMICAL ABSTRACTS,* 1413937-08-2
- *CHEMICAL ABSTRACTS,* 1890157-92-2
- *CHEMICAL ABSTRACTS,* 1415348-93-4
- *CHEMICAL ABSTRACTS,* 1889262-89-8
- *CHEMICAL ABSTRACTS,* 1415348-99-0
- *CHEMICAL ABSTRACTS,* 1890156-90-7
- *CHEMICAL ABSTRACTS,* 1415349-00-6
- *CHEMICAL ABSTRACTS,* 1890156-91-8
- *CHEMICAL ABSTRACTS,* 1415349-01-7
- *CHEMICAL ABSTRACTS,* 1890157-12-6
- *CHEMICAL ABSTRACTS,* 1415349-02-8
- *CHEMICAL ABSTRACTS,* 1890157-13-7
- *CHEMICAL ABSTRACTS,* 1415349-03-9

- *CHEMICAL ABSTRACTS,* 1890157-14-8
- *CHEMICAL ABSTRACTS,* 1415349-04-0
- *CHEMICAL ABSTRACTS,* 1890157-37-5
- *CHEMICAL ABSTRACTS,* 1415349-05-1
- *CHEMICAL ABSTRACTS,* 1415349-06-2
- *CHEMICAL ABSTRACTS,* 1415349-07-3
- *CHEMICAL ABSTRACTS,* 1890157-41-1
- *CHEMICAL ABSTRACTS,* 1890157-42-2
- *CHEMICAL ABSTRACTS,* 1890157-43-3
- *CHEMICAL ABSTRACTS,* 1422451-48-6
- *CHEMICAL ABSTRACTS,* 1890157-64-8
- *CHEMICAL ABSTRACTS,* 1445952-53-3
- *CHEMICAL ABSTRACTS,* 1445952-58-8
- *CHEMICAL ABSTRACTS,* 1450933-86-4
- *CHEMICAL ABSTRACTS,* 1919031-93-8
- *CHEMICAL ABSTRACTS,* 1919031-92-7
- *CHEMICAL ABSTRACTS,* 1919031-95-0
- *CHEMICAL ABSTRACTS,* 1919031-94-9
- *CHEMICAL ABSTRACTS,* 1919031-97-2
- *CHEMICAL ABSTRACTS,* 1919031-96-1
- *CHEMICAL ABSTRACTS,* 1919031-99-4
- *CHEMICAL ABSTRACTS,* 1919031-98-3
- *CHEMICAL ABSTRACTS,* 1598389-98-0
- *CHEMICAL ABSTRACTS,* 1919032-02-2
- *CHEMICAL ABSTRACTS,* 1604034-14-1
- *CHEMICAL ABSTRACTS,* 1604034-02-7
- *CHEMICAL ABSTRACTS,* 1604034-07-2
- *CHEMICAL ABSTRACTS,* 1604034-12-9
- *CHEMICAL ABSTRACTS,* 1622931-00-3
- *CHEMICAL ABSTRACTS,* 1604034-15-2
- *CHEMICAL ABSTRACTS,* 1622931-01-4
- *CHEMICAL ABSTRACTS,* 1622931-04-7
- *CHEMICAL ABSTRACTS,* 1630029-28-5
- *CHEMICAL ABSTRACTS,* 1630029-29-6
- *CHEMICAL ABSTRACTS,* 1643479-51-9
- *CHEMICAL ABSTRACTS,* 1643479-52-0
- *CHEMICAL ABSTRACTS,* 1643479-54-2
- *CHEMICAL ABSTRACTS,* 1643479-59-7
- *CHEMICAL ABSTRACTS,* 1643479-62-2
- *CHEMICAL ABSTRACTS,* 1643479-68-8
- *CHEMICAL ABSTRACTS,* 1643479-69-9
- *CHEMICAL ABSTRACTS,* 1643479-74-6
- *CHEMICAL ABSTRACTS,* 2018307-43-0
- *CHEMICAL ABSTRACTS,* 1643479-75-7
- *CHEMICAL ABSTRACTS,* 2018307-47-4
- *CHEMICAL ABSTRACTS,* 2018307-50-9
- *CHEMICAL ABSTRACTS,* 2018307-49-6
- *CHEMICAL ABSTRACTS,* 1656982-30-7
- *CHEMICAL ABSTRACTS,* 1680184-58-0
- *CHEMICAL ABSTRACTS,* 1799483-56-9
- *CHEMICAL ABSTRACTS,* 1799519-35-9
- *CHEMICAL ABSTRACTS,* 1799678-37-7
- *CHEMICAL ABSTRACTS,* 2073116-97-7
- *CHEMICAL ABSTRACTS,* 2048236-10-6
- *CHEMICAL ABSTRACTS,* 1890157-93-3
- *CHEMICAL ABSTRACTS,* 1890157-94-4
- *CHEMICAL ABSTRACTS,* 1431151-34-6
- *CHEMICAL ABSTRACTS,* 1890157-95-5
- *CHEMICAL ABSTRACTS,* 1446411-07-9
- *CHEMICAL ABSTRACTS,* 1894193-97-5
- *CHEMICAL ABSTRACTS,* 1894194-10-5
- *CHEMICAL ABSTRACTS,* 1894194-11-6
- *CHEMICAL ABSTRACTS,* 1497337-43-5
- *CHEMICAL ABSTRACTS,* 1499917-70-2
- *CHEMICAL ABSTRACTS,* 1588866-10-7
- *CHEMICAL ABSTRACTS,* 1934252-94-4
- *CHEMICAL ABSTRACTS,* 1598389-99-1
- *CHEMICAL ABSTRACTS,* 1943719-77-4
- *CHEMICAL ABSTRACTS,* 1613752-14-9
- *CHEMICAL ABSTRACTS,* 1943719-78-5
- *CHEMICAL ABSTRACTS,* 2018307-45-2
- *CHEMICAL ABSTRACTS,* 2018307-44-1
- *CHEMICAL ABSTRACTS,* 1643479-80-4
- *CHEMICAL ABSTRACTS,* 1643479-84-8
- *CHEMICAL ABSTRACTS,* 1643479-88-2
- *CHEMICAL ABSTRACTS,* 2018307-52-1
- *CHEMICAL ABSTRACTS,* 1643480-02-7
- *CHEMICAL ABSTRACTS,* 2018307-51-0
- *CHEMICAL ABSTRACTS,* 2018307-53-2
- *CHEMICAL ABSTRACTS,* 2018307-54-3
- *CHEMICAL ABSTRACTS,* 1656982-32-9
- *CHEMICAL ABSTRACTS,* 2018307-80-5
- *CHEMICAL ABSTRACTS,* 2018307-79-2
- *CHEMICAL ABSTRACTS,* 1799483-31-0
- *CHEMICAL ABSTRACTS,* 1792238-01-7
- *CHEMICAL ABSTRACTS,* 1799483-43-4
- *CHEMICAL ABSTRACTS,* 2020391-63-1
- *CHEMICAL ABSTRACTS,* 1799483-44-5
- *CHEMICAL ABSTRACTS,* 2020391-71-1
- *CHEMICAL ABSTRACTS,* 2020391-73-3
- *CHEMICAL ABSTRACTS,* 2020391-72-2
- *CHEMICAL ABSTRACTS,* 2020391-75-5
- *CHEMICAL ABSTRACTS,* 2020391-74-4
- *CHEMICAL ABSTRACTS,* 2079874-13-6
- *CHEMICAL ABSTRACTS,* 2075738-96-2
- *CHEMICAL ABSTRACTS,* 2075738-98-4
- *CHEMICAL ABSTRACTS,* 2075738-97-3
- *CHEMICAL ABSTRACTS,* 2075738-99-5
- *CHEMICAL ABSTRACTS,* 2075739-04-5
- *CHEMICAL ABSTRACTS,* 2075739-05-6
- *CHEMICAL ABSTRACTS,* 2075739-06-7
- *CHEMICAL ABSTRACTS,* 2075739-07-8
- *CHEMICAL ABSTRACTS,* 1269508-30-6
- *CHEMICAL ABSTRACTS,* 1989601-68-4
- *CHEMICAL ABSTRACTS,* 1989602-19-8
- *CHEMICAL ABSTRACTS,* 1989602-70-1
- *CHEMICAL ABSTRACTS,* 1215692-34-4
- *CHEMICAL ABSTRACTS,* 1989601-69-5
- *CHEMICAL ABSTRACTS,* 1989602-20-1
- *CHEMICAL ABSTRACTS,* 1989602-71-2
- *CHEMICAL ABSTRACTS,* 1370364-40-1
- *CHEMICAL ABSTRACTS,* 1989601-70-8
- *CHEMICAL ABSTRACTS,* 1989602-21-2
- *CHEMICAL ABSTRACTS,* 1989602-72-3

- *CHEMICAL ABSTRACTS,* 1370364-42-3
- *CHEMICAL ABSTRACTS,* 1989601-71-9
- *CHEMICAL ABSTRACTS,* 1989602-22-3
- *CHEMICAL ABSTRACTS,* 1989602-73-4
- *CHEMICAL ABSTRACTS,* 1989600-74-9
- *CHEMICAL ABSTRACTS,* 1989601-72-0
- *CHEMICAL ABSTRACTS,* 1989602-23-4
- *CHEMICAL ABSTRACTS,* 1989602-74-5
- *CHEMICAL ABSTRACTS,* 1989600-75-0
- *CHEMICAL ABSTRACTS,* 1989601-73-1
- *CHEMICAL ABSTRACTS,* 1989602-24-5
- *CHEMICAL ABSTRACTS,* 1989602-75-6
- *CHEMICAL ABSTRACTS,* 1989600-77-2
- *CHEMICAL ABSTRACTS,* 1989601-74-2
- *CHEMICAL ABSTRACTS,* 1989602-25-6
- *CHEMICAL ABSTRACTS,* 1989602-76-7
- *CHEMICAL ABSTRACTS,* 1989600-78-3
- *CHEMICAL ABSTRACTS,* 1989601-75-3
- *CHEMICAL ABSTRACTS,* 1989602-26-7
- *CHEMICAL ABSTRACTS,* 1989602-77-8
- *CHEMICAL ABSTRACTS,* 1989600-79-4
- *CHEMICAL ABSTRACTS,* 1989601-76-4
- *CHEMICAL ABSTRACTS,* 1989602-27-8
- *CHEMICAL ABSTRACTS,* 1989602-78-9
- *CHEMICAL ABSTRACTS,* 1989600-82-9
- *CHEMICAL ABSTRACTS,* 1989601-77-5
- *CHEMICAL ABSTRACTS,* 1989602-28-9
- *CHEMICAL ABSTRACTS,* 1989602-79-0
- *CHEMICAL ABSTRACTS,* 1989600-83-0
- *CHEMICAL ABSTRACTS,* 1989601-78-6
- *CHEMICAL ABSTRACTS,* 1989602-29-0
- *CHEMICAL ABSTRACTS,* 1989602-80-3
- *CHEMICAL ABSTRACTS,* 1989600-84-1
- *CHEMICAL ABSTRACTS,* 1989601-79-7
- *CHEMICAL ABSTRACTS,* 1989602-30-3
- *CHEMICAL ABSTRACTS,* 1989602-82-5
- *CHEMICAL ABSTRACTS,* 1989600-85-2
- *CHEMICAL ABSTRACTS,* 1989601-80-0
- *CHEMICAL ABSTRACTS,* 1989602-31-4
- *CHEMICAL ABSTRACTS,* 1989602-84-7
- *CHEMICAL ABSTRACTS,* 1989600-86-3
- *CHEMICAL ABSTRACTS,* 1989601-81-1
- *CHEMICAL ABSTRACTS,* 1989602-32-5
- *CHEMICAL ABSTRACTS,* 1989602-85-8
- *CHEMICAL ABSTRACTS,* 1989600-87-4
- *CHEMICAL ABSTRACTS,* 1989601-82-2
- *CHEMICAL ABSTRACTS,* 1989602-33-6
- *CHEMICAL ABSTRACTS,* 1989602-86-9
- *CHEMICAL ABSTRACTS,* 1989600-88-5
- *CHEMICAL ABSTRACTS,* 1989601-83-3
- *CHEMICAL ABSTRACTS,* 1989602-34-7
- *CHEMICAL ABSTRACTS,* 1989602-87-0
- *CHEMICAL ABSTRACTS,* 1989600-89-6
- *CHEMICAL ABSTRACTS,* 1989601-84-4
- *CHEMICAL ABSTRACTS,* 1989602-35-8
- *CHEMICAL ABSTRACTS,* 1989602-88-1
- *CHEMICAL ABSTRACTS,* 1989601-11-7
- *CHEMICAL ABSTRACTS,* 1989601-85-5
- *CHEMICAL ABSTRACTS,* 1989602-36-9
- *CHEMICAL ABSTRACTS,* 1989604-00-3
- *CHEMICAL ABSTRACTS,* 1989601-23-1
- *CHEMICAL ABSTRACTS,* 1989601-86-6
- *CHEMICAL ABSTRACTS,* 1989602-37-0
- *CHEMICAL ABSTRACTS,* 1989604-01-4
- *CHEMICAL ABSTRACTS,* 1989601-26-4
- *CHEMICAL ABSTRACTS,* 1989601-87-7
- *CHEMICAL ABSTRACTS,* 1989602-38-1
- *CHEMICAL ABSTRACTS,* 1989604-02-5
- *CHEMICAL ABSTRACTS,* 1989601-28-6
- *CHEMICAL ABSTRACTS,* 1989601-88-8
- *CHEMICAL ABSTRACTS,* 1989602-39-2
- *CHEMICAL ABSTRACTS,* 1989604-03-6
- *CHEMICAL ABSTRACTS,* 1989601-29-7
- *CHEMICAL ABSTRACTS,* 1989601-89-9
- *CHEMICAL ABSTRACTS,* 1989602-40-5
- *CHEMICAL ABSTRACTS,* 1989604-04-7
- *CHEMICAL ABSTRACTS,* 1989601-33-3
- *CHEMICAL ABSTRACTS,* 1989601-90-2
- *CHEMICAL ABSTRACTS,* 1989602-41-6
- *CHEMICAL ABSTRACTS,* 1989604-05-8
- *CHEMICAL ABSTRACTS,* 1989601-40-2
- *CHEMICAL ABSTRACTS,* 1989601-91-3
- *CHEMICAL ABSTRACTS,* 1989602-42-7
- *CHEMICAL ABSTRACTS,* 1989604-06-9
- *CHEMICAL ABSTRACTS,* 1989601-41-3
- *CHEMICAL ABSTRACTS,* 1989601-92-4
- *CHEMICAL ABSTRACTS,* 1989602-43-8
- *CHEMICAL ABSTRACTS,* 1989604-07-0
- *CHEMICAL ABSTRACTS,* 1989601-42-4
- *CHEMICAL ABSTRACTS,* 1989601-93-5
- *CHEMICAL ABSTRACTS,* 1989602-44-9
- *CHEMICAL ABSTRACTS,* 1989604-08-1
- *CHEMICAL ABSTRACTS,* 1989601-43-5
- *CHEMICAL ABSTRACTS,* 1989601-94-6
- *CHEMICAL ABSTRACTS,* 1989602-45-0
- *CHEMICAL ABSTRACTS,* 1989604-09-2
- *CHEMICAL ABSTRACTS,* 1989601-44-6
- *CHEMICAL ABSTRACTS,* 1989601-95-7
- *CHEMICAL ABSTRACTS,* 1989602-46-1
- *CHEMICAL ABSTRACTS,* 1989604-10-5
- *CHEMICAL ABSTRACTS,* 1989601-45-7
- *CHEMICAL ABSTRACTS,* 1989601-96-8
- *CHEMICAL ABSTRACTS,* 1989602-47-2
- *CHEMICAL ABSTRACTS,* 1989604-11-6
- *CHEMICAL ABSTRACTS,* 1989601-46-8
- *CHEMICAL ABSTRACTS,* 1989601-97-9
- *CHEMICAL ABSTRACTS,* 1989602-48-3
- *CHEMICAL ABSTRACTS,* 1989604-13-8
- *CHEMICAL ABSTRACTS,* 1989601-47-9
- *CHEMICAL ABSTRACTS,* 1989601-98-0
- *CHEMICAL ABSTRACTS,* 1989602-49-4
- *CHEMICAL ABSTRACTS,* 1989604-14-9
- *CHEMICAL ABSTRACTS,* 1989601-48-0
- *CHEMICAL ABSTRACTS,* 1989601-99-1
- *CHEMICAL ABSTRACTS,* 1989602-50-7
- *CHEMICAL ABSTRACTS,* 1989604-15-0

- *CHEMICAL ABSTRACTS,* 1989601-49-1
- *CHEMICAL ABSTRACTS,* 1989602-00-7
- *CHEMICAL ABSTRACTS,* 1989602-51-8
- *CHEMICAL ABSTRACTS,* 1989604-16-1
- *CHEMICAL ABSTRACTS,* 1989601-50-4
- *CHEMICAL ABSTRACTS,* 1989602-01-8
- *CHEMICAL ABSTRACTS,* 1989602-52-9
- *CHEMICAL ABSTRACTS,* 1989604-17-2
- *CHEMICAL ABSTRACTS,* 1989601-51-5
- *CHEMICAL ABSTRACTS,* 1989602-02-9
- *CHEMICAL ABSTRACTS,* 1989602-53-0
- *CHEMICAL ABSTRACTS,* 1989604-18-3
- *CHEMICAL ABSTRACTS,* 1989601-52-6
- *CHEMICAL ABSTRACTS,* 1989602-03-0
- *CHEMICAL ABSTRACTS,* 1989602-54-1
- *CHEMICAL ABSTRACTS,* 1989604-19-4
- *CHEMICAL ABSTRACTS,* 1989601-53-7
- *CHEMICAL ABSTRACTS,* 1989602-04-1
- *CHEMICAL ABSTRACTS,* 1989602-55-2
- *CHEMICAL ABSTRACTS,* 1989604-20-7
- *CHEMICAL ABSTRACTS,* 1989601-54-8
- *CHEMICAL ABSTRACTS,* 1989602-05-2
- *CHEMICAL ABSTRACTS,* 1989602-56-3
- *CHEMICAL ABSTRACTS,* 1989604-21-8
- *CHEMICAL ABSTRACTS,* 1989601-55-9
- *CHEMICAL ABSTRACTS,* 1989602-06-3
- *CHEMICAL ABSTRACTS,* 1989602-57-4
- *CHEMICAL ABSTRACTS,* 1989604-22-9
- *CHEMICAL ABSTRACTS,* 1989601-56-0
- *CHEMICAL ABSTRACTS,* 1989602-07-4
- *CHEMICAL ABSTRACTS,* 1989602-58-5
- *CHEMICAL ABSTRACTS,* 1989604-23-0
- *CHEMICAL ABSTRACTS,* 1989601-57-1
- *CHEMICAL ABSTRACTS,* 1989602-08-5
- *CHEMICAL ABSTRACTS,* 1989602-59-6
- *CHEMICAL ABSTRACTS,* 1989604-24-1
- *CHEMICAL ABSTRACTS,* 1989601-58-2
- *CHEMICAL ABSTRACTS,* 1989602-09-6
- *CHEMICAL ABSTRACTS,* 1989602-60-9
- *CHEMICAL ABSTRACTS,* 1989604-25-2
- *CHEMICAL ABSTRACTS,* 1989601-59-3
- *CHEMICAL ABSTRACTS,* 1989602-10-9
- *CHEMICAL ABSTRACTS,* 1989602-61-0
- *CHEMICAL ABSTRACTS,* 1989604-26-3
- *CHEMICAL ABSTRACTS,* 1989601-60-6
- *CHEMICAL ABSTRACTS,* 1989602-11-0
- *CHEMICAL ABSTRACTS,* 1989602-62-1
- *CHEMICAL ABSTRACTS,* 1989604-27-4
- *CHEMICAL ABSTRACTS,* 1989601-61-7
- *CHEMICAL ABSTRACTS,* 1989602-12-1
- *CHEMICAL ABSTRACTS,* 1989602-63-2
- *CHEMICAL ABSTRACTS,* 1989604-28-5
- *CHEMICAL ABSTRACTS,* 1989601-62-8
- *CHEMICAL ABSTRACTS,* 1989602-13-2
- *CHEMICAL ABSTRACTS,* 1989602-64-3
- *CHEMICAL ABSTRACTS,* 1989604-29-6
- *CHEMICAL ABSTRACTS,* 1989601-63-9
- *CHEMICAL ABSTRACTS,* 1989602-14-3
- *CHEMICAL ABSTRACTS,* 1989602-65-4
- *CHEMICAL ABSTRACTS,* 1989604-30-9
- *CHEMICAL ABSTRACTS,* 1989601-64-0
- *CHEMICAL ABSTRACTS,* 1989602-15-4
- *CHEMICAL ABSTRACTS,* 1989602-66-5
- *CHEMICAL ABSTRACTS,* 1989604-31-0
- *CHEMICAL ABSTRACTS,* 1989601-65-1
- *CHEMICAL ABSTRACTS,* 1989602-16-5
- *CHEMICAL ABSTRACTS,* 1989602-67-6
- *CHEMICAL ABSTRACTS,* 1989604-32-1
- *CHEMICAL ABSTRACTS,* 1989601-66-2
- *CHEMICAL ABSTRACTS,* 1989602-17-6
- *CHEMICAL ABSTRACTS,* 1989602-68-7
- *CHEMICAL ABSTRACTS,* 1989604-33-2
- *CHEMICAL ABSTRACTS,* 1989601-67-3
- *CHEMICAL ABSTRACTS,* 1989602-18-7
- *CHEMICAL ABSTRACTS,* 1989602-69-8
- *CHEMICAL ABSTRACTS,* 1989604-34-3
- *CHEMICAL ABSTRACTS,* 1989604-35-4
- *CHEMICAL ABSTRACTS,* 1989604-88-7
- *CHEMICAL ABSTRACTS,* 1989605-52-8
- *CHEMICAL ABSTRACTS,* 1989606-07-6
- *CHEMICAL ABSTRACTS,* 1989604-36-5
- *CHEMICAL ABSTRACTS,* 1989604-89-8
- *CHEMICAL ABSTRACTS,* 1989605-53-9
- *CHEMICAL ABSTRACTS,* 1989606-08-7
- *CHEMICAL ABSTRACTS,* 1989604-37-6
- *CHEMICAL ABSTRACTS,* 1989604-90-1
- *CHEMICAL ABSTRACTS,* 1989605-54-0
- *CHEMICAL ABSTRACTS,* 1989606-09-8
- *CHEMICAL ABSTRACTS,* 1989604-38-7
- *CHEMICAL ABSTRACTS,* 1989604-92-3
- *CHEMICAL ABSTRACTS,* 1989605-55-1
- *CHEMICAL ABSTRACTS,* 1989606-10-1
- *CHEMICAL ABSTRACTS,* 1989604-39-8
- *CHEMICAL ABSTRACTS,* 1989604-93-4
- *CHEMICAL ABSTRACTS,* 1989605-56-2
- *CHEMICAL ABSTRACTS,* 1989606-11-2
- *CHEMICAL ABSTRACTS,* 1989604-40-1
- *CHEMICAL ABSTRACTS,* 1989604-94-5
- *CHEMICAL ABSTRACTS,* 1989605-57-3
- *CHEMICAL ABSTRACTS,* 1989606-12-3
- *CHEMICAL ABSTRACTS,* 1989604-41-2
- *CHEMICAL ABSTRACTS,* 1989604-95-6
- *CHEMICAL ABSTRACTS,* 1989605-58-4
- *CHEMICAL ABSTRACTS,* 1989606-13-4
- *CHEMICAL ABSTRACTS,* 1989604-42-3
- *CHEMICAL ABSTRACTS,* 1989604-96-7
- *CHEMICAL ABSTRACTS,* 1989605-59-5
- *CHEMICAL ABSTRACTS,* 1989606-14-5
- *CHEMICAL ABSTRACTS,* 1989604-43-4
- *CHEMICAL ABSTRACTS,* 1989604-97-8
- *CHEMICAL ABSTRACTS,* 1989605-61-9
- *CHEMICAL ABSTRACTS,* 1989606-15-6
- *CHEMICAL ABSTRACTS,* 1989604-45-6
- *CHEMICAL ABSTRACTS,* 1989605-09-5
- *CHEMICAL ABSTRACTS,* 1989605-62-0
- *CHEMICAL ABSTRACTS,* 1989606-16-7

- *CHEMICAL ABSTRACTS,* 1989604-46-7
- *CHEMICAL ABSTRACTS,* 1989605-10-8
- *CHEMICAL ABSTRACTS,* 1989605-63-1
- *CHEMICAL ABSTRACTS,* 1989606-17-8
- *CHEMICAL ABSTRACTS,* 1989604-47-8
- *CHEMICAL ABSTRACTS,* 1989605-11-9
- *CHEMICAL ABSTRACTS,* 1989605-64-2
- *CHEMICAL ABSTRACTS,* 1989606-18-9
- *CHEMICAL ABSTRACTS,* 1989604-48-9
- *CHEMICAL ABSTRACTS,* 1989605-13-1
- *CHEMICAL ABSTRACTS,* 1989605-65-3
- *CHEMICAL ABSTRACTS,* 1989606-19-0
- *CHEMICAL ABSTRACTS,* 1989604-49-0
- *CHEMICAL ABSTRACTS,* 1989605-14-2
- *CHEMICAL ABSTRACTS,* 1989605-66-4
- *CHEMICAL ABSTRACTS,* 1989606-20-3
- *CHEMICAL ABSTRACTS,* 1989604-50-3
- *CHEMICAL ABSTRACTS,* 1989605-15-3
- *CHEMICAL ABSTRACTS,* 1989605-67-5
- *CHEMICAL ABSTRACTS,* 1989606-21-4
- *CHEMICAL ABSTRACTS,* 1989604-52-5
- *CHEMICAL ABSTRACTS,* 1989605-16-4
- *CHEMICAL ABSTRACTS,* 1989605-68-6
- *CHEMICAL ABSTRACTS,* 1989606-22-5
- *CHEMICAL ABSTRACTS,* 1989604-53-6
- *CHEMICAL ABSTRACTS,* 1989605-17-5
- *CHEMICAL ABSTRACTS,* 1989605-69-7
- *CHEMICAL ABSTRACTS,* 1989606-23-6
- *CHEMICAL ABSTRACTS,* 1989604-54-7
- *CHEMICAL ABSTRACTS,* 1989605-18-6
- *CHEMICAL ABSTRACTS,* 1989605-70-0
- *CHEMICAL ABSTRACTS,* 1989606-24-7
- *CHEMICAL ABSTRACTS,* 1989604-55-8
- *CHEMICAL ABSTRACTS,* 1989605-19-7
- *CHEMICAL ABSTRACTS,* 1989605-71-1
- *CHEMICAL ABSTRACTS,* 1989606-26-9
- *CHEMICAL ABSTRACTS,* 1989604-56-9
- *CHEMICAL ABSTRACTS,* 1989605-20-0
- *CHEMICAL ABSTRACTS,* 1989605-72-2
- *CHEMICAL ABSTRACTS,* 1989606-27-0
- *CHEMICAL ABSTRACTS,* 1989604-57-0
- *CHEMICAL ABSTRACTS,* 1989605-21-1
- *CHEMICAL ABSTRACTS,* 1989605-73-3
- *CHEMICAL ABSTRACTS,* 1989606-28-1
- *CHEMICAL ABSTRACTS,* 1989604-58-1
- *CHEMICAL ABSTRACTS,* 1989605-22-2
- *CHEMICAL ABSTRACTS,* 1989605-74-4
- *CHEMICAL ABSTRACTS,* 1989606-29-2
- *CHEMICAL ABSTRACTS,* 1989604-59-2
- *CHEMICAL ABSTRACTS,* 1989605-23-3
- *CHEMICAL ABSTRACTS,* 1989605-75-5
- *CHEMICAL ABSTRACTS,* 1989606-30-5
- *CHEMICAL ABSTRACTS,* 1989604-60-5
- *CHEMICAL ABSTRACTS,* 1989605-24-4
- *CHEMICAL ABSTRACTS,* 1989605-76-6
- *CHEMICAL ABSTRACTS,* 1989606-31-6
- *CHEMICAL ABSTRACTS,* 1989604-61-6
- *CHEMICAL ABSTRACTS,* 1989605-25-5
- *CHEMICAL ABSTRACTS,* 1989605-77-7
- *CHEMICAL ABSTRACTS,* 1989606-32-7
- *CHEMICAL ABSTRACTS,* 1989604-62-7
- *CHEMICAL ABSTRACTS,* 1989605-26-6
- *CHEMICAL ABSTRACTS,* 1989605-78-8
- *CHEMICAL ABSTRACTS,* 1989606-33-8
- *CHEMICAL ABSTRACTS,* 1989604-63-8
- *CHEMICAL ABSTRACTS,* 1989605-27-7
- *CHEMICAL ABSTRACTS,* 1989605-79-9
- *CHEMICAL ABSTRACTS,* 1989606-34-9
- *CHEMICAL ABSTRACTS,* 1989604-64-9
- *CHEMICAL ABSTRACTS,* 1989605-28-8
- *CHEMICAL ABSTRACTS,* 1989605-81-3
- *CHEMICAL ABSTRACTS,* 1989606-35-0
- *CHEMICAL ABSTRACTS,* 1989604-65-0
- *CHEMICAL ABSTRACTS,* 1989605-29-9
- *CHEMICAL ABSTRACTS,* 1989605-82-4
- *CHEMICAL ABSTRACTS,* 1989606-36-1
- *CHEMICAL ABSTRACTS,* 1989604-66-1
- *CHEMICAL ABSTRACTS,* 1989605-30-2
- *CHEMICAL ABSTRACTS,* 1989605-83-5
- *CHEMICAL ABSTRACTS,* 1989606-37-2
- *CHEMICAL ABSTRACTS,* 1989604-67-2
- *CHEMICAL ABSTRACTS,* 1989605-31-3
- *CHEMICAL ABSTRACTS,* 1989605-84-6
- *CHEMICAL ABSTRACTS,* 1989606-38-3
- *CHEMICAL ABSTRACTS,* 1989604-68-3
- *CHEMICAL ABSTRACTS,* 1989605-32-4
- *CHEMICAL ABSTRACTS,* 1989605-85-7
- *CHEMICAL ABSTRACTS,* 1989606-39-4
- *CHEMICAL ABSTRACTS,* 1989604-69-4
- *CHEMICAL ABSTRACTS,* 1989605-33-5
- *CHEMICAL ABSTRACTS,* 1989605-86-8
- *CHEMICAL ABSTRACTS,* 1989606-40-7
- *CHEMICAL ABSTRACTS,* 1989604-70-7
- *CHEMICAL ABSTRACTS,* 1989605-34-6
- *CHEMICAL ABSTRACTS,* 1989605-87-9
- *CHEMICAL ABSTRACTS,* 1989606-41-8
- *CHEMICAL ABSTRACTS,* 1989604-71-8
- *CHEMICAL ABSTRACTS,* 1989605-35-7
- *CHEMICAL ABSTRACTS,* 1989605-88-0
- *CHEMICAL ABSTRACTS,* 1989606-42-9
- *CHEMICAL ABSTRACTS,* 1989604-72-9
- *CHEMICAL ABSTRACTS,* 1989605-36-8
- *CHEMICAL ABSTRACTS,* 1989605-89-1
- *CHEMICAL ABSTRACTS,* 1989606-43-0
- *CHEMICAL ABSTRACTS,* 1989604-73-0
- *CHEMICAL ABSTRACTS,* 1989605-37-9
- *CHEMICAL ABSTRACTS,* 1989605-90-4
- *CHEMICAL ABSTRACTS,* 1989606-44-1
- *CHEMICAL ABSTRACTS,* 1989604-74-1
- *CHEMICAL ABSTRACTS,* 1989605-38-0
- *CHEMICAL ABSTRACTS,* 1989605-91-5
- *CHEMICAL ABSTRACTS,* 1989606-45-2
- *CHEMICAL ABSTRACTS,* 1989604-75-2
- *CHEMICAL ABSTRACTS,* 1989605-39-1
- *CHEMICAL ABSTRACTS,* 1989605-92-6
- *CHEMICAL ABSTRACTS,* 1989606-46-3

- *CHEMICAL ABSTRACTS,* 1989604-76-3
- *CHEMICAL ABSTRACTS,* 1989605-40-4
- *CHEMICAL ABSTRACTS,* 1989605-93-7
- *CHEMICAL ABSTRACTS,* 1989606-48-5
- *CHEMICAL ABSTRACTS,* 1989604-77-4
- *CHEMICAL ABSTRACTS,* 1989605-41-5
- *CHEMICAL ABSTRACTS,* 1989605-94-8
- *CHEMICAL ABSTRACTS,* 1989606-49-6
- *CHEMICAL ABSTRACTS,* 1989604-78-5
- *CHEMICAL ABSTRACTS,* 1989605-42-6
- *CHEMICAL ABSTRACTS,* 1989605-95-9
- *CHEMICAL ABSTRACTS,* 1989606-53-2
- *CHEMICAL ABSTRACTS,* 1989604-79-6
- *CHEMICAL ABSTRACTS,* 1989605-43-7
- *CHEMICAL ABSTRACTS,* 1989605-96-0
- *CHEMICAL ABSTRACTS,* 1989606-55-4
- *CHEMICAL ABSTRACTS,* 1989604-80-9
- *CHEMICAL ABSTRACTS,* 1989605-44-8
- *CHEMICAL ABSTRACTS,* 1989605-97-1
- *CHEMICAL ABSTRACTS,* 1989606-56-5
- *CHEMICAL ABSTRACTS,* 1989604-81-0
- *CHEMICAL ABSTRACTS,* 1989605-45-9
- *CHEMICAL ABSTRACTS,* 1989605-98-2
- *CHEMICAL ABSTRACTS,* 1989606-61-2
- *CHEMICAL ABSTRACTS,* 1989604-82-1
- *CHEMICAL ABSTRACTS,* 1989605-46-0
- *CHEMICAL ABSTRACTS,* 1989605-99-3
- *CHEMICAL ABSTRACTS,* 1989606-62-3
- *CHEMICAL ABSTRACTS,* 1989604-83-2
- *CHEMICAL ABSTRACTS,* 1989605-47-1
- *CHEMICAL ABSTRACTS,* 1989606-00-9
- *CHEMICAL ABSTRACTS,* 1989606-63-4
- *CHEMICAL ABSTRACTS,* 1989604-84-3
- *CHEMICAL ABSTRACTS,* 1989605-48-2
- *CHEMICAL ABSTRACTS,* 1989606-01-0
- *CHEMICAL ABSTRACTS,* 1989606-67-8
- *CHEMICAL ABSTRACTS,* 1989604-85-4
- *CHEMICAL ABSTRACTS,* 1989605-49-3
- *CHEMICAL ABSTRACTS,* 1989606-04-3
- *CHEMICAL ABSTRACTS,* 1989606-69-0
- *CHEMICAL ABSTRACTS,* 1989604-86-5
- *CHEMICAL ABSTRACTS,* 1989605-50-6
- *CHEMICAL ABSTRACTS,* 1989606-05-4
- *CHEMICAL ABSTRACTS,* 1989606-70-3
- *CHEMICAL ABSTRACTS,* 1989604-87-6
- *CHEMICAL ABSTRACTS,* 1989605-51-7
- *CHEMICAL ABSTRACTS,* 1989606-06-5
- *CHEMICAL ABSTRACTS,* 1989606-74-7
- *CHEMICAL ABSTRACTS,* 1989658-39-0
- *CHEMICAL ABSTRACTS,* 2088184-56-7
- *CHEMICAL ABSTRACTS,* 2088185-07-1
- *CHEMICAL ABSTRACTS,* 2088185-66-2
- *CHEMICAL ABSTRACTS,* 1989658-41-4
- *CHEMICAL ABSTRACTS,* 2088184-57-8
- *CHEMICAL ABSTRACTS,* 2088185-08-2
- *CHEMICAL ABSTRACTS,* 2088185-67-3
- *CHEMICAL ABSTRACTS,* 1989658-43-6
- *CHEMICAL ABSTRACTS,* 2088184-58-9
- *CHEMICAL ABSTRACTS,* 2088185-09-3
- *CHEMICAL ABSTRACTS,* 2088185-68-4
- *CHEMICAL ABSTRACTS,* 1989658-47-0
- *CHEMICAL ABSTRACTS,* 2088184-59-0
- *CHEMICAL ABSTRACTS,* 2088185-10-6
- *CHEMICAL ABSTRACTS,* 2088185-69-5
- *CHEMICAL ABSTRACTS,* 1989658-49-2
- *CHEMICAL ABSTRACTS,* 2088184-60-3
- *CHEMICAL ABSTRACTS,* 2088185-11-7
- *CHEMICAL ABSTRACTS,* 2088185-70-8
- *CHEMICAL ABSTRACTS,* 2088184-07-8
- *CHEMICAL ABSTRACTS,* 2088184-61-4
- *CHEMICAL ABSTRACTS,* 2088185-12-8
- *CHEMICAL ABSTRACTS,* 2088185-71-9
- *CHEMICAL ABSTRACTS,* 2088184-08-9
- *CHEMICAL ABSTRACTS,* 2088184-62-5
- *CHEMICAL ABSTRACTS,* 2088185-13-9
- *CHEMICAL ABSTRACTS,* 2088185-72-0
- *CHEMICAL ABSTRACTS,* 2088184-09-0
- *CHEMICAL ABSTRACTS,* 2088184-63-6
- *CHEMICAL ABSTRACTS,* 2088185-14-0
- *CHEMICAL ABSTRACTS,* 2088185-73-1
- *CHEMICAL ABSTRACTS,* 2088184-10-3
- *CHEMICAL ABSTRACTS,* 2088184-64-7
- *CHEMICAL ABSTRACTS,* 2088185-15-1
- *CHEMICAL ABSTRACTS,* 2088185-74-2
- *CHEMICAL ABSTRACTS,* 2088184-11-4
- *CHEMICAL ABSTRACTS,* 2088184-65-8
- *CHEMICAL ABSTRACTS,* 2088185-16-2
- *CHEMICAL ABSTRACTS,* 2088185-75-3
- *CHEMICAL ABSTRACTS,* 2088184-13-6
- *CHEMICAL ABSTRACTS,* 2088184-66-9
- *CHEMICAL ABSTRACTS,* 2088185-17-3
- *CHEMICAL ABSTRACTS,* 2088185-76-4
- *CHEMICAL ABSTRACTS,* 2088184-14-7
- *CHEMICAL ABSTRACTS,* 2088184-67-0
- *CHEMICAL ABSTRACTS,* 2088185-18-4
- *CHEMICAL ABSTRACTS,* 2088185-77-5
- *CHEMICAL ABSTRACTS,* 2088184-15-8
- *CHEMICAL ABSTRACTS,* 2088184-68-1
- *CHEMICAL ABSTRACTS,* 2088185-19-5
- *CHEMICAL ABSTRACTS,* 2088185-78-6
- *CHEMICAL ABSTRACTS,* 2088184-16-9
- *CHEMICAL ABSTRACTS,* 2088184-69-2
- *CHEMICAL ABSTRACTS,* 2088185-20-8
- *CHEMICAL ABSTRACTS,* 2088185-79-7
- *CHEMICAL ABSTRACTS,* 2088184-17-0
- *CHEMICAL ABSTRACTS,* 2088184-70-5
- *CHEMICAL ABSTRACTS,* 2088185-21-9
- *CHEMICAL ABSTRACTS,* 2088185-80-0
- *CHEMICAL ABSTRACTS,* 2088184-18-1
- *CHEMICAL ABSTRACTS,* 2088184-71-6
- *CHEMICAL ABSTRACTS,* 2088185-22-0
- *CHEMICAL ABSTRACTS,* 2088185-81-1
- *CHEMICAL ABSTRACTS,* 2088184-19-2
- *CHEMICAL ABSTRACTS,* 2088184-72-7
- *CHEMICAL ABSTRACTS,* 2088185-23-1
- *CHEMICAL ABSTRACTS,* 2088185-82-2

- *CHEMICAL ABSTRACTS,* 2088184-20-5
- *CHEMICAL ABSTRACTS,* 2088184-73-8
- *CHEMICAL ABSTRACTS,* 2088185-32-2
- *CHEMICAL ABSTRACTS,* 2088185-83-3
- *CHEMICAL ABSTRACTS,* 2088184-21-6
- *CHEMICAL ABSTRACTS,* 2088184-74-9
- *CHEMICAL ABSTRACTS,* 2088185-33-3
- *CHEMICAL ABSTRACTS,* 2088185-84-4
- *CHEMICAL ABSTRACTS,* 2088184-22-7
- *CHEMICAL ABSTRACTS,* 2088184-75-0
- *CHEMICAL ABSTRACTS,* 2088185-34-4
- *CHEMICAL ABSTRACTS,* 2088185-85-5
- *CHEMICAL ABSTRACTS,* 2088184-23-8
- *CHEMICAL ABSTRACTS,* 2088184-76-1
- *CHEMICAL ABSTRACTS,* 2088185-35-5
- *CHEMICAL ABSTRACTS,* 2088185-86-6
- *CHEMICAL ABSTRACTS,* 2088184-24-9
- *CHEMICAL ABSTRACTS,* 2088184-77-2
- *CHEMICAL ABSTRACTS,* 2088185-36-6
- *CHEMICAL ABSTRACTS,* 2088185-87-7
- *CHEMICAL ABSTRACTS,* 2088184-25-0
- *CHEMICAL ABSTRACTS,* 2088184-78-3
- *CHEMICAL ABSTRACTS,* 2088185-37-7
- *CHEMICAL ABSTRACTS,* 2088185-88-8
- *CHEMICAL ABSTRACTS,* 2088184-26-1
- *CHEMICAL ABSTRACTS,* 2088184-79-4
- *CHEMICAL ABSTRACTS,* 2088185-38-8
- *CHEMICAL ABSTRACTS,* 2088185-89-9
- *CHEMICAL ABSTRACTS,* 2088184-27-2
- *CHEMICAL ABSTRACTS,* 2088184-80-7
- *CHEMICAL ABSTRACTS,* 2088185-39-9
- *CHEMICAL ABSTRACTS,* 2088185-90-2
- *CHEMICAL ABSTRACTS,* 2088184-28-3
- *CHEMICAL ABSTRACTS,* 2088184-81-8
- *CHEMICAL ABSTRACTS,* 2088185-40-2
- *CHEMICAL ABSTRACTS,* 2088185-91-3
- *CHEMICAL ABSTRACTS,* 2088184-29-4
- *CHEMICAL ABSTRACTS,* 2088184-82-9
- *CHEMICAL ABSTRACTS,* 2088185-41-3
- *CHEMICAL ABSTRACTS,* 2088185-92-4
- *CHEMICAL ABSTRACTS,* 2088184-30-7
- *CHEMICAL ABSTRACTS,* 2088184-83-0
- *CHEMICAL ABSTRACTS,* 2088185-42-4
- *CHEMICAL ABSTRACTS,* 2088185-93-5
- *CHEMICAL ABSTRACTS,* 2088184-32-9
- *CHEMICAL ABSTRACTS,* 2088184-84-1
- *CHEMICAL ABSTRACTS,* 2088185-43-5
- *CHEMICAL ABSTRACTS,* 2088185-94-6
- *CHEMICAL ABSTRACTS,* 2088184-34-1
- *CHEMICAL ABSTRACTS,* 2088184-85-2
- *CHEMICAL ABSTRACTS,* 2088185-44-6
- *CHEMICAL ABSTRACTS,* 2088185-95-7
- *CHEMICAL ABSTRACTS,* 2088184-35-2
- *CHEMICAL ABSTRACTS,* 2088184-86-3
- *CHEMICAL ABSTRACTS,* 2088185-45-7
- *CHEMICAL ABSTRACTS,* 2088185-96-8
- *CHEMICAL ABSTRACTS,* 2088184-36-3
- *CHEMICAL ABSTRACTS,* 2088184-87-4
- *CHEMICAL ABSTRACTS,* 2088185-46-8
- *CHEMICAL ABSTRACTS,* 2088185-97-9
- *CHEMICAL ABSTRACTS,* 2088184-37-4
- *CHEMICAL ABSTRACTS,* 2088184-88-5
- *CHEMICAL ABSTRACTS,* 2088185-47-9
- *CHEMICAL ABSTRACTS,* 2088185-98-0
- *CHEMICAL ABSTRACTS,* 2088184-38-5
- *CHEMICAL ABSTRACTS,* 2088184-89-6
- *CHEMICAL ABSTRACTS,* 2088185-48-0
- *CHEMICAL ABSTRACTS,* 2088185-99-1
- *CHEMICAL ABSTRACTS,* 2088184-39-6
- *CHEMICAL ABSTRACTS,* 2088184-90-9
- *CHEMICAL ABSTRACTS,* 2088185-49-1
- *CHEMICAL ABSTRACTS,* 2088186-00-7
- *CHEMICAL ABSTRACTS,* 2088184-40-9
- *CHEMICAL ABSTRACTS,* 2088184-91-0
- *CHEMICAL ABSTRACTS,* 2088185-50-4
- *CHEMICAL ABSTRACTS,* 2088186-01-8
- *CHEMICAL ABSTRACTS,* 2088184-41-0
- *CHEMICAL ABSTRACTS,* 2088184-92-1
- *CHEMICAL ABSTRACTS,* 2088185-51-5
- *CHEMICAL ABSTRACTS,* 2088186-02-9
- *CHEMICAL ABSTRACTS,* 2088184-42-1
- *CHEMICAL ABSTRACTS,* 2088184-93-2
- *CHEMICAL ABSTRACTS,* 2088185-52-6
- *CHEMICAL ABSTRACTS,* 2088195-88-2
- *CHEMICAL ABSTRACTS,* 2088184-43-2
- *CHEMICAL ABSTRACTS,* 2088184-94-3
- *CHEMICAL ABSTRACTS,* 2088185-53-7
- *CHEMICAL ABSTRACTS,* 2088195-89-3
- *CHEMICAL ABSTRACTS,* 2088184-44-3
- *CHEMICAL ABSTRACTS,* 2088184-95-4
- *CHEMICAL ABSTRACTS,* 2088185-54-8
- *CHEMICAL ABSTRACTS,* 2088195-90-6
- *CHEMICAL ABSTRACTS,* 2088184-45-4
- *CHEMICAL ABSTRACTS,* 2088184-96-5
- *CHEMICAL ABSTRACTS,* 2088185-55-9
- *CHEMICAL ABSTRACTS,* 2088195-91-7
- *CHEMICAL ABSTRACTS,* 2088184-46-5
- *CHEMICAL ABSTRACTS,* 2088184-97-6
- *CHEMICAL ABSTRACTS,* 2088185-56-0
- *CHEMICAL ABSTRACTS,* 861806-70-4
- *CHEMICAL ABSTRACTS,* 2088184-47-6
- *CHEMICAL ABSTRACTS,* 2088184-98-7
- *CHEMICAL ABSTRACTS,* 2088185-57-1
- *CHEMICAL ABSTRACTS,* 2088184-48-7
- *CHEMICAL ABSTRACTS,* 2088184-99-8
- *CHEMICAL ABSTRACTS,* 2088185-58-2
- *CHEMICAL ABSTRACTS,* 2088184-49-8
- *CHEMICAL ABSTRACTS,* 2088185-00-4
- *CHEMICAL ABSTRACTS,* 2088185-59-3
- *CHEMICAL ABSTRACTS,* 2088184-50-1
- *CHEMICAL ABSTRACTS,* 2088185-01-5
- *CHEMICAL ABSTRACTS,* 2088185-60-6
- *CHEMICAL ABSTRACTS,* 2088184-51-2
- *CHEMICAL ABSTRACTS,* 2088185-02-6
- *CHEMICAL ABSTRACTS,* 2088185-61-7
- *CHEMICAL ABSTRACTS,* 2088184-52-3

- *CHEMICAL ABSTRACTS,* 2088185-03-7
- *CHEMICAL ABSTRACTS,* 2088185-62-8
- *CHEMICAL ABSTRACTS,* 2088184-53-4
- *CHEMICAL ABSTRACTS,* 2088185-04-8
- *CHEMICAL ABSTRACTS,* 2088185-63-9
- *CHEMICAL ABSTRACTS,* 2088184-54-5
- *CHEMICAL ABSTRACTS,* 2088185-05-9
- *CHEMICAL ABSTRACTS,* 2088185-64-0
- *CHEMICAL ABSTRACTS,* 2088184-55-6
- *CHEMICAL ABSTRACTS,* 2088185-06-0
- *CHEMICAL ABSTRACTS,* 2088185-65-1
- **T. MATSUMOTO ; T. NAKADA ; J. ENDO ; K. MORI ; N. KAWAMURA ; A. YOKOI ; J. KIDO.** Multiphoton Organic EL Device Having Charge Generation Layer. *IDMC 2003, Taiwan*
- **Y. SHIROTA et al.** *Chem. Rev.,* 2007, vol. 107 (4), 953-1010
- **M. S. ARNOLD et al.** *Appl. Phys. Lett.,* 2008, vol. 92, 053301
- *Physical Chemistry Chemical Physics,* 2015, vol. 17 (37), 24468-24474